# EUROPEAN PATENT SPECIFICATION

(11) **EP 1 432 701 B1**
(45) Date of publication and mention of the grant of the patent: **21.12.2005**
(21) Application number: 02782863.1
(22) Date of filing: 07.10.2002
(51) Int. Cl.: C07D 401/12, A61K 31/47, A61K 31/404, A61P 13/00, C07D 215/12, C07D 215/18, C07D 215/20, C07D 405/12, C07D 215/40, C07D 215/42, C07D 215/26, C07D 401/06, C07D 417/12, C07D 413/12, C07D 409/08, C07D 413/08, C07D 401/14, C07D 405/14, C07D 413/14, C07D 409/14

(54) **HETEROCYCLIC COMPOUNDS FOR USE IN THE TREATMENT OF DISORDERS OF THE URINARY TRACT**
HETEROZYKLISCHE VERBINDUNGEN ZUR BEHANDLUNG VON HARNWEGSERKRANKUNGEN
COMPOSES HETEROCYCLIQUES UTILISES POUR TRAITER DES TROUBLES DE LA VOIE URINAIRE

(30) Priority: 05.10.2001 IT MI20012060
(43) Date of publication of application: 30.06.2004
(73) Proprietor: Recordati Ireland Limited, Ringaskiddy County Cork (IE); RECORDATI INDUSTRIA CHIMICA E FARMACEUTICA S.p.a., 20148 Milano (IT)
(72) Inventor: LEONARDI, Amedeo, I-20154 Milano (IT); MOTTA, Gianni, I-20030 Barlassina (IT); RIVA, Carlo, I-21100 Varese (IT); TESTA, Rodolfo, I-20060 Vignate (IT); CORBETT, Jeff, W., Portage, MI 49024 (US)
(74) Representative: McKelvey, Ian Edward
(86) International application number: PCT/EP2002/011282
(87) International publication number: WO 2003/031436

(56) References cited:
- EP-A- 0 846 683
- WO-A-99/06382
- WO-A-99/06383
- FR-A- 2 729 144

## Description

### FIELD OF THE INVENTION

The invention relates to compounds having affinity for serotonergic receptors, pharmaceutical compositions thereof and uses for such compounds and compositions.

### BACKGROUND OF THE INVENTION

In mammals, micturition (urination) is a complex process that requires the integrated action of the bladder, its internal and external sphincters, the musculature of the pelvic floor and neurological control over these muscles at three levels (in the bladder wall or sphincter itself, in the autonomic centres of the spinal cord and in the central nervous system at the level of the pontine micturition centre (PMC) in the brainstem (pons) under the control of the cerebral cortex) (De Groat, *Neurobiology of Incontinence,* Ciba Foundation Symposium **151**:27, 1990). Micturition results from contraction of the detrusor muscle, which consists of interlacing smooth-muscle fibres, under the control of the parasympathetic autonomic system originating from the sacral spinal cord. A simple voiding reflex is triggered by sensory nerves for pain, temperature and distension that run from the bladder to the sacral spinal cord. However, sensory tracts from the bladder reach the PMC too, generating nerve impulses that normally suppress the sacral spinal suppression of cortical inhibition of the reflex arc, and relaxing the muscles of the pelvic floor and external sphincter. Finally, the detrusor muscle contracts and voiding occurs. Abnormalities of lower-urinary tract function, e.g. dysuria, incontinence and enuresis, are common in the general population. Dysuria includes urinary frequency, nocturia and urgency, and may be caused by cystitis (including interstitial cystitis), prostatitis or benign prostatic hyperplasia (BPH) (which affects about 70% of elderly males), or by neurological disorders. Incontinence syndromes include stress incontinence, urgency incontinence, overflow incontinence and mixed incontinence. Enuresis refers to the involuntary passage of urine at night or during sleep.

Previously, treatment of neuromuscular dysfunction of the lower urinary tract involved administration of compounds that act directly on the bladder muscles, such as flavoxate, a spasmolytic drug (Ruffman, *J. Int. Med. Res.* **16**:317, 1988) which is also active on the PMC (Guarneri *et al., Drugs of Today,* **30**:91, 1994), or anticholinergic compounds such as oxybutynin (Andersson, *Drugs* **36**:477, 1988) and tolterodine (Nilvebrant, *Life Sci.* **68**(22-23): 2549, 2001). The use of α₁-adrenergic receptor antagonists for the treatment of BPH is common too, but is based on a different mechanism of action (Lepor, *Urology,* **42**:483, 1993). However, treatments that involve direct inhibition of the pelvic musculature (including the detrusor muscle) may have unwanted side effects, such as incomplete voiding or accommodation paralysis, tachycardia and dry mouth (Andersson, *Drugs* **35**:477, 1988).

Thus, it would be preferable to utilize compounds that act via the central nervous system to, for example, affect the sacral spinal reflex and/or the PMC inhibition pathways in a manner that restores normal functioning of the micturition mechanism. 1-(4-Fluorophenyl)-4-(6-trifluoromethyl-1,2,3,4-tetrahydroquinoline-2-ylmethyl)-piperazine is described in WO 98/38194 as an intermediate in the synthesis of 2-iminothiazole[2,4,3-yl]quinoline having a glutaminergic antagonistic activity.

1-Phenyl-4-(1,2,3,4-tetrahydroquinoline-2-ylmethyl)-piperazine is described as an antagonist of α-adrenergic receptors in *Indian J. Exp. Biol.***10** (5): 368-370 (1972).

A series of 1-aryl-4-[2-(1,2,3,4-tetrahydroquinoline-2-yl)-ethyl]piperazines is described in US 3983121 as having a hypotensive activity.

1-Acetyl-2,β-(N-piperidinoethyl)-2H-indole-3-one is described in *Chem. Pharm. Bull.* **29**:1900-1911 (1981).

Structurally complex N-acylated tetrahydroquinolines having a somatostatin receptor agonistic or antagonistic activity are disclosed in WO 99/52875.

WO 01/49678 discloses a class of phenylpiperazine derivatives which are described as having a high affinity for 5HT_{1A} receptor.

WO 99/06382 and WO 99/06383 disclose piperazine derivatives, which bind to 5HT_{1A} receptors and are useful in the treatment of neuromuscular dysfunctions of the lower urinary tract.

### DESCRIPTION OF THE INVENTION

The invention provides compounds having the general formula I 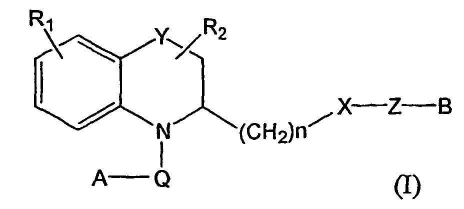 wherein
R₁ represents one or more substituents selected from hydrogen and halogen atoms and hydroxy, alkyl, haloalkyl, alkoxy, haloalkoxy, nitro, phenyl, substituted phenyl, heterocycle, substituted heterocycle and NR₃R₄ groups wherein each of R₃ and R₄ independently represents a hydrogen atom or an alkyl, acyl or alkoxycarbonyl group;
R₂ represents one or two substituents selected from hydrogen atoms and alkyl groups;
Y represents a CH₂ group or a bond;
Q represents a carbonyl, thiocarbonyl or sulphonyl group;
A represents an alkyl, alkenyl, cycloalkyl, cycloalkenyl, aryl, heterocyclic, alkylamino, dialkylamino, arylamino or aralkylamino group, each of which is optionally substituted;
n is 1 or 2;
X represents an amino moiety selected from 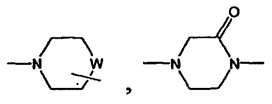 and -N(R₅)-CH₂- wherein R₅ represents a hydrogen atom or a methyl or benzyl group and W represents a bond, a nitrogen atom or a CH, CH₂, C(CN) C(OH) or C(COCH₃) group (when W represents a nitrogen atom or a CH, C(CN) C(OH) or C(COCH₃) group, the group Z-B attaches to the W ring atom);
when X represents a 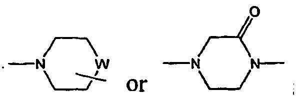 group, Z represents a bond, an oxygen or sulphur atom or a CH₂, CH₂CH₂, CO, CHOH, OCH₂, NH, NHCO or NHCONHCH₂ group, when X represents a -N(R₅)-CH₂- group, Z represents a CH₂CH₂ or CH₂O group or Z and B together represent a 2,3-dihydro-benzo[1,4]dioxin-2-yl group; and
except as aforesaid, B represents a monocyclic or bicyclic aryl group or a monocyclic or bicyclic heterocycle, each of which is optionally substituted.

Enantiomers, diastereomers, N-oxides, crystalline forms, hydrates, solvates and pharmaceutically acceptable salts of such compounds are also included in the invention.

The invention also includes metabolites of the compounds of formula I having the same type of activity, hereinafter referred to as active metabolites. A "metabolite" of a compound disclosed herein is a derivative of a compound which is formed when the compound is metabolised. The term "active metabolite" refers to a biologically active derivative of a compound which is formed when the compound is metabolised. The term "metabolised" refers to the sum of the processes by which a particular substance is changed in the living body. In brief, all compounds present in the body are manipulated by enzymes within the body in order to derive energy and/or to remove them from the body. Specific enzymes produce specific structural alterations to the compound. For example, cytochrome P450 catalyses a variety of oxidative and reductive reactions while uridine diphosphate glucuronyltransferases catalyse the transfer of an activated glucuronic-acid molecule to aromatic alcohols, aliphatic alcohols, carboxylic acids, amines and free sulphhydryl groups. Further information on metabolism may be obtained from *The Pharmacological Basis of Therapeutics,* 9^{th} Edition, McGraw-Hill (1996), pages 11-17. The metabolites of the compounds disclosed herein can be identified either by administration of compounds to a host and analysis of tissue samples from the host, or by incubation of compounds with hepatic cells *in vitro* and analysis of the resulting compounds. Both methods are well known in the art.

The invention also contemplates prodrugs which are metabolised in the body to generate the compounds of formula I

In another embodiment, the invention provides pharmaceutical compositions comprising compounds of formula I, or enantiomers, diastereomers, N-oxides, crystalline forms, hydrates, solvates or pharmaceutically acceptable salts of such compounds, in admixture with pharmaceutically acceptable diluents or carriers.

In yet another embodiment, the invention provides the use of at least one compound of formula I in an amount effective for reducing the frequency of bladder contractions due to bladder distension by administering it to a mammal, including a human, in need of such treatment. Also, the present invention contemplates a method of administering a compound of formula I.

In a further embodiment, the invention provides the use of at least one compound of formula I in an amount effective for increasing urinary bladder capacity by administering it to a mammal, including a human, in need of such treatment.

In a still further embodiment, this invention provides the use of at least one compound of formula I in an amount effective for treating disorders of the urinary tract in a patient in need of such treatment to ameliorate at least one condition among urinary urgency, overactive bladder, increased urinary frequency, decreased urinary compliance (decreased bladder storage capacity), cystitis (including interstitial cystitis), incontinence, urine leakage, enuresis, dysuria, urinary hesitancy and difficulty in emptying the bladder.

For treating the above disorders, the compounds of the invention may be administered in combination with known antimuscarinic drugs such as oxybutynin, tolterodine, darifenacin and temiverine. Analogously, the compounds of the invention may be associated to α1-adrenergic antagonists such as prazosin, doxazosin, terazosin, alfuzosin and tamsulosin, for the therapy of lower urinary tract symptoms, whether or not these are associated with BPH.

In a yet further embodiment, the invention covers the use of at least one compound of formula I in an amount effective for the treatment of central nervous system disorders due to serotonergic dysfunction. Such dysfunctions include anxiety, depression, hypertension, sleep/wake cycle disorders, feeding, behaviour, sexual function and cognition disorders in mammals (particularly in humans) associated with stroke, injury, dementia, and originated by neurological development, attention-deficit hyperactivity disorders (ADHD), drug addiction, drug withdrawal, irritable-bowel syndrome. Treatment may be effected by delivering to the environment of 5-HT_{1A} serotonergic receptor, for example to the extracellular medium, (or by systemically or locally administering to a mammal possessing such receptor) an amount of a compound of the invention effective to increase the duration of bladder quiescence with no contractions. The present invention refers to a method of administering a compound of the above formula with the previously-disclosed substituent patterns and combinations of such substituents.

### All variables:

The term "alkyl" refers to a straight- or branched-chain saturated hydrocarbon group having from 1 to 7 carbon atoms, and preferably from 1 to 5 carbon atoms. This meaning is retained in complex groups including alkoxy, alkoxycarbonyl, haloalkyl, haloalkoxy, alkylamino, dialkylamino, alkylsulphonyl and aralkyl.

The term "alkenyl" refers to a straight- or branched-chain unsaturated hydrocarbon group having from 2 to 7 carbon atoms, and preferably from 2 to 5 carbon atoms, and at least one double bond.

The term "cycloalkyl" refers to a saturated hydrocarbon ring system having from 3 to 10 carbon atoms. Cycloakyl groups may be monocyclic, fused or bridged. Monocyclic cycloalkyl groups preferably have from 3 to 7 carbon atoms.

The term "cycloalkenyl" refers to an unsaturated non-aromatic hydrocarbon ring system having from 5 to 7 carbon atoms and at least one double bond.

The term "monocyclic aryl" refers to an aromatic carbocyclic ring structure having from 5 to 7 carbon atoms.

The term "bicyclic aryl" refers to an aromatic carbocyclic ring structure having from 9 to 12 carbon atoms.

The unqualified term "aryl" refers to either monocyclic aryl or bicyclic aryl.

The term "monocyclic heterocycle" means a saturated or unsaturated ring structure having from 5 to 7 ring atoms of which at least one is a heteroatom, that is not a carbon atom. Preferred heteroatoms include oxygen, nitrogen and sulphur.

The term "bicyclic heterocycle" means a saturated or unsaturated ring structure having from 9 to 12 ring atoms of which at least one is a heteroatom, that is not a carbon atom. Preferred heteroatoms include oxygen, nitrogen and sulphur.

The unqualified term "heterocycle" refers to either monocyclic heterocycle or bicyclic heterocycle.

### Variable A:

In the general formula I, variable A represents an alkyl, alkenyl, cycloalkyl, cycloalkenyl, aryl, heterocyclic, alkylamino, dialkylamino, arylamino or aralkylamino group, each of which is optionally substituted.

Suitable alkyl groups include, but are not limited to, methyl, ethyl, propyl, isopropyl, butyl, isobutyl, t-butyl, pentyl, neopentyl and 1-ethylpropyl. Substituents for substituted alkyl groups A include, but are not limited to, fluorine atoms and hydroxy, cyano, alkoxy, aryl, aryloxy, aralkoxy, acyloxy, cycloalkyl, heterocyle and amino groups. The preferred aryl group is phenyl, the preferred aryloxy group is phenoxy and the preferred aralkoxy group is benzyloxy. These groups may themselves be substituted, especially with small substituents such as methyl, trifluoromethyl, fluoro, chloro, bromo, nitro, cyano, hydroxy, methoxy, ethoxy, isopropoxy, butoxy, trifluoromethoxy, 2,2,2-trifluoroethoxy, acetyl, methylthio and dimethylamino groups.

Preferred cycloalkyl groups are cyclopropyl and cyclohexyl. Preferred heterocyles are 2-thienyl, 3-thienyl, 3-pyridyl and 5-benzo[1,3]dioxolyl groups. Amino groups may also be substituted with alkyl (especially methyl), aralkyl (especially benzyl), aroyl (especially benzoyl), alkylsulphonyl (especially methylsulphonyl), acyl (especially acetyl), cyano, carbamoyl and alkoxycarbonyl (especially *t*-butoxycarbonyl) groups.

Suitable alkenyl groups include vinyl, preferably substituted by phenyl or fluorophenyl.

Suitable cycloalkyl groups are cyclopropyl, cyclobutyl, cyclopentyl, cyclohexyl, cycloheptyl, adamantyl and bicyclo[2,2,2]octyl groups. The preferred substituents for substituted cycloalkyl groups are hydroxy and trifluoromethyl groups.

The preferred cycloalkenyl group is cyclohex-3-enyl.

Aryl groups A are preferably phenyl groups, and these may be substituted with small substituents such as methyl, trifluoromethyl, fluoro, chloro, bromo, nitro, cyano, hydroxy, methoxy, ethoxy, isopropoxy, butoxy, trifluoromethoxy, 2,2,2-trifluoroethoxy, acetyl, methylthio and dimethylamino groups.

Preferred heterocycle groups A include furyl, tetrahydrofuryl, thienyl, pyrrolyl, pyrrolidinyl, pyridyl, piperidinyl, morpholino, isoxazolyl, indolyl and 5-benzo[1,3]dioxazolyl groups. These may be substituted with small groups such as acetyl, C₁-C₄ alkyl and oxo, and with larger groups such as alkoxyalkyl, 1,2,4-triazol-1-ylmethyl, p-tolylsulphonyl, t-butoxycarbonyl, dimethylaminosulphonyl, 1-pyrrolidinylsulphonyl, morpholinosulphonyl and piperidinosulphonyl.

Alkylamino groups A may include methylamino, ethylamino, isopropylamino, t-butylamino and pentylamino groups. The preferred dialkylamino group A is dimethylamino. The prefered arylamino group is aniline, optionally substituted with a halogen atom or a methyl group. The preferred aralkylamino groups are benzylamino, 1-phenylethylamino and 2 phenylethylamino, optionally substituted with a methoxy group.

Most preferably, A is a cyclohexyl group.

### Variable B:

In the general formula I, variable B represents a monocyclic or bicyclic aryl group or a monocyclic or bicyclic heterocycle, each of which is optionally substituted.

The preferred monocyclic aryl group is phenyl. This may be substituted with small substituents such as methyl, trifluoromethyl, fluoro, chloro, bromo, nitro, cyano, hydroxy, methoxy, ethoxy, isopropoxy, butoxy, trifluoromethoxy, 2,2,2-trifluoroethoxy, acetyl, methylthio and dimethylamino groups. More than one substituent may be included. The most preferred substituted monocyclic aryl groups are 2-methoxyphenyl and 4-fluoro-2-methoxyphenyl groups.

Preferred bicyclic aryl groups are naphthyl and 1,2,3,4-tetrahydronaphthyl groups.

Monocyclic heterocycles suitable for variable B include furyl, pyrazolyl, pyridyl, pyrazinyl, pyrimidinyl and thienyl groups. Bicyclic heterocycles are preferred, and include indolyl, quinolyl, isoquinolyl, benzimidazolyl, 2,3-dihydrobenzo[1,4]dioxinyl, 2,1,3-benzothiadiazolyl, 2,1,3-benzoxadiazolyl, benzo[1,3]dioxolyl, benzofuranyl and 3,4-dihydro-2H-benzo{b][1,4]dioxepinyl. Heterocycles B may be substituted with small groups such as methyl, ethyl, isopropyl, halo, trifluoromethyl, cyano, acetonyl and acetyl groups.

### Variable X:

In the general formula I, X represents an amino moiety selected from 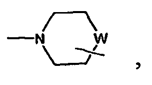 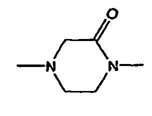 and -N(R₅)-CH₂- wherein R₅ represents a hydrogen atom or a methyl or benzyl group and W represents a bond, a nitrogen atom or a CH, CH₂, C(CN) C(OH) or C(COCH₃) group (when W represents a nitrogen atom or a CH, C(CN) C(OH) or C(COCH₃) group, the group Z-B attaches to the W ring atom). The most preferred of these is that in which W represents a nitrogen atom, namely 1,4-piperazine-di-yl.

### Variable Z:

In the general formula I, when X represents a 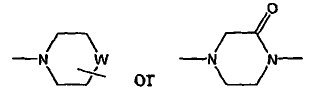 group, Z represents a bond, an oxygen or sulphur atom or a CH₂, CH₂CH₂, CO, CHOH, OCH₂, NH, NHCO or NHCONHCH₂ group. Of these, a bond is preferred. However, when X represents a -N(R₅)-CH₂- group, Z represents a CH₂CH₂ or CH₂O group or Z and B together represent a 2,3-dihydro-benzo[1,4]dioxin-2-yl group. Of these, a CH₂O group is preferred.

### Variable R₁:

Most preferably R₁ represents a hydrogen atom. When R₁ is other than a hydrogen atom, it preferably represents a single substituent which may be in the 5-, 6-, 7- or 8-position, but is preferably in the 6-position. When R₁₁ is other than a hydrogen atom, it is preferably a fluorine, chlorine or bromine atom or a hydroxy, methyl, trifluoromethyl, methoxy, trifluoromethoxy, nitro, phenyl, or amino group.

### Other variables:

R₂ is preferably a hydrogen atom, but may be a methyl group. Variable n is preferably 1. Q preferably represents a carbonyl group. Y preferably represents a CH₂ group.

As previously indicated, the invention further provides pharmaceutical compositions comprising a compound having the general formula I or an enantiomer, diastereomer, N-oxide, crystalline form, hydrate, solvate, active metabolite or pharmaceutically acceptable salt of such a compound in admixture with a pharmaceutically acceptable carrier or diluent. The pharmaceutical composition may also include optional additives, such as a flavouring, a sweetener, a preservative, a dye, a binder, a suspending agent, a dispersing agent, a colourant, a disintegrator, an excipient, a lubricant, an absorption enhancer, a bactericide and the like, a stabiliser, a plasticizer, an edible oil, or any combination of two or more of said additives.

Suitable pharmaceutically acceptable carriers or diluents include, but are not limited to, ethanol, water, glycerol, aloe vera gel, allantoin, glycerine, vitamin-A and E oils, mineral oil, phosphate buffered saline, PPG2 myristyl propionate, magnesium carbonate, potassium phosphate, vegetable oil, animal oil and solketal.

Suitable binders include, but are not limited to, starch, gelatine, natural sugars such as glucose, sucrose and lactose, corn sweeteners, natural and synthetic gums such as acacia, tragacanth, vegetable gum, sodium alginate, carboxymethylcellulose, polyethylene glycol, waxes and the like.

Suitable disintegrators include, but are not limited to, starch such as corn starch, methyl cellulose, agar, bentonite, xanthan gum and the like.

Suitable lubricants include, but are not limited to, sodium oleate, sodium stearate, magnesium stearate, sodium benzoate, sodium acetate, sodium chloride and the like.

Suitable suspending agents include, but are not limited to, bentonite.

Suitable dispersing and suspending agents include, but are not limited to, synthetic and natural gums such as vegetable gum, tragacanth, acacia, alginate, dextran, sodium carboxymethylcellulose, methylcellulose, polyvinylpyrrolidone and gelatine.

Suitable edible oils include, but are not limited to, cottonseed oil, sesame oil, coconut oil and peanut oil.

Examples of additional additives include, but are not limited to, sorbitol, talc, stearic acid and dicalcium phosphate.

The pharmaceutical composition may be formulated as unit dosage forms, such as tablets, pills, capsules, boluses, powders, granules, sterile parenteral solutions, sterile parenteral suspensions, sterile parenteral emulsions, elixirs, tinctures, metered aerosol or liquid sprays, drops, ampoules, autoinjector devices or suppositories. The unit dosage forms may be used for oral, parenteral, intranasal, sublingual or rectal administration, or for administration by inhalation or insufflation, transdermal patches, and a lyophilized composition. In general, any delivery of active ingredients that results in systemic availability of such ingredients can be used. Preferably the unit dosage form is an oral dosage form, most preferably a solid oral dosage; therefore the preferred dosage forms are tablets, pills and capsules. However, parenteral preparations are preferred too.

Solid unit dosage forms may be prepared by mixing the active agents of the present invention with a pharmaceutically acceptable carrier and any other desired additives as described above. The mixture is typically mixed until a homogeneous mixture of the active agents of the present invention is obtained and the carrier and any other desired additives are formed, i.e. the active agents are dispersed evenly throughout the composition. In this case, the composition can be formed as dry or moist granules.

Tablets or pills can be coated or otherwise prepared so as to form a unit dosage form that has delayed and/or sustained action, such as controlled release and delayed release unit dosage forms. For example, the tablet or pill can comprise an inner dosage and an outer dosage component, the latter being in the form of a layer or envelope over the former. The two components can be separated by an enteric layer which serves to resist disintegration in the stomach and permits the inner component to pass intact into the duodenum or to be delayed in release.

Biodegradable polymers for controlling the release of the active agents include, but are not limited to, polylactic acid, polyepsilon caprolactone, polyhydroxybutyric acid, polyorthoesters, polyacetals, polydihydropyrans, polycyanoacrylates and crosslinked or amphipathic block copolymers of hydrogels.

For liquid dosage forms, the active substances or their physiologically acceptable salts are dissolved, suspended or emulsified, optionally with the usually employed substances such as solubilizers, emulsifiers or other auxiliaries. Solvents for the active combinations and the corresponding physiologically acceptable salts can include water, physiological salt solutions or alcohols, e.g. ethanol, propanediol or glycerol. Additionally, sugar solutions such as glucose or mannitol solutions may be used. A mixture of the various solvents mentioned may be used in the present invention too.

A transdermal dosage form is contemplated by the present invention too. Transdermal forms may be a diffusion transdermal system (transdermal patch) using either a fluid reservoir or a drug-in-adhesive matrix system. Other transdermal dosage forms include, but are not limited to, topical gels, lotions, ointments, transmucosal systems and devices, and iontophoretic (electrical diffusion) delivery systems. Transdermal dosage forms may be used for delayed release and sustained release of the active agents of the present invention.

The pharmaceutical compositions and unit dosage forms of the present invention for parenteral administration, and in particular by injection, typically include a pharmaceutically acceptable carrier, as described above. A preferred liquid carrier is vegetable oil. Injection may be, for example, intravenous, epidural, intrathecal, intramuscular, intraluminal, intratracheal or subcutaneous.

The active agents can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The active agents of the present invention may also be coupled with soluble polymers such as targetable drug carriers. Such polymers include, but are not limited to, polyvinylpyrrolidone, pyran copolymers, polyhydroxypropylmethacrylamidophenol, polyhydroxyethylaspartamidophenol, and polyethylenoxypolylysine substituted with palmitoyl residues.

The pharmaceutical composition or unit dosage forms of the present invention may be administered by a variety of routes, such as the oral and enteral, intravenous, intramuscular subcutaneous, transdermal, transmucosal (including rectal and buccal) and by inhalation routes.

Preferably, the oral or transdermal route is used (i.e. with solid or liquid formulations or skin patches, respectively).

The pharmaceutical composition or unit dosage forms comprising an effective amount of the present invention may be administered to an animal, preferably a human, in need of treatment of neuromuscular dysfunction of the lower urinary tract described by E. J. McGuire in "Campbell's UROLOGY", 5^{th} Ed. 616-638, 1986, W.B. Saunders Company, and patients affected by any physiological dysfunction related to impairment of 5-HT_{1A} receptor function. Such dysfunctions include, without limitation, central-nervous-system disorders such as depression, anxiety, eating disorders, sexual dysfunction, addiction and related problems.

As used herein, the term "effective amount" refers to an amount that results in measurable amelioration of at least one symptom or parameter of a specific disorder. In a preferred embodiment, the compound treats disorders of the urinary tract, such as urinary urgency, overactive bladder, increased urinary frequency, reduced urinary compliance (reduced bladder storage capacity), cystitis (including interstitial cystitis), incontinence, urine leakage, enuresis, dysuria, urinary hesitancy and difficulty in emptying the bladder, or central nervous system disorders due to serotonergic dysfunction (such as anxiety, depression, hypertension, sleep/wake cycle disorders, feeding behaviour, sexual function and cognition disorders in mammals (particularly a human) associated to stroke, injury, dementia and due to neurological development, disorders from hyperactivity related to an attention deficit (ADHD), drug addiction, drug withdrawal, irritable bowel syndrome.

The pharmaceutical composition or unit dosage form of the present invention may be administered according to a dosage and administration regimen defined by routine testing in the light of the guidelines given above in order to obtain optimal activity while minimising toxicity or side effects for a particular patient. However, such fine tuning of the therapeutic regimen is routine in the light of the guidelines given herein.

The dosage of the active agents of the present invention may vary according to a variety of factors such as underlying disease conditions, the individual's condition, weight, sex and age, and the mode of administration. An effective amount for treating a disorder can easily be determined by empirical methods known to those of ordinary skill in the art, for example by establishing a matrix of dosages and frequencies of administration and comparing a group of experimental units or subjects at each point in the matrix. The exact amount to be administered to a patient will vary depending on the state and severity of the disorder and the physical condition of the patient A measurable amelioration of any symptom or parameter can be determined by a person skilled in the art or reported by the patient to the physician. It will be understood that any clinically or statistically significant attenuation or amelioration of any symptom or parameter of urinary tract disorders is within the scope of the invention. Clinically significant attenuation or amelioration means perceptible to the patient and/or to the physician.

For example, a single patient may suffer from several symptoms of dysuria simultaneously, such as, for example, urgency and excessive frequency of urination or both, and these may be reduced using the methods of the present invention. In the case of incontinence, any reduction in the frequency or volume of unwanted passage of urine is considered a beneficial effect of the present method of treatment.

The amount of the agent to be administered can range between about 0.01 and about 25 mg/kg/day, preferably between about 0.1 and about 10 mg/kg/day and most preferably between 0.2 and about 5 mg/kg/day. It will be understood that the pharmaceutical formulations of the present invention need not necessarily contain the entire amount of the agent that is effective in treating the disorder, as such effective amounts can be reached by administration of a plurality of doses of such pharmaceutical formulations.

In a preferred embodiment of the present invention, the compounds are formulated in capsules or tablets, preferably containing 50 to 200 mg of the compounds, of the invention, and are preferably administered to a patient at a total daily dose of 50 to 400 mg, preferably 150 to 250 mg and most preferably about 200 mg, for relief of urinary incontinence and dysfunctions under treatment with 5-HT_{1A} receptor ligand.

A pharmaceutical composition for parenteral administration contains from about 0.01% to about 100% by weight of the active agents of the present invention, based upon 100% weight of total pharmaceutical composition.

Generally, transdermal dosage forms contain from about 0.01% to about 100% by weight of the active agents versus 100% total weight of the dosage form.

The pharmaceutical composition or unit dosage form may be administered in a single daily dose, or the total daily dosage may be administered in divided doses. In addition, co-administration or sequential administration of another compound for the treatment of the disorder may be desirable. For example, the compounds of the invention may be administered in combination with known antimuscarinic drugs such as oxybutynin, tolterodine, darifenacin and temiverine. Analogously, the compounds of the invention may be associated to α₁-adrenergic antagonists, such as prazosin, doxazosin, terazosin, alfuzosin and tamsulosin for the therapy of the lower urinary tract symptoms.

For combination treatment where the compounds are in separate dosage formulations, the compounds can be administered concurrently, or each can be administered at separate staggered times. For example, the compound of the invention may be administered in the morning and the antimuscarinic compound may be administered in the evening, or vice versa. Additional compounds may be administered at specific intervals too. The order of administration will depend upon a variety of factors including age, weight, sex and medical condition of the patient; the severity and aetiology of the disorders to be treated, the route of administration, the renal and hepatic function of the patient, the treatment history of the patient, and the responsiveness of the patient. Determination of the order of administration may be fine-tuned and such fine-tuning is routine in the light of the guidelines given herein.

Without wishing to be bound by theory, it is believed that administration of 5-HT_{1A} receptor antagonists prevents unwanted activity of the sacral reflex and/or cortical mechanisms that control micturition. Thus, it is contemplated that a wide range of neuromuscular dysfunctions of the lower urinary tract can be treated using the compounds of the present invention, including without limitation dysuria, incontinence and enuresis (overactive bladder). Dysuria includes urinary frequency, nocturia, urgency, reduced urinary compliance (reduced bladder storage capacity), difficulty in emptying the bladder, i.e. a suboptimal volume of urine is expelled during micturition. Incontinence syndromes include stress incontinence, urgency incontinence and enuresis incontinence, as well as mixed forms of incontinence. Enuresis refers to the involuntary passage of urine at night or during sleep.

The compounds of the present invention may also be useful for the treatment of central nervous system disorders due to serotonergic dysfunction.

### SYNTHESIS OF THE COMPOUNDS OF THE INVENTION

The compounds according to the invention may generally be prepared as follows:

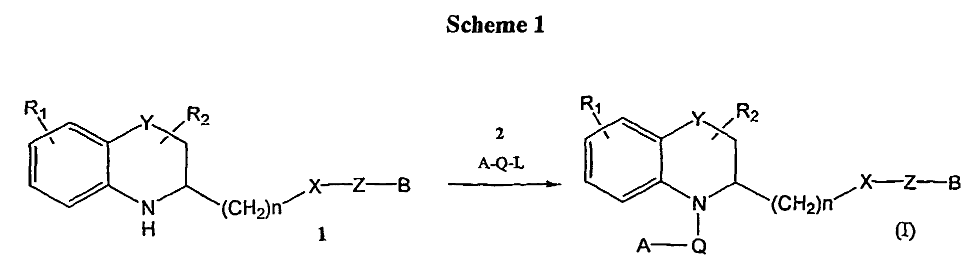

The compounds of the invention are obtained by direct condensation of compounds **1** with compounds **2**. When A is alkyl, cycloalkyl, cycloalkenyl, aryl, heterocycle, (di)alkylamino or cyclic amino and L is a halogen atom, this step is the well-known reaction between an acyl, sulphonyl, thiocarbonyl or carbamoyl chloride (chloroformamide) and amine **1**. Generally, the said chlorides are commercially-available items or are prepared by conventional procedures well documented in the literature and very well known to those skilled in the art. Condensation is carried out as usual in an aprotic solvent (e.g. a chlorinated solvent or tetrahydrofuran or toluene) in the presence of an organic or inorganic base, such as triethylamine or diisopropylethylamine, as a proton scavenger at a temperature in the range of between-20°C and the reflux of the solvent.

If compound **2** is a carboxylic acid (L = OH), condensation can be carried out in the presence of a condensing agent (e.g. dicyclohexylcarbodiimide or diethyl cyanophosphonate), optionally in the presence of a promoting agent (e.g. N-hydroxysuccinimide, 4-dimethylaminopyridine or N,N'-carbonyldiimidazole) in an aprotic or chlorinated solvent (e.g. N,N-dimethylformamide or chloroform) at between-10 and 140°C (Albertson N. F., *Org. React.* **12**, 205-218 (1962); Doherty A. M. et al., *J. Med. Chem.* **35****,** 2-14 (1992); Ishihara Y. et al., *Chem Pharm. Bull.* **39****,** 3236-3243 (1991)). In some cases the activated intermediate esters or amides (such as O-(N-succinimidyl) esters or N-acyl imidazolides) can be isolated and further reacted with compound **1** to be transformed into the corresponding amides (**I**) in an aprotic or chlorinated solvent at between 10 and 100°C. Another activated intermediate which can be used is the mixed anhydride of the carboxylic acid **2**, obtainable by reacting **2** (L = OH) with an alkyl chloroformate (optionally supported on resin) in the presence of a tertiary amine (e.g. triethylamine or N-methylmorpholine), then reacting it with compound 1 at between 0 and 80°C; optionally a promoting agent (e.g. 1-hydroxypiperidine) may be added before the amine addition (Albertson N. F., *Org. React.* **12****,** 157 (1962)). Alternatively, condensation can be carried out without a solvent at between 150 and 220°C (Mitchell J. A. et al., *J. Am. Chem. Soc.* **53**, 1879 (1931)) or in high-boiling ethereal solvents (e.g. diglyme). Less reactive derivatives of the carboxylic acid **2** can be used too, such as alkyl esters, which, in turn, can be converted into (**I**) in the presence of a condensing agent (e.g. trimethylaluminum) in an aprotic and/or chlorinated solvent (e.g. hexane, dichloromethane) at between -10 and 80°C, or without solvents at between 80 and 180°C (Weinreb S. M. et al., *Tetrahedron Lett.* 4171 (1977); Lipton M.F. et al., *Org. Synth.* **59**, 49 (1979)). An alternative procedure to prepare compounds (**I**) where A is alkylamino, dialkylamino, cyclic amino, arylamino or arylalkylamino, consists in the use of isocyanates, which are commercially available or prepared in situ or a priori by conventional procedures well known to those skilled in the art, or in the use of carbonyldiimidazole or phosgene or other phosgene-like compounds subsequently reacting them in a suitable solvent (e.g. THF or toluene or a chlorinated solvent) with compound **1** followed by the proper amine (or vice-versa).

Intermediates **2** (L = OH, Cl) can be prepared by standard procedures well known to those skilled in the art and documented in the literature. Compounds (**I**) where A is an alkyl optionally substituted with one or more hydroxy, alkoxy, arylalkoxy, amino, acylamino, cyanoamino, aminocarbonyl, alkylaminocarbonyl groups can be alternatively prepared from compounds (**I**) where AQ is Hal-Alkyl-Q or CH₂=CHCO (**Ia**) by simple nucleophilic substitutions or 1,4 additions (Michael reactions) with the appropriate reactant (e.g. sodium cyanamide, sodium cyanide, sodium alkoxides). The synthesis of compounds (**I**) where A contains an OH group or an amino or acylamino group requires a supplementary step of deprotection (and acylation). For example, compounds (**I**) with a masked or protected amino group or a protected hydroxy group can be obtained by reaction of (**Ia**) with sodium benzylate or sodium azide or potassium phthalimide or benzylamine or others (see, for example; T. W. Greene et al., Protective group in organic synthesis, 3^{rd} Ed., Wiley Interscience, New York). Compounds (**I**) are obtained after deprotection or reduction in the case of azido group by standard methods. The preferred method for the synthesis of compounds (**I**) where A contains an OH group consists anyway in the reaction of a O-protected O-alkylCOOH derivative (or its activated analogue) with compounds **1**. Preferred protecting group is benzyl which can be removed by hydrogenolysis.

When R₁ and the substituents on ring B in compounds **I** represent reactive groups (e.g. hydroxyl or amino), these should be protected before the acylation of Scheme 1 and then deprotected following the known methods described in the foregoing T. W. Greene et al..

Preparation of Intermediates **1** can be carried out by several methods. The main procedures are described below.

The first is detailed in Scheme 2 and includes nucleophilic substitution of the appropriate amine **14** on a 2-halomethylquinoline **4** or 1,2-addition to a 2-vinylquinoline **6** in a suitable solvent (e.g. acetonitrile, N,N-dimethylformamide, a chlorinated solvent, toluene or other protic or aprotic polar solvent) at a temperature of between 0°C and the reflux temperature of the solvent in the presence or not of a base such as N,N-diisopropylethylamine, TEA, potassium carbonate, 1,8-diazabicycloundec-7-ene or others. Intermediates **4** can be prepared by halogenation of **3** with N-bromosuccinimide or N-chlorosuccinimide by conventional procedures well known to those skilled in the art and documented in the experimental part.

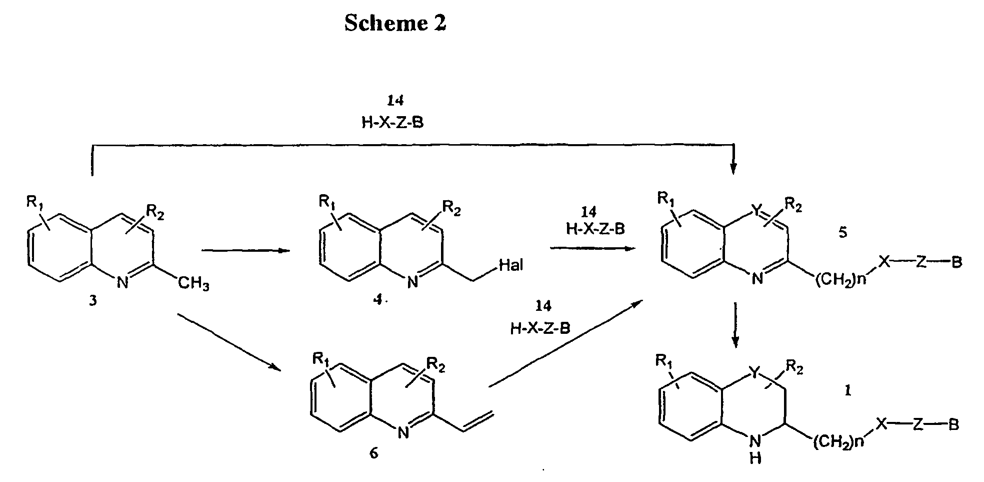

Those of compounds **3** which are not commercially available can be prepared from the corresponding substituted anilines by the methods cited by O. Foye et al., *J. Pharm. Sci.* **68**(3), 336-338, (1979) or by J. C. Hardy et al. WO 98/38194 or by other suitable methods disclosed in the literature.

Those of compounds **6** which are not commercially available can be prepared from the corresponding compounds 3 by the method of Buchmann G. et al., *J. Prakt. Chem.* **24**(4), 101-112, (1964) or by other suitable methods.

An alternative procedure for the preparation of the compounds 5 where n is 2 is a reaction between the 2-methylquinolines 3 and formaldehyde in the presence of an appropriate cyclic amine. This is the well-known Mannich reaction, which is generally carried out in the following manner, viz. the amine in the form of an acid addition salt is dissolved in a 40% aqueous formaldehyde solution and then added to an alcoholic solution of 2-methylquinoline, and the resulting mixture is warmed. Alternative Mannich procedures which can be applied if necessary are available and described in the literature. The tertiary amine 5 is then converted to the desired intermediate **1** by standard hydrogenation methods in the presence of a catalyst such as platinum, platinum dioxide, palladium or rodhium or nickel, with or without a support such as charcoal or alumina.

This hydrogenation can also be carried out using nascent hydrogen generated by sodium, lithium or potassium metals and a lower alkanol, e.g. methanol, ethanol, propanol, n-butanol, at a temperature ranging between the room temperature and the boiling point of the solvent. Other methods of reduction of the pyridine ring can be used too, as detailed in the literature (C. J. Moody, *SYNLETT* **9**, 1029-1030, (1998); B. C. Ranu, *Synth. Comm.* **28**(3), 485-492, (1998); P. Balczewsky, *Synth. Comm.* **20** (18), 2815-2819 (1990); A Srikrishna, *Tetrahedron* **52** (5), 1631-1636 (1996)).

The same synthetic approach can be used to prepare the corresponding indolyl derivatives (equivalent to **5**) from 2-methylindole analogues, subsequently reducing them to the indoline derivatives corresponding to **1**.

An alternative procedure to prepare compounds **1** is illustrated in Scheme 3.

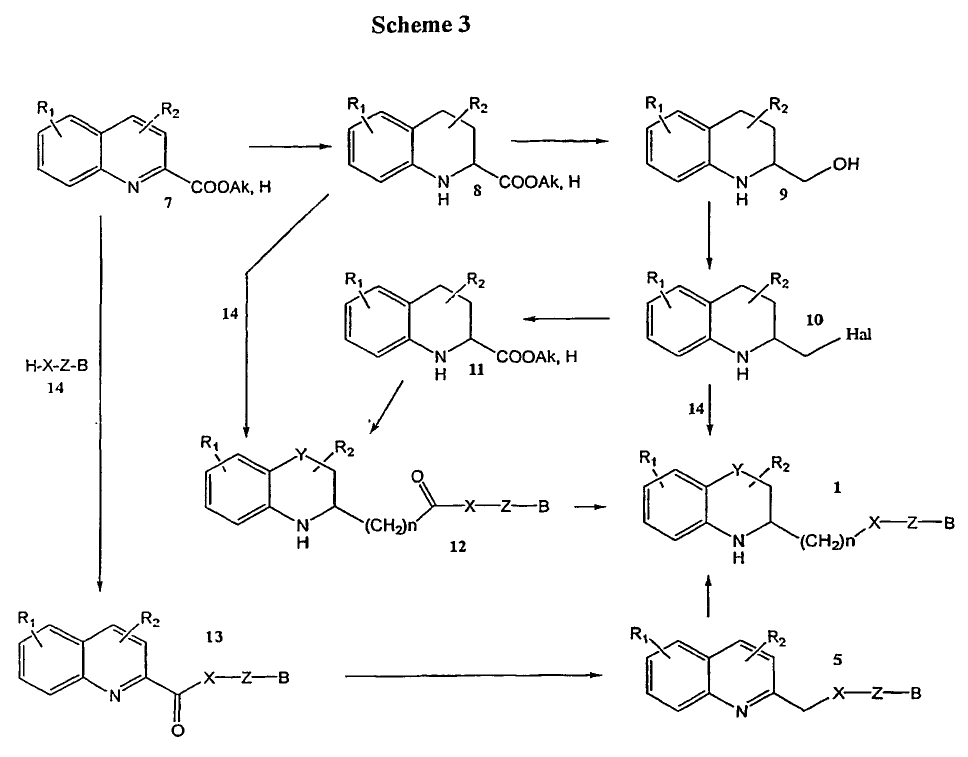

Intermediates 7 are commercially available or can be prepared by methods very well known to those skilled in the art and cited in the literature e.g. by Reissert reaction, treating the appropriate quinoline with cyanide ion or equivalent (for example trimethylsilylcyanide Popp, F.D. *Heterocycles,* 23, 731, **1985**; D. E. Portlock, US 4,461,896; Renaud, A. et al, EP322263) or by haloform reaction from the appropriate 2-methylquinoline by exhaustive halogenation followed by hydrolysis (Ejima,A. et al USP 6169086) or by reacting an appropriately substuted aniline with dimethyl acetylenedicarboxylate in a high boiling solvent (e.g. diphenyl ether) affording compound **7** as a 4-hydroxy derivative which can be on turn converted into a 4-halo derivative (phosporus oxychloride). Compound **7** can be reduced to the corresponding 1,2,3,4-tetrahydroderivatives (**8**) by the same hydrogenation procedures cited above and condensed by standard methods with the appropriate amines to afford amides **12** (see above for condensation reactions). The next step is a standard reduction with lithium aluminium hydride or borane or, where necessary, non standard reduction (e.g. conversion into a thioamide and reduction with boron and sodium hydride) to convert the amide carbonyl function to a methylene group. The reduction is accomplished by dissolving the amide in a solvent such as dioxane and then adding the organic solution slowly to a thick suspension of lithium aluminium hydride in anhydrous dioxane or tetrahydrofuran or 1,2-dimethoxyethane or other solvent: The catalytic-hydrogenation step that reduces the hetero ring in the quinoline nucleus can also be performed on the reduction products **5** obtained by the same procedure as above by reduction of intermediates **13**, in turn obtained from **7**. The carboxy group of intermediates 8 can also be reduced by standard fashions (see R. Nagata et al., *J*. *Med. Chem.* **37**, 3956-3968 (1994)) to give alcohols **9** which, in turn, can be converted into haloderivatives **10** by standard halogenation procedures (SOCl₂, PBr₃ or others) or by Mitsunobu reaction (R. Nagata or carbon tetrachloride, triphenylphosphine and a chlorinated solvent or others, between room temperature and reflux). The compounds **10** can be reacted through classical nucleophilic-substitution methods, very well-known to those skilled in the art, with the appropriate amines to afford compounds **1**. The compounds **10** can also be used in the homologation reactions which lead to formation of **11** (for example via cyanide nucleophilic substitution, hydrolysis and reesterification). The entire last reaction pathway can be carried out by conventional methods which are well documented in the literature (see for example R. Nagata et al.).

In addition to the above procedures, there are two similar related methods for preparing the same compounds. The first variation utilises a 2-formylquinoline (obtained, for example, by selenium-dioxide oxidation of derivatives **3 (Scheme 2)** as starting material instead of the corresponding 2-carboxylic acid. The 2-formyl compound can be simultaneously reacted with amine **14** and reduced via hydrogenation over Raney nickel, platinum oxide (Adams catalyst) or a catalyst made up of 5% palladium on barium sulphate. The combined reaction is generally carried out in a suitable solvent such as absolute alcohol. The last procedure can also be performed by using a classical reductive-amination method and a reducing agent such as sodium triacetoxyborohydride or sodium cyanoborohydride followed by hydrogenation of the pyridine nucleus. If R₁ is nitro, a nitration step should be introduced, for example, on compounds **10** or **11** (preferred) in which R₁ is H. The best mode to carry out the nitration step is to use a N-protected **11** (e.g. N-cyclohexylcarbonyl derivative **15** (see Scheme 4). The compounds of the invention in which R₁ is nitro can also be prepared by nitro-dediazosubstitution (Sandmeyer reaction) on diazonium salt from aniline compounds, as well as compounds where R₁ is halogen, which can be obtained from the corresponding compounds where R₁ is nitro by reduction to the amine, followed by diazotization and subsequent conversion to the halo compound via well-known methods.

If R₁ is Br, a bromination step e.g. (with N-bromosuccinimide in DMF) should be introduced, for example, on compounds **10** or **11** (preferred) in which R₁ is H. Bromination on compounds **15** (Scheme 4) requires harsher conditions (e.g. Br₂ with Fe catalysis in dichloromethane or other methods from the literature (see Nagata R. above)). It should be noted that the starting materials required for all these reactions are either commercially available or easily prepared using standard techniques and normal organic-synthesis procedures. Such techniques are disclosed in standard organic-synthesis textbooks and/or published in specific literature references.

The compounds **I** where Y is a bond (2,3-dihydroindoles) can be prepared in analogy with the procedures detailed above for tetrahydroquinoline derivatives (see Nagata R. above). Harsher conditions are required for the acylations that lead to compounds (**I**) when the heterocycle is an indole derivative. The use of strong bases is needed to deprotonate the indole NH group to make it a good nucleophile (see T. W. Greene et al., Protective Group in Organic Synthesis, 3^{rd} Ed., Wiley Interscience, New York, 1999). When B is not an N-protected indole, the last acylation step to form **I** must be carried out after protecting the B indole with a proper group such as a silyl or tertbutoxycarbonyl or other group (see T. W. Greene above). The obtained protected compound **I** must then be deprotected by standard methods. Alternatively, the compounds **I** where B is indole or other potentially-reactive hetero ring can be prepared starting from, for example, a methyl or ethyl indole-2-carboxylate, N-acylating it and reducing the carboxylate group, avoiding harsh reaction conditions, for example by using CaBH₄ or hydrolyzing the ester moiety, and carrying out the reduction on the mixed anhydride obtained from indole-2-carboxylic acid and ethylchloroformate with boron sodium hydride. These N-acyl-2-indolylmethanol derivatives can be converted to the final compounds **I** having an indole as B, as described for intermediate 9 (Scheme 3).

The compounds where Y is a CH bearing a double bond can be obtained as described in US 3,929,784 by the method of Reissert starting from a proper quinoline or other known method or its variations. For example, reacting a substituted quinoline with benzoyl chloride and potassium cyanide one can obtain 1-benzoyl-2-cyano(substituted)-1,2-dihydroquinoline, or by reaction with benzoyl chloride and diethyl malonate (CA76:59409) followed by hydrolysis/decarboxylation, one can prepare the 2-quinoline acetic derivative. Afterwards, classical reaction methodologies can afford the same type of intermediates described for the tetrahydro compounds.

An alternative procedure to obtain the compounds **I** of the invention, especially amenable when dealing with optically active compounds, is described in Scheme 4.

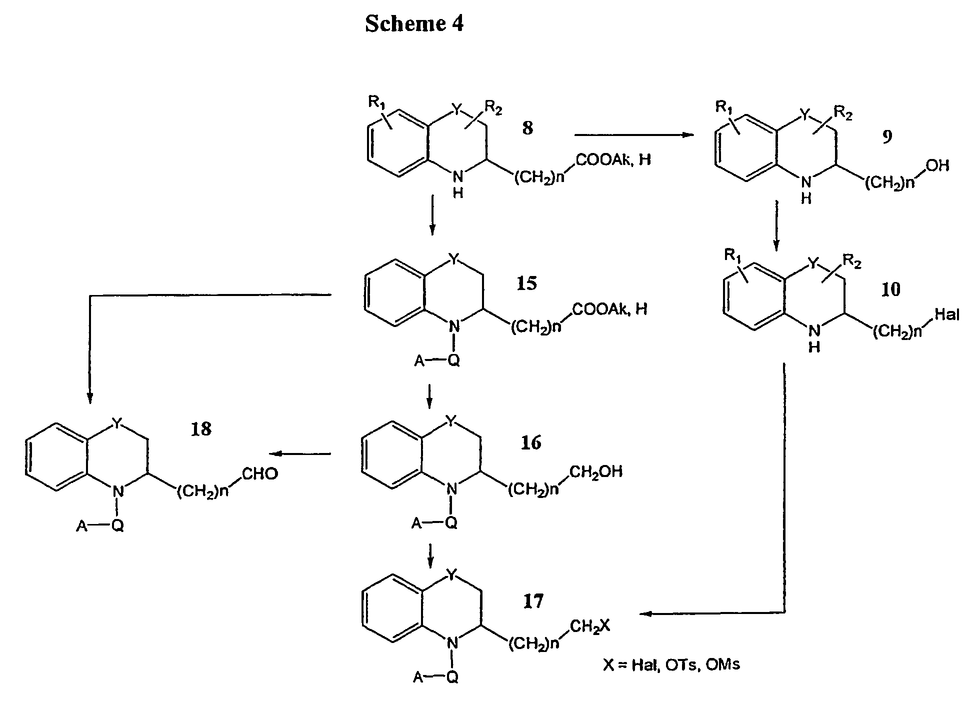

The NH group of Intermediates **8** and **11** can be acylated by standard fashions (see above for the acylation of Intermediates 1) to give polyfunctionalized compounds **15** which, in turn, can be selectively reduced by methods known from the literature (e.g. lithium borohydride, sodium borohydride in alcohols or water, sodium borohydride-lithium chloride or sodium borohydride-calcium chloride, calcium borohydride, borane-THF complex or borane-dimethyl sulfide complex or, only when **8** or **11** is a carboxylic acid, generating the mixed anhydride with a chloroformate and a base and reducing it with sodium borohydride). Alternatively alcohols **16** can be prepared by reacting Intermediates **9** with excess acylating reagent to afford the corresponding O,N-diacylated derivatives, which in turn can be selectively O-monohydrolized by known methods. The so obtained alcohols **16** can be converted into the haloderivative **17** by standard halogenation procedures (SOCl₂, PBr₃ or other) or by Mitsunobu reaction (R. Nagata or CCl₄, triphenylphosphine/ chlorinated solvent or other, r.t. - reflux). **17** can be reacted through classical nucleophilic substitution methods very well known to those skilled in the art with the appropriate amine to afford compounds **I.** Compounds **17** can also be prepared carrying out the acylation reactions on intermediate **10**.

An alternative procedure to obtain the compounds **I** of the invention consists in reacting in a reductive amination fashion the aldehyde **18** with amines **14**. The Intermediate **18** can be generated by selectively reducing compounds **15** (e.g. with diisobutylaluminumhydride or by Rosemund reaction *(Bull. Chem. Soc. Jpn.* **58** (11), 3337-45, (1985)) or other methods (*J.Org. Chem.* **64** (24), 8962-8964, (1999) or *J. Org. Chem.* **51** (5), 705-12, (1986)). Alternatively, alcohols **16** can be oxidized to compounds **18** by known methods (e.g. Swern's oxidation or DMSO-based oxidation methods (*Synthesis,* 857 **(1990)** or pyridinium dichromate or Martin's reagent or manganese dioxide).

Compounds **15** are very useful intermediate for the synthesis of compounds **(I)** where R₂ is a 2-alkyl group. This group can be introduced by a proton abstraction at the 2-position of the tetrahydroquinoline ring with a strong base (e.g. NaNH₂, NaH, BuLi etc.) and alkylation with the proper alkyl halogenide (see *Bioorg. Med. Chem. Lett.* 5 1527, (1995)). Compounds **(I)** where R₁ is aryl or heteroaryl can be obtained by very well known Suzuki coupling of compounds **(I)** with R₁=Br. The chemical procedures are very well documented in the experimental part.

The N-oxides of compounds **I** may be synthesised by simple oxidation procedures known to those skilled in the art. The oxidation procedure described by Brougham *P.,Synthesis,* 1015-1017 (**1987**), allows differentiation of the two nitrogen atoms of the piperazine ring, permitting both the N-oxides and N, N'-dioxide to be obtained.

The above compounds **I** (tetrahydro or dihydroquinoline and 2,3-dihydroindole compounds), carry a stereocenter at position 2 of the hetero ring and can be obtained as two enantiomers.

Resolution of the racemic form of **(I)** can be carried out by fractional crystallization of the diastereoisomeric salt prepared by salification of **(I)** with an optically-active acid or by preparative-chiral-column chromatography methods as described in the experimental part.

The enantiomers of **(I)** can be obtained by stereospecific synthesis starting from the homochiral compounds **8** (Scheme 3). These can be obtained by known methods which include N-derivatization of the esters with chiral compounds, separation by chromatographic column or fractional crystallization or hydrolysis of the diastereomeric-acid mixture followed by fractional crystallization and deprotection (M. Paglialunga et al, *J*. *Chem. Soc. Perkin Trans. I.,* 596-600, (**1976**)). Alternatively direct fractional crystallization of the salt of the N-protected acids 8 with optically-active bases can be performed (D. E. Portlock US 4.461.896).

The homochiral compounds **8** (Scheme 3) can also be obtained by enzymatic resolution (S. Katayama et al., *Tetrahedron Asymmetry* **9**, 4295-4299, (1998)).

Preparation of the intermediate amines **14,** not yet known in the literature, uses synthesis procedures very well-known to those skilled in the art. Piperazine preparation methods comprise the synthesis of a proper aniline through standard reactions and the subsequent cyclization with bis-(2-chloroethyl)amine to afford piperazine following the method of Prelog V. et al., *Collect. Czech. Chem. Comm.* **5****,** 497-502 (1933)) or its variations (Elworthy T. R., *J*. *Med. Chem.* **40,**. 2674-2687 (1997)).

Preparation of the intermediate aryl or heterocyclopiperidines not commercially available can be carried out by known methods which include reaction of aryl or heterocycle organometallic compounds with N-protected piperidones (e.g. carbobenzyloxy or tertbutoxycarbonyl piperidones or ethoxycarbonyl piperidones) to afford compounds 4-aryl or heterocyclopiperidinol which can be dehydrated and reduced or deoxygenated to compounds **14.** Alternatively, N-protected piperidones (e.g. carbobenzyloxv and tertbutoxycarbonyl or ethoxycarbonyl piperidones) can be converted to their silyl enol ethers and reacted, by palladium-catalyzed reactions, with aryl or heterocycle organometallic compounds or aryl or heterocycloboronic acids or esters to afford the ene compounds which are then reduced to compounds **14**. The other required cyclic amines **14** can in turn be prepared by known methods.

### BRIEF DESCRIPTION OF DRAWINGS

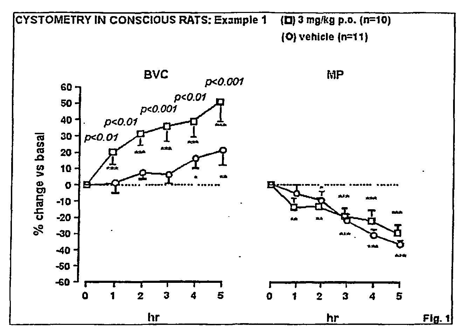

**Figure 1.** Time course of BVC and MP changes in rats after oral administration of vehicle (circles) or 3.0 mg/kg of the racemic compound ((±)1-(1-cyclohexanecarbonyl-1,2,3,4-tetrahydroquinoline-2-ylmethyl)-4-(4-indolyl)piperazine) of Example 1 (squares). Data represent the % changes *versus* basal values at different times from treatment. "n" = number of rats/group. Significance shown as P<... (between treatments: ANOVA of CONTRAST VARIABLES) indicates the difference between the trend observed in the control (vehicle) and treated groups. Asterisks (* = p < 0.05, ** = p < 0.01 and *** = p < 0.001) indicate significance between the value observed at the time reported and the baseline value (within treatment).

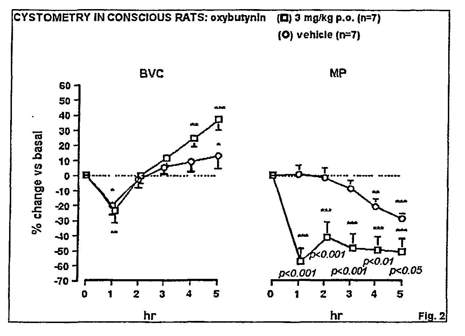

**Figure 2.** Time-course of BVC and MP changes in rats after oral administration of vehicle (circles) or 3.0 mg/kg of oxybutynin (squares). Data are expressed as in Figure 1.

### EXAMPLES

The following examples illustrate the invention but do not limit it. ¹H-NMR spectra were taken at 200 MHz in CDCl₃ unless otherwise specified. The main abbreviations used in the examples are as follows: CHCl₃ stands for alcohol-free chloroform, THF stands for tetrahydrofuran, Me stands for methyl, Et stands for ethyl, Ph stands for phenyl, Ac stands for acetyl, DMF stands for N,N-dimethylformamide, DIPEA stands for N,N--diisopropylethylamine, TEA stands for triethylamine, NBS stands for N-bromosuccinimide, AIBN stands for azoisobutyronitrile, DMAP stands for 4-dimethylaminopyridine.

### Example 1

1-(1-Cyclohexanecarbonyl-1,2,3,4-tetrahydroquinoline-2-ylmethyl)-4-(4-indolyl)-piperazine,
(+)-1-(1-Cyclohexanecarbonyl-1,2,3,4-tetrahydroquinoline-2-ylmethyl)-4-(4-indolyl)-piperazine,
(-)-1-(1-Cyclohexanecarbonyl-1,2,3,4-tetrahydroquinoline-2-ylmethyl)-4-(4-indolyl)-piperazine

### a) 2-Chloromethyl-1,2,3,4-tetrahydroquinoline (Compound 1A)

A solution of 0.13 g of 2-hydroxymethyl-1,2,3,4-tetrahydroquinoline (prepared as described in Nagata R. et aL, *J. Med. Chem.* **37**, 3956-3968 (1994)), 0.39 g of Ph₃P in 5 ml of CHCl₃ and 3 ml of CCl₄ was stirred at 50°C for 6 hours. The mixture was evaporated to dryness and the residue was purified by flash chromatography (petroleum ether- ethyl acetate 97:3) to give 0.09 g (66%) of the title compound.
¹H-NMR (δ): 1.00-2.00 (b, 1H), 1.65-1.85 (m, 1H), 1.90-2.10 (m, 1H), 2.60-2.95 (m, 2H), 3.45-3.70 (m, 3H), 6.50-6.70 (m, 2H), 6.90-7.10 (m, 2H)

### b) 1-(4-Indolyl)-4-(1,2,3,4-tetrahydroquinoline-2-ylmethyl)-piperazine (Compound 1B)

0.13 g of Compound 1A and 0.20 g of 1-(4-indolyl)-piperazine (W0 99/67237) in 1.5 ml of DMF was stirred at 100°C under nitrogen atmosphere for 2 hours. The cooled mixture was poured into 20 ml of water and extracted with ethyl acetate (3 x 5 ml). The organic layer was dried on anhydrous sodium sulphate and evaporated to dryness. The residue was purified by flash chromatography (petroleum ether - ethyl acetate 7:3) to give 0.08 g (32%) of the title compound.
¹H-NMR (δ): 1.45-1.75 (m, 1H), 1.85-2.00 (m, 1H), 2.45-3.10 (m, 8H), 3.20-3.60 (m, 6H), 6.50-6.70 (m, 4H), 6.90-7.05 (m, 2H), 7.05-7.20 (m, 3H), 8.25 (bs, 1H)

### c) 1-(1-Cyclohexanecarbonyl-1,2,3,4-tetrahydroquinoline-2-ylmethyl)-4-(4-indolyl)-piperazine

A mixture of 0.07 g of Compound 1B, 0.05 ml of DIPEA, 0.04 ml of cyclohexanecarbonyl chloride and 1.5 ml of toluene was stirred at 20 to 25°C under nitrogen atmosphere for 1 hour. The solution was washed with a 5% aqueous solution of sodium bicarbonate (3 x 3 ml) and 3 ml of water. The organic layer, dried on anhydrous sodium sulphate, was evaporated to dryness and the residue purified by flash chromatography (dichloromethane - methanol 95:5) to give 0.03 g (33%) of the title compound.
¹H-NMR (δ): 0.80-2.05 (m, 12H), 2.05-2.25 (m, 1H), 2.25-2.85 (m, 8H), 3.10-3.35 (m, 4H), 5.00-5.25 (m, 1H), 6.45-6.60 (m, 2H), 7.00-7.30 (m, 7H), 8.20 (bs, 1H)

Alternatively, the compound of Example 1 can be prepared by the following procedure:

### d) 1-(4-Indolyl)-4-(quinoline-2-ylmethyl)-piperazine (Compound 1C)

A mixture of 2-chloromethylquinoline (2.56 g), DIPEA (4.16 ml) and 1-(4-indolyl)-piperazine (2.65 g) in DMF (4 ml) was heated at 120-130°C for 3-4 hours. After cooling to room temperature, the mixture was diluted with water (50-60 ml); the liquids were decanted and extracted with diethyl ether (3 x 40 ml), washed with water, dried on sodium sulphate and filtered; the solid residue was dissolved in dichloromethane, washed with water, dried on sodium sulphate and filtered.

The combined organic layers were evaporated to dryness at reduced pressure. The crude was purified by flash chromatography eluting with ethyl acetate-petroleum ether 75:25 to give 2.87 g (70%) of the desired compound as a solid.
¹H-NMR (δ): 2.65-2.96 (m, 4H), 3.28-3.42 (m, 4H), 3.99 (s, 2H), 6.51-6.66 (m, 2H), 6.98-7.18 (m, 3H), 7.48-7.61 (m, 1H), 7.61-7.89 (m, 3H), 8.078.28 (m, 3H)

### e) 1-(4-Indolyl)-4-(1,2,3,4-tetrahydroquinoline-2-ylmethyl)-piperazine (Compound 1B)

A mixture of Compound 1C (2.87 g), PtO₂ (101 mg) and acetic acid (50 ml) was hydrogenated at a hydrogen pressure of 15 psi (103455 pascals) in a Parr apparatus at room temperature. The catalyst was separated by filtration and washed with methanol. The solvent was evaporated at reduced pressure, the residue was taken up with dichloromethane (20 ml) and alkalinised with 1N sodium hydroxide (pH > 8). The aqueous extract was extracted twice with dichloromethane and the combined organic layers were dried on sodium sulphate, filtered and evaporated *in vacuo.* The crude was purified by flash chromatography eluting with dichloromethane - methanol 97:3 to give 2.22 g (76.5%) of the title compound.

### f) 1-(1-Cyclohexanecarbonyl-1,2,3,4-tetrahydroquinoline-2-ylmethyl)-4-(4-indolyl)-piperazine

Cyclohexanecarbonyl chloride (1.03 ml) was added to a solution of Compound 1B (2.22 g), TEA (1.33 ml) in dichloromethane (30 ml) cooled to 0°C, under nitrogen atmosphere. After 2-3 hours of stirring at room temperature, the resulting mixture was washed with 1N sodium hydroxide (pH > 8) and the organic phase was dried on sodium sulphate, filtered and evaporated *in vacuo.*

The crude was purified by flash chromatography eluting with toluene-acetone 8:2 affording 2.41 g (81 %) of the title compound as a pale yellow powder. Crystallisation from MeCN afforded the title compound as a white solid. M.p. 180-181°C.

Alternatively the crude compound was purified directly by crystallisation from MeCN affording the pure title compound.

Alternatively Compound 1B can be prepared by the synthetic procedure described below:

### g) 1-(4-Indolyl)-4-(1,2,3,4-tetrahydroquinoline-2-ylcarbonyl)-piperazine (Compound 1D)

TEA (0.48 ml) and diethyl cyanophosphonate (0.53 ml) were added to a stirred solution of 1,2,3,4-tetrahydroquinoline-2-carboxylic acid (0.594 g) and 1-(4-indolyl)-piperazine (0.675 g) in DMF (24 ml) at 0°C. The reaction mixture was stirred at room temperature for 3 hours. It was diluted with H₂O (300 ml) and the white precipitate which formed was filtered and dried on sodium sulphate by dissolving in CH₂Cl₂, and the solution was evaporated to dryness *in vacuo.* The residue was purified by flash chromatography eluting with petroleum ether - EtOAc 1:1 to give 0.7 g (58%) of the title compound.
¹H-NMR (δ): 1.60-1.90 (m, 1H), 2.10-2.25 (m, 1H), 2.70-3.00 (m, 2H), 3.20-3.35 (m, 4H), 3.65-4.10 (m, 4H), 4.40 (dd, 1H), 4.50 (s, 1H), 6.51-6.75 (m, 4H), 6.95-7.30 (m, 5H), 8.25 (s,1H)

### h) 1-(4-Indolyl)-4-(1,2,3,4-tetrahydroquinoline-2-ylmethyl)-piperazine (Compound 1B)

10M Borane-dimethylsulphide (0.21 ml) was added to a stirred solution of Compound 1D (0.25 g) in THF (5 ml) at 0°C. The solution was refluxed for 1 hour, cooled at room temperature and diluted with a saturated solution of ammonium chloride. The resulting solution was extracted with CH₂Cl₂ (20 ml), dried on sodium sulphate and evaporated *in vacuo.* The residue was purified by flash chromatography eluting with petroleum ether - EtOAc 1:1 to give 0.12 g (49%) of the title compound.

### (+)-1-(1-Cyclohexanecarbonyl-1,2,3,4-tetrahydroquinoline-2-ylmethyl)-4-(4-indolyl)-piperazine and (-)-1-(1-Cyclohexanecarbonyl-1,2,3,4-tetrahydroquinoline-2-ylmethyl)-4-(4-indolyl)-piperazine

The racemic compound of Example 1 was subjected to chiral-column chromatography (Chiralpack AD 0.46 x 25 cm; eluent 0.1 % diethylamine in ethanol; flow 65 ml/min; λ = 235 nM affording the two enantiomers:
Ex. (+)-1 α_{D}: + 142° (c = 0.5, CHCl₃)
Ex. (-)-1 α_{D}: - 147° (c = 0.57, CHCl₃)

The compound of Example (+)-1 was converted into the corresponding monomethanesulphonate salt by conventional methods and, after crystallisation from i-propanol and mixtures of i-propanol-water 4:1, 1:1 and 1:4, showed a DSC melting peak of 143°C.

Similarly, after crystallisation from mixtures of ethanol-water 4:1, 1:1 and 1:4, it showed a DSC melting peak of 151°C.

### Example 2

### 1-(1-Cyclohexanecarbonyl-1,2,3,4-tetrahydroquinoline-2-ylmethyl)-4-(2-methoxyphenyl)-piperazine

### a) 2-[4-(2-Methoxyphenyl)-1-piperazinylmethyl]-quinoline (Compound 2A)

The title compound was prepared as described for Compound 1C substituting 1-(4-indolyl)-piperazine with 1-(2-methoxyphenyl)-piperazine. Purification was carried out by flash chromatography eluting with petroleum ether-ethyl acetate 4:6 to give the title compound (63.6%).
¹H-NMR (δ): 2.69-2.86 (m, 4H), 2.98-3.27 (m, 4H), 3.85 (s, 2H), 3.97 (s, 3H), 6.797.04 (m, 4H), 7.49-7.58 (m, 1H), 7.63-7.79 (m, 3H), 8.03-8.21 (m, 2H)

### b) 1-(1,2,3,4-Tetrahydroquinoline-2-ylmethyl)-4-(2-methoxyphenyl)-piperazine (Compound 2B)

Compound 2B was obtained in the same way as described for Compound 1B (step e) but starting from Compound 2A instead of Compound 1C. The crude was purified by flash chromatography eluting with dichloromethane-methanol 97:3 to give the title compound (57%).
¹H-NMR (δ): 1.48-1.67 (m, 1H), 1.80-1.99 (m, 1H), 2.47 (m, 2H), 2.51-2.67 (m, 4H), 2.72 2.98 (m, 4H), 3.02-3.27 (m, 4H), 3.37-3.59 (m, 1H), 3.90 (m, 3H), 4.56-4.77 (b, 1H), 6.48 6.67 (m, 2H), 6.83-7.08 (m, 6H)

### c) 1-(1-Cyclohexanecarbonyl-1,2,3,4-tetrahydroquinoline-2-ylmethyl)-4-(2-methoxyphenyl)-piperazine

The title compound was synthesised by the alternative acylation procedure (step f) described for the compound of Example 1 starting from Compound 2B instead of Compound 1B. The crude was purified twice by flash chromatography eluting with dichloromethane-methanol 95:5 and then with toluene-acetone 85:15 affording the title compound (69 %). M.p. 45.5°C (dec.).
¹H-NMR (δ): 0.86-2.00 (m, 11H), 2.02-2.21 (m, 1H), 2.25-2.81 (m, 9H), 2.92-3.16 (m, 4H), 3.81 (s, 3H), 4.99-5.21 (m, 1H), 6.81-8.04 (m, 4H), 7.087.29 (m, 4H)

### Example 3

### 1-[2-(1-Cyclohexanecarbonyl-1,2,3,4-tetrahydroquinoline-2-yl)-ethyl]-4-(2-methohyphenyl)-piperazine

### a) 1-[2-(1,2,3,4-Tetrahydroquinoline-2-yl)-ethyl]-4-(2-methoxyphenyl)-piperazine (Compound 3A)

Compound 3A was obtained in the manner described for Compound 1B (step e) but starting from 1-[2-(2-quinolineyl)-ethyl]-4-(2-methoxyphenyl)-piperazine (US 3,983,121) instead of Compound 1C. The crude was purified by flash chromatography eluting with dichloromethane-methanol 93:7 to give 0.115 g (43 %) of the title compound.
¹H-NMR (δ): 1.54-2.03 (m, 4H), 2.51-2.72 (m, 4H), 2.73-2.97 (m, 4H), 3.01-3.40 (m, 4H), 3.31-3.49 (m, 1H), 3.89 (s, 3H), 6.47 (d, 1H), 6.51-6.62 (m, 1H), 6.81-7.09 (m, 6H)

### b) 1-[2-(1-Cyclohexanecarbonyl-1,2,3,4-tetrahydroquinoline-2-yl)-ethyl]-4-(2-methoxyphenyl)-piperazine

The title compound was synthesised following the alternative acylation procedure (step f) described for the compound of Example 1 starting from Compound 3A instead of Compound 1B. The crude was purified twice by flash chromatography eluting first with dichloromethane-methanol 94:6, then with ethyl acetate-2N methanolic ammonia 98:2, affording 0.13 g of the title compound as a solid containing cyclohexanecarboxylic acid as the main impurity. This solid was dissolved in dichloromethane (5 ml) and washed with 1N sodium hydroxide (2 x 3 ml). The organic part was dried on sodium sulphate, filtered and evaporated at reduced pressure to give 0.091 g (62%) of the designated compound.
¹H-NMR (δ): 0.89-2.00 (m, 13H), 2.23-2.81 (m, 10H), 2.94-3.15 (m, 4H), 3.87 (s, 3H), 4.78-5.04 (m, 1H), 6.76-7.28 (m, 8H)

### Example 4

### 1-(1-Cyclohexanecarbonyl-1,2,3,4-tetrahydroquinoline-2-ylmethyl)-4-[2-(2,2,2-trifluoroethoxy)-phenyl]-piperazine

### a) 2-[4-[2-(2,2,2-trifluoroethoxy)-phenyl]-1-piperazinylmethyl]quinoline (Compound 4A

Compound 4A was obtained in the manner described for Compound 1C (procedure d) but using 1-[2-(2,2,2-trifluoroethoxyphenyl)]-piperazine instead of 1-(4-indolyl)-piperazine. The crude was purified by flash chromatography eluting with petroleum ether-ethyl acetate 55:45 to give the title compound (88%).
¹H-NMR (δ):2.60-2.83 (m, 4H), 3.06-3.27 (m, 4H), 3.91 (s, 2H), 4.40 (q, 4H), 6.88-7.09 (m, 4H), 7.46-7.62 (m, 1H), 7.66-7.85 (m, 3H), 8.03-8.21 (m, 2H)

### b) 1-(1,2,3,4-Tetrahydroquinoline-2-ylmethyl)-4-[2-(2,2,2-trifluoroethoxy)-phenyl]-piperazine (Compound 4B)

The title compound was prepared as described for Compound 1B (procedure e) starting from Compound 4A instead of Compound 1C. The crude was purified by flash chromatography eluting with dichloromethane-methanol 97:3 to give the title compound (61%).
¹H-NMR (δ): 1.45-1.71 (m, 1H), 1.82 2.00 (m, 1H), 2.40-2.64 (m, 4H), 2.69-2.91 (m, 4H), 3.04-3.26 (m, 4H), 3.37-3.57 (m, 1H), 4.41 (q, 2H), 4.56-4.76 (br, 1H), 6.48-6.67 (m, 2H), 6.85-7.09 (m, 6H)

### c) 1-(1-Cyclohexanecarbonyl-1,2,3,4-tetrahydroquinoline-2-ylmethyl)-4-(2-(2,2,2-trifluoroethoxy)-phenyl]-piperazine

The title compound was synthesised following the alternative acylation procedure described for the compound of Example 1 (step f) but starting from Compound 4B instead of Compound 1B. The crude was purified by flash chromatography eluting with petroleum ether-ethyl acetate 7:3 to give the title compound (89%). M.p. 46°C (dec.).
¹H-NMR (δ): 0.81-1.99 (m, 12H), 2.01-2.19 (m, 1H), 2.24-2.78 (m, 8H), 2.92-3.13 (m, 4H), 4.39 (q, 2H), 4.99-5.20 (m, 1H), 6.84-7.28 (m, 8H)

### Example 5

### 1-(1-Cyclohexanecarbonyl-6-fluoro-1,2,3,4-tetrahydroquinoline-2-ylmethyl)-4-[2-(2,2,2-trifluoroethoxy)-phenyl]-piperazine

### a) 2-Bromomethyl-6-fluoroquinoline (Compound 5A)

A mixture of 6-fluoro-2-methylquinoline (3 g), NBS (1.65 g), AIBN (25 mg) and CCl₄ (10 ml) was refluxed for 5 hours, then additional NBS (0.825 g) was added and the mixture was refluxed for 5 hours. After cooling at room temperature, the solid was separated by filtration and the filtrate evaporated at reduced pressure. The crude was purified by flash chromatography eluting with petroleum ether-ethyl acetate 85:15 to give, as a first eluted product, 0.71 g (17%) of the title compound and 2.13 g of starting material.
¹H-NMR (δ): 4.71 (s, 2H), 7.36-7.65 (m, 3H), 8.00-8.19 (m, 2H)

### b) 6-Fluoro-2-{4-[2-(2,2,2-trifluoroethoxy)-phenyl]-1-piperazinylmethyl}-quinoline (Compound 5B)

Compound 5B was obtained in the manner described for Compound 1C (procedure d) but using 1-[2-(2,2,2-trifluoroethoxy)-phenyl]-piperazine instead of 1-(4-indolyl)-piperazine and Compound 5A instead of 2-chloromethylquinoline. The crude was purified by flash chromatography eluting with petroleum ether-ethyl acetate 6:4 to give the title compound (80%).
¹H-NMR (δ): 2.67-2.86 (m, 4H), 3.05-3.26 (m, 4H), 3.91 (s, 2H), 4.41 (q, 4H), 6.88-7.09 (m, 4H), 7.37-7.56 (m, 2H), 7.71 (d, 3H), 7.99-8.18 (m, 2H)

### c) 1-(6-Fluoro-1,2,3,4-tetrahydroquinoline-2-ylmethyl)-4-[2-(2,2,2-trifluoroethoxy)-phenyl]-piperazine (Compound 5C)

The title compound was prepared as described for Compound 1B (procedure e) starting from Compound 5B instead of Compound 1C. The crude was purified by flash chromatography eluting with petroleum ether-ethyl acetate 8:2 to give the title compound (57%).
¹H-NMR (δ): 1.42-1.80 (m, 1H), 1.80-1.99 (m, 1H), 2.39-2.61 (m, 4H), 2.63-2.94 (m, 4H), 3.02-3.26 (m, 4H), 3.31-3.52 (m, 1H), 4.41 (q, 2H), 4.41-4.68 (br, 1H), 6.37-6.54 (m, 1H), 6.61-6.79 (m, 2H), 6.88-7.11 (m, 6H)

### d) 1-(1-Cyclohexanecarbonyl-6-fluoro-1,2,3,4-tetrahydroquinoline-2-ylmethyl)-4-[2-(2,2,2-trifluoroethoxy)-phenyl]-piperazine

The title compound was synthesised following the alternative acylation procedure described for the compound of Example 1 (step f) but starting from Compound 5C instead of Compound 1B. The crude was purified by flash chromatography eluting with petroleum ether-ethyl acetate 7:3 affording the title compound (92%). M.p. 51 °C
¹H-NMR (δ): 0.80-1.99 (m, 11H), 2.00-2.17 (m, 1H), 2.21-2.77 (m, 9H), 2.91-3.12 (m, 4H), 4.40 (q, 2H), 4.98-5.22 (m, 1H), 6.83-7.16 (m, 8H)

### Example 6

### 1-[1-(2-Ethylbutanoyl)-1,2,3,4-tetrahydroquinoline-2-ylmethyl]-4-[2-(2,2,2-trifluoroethoxy)-phenyl]-piperazine

The title compound was synthesised following the alternative acylation procedure described for the compound of Example 1 (step f) but starting from Compound 4B instead of Compound 1B and using 2-ethylbutanoyl chloride instead of cyclohexanecarbonyl chloride. The crude was purified by flash chromatography eluting with petroleum ether-ethyl acetate 75:25 affording the title compound (93%).
¹H-NMR (δ): 0.61 and 1.05 (2t, 6H), 1.21-1.67 (m, 5H), 1.74-1.93 (m, 1H), 2.00-2.19 (m, 1H), 2.23-2.84 (m, 8H), 2.92-3.24 (m, 4H), 4.41 (m, 2H), 5.08-5.29 (m, 1H), 6.87-7.29 (m, 8H)

### Example 7

### 1-[1-(3-Methoxypropionyl)-1,2,3,4-tetrahydroquinoline-2-ylmethyl]-4-[2-(2,2,2-trifluoroethoxy)-phenyl]-piperazine

The title compound was synthesised following the alternative acylation procedure described for the compound of Example 1 (step f) but starting from Compound 4B instead of Compound 1B and using 3-methoxypropionyl chloride instead of cyclohexanecarbonyl chloride. The crude was purified by flash chromatography eluting with petroleum ether-ethyl acetate 4:6 affording the title compound (56.5%).
¹H-NMR (δ): 1.41-1.55 (m, 1H), 2.04-2.21 (m, 1H), 2.31-2.95 (m, 10H), 2.97-3.16 (m, 4H), 3.32 (s, 3H), 3.71 (t, 2H), 4.41 (q, 2H), 5.06-5.21 (m, 1H), 6.88-7.08 (m, 4H), 7.00-7.18 (m, 4H)

### Example 8

### 1-[1-(3-Benzyloxypropionyl)-1,2,3,4-tetrahydroquinoline-2-ylmethyl]-4-[2-(2,2,2-trifluoroethoxy)-phenyl]-piperazine

The title compound was synthesised following the alternative acylation procedure described for the compound of Example 1 (step f) but starting from Compound 4B instead of Compound 1B and using 3-benzyloxypropionyl chloride instead of cyclohexanecarbonyl chloride. The crude was purified by flash chromatography eluting with toluene-acetone 7.5:21.5 affording the title compound (48%).
¹H-NMR (δ): 1.41-1.60 (m, 1H), 2.05-2.21 (m, 1H), 2.25-2.75 (m, 9H), 2.80-3.16 (m, 5H), 3.70-3.90 (m, 2H), 4.41 (q, 2H), 4.50 (s, 2H), 5.00-5.21 (m, 1H), 6.82-7.08 (m, 4H), 7.10 7.40 (m, 4H)

### Example 9

### 1-[1-(3-Benzyloxypropionyl)-1,2,3,4-tetrahydroquinoline-2-ylmethyl]-4-(4-indolyl)-piperazine

The title compound was synthesised following the alternative acylation procedure described for the compound of Example 1 (step f) using 3-benzyloxypropionyl chloride instead of cyclohexanecarbonyl chloride. The crude was purified by flash chromatography eluting with toluene-acetone 7.5:2.5 to give the title compound (58%).
¹H-NMR (δ): 1.41-1.65 (m, 1H), 2.10-2.30 (m, 1H), 2.32-3.00 (m, 10H), 3.10-3.30 (m, 4H), 3.72-3.92 (m, 2H), 4.50 (s, 2H), 5.05-5.25 (m, 1H), 6.45-6.65 (m, 2H), 7.007.40 (m, 12H), 8.15 (s, 1H)

### Example 10

### 1-[1-(3-Hydroxypropionyl)-1,2,3,4-tetrahydroquinoline-2-ylmethyl]-4-[2-(2,2,2-trifluoroethoxy)-phenyl]-piperazine

10% Pd-C (0.05 mg) and ammonium formate (0.16 g) was added to a solution of the compound of Example 8 (0.202 g) in MeOH (8 ml). The reaction mixture was refluxed for 6 hours under nitrogen atmosphere. Additional 0.2 g of ammonium formate and 0.1 g of 10% Pd-C was added. The mixture was refluxed for 4 more hours. The catalyst was filtered and evaporation *in vacuo* was carried out. The crude was purified by flash chromatography eluting with CHCl₃-MeOH 97:3 to obtain the title compound (48%).
¹H-NMR (δ): 1.30-2.00 (m, 2H), 2.04-2.21 (m, 1H), 2.25-2.80 (m, 9H), 2.80-3.00 (m, 1H), 3.00-3.15 (m, 4H), 3.75-4.00 (m, 2H), 4.40 (q, 2H), 5.05-5.31 (m, 1H), 6.82-7.08 (m, 4H), 7.10-7.35 (m, 4H)

### Example 11

### 1-[1-(3-Methoxypropionyl)-1,2,3,4-tetrahydroquinoline-2-ylmethyl]-4-(4-indolyl)-piperazine

The title compound was synthesised following the alternative acylation procedure described for the compound of Example 1 (step f) using 3-methoxypropionyl chloride instead of cyclohexanecarbonyl chloride. The crude was purified by flash chromatography eluting with petroleum ether-ethyl acetate 3:7 to give the title compound (44%). M.p. 62.8-66°C.
¹H-NMR (δ): 1.41-1.65 (m, 1H), 2.10 -2.25 (m, 1H), 2.30-2.95 (m, 10H), 3.12-3.25 (m, 4H), 3.32 (s, 1H), 3.70 (t, 2H), 5.00-5.25 (m, 1H), 6.45-6.60 (m, 2H), 7.007.30 (m, 12H), 8.15 (s, 1H)

### Example 12

### 1-[1-(3-Isopropoxypropionyl)-1,2,3,4-tetrahydroquinoline-2-ylmethyl]-4-(4-indolyl)-piperazine

The title compound was synthesised following the alternative acylation procedure described for the compound of Example 1 (step f) using 3-isopropoxypropionyl chloride instead of cyclohexanecarbonyl chloride. The crude was purified by flash chromatography eluting with petroleum ether-ethyl acetate 1:1 to give the title compound (20%).
¹H-NMR (δ) : 1.12 (d, 6H), 1.41-1.65 (m, 1H), 2.10-2.25 (m, 1H), 2.30-3.00 (m, 10H), 3.12-3.25 (m, 4H), 3.55 (septet, 1H), 3.65-3.90 (m, 1H), 5.00-5.25 (m, 1H), 6.456.60 (m, 2H), 7.00-7.35 (m, 12H), 8.15 (s, 1H)

### Example 13

### 1-[1-Acetyl-1,2,3,4-tetrahydroquinoline-2-ylmethyl]-4-(4-indolyl)-piperazine

The title compound was synthesised following the alternative acylation procedure described for the compound of Example 1 (step f) using acetyl chloride instead of cyclohexanecarbonyl chloride and CHCl₃ instead of dichloromethane. The crude was purified by flash chromatography eluting with petroleum ether-ethyl acetate-2.5N methanolic ammonia 3:7:0.1 to give the title compound (53%). M.p. 187-189°C.
¹H-NMR (δ): 1.50-1.70 (m, 1H), 2.05-2.29 (m, 4H), 2.31-2.85 (m, 8H), 3.10-3.32 (m, 4H), 4.98-5.21 (m, 1H), 6.48-6.65 (m, 2H), 7.00-7.30 (m, 7H), 8.15, s, 1H

### Example 14

### 1-[1-(4-Morpholinocarbonyl)-1,2,3,4-tetrahydroquinoline-2-ylmethyl]-4-(4-indolyl)-piperazine

The title compound was synthesised following the alternative acylation procedure described for the compound of Example 1 (step f) using 4-morpholinocarbonyl chloride instead of cyclohexanecarbonyl chloride and toluene instead of dichloromethane, and refluxing for 2.5 hours. The crude was purified by flash chromatography eluting with petroleum ether-ethyl acetate-2.5N methanolic ammonia 8:2:0.01 to give the title compound (26%). M.p. 195-205°C.
¹H-NMR (δ): 1.90-2.21 (m, 2H), 2.35 (d, 1H), 2.55-2.90 (m, 7H), 3.12-3.42 (m, 8H), 3.48 3.75 (m, 4H), 4.38-4.50 (m, 1H), 6.48-6.60 (m, 2H), 6.85-7.20 (m, 7H), 8.15 (s, 1H)

### Example 15

### 1-(1-Cyclohexanecarbonyl-1,2,3,4-tetrahydroquinoline-2-ylmethyl)-4-(1-methyl-4-indolyl)-piperazine

The compound of example 1 (0.456 g) was added to a suspension of 50% NaH (0.130 g) in DMF (20 ml), and stirred for 20 minutes at room temperature and then 1 hour at 55°C. Methyl iodide (0.185 ml) was added dropwise, stirred for 4 hours at room temperature, and poured into H₂O. The precipitate was filtered, dissolved in Et₂O, washed with H₂O, dried on sodium sulphate and evaporated *in vacuo.* The crude was purified by flash chromatography eluting with CH₂Cl₂-MeOH 9.7:0.3 to give 0.077 g of the title compound (16%). M.p. 66-70°C.
¹H-NMR (δ): 0.80-2.05 (m, 11H), 2.06-2.25 (m, 1H), 2.25-2.85 (m, 8H), 3.10-3.35 (m, 4H), 5.00-5.25 (m, 1H), 6.45-6.60 (m, 2H), 7.00-7.30 (m, 7H), 8.20 (b, 1H).

### Example 16

### 1-(1-Cyclohexanecarbonyl-6-methoxy-1,2,3,4-tetrahydroquinoline-2-ylmethyl)-4-(4-indolyl)-piperazine

### a) 2-Bromomethyl-6-methoxyquinoline (Compound 16A)

Compound 16A was obtained in the manner described for Compound 5A (procedure a) but using 6-methoxy-2-methylquinoline instead of 6-fluoro-2-methylquinoline. The crude was purified by flash chromatography eluting with petroleum ether-ethyl acetate 85:15 to give, as a first eluted product, 0.45 g (30%) of the title compound, followed by 0.39 g of starting material.
¹H-NMR (δ): 3.93 (s, 3H), 4.70 (s, 2H), 7.09 (d 1H), 7.37 (dd, 1H), 7.37 (d, 1H), 7.96 (d, 1H), 8.09 (d, 1H).

### b) 6-Methoxy-2-[4-(4-indolyl)-1-piperazinylmethyl]-quinoline (Compound 16B)

Compound 16B was obtained in the manner described for Compound 1C (procedure d) but using Compound 16A instead of 2-chloromethylquinoline. The crude was purified by flash chromatography eluting with dichloromethane-methanol 95:5 to give the title compound (80%).
¹H-NMR (δ): 2.73-2.91 (m, 4H), 3.21-3.42 (m, 4H), 3.86-4.02 (m, 5H), 6.49-6.67 (m, 2H), 7.00-7.21 (m, 4H), 7.36 (d, 1H), 7.65 (d, 1H), 7.91-8.10 (m, 2H), 8.12-8.33 (br, 1H).

### c) 1-(6-Methoxy-1,2,3,4-tetrahydroquinoline-2-ylmethyl)-4-(4-indolyl)-piperazine (Compound 16C)

The title compound was prepared as described for Compound 1B (procedure e) starting from Compound 16B instead of Compound 1C. The crude was purified by flash chromatography eluting with dichloromethane-methanol 97:3 to give the title compound (48%).
¹H-NMR (δ): 1.47-1.72 (m, 1H), 1.81-2.01 (m, 1H), 2.36-2.53 (m, 2H), 2.54-2.74 (m, 2H), 2.75-2.98 (m, 4H), 3.18-3.57 (m, 5H), 3.73 (s, 3H), 4.71-4.83 (sa, 1H), 6.47-6.63 (m, 5H), 7.02-7.18 (m, 3H), 8.11-8.26 (br, 1H)

### d) 1-(1-Cyclohexanecarbonyl-6-methoxy-1,2,3,4-tetrahydroquinoline-2-ylmethyl]-4-(4-indolyl)-piperazine

The title compound was synthesised following the alternative acylation procedure described for the compound of Example 1 (step f) but starting from Compound 16C instead of Compound 1B. The crude was purified by flash chromatography eluting with dichloromethane-methanol 97:3 to give the title compound (89%). M.p. 90-93°C.
¹H-NMR (δ): 0.75-2.00 (m, 11H), 2.01-2.21 (m, 1H), 2.22-2.84 (m, 9H), 3.11-3.34 (m, 4H), 3.76 (s, 3H), 5.01-5.23 (m, 1H), 6.46-6.64 (m, 2H), 6.70-6.83 (m, 2H), 6.95-7.19 (m, 4H), 8.07-8.22 (b, 1H).

### Example 17

### 1-[1-(3-Hydroxypropionyl)-1,2,3,4-tetrahydroquinoline-2-ylmethyl]-4-(4-indolyl)-piperazine

The title compound was prepared following the procedure described for the compound of Example 10, but using the compound of Example 9 instead of the compound of Example 8. The crude was purified by flash chromatography eluting with CHCl₃-MeOH 97:3 to give the title compound (63%). M.p. 58-61°C.
¹H-NMR (δ): 1.30-2.30 (br, 1H), 1.41-1.60 (m, 1H), 2.10-2.27 (m, 1H), 2.30-2.99 (m, 10H), 3.11-3.31 (m, 4H), 3.72-3.93 (m, 2H), 5.05-5.28 (m, 1H), 6.506.67 (m, 2H), 7.01-7.28 (m, 7H), 8.01-8.25 (br, 1H)

### Example 18

### 1-(1-Cyclohexanecarbonyl-6-fluoro-1,2,3,4-tetrahydroquinoline-2-ylmethyl)-4-(4-indolyl)-piperazine

### a) 6-Fluoro-2-[4-(4-indolyl)-1-piperazinylmethyl]quinoline (Compound 18A)

Compound 18A was obtained in the same way as described for Compound 1C (procedure d) but using Compound 5A instead of 2-chloromethylquinoline. The crude was purified by flash chromatography eluting with petroleum ether-ethyl acetate 1:1 to give the title compound (63%).
¹H-NMR (δ): 2.65-2.91 (m, 4H), 3.12-3.41 (m, 4H), 3.93 (s, 2H), 6.49-6.62 (m, 2H), 7.00-7.21 (m, 3H), 7.38-7.52 (m, 2H), 7.65-7.80 (m, 1H), 7.93-8.12 (m, 2H), 8.15-8.30 (br, 1H)

### b) 1-(6-Fluoro-1,2,3,4-tetrahydroquinoline-2-ylmethyl)-4-(4-indolyl)-piperazine (Compound 18B)

The title compound was prepared as described for Compound 1B (procedure e) starting from Compound 18A instead of Compound 1C. The crude was purified by flash chromatography eluting with petroleum ether-ethyl acetate 6:4 to give the title compound (59%).
¹H-NMR (δ): 1.45-1.68 (m, 1H), 1.80-1.93 (m, 1H), 2.43 (d, 2H), 2.51-2.69 (m, 2H), 2.81-3.01 (m, 4H), 3.12-3.33 (m, 4H), 3.34-3.51 (m, 1H), 4.20-5.00 (br, 1H), 6.40-6.73 (m, 5H), 7.00-7.19 (m, 3H), 8.07-8.27 (br, 1H)

### c) 1-(1-Cyclohexanecarbonyl-6-fluoro-1,2,3,4-tetrahydroquinoline-2-ylmethyl)-4-(4-indolyl)-piperazine

The title compound was synthesised following the alternative acylation procedure described for the compound of Example 1 (step f) but starting from Compound 18B instead of Compound 1B. The crude was purified by flash chromatography eluting with petroleum ether-ethyl acetate 6:4 to give the title compound (49%). M.p. 82-84°C.
¹H-NMR (δ): 0.75-1.93 (m, 11H), 1.95-2.20 (m, 1H), 2.28-2.83 (m, 9H), 3.073.30 (m, 4H), 5.02-5.23 (m, 1H), 6.42-6.70 (m, 2H), 6.81-7.28 (m, 6H), 8.04-8.22 (br, 1H)

### Example 19

### 1-(1-Dimethylaminocarbonyl-1,2,3,4-tetrahydroquinoline-2-ylmethyl)-4-(4-indolyl)-piperazine

The title compound was synthesised following the alternative acylation procedure described for the compound of Example 1 (step f) using N,N-dimethylaminocarbonyl chloride instead of cyclohexanecarbonyl chloride and toluene instead of dichloromethane, and refluxing for 2 hours. The crude was purified by flash chromatography eluting with petroleum ether-ethyl acetate-2.5 N methanolic ammonia 8:2:0.01 to give the title compound (39 %). M.p. 190-227°C.
¹H-NMR (δ): 1.88-2.10 (m, 1H), 2.11-2.27 (m, 1H), 2.28-2.50 (m, 1H), 2.60-2.95 (m, 13H), 3.10-3.48 (m, 4H), 4.32-4.50 (s, 1H), 6.50-6.65 (m, 2H), 6.75-6.98 (m, 2H), 7.007.22 (m, 5H), 8.20 (bs, 1H).

### Example 20

### 1-(1-Ethylaminocarbonyl-1,2,3,4-tetrahydroquinoline-2-ylmethyl)-4-(4-indolyl)-piperazine

A solution of Compound 1B (0.35 g) and ethyl isocyanate (0.16 ml) in DMF (2 ml) was stirred at 100°C for 2 h. Afterwards, the reaction mixture was poured into H₂O and extracted with EtOAc (2x30 ml). The combined extracts were washed with H₂O, dried (Na₂SO₄) and evaporated to dryness in vacuo. The crude was purified by flash chromatography eluting with petroleum ether-ethyl acetate-2.5 N methanolic ammonia 8:2:0.01 to give 0.22 g of the title compound (49.8 %). M.p. 109-112°C.
¹H-NMR (δ): 1.18 (t, 3H), 1.58-1.78 (m, 1H), 2.11-2.34 (m, 1H), 2.36 (dd, 1H), 2.58-2.98 (m, 7H), 3.10-3.42 (m, 4H), 4.45-4.65 (s, 1H), 6.47-6.65 (m, 2H), 6.95-7.25 (m, 7H), 7.39 (d, 1H), 8.25 (bs, 1H)

### Example 21

### 1-(1-Cyclohexanecarbonyl-1,2,3,4-tetrathydroquinoline-2-ylmethyl)-4-(1-isoquinolinyl)-piperazine

### a) 1-(1-Isoquinolinyl)-4-(1,2,3,4-tetrahydroquinoline-2-ylcarbonyl)-piperazine (Compound 21A)

The title compound was synthesised following the procedure described for Compound 1D (step g) but using 1-(1-isoquinolinyl)-piperazine (WO 00/40554) instead of 1-(4-indolyl)-piperazine. The residue was purified by flash chromatography eluting with petroleum ether - EtOAc 1:1 to give of the title compound (75.6 %)
¹H-NMR (δ): 1.61-1.89 (m, 1H), 2.22-2.30 (m, 1H), 2.78-2.96 (m, 2H), 3.35-3.54 (m, 4H), 3.71-4.10 (m, 4H), 4.29 (dd, 1H), 4.41-4.68 (br, 1H), 6.59-6.76 (m, 2H), 6.95-7.13 (m, 2H), 7.32 (d, 1H), 7.50-7.71 (m, 2H), 7.80 (d, 1H), 8.04-8.23 (m, 2H).

### b) 1-(1-Isoquinolinyl)-4-1,2,3,4-tetrahydroquinoline-2-ylmethyl)-piperazine (Compound 21B)

The title compound was synthesised following the procedure described for Compound 1B (step h), but using Compound 21A instead of Compound ID. The residue was purified by flash chromatography eluting with petroleum ether- EtOAc 5.5:4.5 to give 0.12 g (73.6 %) of the title compound.
¹H-NMR (δ): 1.41-1.80 (m, 1H), 1.81-1.99 (m, 1H), 2.50 (d, 2H), 2.58-2.76 (m, 2H), 2.78-3.01 (m, 4H), 3.39-3.58 (m, 4H), 4.59-4.90 (br, 1H), 6.48-6.67 (m, 2H), 6.87-7.08 (m, 2H), 7.16-7.3 (m, 1H), 7.47-7.66 (m, 2H), 7.78 (d, 1H), 8.04-8.22 (m, 2H).

### c) 1-(1-Cyclohexanecarbonyl-1,2,3,4-tetrahydroquinoline-2-ylmethyl)-4-(1-isoquinolinyl)-piperazine

The title compound was synthesised following the alternative acylation procedure described for the compound of Example 1 (step f) but starting from Compound 21B instead of Compound 1B. The crude was purified by flash chromatography eluting with petroleum ether-ethyl acetate 6:4 to give the title compound (88 %). M.p. (63.9) 70.4-72°C.
¹H-NMR (δ): 0.86-2.01 (m, 11H), 2.10-2.31 (m, 1H), 2.33-2.87 (m, 9H), 3.12-3.56 (m, 4H), 4.94-5.29 (m, 1H), 7.08-7.27 (m, 5H), 7.41-7.67 (m, 2H), 7.26 (d, 1H), 8.00-8.19 (m, 2H).

### Example 22

### 1-(1-Cyclohexanecarbonyl-1,2,3,4-tetrahydroquinoline-2-ylmethyl)-4-(2-methoxyphenyl)-piperidine

### a) 2-[4-(2-Methoxyphenyl)-1-piperidinylmethyl]-quinoline (Compound 22A)

The title compound was prepared as described for Compound 1C substituting 1-(4-indolyl)-piperazine with 1-(2-methoxyphenyl)-piperidine. Purification was carried out by flash chromatography eluting with petroleum ether-ethyl acetate 6:4 to give the title compound (53 %). Oil.
¹H-NMR (δ): 1.70-1.90 (m, 4H), 2.18-2.35 (m, 2H), 2.85-3.10 (m, 3H), 3.82 (s, 3H), 3.90 (s, 2H), 6.78-7.00 (m, 2H), 7.08-730 (m, 2H), 7.51 (t, 1H), 7.60-7.85 (m, 3H), 8.10 (t, 1H).

### b) 1-(1,2,3,4-Tetrahydroquinoline-2-ylmethyl)-4-(2-methoxyphenyl)-piperidine (Compound 22B)

Compound 22B was obtained in the same way as described for Compound 1B (step e) but starting from Compound 22A instead of Compound 1C. The crude was purified by flash chromatography eluting with CH₂Cl₂ - 2.5 N NH₃ in MeOH 100:1 to 100:2 affording the title compound (50.3 %). Oil.
¹H-NMR (δ): 1.40-2.20 (m, 6H), 2.20-3.20 (m, 9H), 3.30-3.60 (m, 1H), 3.90-4.50 (br, 1H), 6.45-6.65 (m, 2H), 6.80-7.05 (m, 4H), 7.05-7.30 (m, 2H).

### c) 1-(1-Cyclohexanecarbonyl-1,2,3,4-tetrahydroquinoline-2-ylmethyl)-4-(2-methoxyphenyl)-piperidine

The title compound was synthesised by the alternative acylation procedure (step f) described for the compound of Example 1 starting from Compound 22B instead of Compound 1B. The crude was purified by flash chromatography eluting with dichloromethane-methanol 95:5 and then with CH₂Cl₂ - 2.5 N NH₃ in MeOH 100:2 to 100:3 affording the title compound (72 %). Oil.
¹H-NMR (δ): 1.40-2.20 (m, 16H), 2.20-3.30 (m, 10H), 3.80 (s, 3H), 4.85-5.25 (br, 2H), 6.75-7.00 (m, 2H), 7.00-7.35 (m, 6H).

### Example 23

### 1-(7-Benzofuranyl)-4-(1-cyclohexanecarbonyl-1,2,3,4-tetrahydroquinoline-2-ylmethyl)-piperazine

### a) 1-(7-Benzofuranyl)-4-(1,2,3,4-tetrahydroquinoline-2-ylcarbonyl)-piperazine (Compound 23A)

The title compound was synthesised following the procedure described for Compound 1D (step g) but using 1-(7-benzofuranyl)-piperazine instead of 1-(4-indolyl)-piperazine. The residue was purified by flash chromatography eluting with petroleum ether-EtOAc 55:45 to give the title compound (69 %).
¹H-NMR (δ): 1.60-1.56 (m, 1H), 2.10-2.30 (m, 1H), 2.74-3.01 (m, 2H), 3.26-3.45 (m, 4H), 3.62-4.08 (m, 4H), 4.27 (d, 1H), 4.39-4.46 (br, 1H), 6.636.81 (m, 2H), 6.86-7.05 (m, 2H), 7.11-7.32 (m, 2H), 7.62 (d, 1H).

### b) 1-(7-Benzofuranyl)-4-(1,2,3,4-tetrahydroquinoline-2-ylmethyl)-piperazine (Compound 23B)

The title compound was synthesised following the procedure described for Compound 1B (step h), but using Compound 23A instead of Compound 1D. The residue was purified by flash chromatography eluting with petroleum ether- EtOAc 8:2 to give the title compound (62%).
¹H-NMR (δ): 1.49-1.71 (m, 1H), 1.79-1.98 (m, 1H), 2.50 (d, 2H), 2.57-2.76 (m, 2H), 2.77-2.98 (m, 4H), 4.40-4.99 (br, 1H), 6.48-6.66 (m, 2H), 6.72-6.85 (m, 2H), 6.91-7.08 (m, 2H), 7.09-7.29 (m, 2H), 7.62 (s, 1H).

### c) 1-(7-Benzofuranyl)-4-(1-cyclohexanecarbonyl-1,2,3,4-tetrahydroquinoline-2-ylmethyl)-piperazine

The title compound was synthesised following the alternative acylation procedure described for the compound of Example 1 (step f) but starting from Compound 23B instead of Compound 1B. The crude was purified by flash chromatography eluting with petroleum ether-ethyl acetate 6:4 to give the title compound (61.5%). M.p. 99.9-104°C.
¹H-NMR (δ): 0.83-1.99 (m, 11H), 2.02-2.21 (m, 1H), 2.29-2.85 (m, 9H), 3.19-3.89 (m, 4H), 5.03-5.23 (m, 1H), 6.64-6.85 (m, 2H), 7.06-7.27 (m, 6H), 6.72-6.85 (m, 2H), 7.59 (d, 1H).

### Example 24A

### 1-(2-Ethylbutanoyl-1,2,3,4-tetrahydroquinoline-2-ylmethyl)-4-(4-indolyl)-piperazine

The title compound was synthesised following the alternative acylation procedure described for the compound of Example 1 (step f) starting from Compound 1B but using 2-ethylbutanoyl chloride instead of cyclohexanecarbonyl chloride. Purification (flash chromatography): petroleum ether - EtOAc 6:4. Yield: 88%. M.p. 145-148°C.
¹H-NMR (δ): 0.61 (t, 3H), 1.05 (t, 3H), 1.20-1.70 (m, 4H), 1.71-1.98 (m, 1H), 2.032.25 (m, 1H), 2.30-2.90 (m, 9H), 3.10-3.32 (m, 4H), 5.18-5.35 (m, 1H), 6.48-6.63 (m, 2H), 7.00-7.32 (m, 7H), 8.12 (s,1H).

The following compounds were synthesised following the above acylation procedure starting from Compound 1B but using the appropriate acyl or aroyl chloride instead of 2-ethylbutanoyl chloride. Flash chromatography purification with the given eluting mixtures afforded the title compounds.

### Example 24A1

### 1-(1-Cyclohex-3-enylcarbonyl-1,2,3,4-tetrahydroquinoline-2-ylmethyl)-4-(4-indolyl)-piperazine

From 1-cyclohex 3-enecarbonyl chloride. Purification: petroleum ether- EtOAc- 2.5 N NH₃ in MeOH 6:4:0.15. Yield: 40%. M.p. 156-160°C.
¹H-NMR (δ): 1.32-2.83 (m, 14H), 2.85-3.10 (m, 1H), 5.00-525 (m, 1H), 5.48-5.65 (m, 1H), 5.66-5.81 (m, 1H), 6.48-6.61 (m, 2H), 7.00-7.30 (m, 7H), 8.14 (s, 1H).

### Example 24A2

### 1-(1-Cycloheptanecarbonyl-1,2,3,4-tetrahydroquinoline-2-ylmethyl)-4-(4-indolyl)-piperazine

From 1-cycloheptanecarbonyl chloride. Purification: petroleum ether - EtOAc- 2.5 N NH₃ in MeOH 7:3:0.1. Yield: 30%. M.p. 167-173°C.
¹H-NMR (δ): 1.02-2.04 (m, 13H), 2.03-2.22 (m, 1H), 2.30-2.80 (m, 8H), 2.81-2.98 (m, 1H), 3.11-3.22 (m, 1H), 4.95-5.22 (m, 1H), 6.48-6.61 (m, 2H), 7.047.30 (m, 7H), 8.14 (s, 1H).

### Example 24A3

### 1-(1-Cyclopentanecarbonyl-1,2,3,4-tetrabydroquinoline-2-ylmethyl)-4-(4-indolyl)-piperazine

From 1-cyclopentanecarbonyl chloride. Purification: petroleum ether - EtOAc- 2.5 N NH₃ in MeOH 6:4:0.2. Yield: 82 %. M.p. 150-153°C.
¹H-NMR (δ): 1.30-2.25 (m, 10H), 2.32-2.82 (m, 8H), 2.96-3.32 (m, 5H), 3.11-3.22 (m, 1H), 4.98-5.22 (m, 1H), 6.45-6.61 (m, 2H), 7.00-7.27 (m, 7H), 8.14 (s, 1H).

### Example 24A4

### 1-(1-Benzoyl-1,2,3,4-tetrahydroquinoline-2-ylmethyl)-4-(4-indolyl)-piperazine

From benzoyl chloride. Purification: petroleum ether- EtOAc- 2.5 N methanolic ammonia 6:4:0.2. Yield: 91 %. Oil
¹H-NMR (δ): 1.78-1.98 (m, 1H), 2.30 (dd, 1H), 2.34-2.55 (m, 1H), 2.66 (dd, 1H), 2.69-2.88 (m, 4H), 3.12-3.38 (m, 4H), 4.98-5.14 (m, 1H), 6.50-6.73 (m, 3H), 6.80-6.97 (m, 1H), 7.00 7.37 (m, 10H), 8.14 (s, 1H).

### Example 24A5

### 1-(4-Indolyl)-4-(1-pentanoyl-1,2,3,4-tetrahydroquinoline-2-ylmethyl)-piperazine

From pentanoyl chloride. Purification: petroleum ether-ethyl acetate-2N NH₃ in MeOH 6:4:0.1. Yield: 80 %.
¹H-NMR (δ): 1.85 (s, 3H), 1.12-1.40 (m, 2H), 1.42-1.72 (m, 3H), 2.16 (dd, 1H), 2.27-2.82 (m, 10H), 3.10-3.22 (m, 4H), 4.98-5.20 (m, 1H), 6.48-6.60 (m, 2H), 6.80-6.97 (m, 1H), 7.00-7.30 (m, 7H), 8.14 (s, 1H).

### Example 25

### 1-(1-Cyclohexanecarbonyl-6-trifluoromethyl-1,2,3,4-tetrahydroquinoline-2-ylmethyl)-4-(4-indolyl)-piperazine

### a) 2-Methyl-6-trifluoromethylquinoline (Compound 25A)

A mixture of 4-trifluoromethylaniline (3 g), chloranil (4.6 g), 37% HCl (4.77 ml) and n-BuOH (4.77 ml) was heated to reflux; then crotonaldehyde (1.89 ml) in n-BuOH (1.88 ml) was added dropwise and the reaction mixture was stirred at reflux for 40 min. After cooling to r.t., it was diluted with H₂O and extracted with Et₂O (2x30 ml). The aqueous layer was alkalinized with 37% NaOH (pH >9) cooling with an ice bath, extracted with Et₂O (3x60 ml), dried (Na₂SO₄) and evaporated to dryness in vacuo. The crude was purified by flash chromatography eluting with petroleum ether-ethyl acetate 1:1 to give 3.8 g (72 %) of the title compound.
¹H-NMR (δ): 2.73 (s, 3H), 7.39 (d, 1H), 7.86 (dd, 1H), 8.02-8.21 (m, 3H).

### b) 2-Bromomethyl-6-trifluoromethylquinoline (Compound 25B)

Compound 25B was obtained in the manner described for Compound 5A (procedure a) but using 2-methyl-6-trifluoromethylquinoline (Compound 25A) instead of 6-fluoro-2-methylquinoline. The crude was purified by flash chromatography eluting with petroleum ether - ethyl acetate 8:2 to give, as a first eluted product, the title compound (44%), followed by a partial recovery of unreacted starting material.
¹H-NMR (δ): 4.71 (s, 2H), 7.69 (d, 1H), 7.91 (dd, 1H), 8.12-8.21 (m, 3H).

### c) 6-Trifluoromethyl-2-[4-(4-indolyl)-1-piperazinylmethyl]-quinoline (Compound 25C)

Compound 25C was obtained in the manner described for Compound 1C (procedure d) but using Compound 25B instead of 2-chloromethylquinoline. The crude was purified by flash chromatography eluting with petroleum ether - ethyl acetate 55:45 to give the title compound (44%).
¹H-NMR (δ):2.78-2.92 (m, 4H), 3.18-3.37 (m, 4H), 3.96 (s, 2H), 6-49-6.68 (m, 2H), 7.03-7.22 (m, 3H), 7.26-7.97 (m, 2H), 8.08-8.27 (m, 4H).

### d) 1-(6-Trifluoromethyl-1,2,3,4-tetrahydroquinoline-2-ylmethyl)-4-(4-indolyl)-piperazine (Compound 25D)

The title compound was prepared as described for Compound 1B (procedure e) starting from Compound 25C instead of Compound 1C. The crude was purified by flash chromatography eluting with petroleum ether - ethyl acetate 7:3 to give the title compound (46%).
¹H-NMR (δ): 1.43-1.66 (m, 1H), 1.84-2.01(m, 1H), 2.43-2.71 (m, 4H), 2.80-3.01 (m, 4H), 3.20-3.41 (m, 4H), 3.45-3.54 (m, 1H), 5.10 (bs, 3H), 6.48-6.68 (m, 3H), 7.06-7.24 (m, 5H), 8.19 (s, 1H).

### e) 1-(1-Cyclohexanecarbonyl-6-trifluoromethyl-1,2,3,4-tetrahydroquinoline-2-ylmethyl)-4-(4-indolyl)-piperazine

The title compound was synthesised following the alternative acylation procedure described for the compound of Example 1 (step f) but starting from Compound 26D instead of Compound 1B. The crude was purified by flash chromatography eluting with petroleum ether - ethyl acetate 6:4 to give the title compound (59 %).
¹H-NMR (δ): 1.00-1.99 (m, 11H), 2.11-2.28 (m, 1H), 2.30-2.51 (m, 2H), 2.61-2.83 (m, 7H), 3.11-3.30 (m, 4H), 4.96-5.17 (m, 1H), 6.45-6.63 (m, 2H), 7.00-7.19 (m, 3H), 7.21-7.38 (m, 1H), 7.41-7.59 (m, 2H), 8.17 (bs, 1H).

### Example 26

### 1-(1-Cyclohexanecarbonyl-6-trifluoromethoxy-1,2,3,4-tetrahydroquinoline-2-ylmethyl)-4-(4-indolyl)-piperazine

### a) 2-Methyl-6-trifluoromethoxyquinoline (Compound 26A)

The title compound was prepared according to the procedure for compound 25A but using 4-trifluoromethoxyaniline instead of 4-trifluoromethylaniline. The crude was purified by flash chromatography eluting with CH₂Cl₂ to give compound 26A (42 %).

### b) 2-Bromomethyl-6-trifluoromethoxyquinoline (Compound 26B)

Compound 26B was obtained in the manner described for Compound 5A (procedure a) but using compound 2-methyl-6-trifluoromethoxyquinoline (Compound 26A) instead of 6-fluoro-2-methylquinoline. The crude was purified by flash chromatography eluting with petroleum ether - CH₂Cl₂ 7:3 to afford the title compound (44%) accompanied by a small amount of 2,2-dibromomethyl-6-trifluoromethoxyquinoline.
¹H-NMR (δ): 4.70 (s, 2H), 7.50-7.70 (m, 3H), 8.05-8.25 (m, 2H).

### c) 6-Trifluoromethoxy-2-[4-(4-indolyl)-1-piperazinylmethyl]-quinoline (Compound 26C)

Compound 26C was obtained in the same way as described for Compound 1C (procedure d) but using Compound 26B instead of 2-chloromethylquinoline. The crude was purified by flash chromatography eluting with petroleum ether - ethyl acetate 6:4 to 5:5 to give the title compound (44%).
¹H-NMR (δ): 2.70-2.93 (m, 4H), 3.203.45 (m, 4H), 3.95 (s, 2H), 6.50-6.65 (m, 2H), 7.00-7.20 (m, 3H), 7.45-7.70 (m, 2H), 7.75 (d, 1H), 8.05-8.30 (m, 3H).

### d) 1-(6-Trifluoromethoxy-1,2,3,4-tetrahydroquinoline-2-ylmethyl)-4-(4-indolyl)-piperazine (Compound 26 D)

The title compound was prepared as described for Compound 1B (procedure e) starting from Compound 26C instead of Compound 1C. The crude was purified by flash chromatography eluting with petroleum ether - ethyl acetate 6:4 to give the title compound (29 %).
¹H-NMR (δ): 1.40-1.70 (m, 1H), 1.80-2.00 (m, 1H), 2.40-2.52 (m, 2H), 2.52-2.72 (m, 2H), 2.72-3.00 (m, 4H), 3.15-3.38 (m, 4H), 3.38-3.55 (m, 1H), 4.78 (b, 1H), 6.40-6.65 (m, 3H), 6.75-6.80 (m, 2H), 7.05-7.25 (m, 3H), 8.15 (b, 1H).

### e) 1-(1-Cyclohexanecarbonyl-6-trifluoromethoxy-1,2,3,4-tetrahydroquinoline-2-ylmethyl)-4-(4-indolyl)-piperazine

The title compound was synthesised following the alternative acylation procedure described for the compound of Example 1 (step f) but starting from Compound 26D instead of Compound 1B. The crude was purified by flash chromatography eluting with petroleum ether - ethyl acetate 6:4 to give the title compound (50 %).
¹H-NMR (δ): 1.70-2.02 (m, 11H), 2.02-2.25 (m, 1H), 2.25-2.50 (m, 2H), 2.50-2.85 (m, 7H), 3.05-3.35 (m, 4H), 6.45-6.65 (m, 2H), 7.00-7.25 (m, 6H), 7.00-7.25 (m, 6H), 8.15 (bs, 1H).

### Example 27

### 1-[1-(3-Benzylaminopropionyl)-1,2,3,4-tetrahydroquinoline-2-ylmethyl]-4-(4-indolyl)-piperazine

### a) 1-(1-Acryloyl-1,2,3,4-tetrahydroquinoline-2-ylmethyl)-4-(4-indolyl)-piperazine (Compound 27A)

The title compound was synthesised following the alternative acylation procedure described for the compound of Example 1 (step f) but using acryloyl chloride instead of cyclohexanecarbonyl chloride and carrying out the reaction at -10°C. The crude was purified by flash chromatography eluting with petroleum ether - ethyl acetate - 2.5 N methanolic ammonia 5:5:0.2 to give the title compound (62%) as a yellow solid. M.p. 147-8 °C.
¹H-NMR (δ): 1.60-1.82 (m, 1H), 2.25 (dd, 1H), 2.32-2.89 (m, 9H), 3.08-3.33 (m, 4H), 5.01-5.22 (m, 1H), 5.65 (d, 1H), 6.40-6.63 (m, 4H), 7.00-7.30 (m, 7H), 8.15 (s, 1H).

### b) 1-[1-(3-Benzylaminopropionyl)-1,2,3,4-tetrahydroquinoline-2-ylmethyl]-4-(4-indolyl)-piperazine

A mixture of Compound 27A (0.52 g) and benzylamine (0.71 ml) was stirred at 110°C for 1h. After cooling to 20-25°C, the reaction mixture was diluted with H₂O. The pasty which formed was precipitated, extracted with EtOAc (20 ml), washed with H₂O, dried (Na₂SO₄) and evaporated to dryness *in vacuo.* The crude was purified by flash chromatography eluting with ethyl acetate - 2.5 N methanolic ammonia 95:5 to give the title compound (91%) as a yellow solid.
¹H-NMR (δ): 1.38-1.65 (m, 1H), 2.08-2.25 (m, 2H), 2.31-2.81 (m, 10H), 2.82-2.98 (m, 24H), 3.10-3.32 (m, 4H), 3.88 (s, 2H), 4.95-5.27 (br, 1H), 6.4&6.62 (m, 2H), 7.00-7.40 (m, 12H).

### Example 28

### 1-(3-Aminopropionyl-1,2,3,4-tetrahydroquinoline-2-ylmethyl]-4-(4-indolyl)-piperazine

A mixture of the compound of Example 27 (0.51 g), ammonium formate (0.32 g), 10% palladium on carbon (0.25 g) and MeOH (18 ml) was stirred at reflux for 1.5 h. Afterwards, it was filtered and evaporated to dryness in vacuo. The crude was purified by flash chromatography eluting with CHCl₃ - 2.5 N methanolic ammonia 9:1 to give 0.34 g of the title compound (80 %) as a yellow solid.
¹H-NMR (δ): 1.38-1.78 (m, 3H), 2.06-2.23 (m, 1H), 2.252.88 (m, 12H), 2.89-3.08 (m, 2H), 3.10-3.32 (m, 2H), 5.00-5.30 (br, 1H), 6.47-6.63 (m, 2H), 7.00-7.33 (m, 7H).

### Example 29

### 1-(4-Indolyl)-4-[1-(3-methylaminopropionyl-1,2,3,4-tetrahydroquinoline-2-ylmethyl]-piperazine

The title compound was synthesised following the procedure reported for Compound 27 (step b) but using 40% aqueous methylamine instead of benzylamine and stirring at r.t. in DMF. The crude was purified by flash chromatography eluting with CHCl₃ - 2.5 N methanolic ammonia 92:8 to give the title compound (63 %).
¹H-NMR (δ): 1.40-1.65 (m, 1H), 1.70 (s, 1H), 2.10-2.25 (m, 2H), 2.30-2.95 (m, 15H), 3.10-3.31 (m, 4H), 4.98-5.25 (br, 1H), 6.48-6.62 (m, 2H), 7.00-7.28 (m, 7H).

### Example 30

### 1-[1-(3-Dimethylaminopropionyl)-1,2,3,4-tetrahydroquinoline-2-ylmethyl]-4-(4-indolyl)-piperazine

The title compound was synthesised following the procedure reported for Compound 27 (step b) but using a solution of anhydrous dimethylamine in toluene and stirring at 100°C for 2 h in toluene in a sealed reaction vessel. The crude was purified by flash chromatography eluting with EtOAc-2.5 N methanolic ammonia 9:1 to give the title compound (56 %).
¹H-NMR (δ): 1.38-1.65 (m, 1H), 2.05-2.30 (m, 6H), 2.31-2.87 (m, 12H), 3.20-3.35 (m, 4H), 4.98-5.30 (br, 1H), 6.48-6.62 (m, 2H), 7.00-7.32 (m, 7H), 8.20 (s, 1H).

### Example 31

### 1-(4-Indolyl)-4-(1-anilinocarbonyl-12,3,4-tetrahydroquinoline-2-ylmethyl]-piperazine

The title compound was synthesised as described for the compound of Example 20 but using phenyl isocyanate instead of ethyl isocyanate and stirring at reflux for 11 h in toluene. The crude was purified by flash chromatography eluting with EtOAc - petroleum ether - 2.5 N methanolic ammonia 1:1:0.01 to give the title compound (69.1 %).
¹H-NMR (δ): 1.60-1.815 (m, 1H), 2.20-2.40 (m, 1H), 2.50 (d, 1H), 2.62-3.08 (m, 7H), 3.10-3.50 (m, 4H), 4.48-4.72 (m, 1H), 6.48-6.70 (m, 2H), 6.957.40 (m, 9H), 7.42-7.58 (m, 3H), 8.25 (s, 1H).

### Example 32

### 1-(1-Cyclohexanecarbonyl-6-methyl-1,2,3,4-tetrahydroquinoline-2-ylmethyl]-4-(4-indolyl)-piperazine

### a) 1-Benzoyl-2-cyano-6-methyl-1,2-dihydroquinoline (Compound 32A)

To a solution of 6-methylquinoline (2.83 ml) in CH₂Cl₂ (25.5 ml) was added a solution of potassium cyanide (4.1 g) in water (10.25 ml) followed by dropwise addition of benzoyl chloride (4.86 ml). The reaction was stirred at r.t. for 4h, then the organic layer was separated and the aqueous layer extracted with CH₂Cl₂ (30 ml). The combined organic layers were washed with H₂O, 1 N HCl, H₂O, 1N NaOH, dried and evaporated to dryness in vacuo. The crude was crystallized from EtOH affording 1.84 g of the title product (32%). A second amount (0.86 g; 15%) was obtained by flash chromatography purification of the mother liquors eluting with petroleum ether - EtOAc 8:2.
¹H-NMR (δ): 2.30 (s, 3H), 6.05-6.14 (m, 1H), 6.21 (d, 1H), 6.48 (d, 1H), 6.70-6.89 (m, 2H), 7.03 (d, 1H), 7.27-7.46 (m, 5H).

### b) 6-Methylquinoline-2-carboxylic acid (Compound 32B)

A suspension of 2.7 g of Compound 32A in 48% HBr (3 ml), AcOH (3 ml), and H₂O (17 ml) was stirred at reflux for 40-45'. The solid which precipitated after cooling to r.t was taken up with 32% aq. NH₃ to pH=8-9 at 50°C; afterwards, AcOH was added to lower the pH to about 4. After filtration the title compound was obtained (1.69 g; 90%).
¹H-NMR (δ): 2.53 (s; 3H) 7.70 (d, 1H), 7.86 (d, 1H), 8.01-8.13 (m, 2H), 8.42 (d, 1H), 12.75 13.50 (br, 1H).

### c) 6-Methyl-1,2,3,4-tetrahydroquinoline-2-carboxylic acid (Compound 32C)

The title compound was prepared as described for Compound 1B (step e) starting from Compound 32B instead of Compound 1D. The crude was treated with 37% HCl and taken up with MeCN (5 ml) to afford, after cooling to 0°C and filtration, Compound 32C (53%).
¹H-NMR (δ): 1.94-2.15 (m, 1H), 2.16-2.32 (m, 4H), 2.57-2.91 (m, 2H), 4.15 (dd, 1H), 6.81-7.01 (m, 3H), 7.25-7.69 (br, 2H), 9.31-10.5 (br, 1H)

### d) 1-(4-Indolyl)-4-(6-methyl-1,2,3,4-tetrahydroquinoline-2-ylcarbonyl)-piperazine (Compound 32D)

The title compound was synthesised following the procedure described for Compound 1D (step g) but using Compound 32C instead of 1,2,3,4-tetrahydroquinoline-2-carboxylic acid. The residue was purified by flash chromatography eluting with petroleum ether - EtOAc 1:1 to give the title compound (73 %).
¹H-NMR (δ): 1.61-1.86 (m, 1H), 2.08-2.30 (m, 4H), 2.68-2.96 (m, 2H), 3.18-3.36 (m, 4H), 3.67-4.10 (m, 4H), 4.23 (dd, 1H), 4.45-4.51 (br, 1H), 6.51-6.69 (m, 3H), 6.78-6.93 (m, 2H), 7.05-7.22 (m, 3H), 8.15-8.38 (br, 1H).

### e) 1-(4-Indolyl)-4-(6-methyl-12,3,4-tetrahydroquinoline-2-ylmethyl)-piperazine (Compound 32E)

The title compound was synthesised following the procedure described for Compound 1B (step h). The residue was purified by flash chromatography eluting with petroleum ether-EtOAc 7:3 to give the title compound (35 %).
¹H-NMR (δ): 1.48-1.71 (m, 1H), 1.82-1.97 (m, 1H), 2.21 (s, 3H), 2.50 (d, 2H), 2.55-2.74 (m, 2H), 2.76-2.99 (m, 4H), 3.19-3.37 (m, 4H), 3.38-3.56 (m, 1H), 4.33-4.69 (br, 1H), 6.43-6.69 (m, 3H), 6.74-6.89 (m, 2H), 7.01-7.21 (m, 3H), 8.07-8.26 (br, 2H).

### f) 1-(1-Cyclohexanecarbonyl-6-methyl-1,2,3,4-tetrahydroquinoline-2-ylmethyl)-4-(4-indolyl)-piperazine

The title compound was synthesised following the alternative acylation procedure described for the compound of Example 1 (step f) but starting from Compound 32E instead of Compound 1B. The crude was purified by flash chromatography eluting with petroleum ether - ethyl acetate 1:1 to give the title compound (88 %).
¹H-NMR (δ): 0.86-2.01; (m; 11H), 2.04-2.21 (m; 1H), 2.29-2.86 (m;12H), 3.11-3.31 (m; 4H), 4.99-5.22 (m; 1H), 6.48-6.62(m; 2H), 6.96-7.16 (m; 6H), 8.05-8.24 (bs; 1H).

### Example 33

### 1-(4-Indolyl)-4-(1-pyrrolidinecarbonyl-1,2,3,4-tetrahydroquinoline-2-ylmethyl)-piperazine

The title compound was synthesised following the alternative acylation procedure described for the compound of Example 1 (step f) but using 4-pyrrolidinocarbonyl chloride instead of cyclohexanecarbonyl chloride and chloroform instead of dichloromethane, and refluxing for 8 hours. The crude was purified by flash chromatography eluting with petroleum ether - ethyl acetate - 2.5N methanolic ammonia 8:2:0.01 to give the title compound (10%).
¹H-NMR (δ): 1.51-2.21 (m, 4H), 2.22-2.41 (m, 1H), 2.58-295 (m, 9H), 3.51-3.69 (m, 2H), 4.34-4.59 (m, 1H), 6.49-6.63 (m, 2H), 6.79- 7.28 (m, 7H), 8.14 (bs, 1H)

### Example 34

### 1-(6-Bromo-1-cyclohexanecarbonyl-1,2,3,4-tetrahydroquinoline-2-ylmethyl)-4-(4-indolyl)-piperazine

### a) 6-Bromo-2-methoxycarbonyl-1,2,3,4-tetrahydroquinoline (Compound 34A)

A solution of 0.36 g of *N*-bromosuccinimide in 3 ml of anhydrous DMF was dropped into a solution of 0.37 g of 2-methoxycarbonyl-1,2,3,4-terahydroquinoline stirred at 0-5°C. After 2.5 h at 0-5°C and 20 h at r.t. the mixture was diluted with H₂O (50 ml) and extracted with Et₂O (3x20 ml); the organic layer was washed with H₂O (3x10 ml) and dried (anhydrous Na₂SO₄). The solvent was evaporated and the residue was purified by flash chromatography (petroleum ether - EtOAc 9:1) to afford 0.29 g (55.5%) of Compound 34A as an ivory solid. ¹H-NMR (δ): 1.85-2.38 (2m, 2H), 2.60-2.90 (m, 2H), 3.78 (s, 3H), 3.944.10 (m, 1H), 4.38 (s, 1H), 6.45 (d, 1H), 7.00-7.15 (m, 2H).

### b) 6-Bromo-2-hydroxymethyl-1,2,3,4-tetrahydroquinoline (Compound 34B)

A mixture of 0.27 g of Compound 34A, 5 ml of anhydrous THF and 0.6 ml of 2 M LiBH₄ in THF was stirred at r.t. for 6.5 h, diluted with H₂O (50 ml) and extracted with Et₂O (3x20 ml). The organic layer was dried (anhydrous Na₂SO₄) and evaporated to dryness *in vacuo* to afford 0.21 g (85.1%) of the title compound as an oil.
¹H-NMR (δ): 1.45-2.00 (2m, 3H), 2.60-2.95 (m, 2H), 3.20-3.85 (2m, 3H), 4.00-4.55 (br, 1H), 6.40 (d, 1H), 6.95-7.10 (m, 2H)

### c) 6-Bromo-2-iodomethyl-1,2,3,4-tetrahydroquinoline (Compound 34C)

Into a stirred mixture of 0.31 g of Compound 34B, 0.20 g of imidazole, 0.40 g of Ph₃P and 3 ml of toluene - MeCN (5:1) was added at 0-5°C during 15 min, 0.36 g of I₂. The mixture was stirred at 0-5°C for 15', then at r.t. for 1 h, treated with aq. Na₂S₂O₃ and extracted with EtOAc (3x20 ml); the organic layer was washed with brine, dried (anhydrous Na₂SO₄) and evaporated to dryness *in vacuo* to afford 0.76 g of a mixture of Compound 34C and Ph₃PO and used without further purification in the next reaction step.
¹H-NMR (δ): 1.63-2.15 (2m,1H), 2.58-2.90 (m, 2H), 3.10-3.35 (2m, 2H), 3.35-3.55 (m, 1H), 4.05-4.40 (b, 1H), 6.40 (d, 1H), 6.95-7.20 (m, 2H), 7.40-7.80 (m, 8H).

### d) 6-Bromo-2-[4-(4-indolyl)-1-piperazinyl]-methyl]-1,2,3,4-tetrahydroquinoline (Compound 34 D)

A stirred mixture of 0.76 g of Compound 34C, 0.6 ml of anhydrous DMF, 0.45 ml of DIPEA and 0.29 g (1.39 mmol) of 1-(4-indolyl)-piperazine was heated at 115°C for 4 h, diluted with H₂O (50 ml) and extracted with Et₂O. The organic layer was washed with H₂O (2×10 ml), dried (anhydrous Na₂SO₄) and evaporated to dryness *in vacuo.* The residue was purified by flash chromatography (petroleum ether - EtOAc 7:3) to afford 0.27 g (50.7%) of the title compound as a dense oil.
¹H-NMR (δ): 1.40-1.70 (m, 1H), 1.80-2.00 (m, 1H), 2.35-3.00 (m, 8H), 3.10-3.70 (m, 5H), 4.55-4.90 (b, 1H), 6.40 (d, 1H), 6.50-6.70 (m, 2H), 6.95-7.25 (m, 5H), 8.05-8.35 (b, 1H).

### e) 1-(6-Bromo-1-cyclohexanecarbonyl-1,2,3,4-tetrahydroquinoline-2-ylmethyl)-4-(4-indolyl)-piperazine

The title compound was prepared from Compound 34D following the alternative acylation procedure described for the compound of Example 1 (step f) using chloroform instead of dichloromethane, and refluxing for 8 hours. The residue was purified by flash chromatography (petroleum ether - EtOAc 70 : 30) affording the title compound (65%) as an ivory vitreous solid.
¹H-NMR (δ): 0.85-2.00 (m, 12H), 2.00-2.20 (m, 1H), 2.20-2.85 (m, 8H), 3.00-3.45 (m, 4H), 4.80-5.20 (m, 1H), 6.45-6.65 (m, 2H), 6.85-7.20 (m, 4H), 7.30-7.05. (m, 2H), 8.15 (br, 1H).

### Example 35A

### 1-(1-Cyclohexanecarbonyl-1,2,3,4-tetrahydroquinoline-2-ylmethyl)-4-(4-fluoro-2-methoxyphenyl)-piperazine

### a) 1-Cyclohexanecarbonyl-2-hydroxymethyl-1,2,3,4-tetrahydroquinoline (Compound 35A-A)

Into a solution of methyl 1-cyclohexanecarbonyl-1,2,3,4-tetrahydroquinoline-2-carboxylate (19 g) in anhydrous THF (210 ml) stirred at 0°C was dropped lithium borohydride (31.5 ml of a 2M sol. in THF). The reaction mixture was stirred at 0°C - r.t for 6 h ; afterwards, it was cautiously acidified by adding 2N HCl and extracted with EtOAc (2x500 ml). The organic layers were washed with H₂O, dried (Na₂SO₄), evaporated to dryness *in vacuo* and purified by flash chromatography eluting with petroleum ether - EtOAc 6:4 to give 11.8 g (69 %) of the title compound.
¹H-NMR (δ): 0.80-2.00 (m, 11H), 2.30-2.85 (m, 4H), 3.32 (dd, 1H), 3.60 (dd,1H), 3.95 4.20 (br, 1H), 4.70-4.92 (m, 1H), 7.05-7.35 (m,4H)

### b) 1-Cyclohexanecarbonyl-2-formyl-1,2,3,4-tetrahydroquinoline (Compound35A-B)

9.2 ml of DMSO was added over a period of 15 min to a solution of freshly distilled oxalyl chloride (5.65 ml) stirred at -60°C in anhydrous CH₂Cl₂ (100 ml). A solution of Compound 35A-A (11.8 g) in CH₂Cl₂ (100 ml) was dropped into this mixture and, after 5 min stirring at the same temperature, TEA (35 ml) was added dropwise. The reaction mixture was allowed to warm to 0°C during 3 h.; afterwards, it was quenched by adding H₂O, alkalinised with 1 M NaOH and extracted with CH₂Cl₂ (2x200 ml). The organic layers were washed with H₂O, dried (Na₂SO₄), evaporated to dryness in vacuo and purified by flash chromatography eluting with petroleum ether - EtOAc 7:3 to give 11.2 g (95 %) of the title compound.
¹H-NMR (δ): 0.82-2.08 (m, 11H), 2.28-2.82 (m, 4H), 2.83-3.03 (m, 1H), 5.18 (dd, 1H), 7.08-7.32 (m, 4H), 9.50 (s, 1H)

### c) 1-(1-Cyclohexanecarbonyl-1,2,3,4-tetrahydroquinoline-2-ylmethyl)-4-(4-fluoro-2-methoxyphenyl)-piperazine

To a solution of Compound 35A-B (0.14 g) and 1-(4-fluoro-2-methoxyphenyl)-piperazine (0.17 g) in CHCl₃ (5 ml) at r.t., were added sodium triacetoxyborohydride (0.21 g) and acetic acid (0.14 ml). The solution was stirred at r.t. for 3 h, maintained overnight resting, poured into H₂O (30 ml), alkalinised with 1 N NaOH and extracted with EtOAc (2 x 30 ml). The combined organic layers were washed with H₂O, dried (Na₂SO₄) and the solvent was evaporated under vacuum. The crude was purified by flash chromatography eluting with EtOAc-petroleum ether-2N NH₃ in MeOH 1:1:0.01 to give 0.18 g (74%) of the title compound.
¹H-NMR (δ): 0.82-2.18 (m,11H), 2.21-2.85 (m, 9H), 2.86-3.10 (m, 4H), 3.83 (s, 3H), 4.92 5.12 (m, 1H), 6.486-6.66 (m, 2H), 6.78-6.90 (m, 2H), 6.98-7.30 (m, 4H).

The following compounds were similarly synthesised (Ex. 35A - step c) in a parallel synthesis fashion using dichloromethane instead of chloroform and using the appropriate piperazine instead of 1-(4-fluoro-2-methoxyphenyl)-piperazine. After having alkalinised, diluted with CH₂Cl₂ and removed, the aqueous layer the organic layer was evaporated to dryness in vacuo and the crude purified by flash chromatography (eluent shown):

### Example 35A1

### 1-(1-Cyclohexanecarbonyl-1,2,3,4-tetrahydroquinoline-2-ylmethyl)-4-(2-methyl-4-indolyl)-piperazine

From 1-(2-methyl-4-indolyl)-piperazine.
Flash chromatography: CH₂Cl₂- MeOH: 95-5. Yield: 89%.
¹H-NMR (δ): 0.79-2.01 (m,11H), 2.05-2.22 (m, 1H), 2.30-2.88 (m, 12H), 3.00-3.32 (m, 4H), 4.95-5.25 (m, 1H), 6.20 (s, 1H), 6.55 (d, 1H), 6.90-7.32 (m, 6H), 7.78-8.00 (brs, 1H).

The starting piperazine was obtained as following:

### a) 2-Methyl-7-aminoindole (Compound 35A1-A)

To a solution of 2-methyl-7-nitroindole (N. Moskalev et al, *Tetrahedron Letters* **40**, 5395-5398, (1999)) (1.4 g) and Ni-Raney (40 mg) in MeOH (40 ml) was added hydrazine hydrate (4.8 ml) and the resulting suspension stirred for 4h. The catalyst was filtered off and the solvents evaporated under vacuum. The residue was dissolved in CH₂Cl₂ (80 ml) and washed with H₂O (50 ml). The organic layer was dried (Na₂SO₄) and the residue was purified by flash chromatography (CH₂Cl₂-2N NH₃ in MeOH 97:3) to give 0.62 g (53%) of the title compound.
¹H-NMR (δ): 2.48 (s, 3H), 3.21-4.32 (br, 2H), 6.05 (s, 1H), 6.41 (dd, 1H), 6.78 (dd, 1H), 6.95 (dd, 1H), 7.75-7.93 (br, 1H)

### b) 1-(2-Methyl-4-indolyl)-piperazine (Compound 35A1-B)

A mixture of Compound 35A1-A (0.62 g), bis(2-chloroethyl)amine hydrochloride (0.7 6g), potassium iodide (0.35 g) and DIPEA (0.8 ml) in 1,2-dichlorobenzene (5ml) and *n*-hexanol (0.5ml) was heated at 190°C for 3h. After cooling at r.t, EtOAc (30ml) and 1M NaOH (20 ml) was added; the organic layer was separated and dried (Na₂SO₄) and evaporated to dryness. The crude was purified by flash chromatography (CH₂Cl₂-2N NH₃ in MeOH 97:3) to give 0.43 g (47%) of the title compound.
¹H-NMR (δ): 2.46 (s, 3H), 3.02 (m, 8H), 6.31 (s 1H), 6.55 (dd, 1H), 6.927.08 (m, 2H), 7.83-8.01 (bs 1H).

### Example 35A2

### 1-(1-Cyclohexanecarbonyl-1,2,3,4-tetrahydroquinoline-2-ylmethyl)-4-phenylpiperazine

From 1-phenylpiperazine (commercial)
Flash chromatography: petroleum ether - EtOAc 70:30. Yield: 43%.
¹H-NMR (δ): 0.82-2.18 (m, 11H), 2.02-2.18 (m, 1H), 2.20-2.82 (m, 9H), 3.00-3.22 (m, 4H), 5.00-5.25 (m, 1H), 6.75-6.98 (m, 3H), 6.99-7.32 (m, 6H).

### Example 35A3

### 1-(1-cyclohexanecarbonyl-1,2,3,4-tetrahydroquinoline-2-ylmethyl)-4-(2-ethoxyphenyl)-piperazine

From 1-(2-ethoxyphenyl)-piperazine (commercial)
Flash chromatography: petroleum ether - EtOAc 70:30. Yield: 74%.
¹H-NMR (δ): 0.78-2.18 (m, 12H), 1.45 (t, 3H), 2.28-2.85 (m, 9H), 2.903.22 (m, 4H), 4.08 (q, 2H), 4.95-5.25 (m, 1H), 6.75-7.02 (m, 4H), 7.03-7.32 (m, 4H).

### Example 35A4

### 1-(1-Cyclohexanecarbonyl-1,2,3,4-tetrahydroquinoline-2-ylmethyl)-4-(2,5-dimethoxyphenyl)-piperazine

From 1-(2,5-dimethoxyphenyl)-piperazine (J. Med. Chem. 29, 630, 1986)
Flash chromatography: petroleum ether-EtOAc 50:50. Yield: 56%.
¹H-NMR (δ): 0.75-2.20 (m, 12H), 2.25-2.80 (m, 9H), 2.88-3.15 (m, 4H), 3.78;3,80 (2s, 6H), 4.95-5.25 (m, 1H), 6.40-7.06 (m, 2H), 6,78 (d; 1H), 7.03-7.32 (m, 4H).

### Example 35A5

### 1-(1-Cyclohexanecarbonyl-1,2,3,4-tetrahydroquinoline-2-ylmethyl)-4-(2,3-dihydrobenzofuran-7-yl)-piperazine

From 1-(2,3-dihydrobenzofuran-7-yl)-piperazine (F. Kerrigan et al., *Tetrahedron Letters,* **39**, 2219-2222, (1998)).
Flash chromatography: petroleum ether - EtOAc 70:30. Yield: 86%.
¹H-NMR (δ): 0.75-2.20 (m, 12H), 2.20-280 (m, 9H), 2.90-3.30 (m, 6H), 4.58 (t, 2H), 4.90 5.25 (m, 1H), 6.40-6.90 (m, 3H), 6.95-7.20 (m, 4H).

### Example 35A6

### 1-(1-Cyclohexanecarbonyl-1,2,3,4-tetrahydroquinoline-2-ylmethyl)-4-(5-fluoro-2-methoxyphenyl)-piperazine

From 1-(5-fluoro-2-methoxyphenyl)-piperazine (US 4585773)
Flash chromatography: petroleum ether - EtOAc 6:4. Yield: 32%.
¹H-NMR (δ): 0.75-1.99 (m, 11H), 2.01-2.18 (m, 1H), 2.28-2.80 (m, 9H), 2.893.11 (m, 4H), 3.81 (s, 3H), 4.92-5.21 (m, 1H), 6.55-6.78 (m, 3H) 7.05-7.25 (m, 4H).

### Example 35A7

### 1-(1-Cyclohexanecarbonyl-1,2,3,4-tetrahydroquinoline-2-ylmethyl)-4-(2-methylphenyl) piperazine

From 1-(2-methylphenyl)-piperazine(commercial).

Flash chromatography:- EtOAc 8:2. Yield: 25%.
¹H-NMR (δ): 0.80-1.99 (m, 11H), 2.00-2.21 (m, 1H), 2.22-2.95 (m, 18H), 4.965.22 (m, 1H), 6.95-7.06 (m, 2H), 7.08-7.20 (m, 6H)

### Example 35A8

### 1-(1-Cyclohexanecarbonyl-1,2,3,4-tetrahydroquinoline-2-ylmethyl)-4-(2-trifluoromethylphenyl)-piperazine

From 1-(2-trifluoromethylphenyl)-piperazine (Commercial)
¹H-NMR (δ): 0.8-2.2 (m, 13H), 2.23-2.95 (m, 12H), 4.95-5.2 (m, 1H), 7.00-7.65 (m, 8H).

### Example 35A9

### 1-(1-Cyclohexanecarbonyl-1,2,3,4-tetrahydroquinoline-2-ylmethyl)-4-(2,4-dichlorophenyl)-piperazine

From 1-(2,4-dichlorophenyl)-piperazine (commercial)
Flash chromatography:- EtOAc 7:3. Yield: 77%.
¹H-NMR (δ): 0.8-2.2 (m, 13H), 2.25-3.1 (m, 12H), 5.0-5.25 (m, 1H), 7.00-7.65 (m, 7H).

### Example 35A10

### 1-(1-Cyclohexanecarbonyl-1,2,3,4-tetrahydroquinoline-2-ylmethyl)-4-(2-hydroxyphenyl)-piperazine

From 1-(2-hydroxyphenyl)-piperazine (commercial)
Flash chromatography: petroleum ether - EtOAc 7:3. Yield: 82%.
¹H-NMR (δ): 0.72-2.12 (m, 13H), 2.23-2.85 (m, 13H), 4.96-5.23 (m, 1H), 6.75-6.95 (m, 2H), 7.01-7.30 (m, 6H).

### Example 35A11

### 1-(1-Cyclohexanecarbonyl-1,2,3,4-tetrahydroquinoline-2-ylmethyl)-4-(2-isopropoxyphenyl)-piperazine

From 1-(2-isopropoxyphenyl)-piperazine (Martin, G.E. et al., *J. Med. Chem.* **32**, 1052-1056, (1989)).
Flash chromatography: petroleum ether - EtOAc 7:3. Yield: 77%.
¹H-NMR (δ): 0.76-2.11 (m, 18H), 2.21-2.84 (m, 9H), 2.89-3.14 (m, 4H), 4.49-4.69 (m, 1H), 4.94-5.21 (m, 1H), 6.80-6.92 (m, 4H), 7.03-7.29 (m, 4H).

### Example 35A12

### 1-(1-Cyclohexanecarbonyl-1,2,3,4-tetrahydroquinoline-2-ylmethyl)-4-(2-fluoro-5-methylphenyl)-piperazine

From 1-(2-fluoro-5-methylphenyl)-piperazine (WO 01/05765)
Eluent: petroleum ether - EtOAc 7:3. Yield: 46%.
¹H-NMR (δ): 0.78-2.18 (m, 12H), 2.20-2.85 (m, 12H), 2.95-3.15 (m, 4H), 4.97-5.23 (m, 1H), 6.62-6.80 (m, 1H), 6.63-6.92 (m, 2H), 7.02-7.25 (m, 4H).

### Example 35A13

### 1-(1-Cyclohexanecarbonyl-1,2,3,4-tetrahydroquinoline-2-ylmethyl)-4-(2,3-dimethyl-4-indolyl)-piperazine

From 1-(2,3-dimethyl-4-indolyl)-piperazine
Flash chromatography: petroleum ether - EtOAc 6:4. Yield: 56%.
¹H-NMR (δ): 0.78-2.20 (m, 11H), 2.21-2.82 (m, 16H), 2.843.22 (m, 4H), 5.01-5.26 (m, 1H), 6.58-6.71 (m, 1H), 6.92-7.02 (m, 2H), 7.04-7.32 (m, 4H), 7.65-7.81 (br, 1H).

The starting piperazine was obtained as following:
a) 2,3-Dimethyl-7-aminoindole (Compound 35A13-A)
   The title compound was prepared as described for Compound 35A1-A starting from 2,3-dimethyl-7-nitroindole (N. Moskalev et al, *Tetrahedron Letters* **40**, 5395-5398, (1999)) instead of 2-methyl-7-nitroindole. The crude was purified by flash chromatography eluting with petroleum ether-ethyl acetate 7:3 to give the title compound (70%).
   ¹H-NMR (δ): 2.31 (s, 3H), 2.49 (s, 3H), 3,85-4.21 (br, 2H), 6.27 (dd, 1H), 6.70 (dd, 1H); 6.94 (dd, 1H), 7.48-7.73 (br, 1H)
b) 1-(2,3-Dimethyl-4-indolyl)-piperazine (Compound 35A13-B)
   The title compound was prepared as described for Compound 35A1-B starting from Compound 35A13-A instead of Compound 35A1-A. The crude was purified by flash chromatography eluting with CH₂Cl₂-2N NH₃ in MeOH 97:3 to give the title compound (43%).
   ¹H-NMR (δ): 2.30 (s, 3H), 2.43 (s, 3H), 2.95-3.21 (m, 8H), 6.31 (s, 1H), 6.62-6.75 (m, 1H), 6.91-7.09 (m, 2H), 7.63-7.86 (br, 1H).

### Example 35A14

### 1-(1-Cyclohexanecarbonyl-1,2,3,4-tetrahydroquinoline-2-ylmethyl)-4-(2-chloro-5-fluorophenyl)-piperazine

1-(2-chloro-5-fluorophenyl)-piperazine (WO 01/05765)
Flash chromatography: petroleum ether - EtOAc 8:2. Yield: 25%.
¹H-NMR (δ): 0.8-2.2 (m, 13H), 2.22-2.75 (m, 8H), 2.8-3.2 (m, 4H), 4.94-5.21 (m, 1H), 6.60-6.80 (m, 2H), 7.00-7.30 (m, 5H).

### Example 35A15

### 1-(1-Cyclohexanecarbonyl-1,2,3,4-tetrahydroquinoline-2-ylmethyl)-4-(5-fluoro-2-methylphenyl)-piperazine

From 1-(5-fluoro-2-methylphenyl)-piperazine.
Flash chromatography: petroleum ether - EtOAc 8:2. Yield: 18%.
¹H-NMR (δ): 0.8-2.15 (m, 13H), 2.15-2.25 (s, 3H) 2.30-2.85 (m, 12H), 4.94-5.21 (m, 1H), 6.58-6.78 (m, 2H), 7.00-7.30 (m, 5H).

The starting piperazine was prepared as following:
a) 1-(5-Fluoro-2-methylphenyl)-piperazine (Compound 35A15-A)
   The title compound was prepared as described for Compound 35A1-B starting from 1-(5-fluoro-2-methyl)-aniline instead of Compound 35A1-A. The crude was purified by flash chromatography eluting with CH₂Cl₂-2N NH₃ in MeOH 97:3 to give the title compound (43%).

### Example 35A16

### 1-[(2,3-Dihydro-1,4-benzodioxin-5-yl)]-4-(1-cyclohexanecarbonyl-1,2,3,4-tetrahydroquinoline-2-ylmethyl)-piperazine

From 1-[(2,3-Dihydro-1,4-benzodioxin-5-yl)]-piperazine (F. Kerrigan et al., *Tetrahedron Letters,* **39**, 2219-2222, (1998)).
Flash chromatography: petroleum ether - EtOAc 4:6. Yield: 22%.
¹H-NMR (δ): 0.8-2.2 (m, 13H), 2.25-2.80 (m, 8H) 265-3.25 (m, 4H), 4.16-4.38 (m, 4H), 4.95-5.20 (m, 1H), 6.48-6.80 (m, 3H), 7.00-7.30 (m, 4H).

### Example 35A17

### 1-(1-Cyclohexanecarbonyl-1,2,3,4-tetrahydroquinoline-2-ylmethyl)-4-(2-trifluoromethoxyphenyl)-piperazine

From 1-(2-trifluoromethoxyphenyl)-piperazine (EP 0711757)
Flash chromatography: petroleum ether - EtOAc 7:3. Yield: 35%
¹H NMR (δ): 0.65-2.80 (m, 21H), 2.90-3.15 (m, 4H), 5.10 (bs, 1H), 6.95 (m, 2H), 7.05-7.30 (m, 6H)

### Example 35A18

### 1-(1-Cyclohexanecarbonyl-1,2,3,4-tetrahydroquinoline-2-ylmethyl)-4-(4-fluoro-2-methylphenyl)-piperazine

From 1-(4-fluoro-2-methylphenyl)-piperazine (WO 01/29015)
Flash chromatography: petroleum ether - EtOAc 7:3. Yield: 87%.
¹HNMR (δ): 0.70-2.20 (m, 13H), 2.20 (s, 3H), 2.20-2.90 (m, 12H), 5.10 (bs, 1H), 6.75-7.00 (m, 3H), 7.00-7.30 (m, 4H)

### Example 35A19

### 1-(1-Cyclohexanecarbonyl-1,2,3,4-tetrahydroquinoline-2-ylmethyl)-2,5-dichlorophenyl)-piperazine

From 1-(2,5-dichlorophenyl)-piperazine (commercial)
Flash chromatography: petroleum ether-EtOAc 8:2. Yield: 55%.
¹H-NMR (δ): 0.70-2.85 (m, 22H), 2.85-3.15 (m, 4H), 5.10 (bs, 1H), 6.85-7.00 (m, 2H), 7.00-7.35 (m, 4H)

### Example 35A20

### 1-(1-Cyclohexanecarbonyl-1,2,3,4-tetrahydroquinoline-2-ylmethyl)-4-[4-fluoro-2-(2,2,2-trifluoroethoxyphenyl)]-piperazine

From 1-[4-fluoro-2-(2,2,2-trifluoroethoxyphenyl)]-piperazine (EP 0748800)
Flash chromatography: petroleum ether - EtOAc 4:6. Yield: 28%.
¹H-NMR (δ): 0.70-2.20 (m, 13H), 2.20-2.85 (m, 8H), 2.85-3.15 (m, 4H), 4.40 (q, 2H), 5.10 (bs, 1H), 6.55-6.80 (m,2H), 6.80-6.95 (m, 1H), 6.95-7.35 (m, 4H)

### Example 35A21

### 1-(1-Cyclohexanecarbonyl-1,2,3,4-tetrahydroquinoline-2-ylmethyl)-4-(2-pyrimidinyl)-piperazine

From 1-(2-pyrimidinyl)-piperazine (commercial)
Flash chromatography: petroleum ether - EtOAc 4:6. Yield: 13%.
¹H-NMR (δ): 0.70-2.30 (m, 21H), 3.60-3.90 (m, 4H), 5.10 (bs, 1H), 6.45 (dd, 1H), 7.00-7.30 (m, 4H), 8.30 (dd, 2H)

### Example 35A22

### 1-(1-Cyclohexanecarbonyl-1,2,3,4-tetrahydroquinoline-2-ylmethyl)-4-(8-quinolinyl)-piperazine

From 1-(8-quinolinyl)-piperazine (WO 00/40554)
Flash chromatography: petroleum ether-EtOAc 50:50. Yield: 70%.
¹H-NMR (δ): 0.79-2.25 (m, 13H), 2.26-3.50 (m, 8H), 3.22-3.49 (m, 4H), 5.00-5.31 (m, 1H), 7.05-7.48 (m, 7H), 8.09 (dd, 1H), 8.82 (dd, 1H)

### Example 35A23

### 1-(5-chloro-2-cyanophenyl)-4-(1-cyclohexanecarbonyl-1,2,3,4-tetrahydroquinoline-2-ylmethyl)-piperazine

From 1-(5-chloro-2-cyanophenyl)-piperazine (WO 01/05765)
Flash chromatography: petroleum ether-EtOAc 50:50. Yield: 80%.
¹H-NMR (δ): 0.79-2.01 (m, 12H), 2.02-2.21 (m, 1H), 2.20-2.81 (m, 8H), 3.08-3.29 (m, 4H), 5.00-5.23 (m, 1H), 6.80-7.00 (m, 2H), 7.05-7.30 (m, 4H), 7.48 (d, 1H)

### Example 35A24

### 1-(5-Cyano-2-methoxyphenyl)-4-(1-cyclohexanecarbonyl-1,2,3,4-tetrahydrlquinoline-2-ylmethyl)-piperazine

From 4-(5-cyano-2-methoxyphenyl)-piperazine (WO 01/29022)
Flash chromatography: petroleum ether-EtOAc 50:50. Yield: 63%.
¹H-NMR (δ): 0.75-2.01 (m, 12H), 2.02-2.18 (m, 1H), 2.22-2.81 (m, 8H), 2.92-3.12 (m, 4H), 3.89 (s, 3H), 4.95-5.19 (m, 1H), 6.85 (d, 1H), 7.04-7.32 (m, 6H)

### Example 35A25

### 1-(1-Acetyl-4-indolyl)-4-(1-cyclohexanecarbonyl-1,2,3,4-tetrahydroquinoline-2-ylmethyl)-piperazine

From 1-(1-acetyl-4-indolyl)-piperazine
Flash chromatography: petroleum ether-EtOAc 50:50. Yield: 63.2%.
¹H-NMR (δ): 0.75-2.25 (m,12H), 2.25-2.90 (m, 9H), 3.00-3.40 (m, 4H), 4.90-5.30 (m, 1H), 6.45-6.65 (m, 2H), 6,95-7.30 (m, 7H).

The starting piperazine was prepared as follows:
a) 1-(1-*t*-Butoxycarbonyl)-4-(1-acetyl-4-indolyl)-piperazine (Compound 35A25-A)
   A suspension of 0.6 g of 1-(1-*t*-butoxycarbonyl)-4-(4-indolyl)-piperazine (WO 99/67237) and 0.24 g of 60% NaH oil dispersion in 6 ml of anhydrous DMF was stirred for 30 min. at r.t. under nitrogen stream then for 1 h at 55°C. Afterwards, a solution of 0.29 ml of acetyl chloride in 3 ml of DMF was dropped; stirring was continued for 6 h at 55°C. After cooling, the reaction mixture was diluted with 60 ml of H₂O, extracted with Et₂O (3x30 ml), washed and dried (Na₂SO₄) and evaporated to dryness in vacuo. The residue was purified by flash chromatography (Et₂O - petroleum ether 40:60) affording 0.55 g (79.3%) of the title compound.
   ¹H-NMR (δ): 2.63 (s, 3H), 3.18-3.40 (m, 8H), 6.73-6.90 (m 2H), 7.12-7.38 (m, 1H), 7.81 (d, 1H), 8.00 (dd, 1H), 9.40 (br, 2H), 9.80-11.00 (br, 1H).
b) 1-(1-Acetyl-4-indolyl)-piperazine (Compound 35A25-B)
   A solution of 0.34 g of Compound 35A25-A in 3 ml of MeOH and 15 ml of a 2N solution of HCl in Et₂O was kept overnight resting. The precipitated solid was filtered affording 0.29 g of the title compound as a dihydrochloride.
   ¹H-NI\lR (δ): 1.50 (s, 9H), 2.62 (s, 3H), 2.95-3.22, 3.50-3.80 (2m, 8H), 6.65 (d, 1H), 6.80 (dd, 1H), 7.18-7.33 (m, 1H), 7.40 (d, 1H), 8.14 (dd, 1H).

### Example 35A26

### 1-(1-Cyclohexanecarbonyl-1,2,3,4-tetrahydroquinoline-2-ylmethyl)-4-(7-indolyl)-piperazine

From 1-(7-indolyl)-piperazine (WO 94/15919)
Flash chromatography: petroleum ether-EtOAc 50:50. Yield: 72.4 %
¹H-NMR (δ): 0.75-2.25 (m, 12H), 2.25-2.90 (m, 9H), 2.90-3.20 (m, 4H), 4.905.30 (m, 1H), 6.48-6.68 (m, 1H), 6.80 (dd, 1H), 6,95-7.40 (m, 7H).

### Example 35A27

### 1-(3-Cyano-4-indolyl)-4-(1-cyclohexanecarbonyl-1,2,3,4-tetrahydroquinoline-2-ylmethyl)-piperazine

From 1-(3-cyano-4-indolyl)-piperazine (WO 99/67237)
Flash chromatography: petroleum ether-EtOAc 30:70. Yield: 35.19%.
¹H-NMR (δ): 0.80-2.20 (m,12H), 2.20-2.90 (m, 9H), 2.90-3.20 (m, 4H), 4.90-5.30 (m, 1H), 6.65-6.85 (m, 1H), 7.00-7.35 (m, 6H), 7.70(d, 1H), 8.82 (b, 1H).

### Example 35A28

### 1-(1-Cyclohexanecarbonyl-1,2,3,4-tetrahydroquinoline-2-ylmethyl)-4-(2,4-difluorobenzyl)-piperazine

From 1-(2,4-difluorobenzyl)-piperazine.
Flash chromatography: petroleum ether-EtOAc 70:30. Yield: 58.5%.
¹H-NMR (δ): 0.80-2.15 (m, 12H), 2.15-2.80 (m, 13H), 3.30 (s, 2H), 4.90-5.20 (m, 1H), 6.65-6.95 (m, 2H), 6.65-7.45 (m, 5H).

The starting piperazine was prepared as follows:
a) 1-(2,4-Difluorobenzyl)-piperazine (Compound 35A28-A)
   The title product was prepared following the general procedure described for benzyl piperazines in CA 2188484.
   ¹H-NMR (δ): 2.26-2.55 (m, 4H), 2.79-2.95 (m, 4H), 3.52 (s, 2H), 6.71-6.88 (m, 2H), 7.22 7.41 (m, 1H).

### Example 35A29

### 1-(2-Bromobenzyl)-4-(1-cyclohexanecarbonyl-1,2,3,4-tetrahydroquinoline-2-ylmethyl)-piperazine

From 1-(2-bromobenzyl)-piperazine.
Flash chromatography: petroleum ether - EtOAc 50:50. Yield:81.9%.
¹H-NMR (δ): 0.70-2.10 (m,12H), 2.10-3.00 (m, 13H), 3.40-3.80 (m, 2H), 4.90-5.20 (m, 1H), 6.90-7.40 (m, 6H), 7.40-7.65 (m, 2H)

The starting piperazine was prepared as follows:
a) 1-(2-Bromobenzyl)-piperazine (Compound 35A29-A)
   The title product was prepared following the general procedure described for benzyl piperazines in CA2188484.
   ¹H-NMR (δ): 2.41-2.55 (m, 4H), 2.62-2.98 (m, 4H), 3.64 (s, 2H), 6.71-6.88 (m, 2H), 7.13 (dd, 1H), 7.25 (dd, 1H), 7.41-7.56 (m, 2H).

### Example35A30

### 1-(1-Cyclohexanecarbonyl-1,2,3,4-tetrahydroquinoline-2-ylmethyl)-4-(2,5-difluorobenzyl)-piperazine

From 1-(2,5-difluorobenzyl)-piperazine (CA 2188484).
Eluent: Petroleum Ether-EtOAc 1:1. Yield: 52%.
¹H-NMR (δ):0.8-2.11 (m, 12 H), 2.12-2.83 (m, 13H), 3.52 (s, 2H), 4.95-5.12 (m, 1H), 6.80 7.00 (m, 2H), 7.01-7.32 (m, 5H).

### Example 35A31

### 1-(1-Cyclohexanecarbonyl-1,2,3,4-tetrahydroquinoline-2-ylmethyl)-4-(1-naphthyl)-piperazine

From 1-(1-naphthyl)-piperazine.
Flash chromatography: petroleum ether - EtOAc 7:3. Yield: 83.7%
¹H-NMR (δ): 0.81-2.15 (m, 11H), 2.10-2.29 (m, 1H), 2.32-2.91 (m, 9H), 3.00-3.28 (m, 4H), 4.98-5.25 (m, 1H), 7.01-7.32 (m, 5H), 7.34-7.59 (m; 4H), 7.71-7.92 (m, 1H), 8.08.8.27 (m, 1H).

The starting piperazine was prepared as follows:
a) 1-Benzyl-4-(1-naphthyl)-piperazine (Compound 35A31-A)
   A reaction flask was charged with cesium carbonate (11 g), BINAP (0.22 g) and palladium acetate (53 mg), under N₂ atmosphere; afterwards, was added 1-bromonaphthalene (5 g), 1-benzylpiperazine (5 ml) and toluene (70 ml) and the suspension was stirred at reflux for 20h. After cooling to r.t., the mixture was diluted with diethyl ether (20 ml), filtered through a Celite bed and concentrated *in vacuo.* The residue was purified by flash chromatography eluting with petroleum ether - ethyl acetate 9:1 to give 4.16 g (57%) of the title compound.
   ¹H-NMR (δ): 2.67-2.84 (m, 4H), 3.07-3.24 (m, 4H), 3.66 (s; 2H), 7.09 (dd, 1H), 7.24-7.61 (m, 9H), 7.76-7.88 (m, 1H), 8.12-8.30 (m, 1H)
b) 1-Naphthyl-piperazine (Compound 35A31-B)
   To a solution of Compound 35A31-A (4.16 g) in MeOH (250 ml) were added 10% Pd-C (0.65 g) and ammonium formate (6.12 g) and the mixture stirred at reflux under N₂ atmosphere for 5 h. After cooling to r.t., the catalyst was filtered off, the solvent was evaporated to dryness and the residue dissolved in CH₂Cl₂. The solution was washed with aqueous 5% NaHCO₃, dried (Na₂SO₄), filtered and evaporated at reduced pressure to give 2.5 g (85%) of the title compound.
   ¹H-NMR (δ): 1.71 (bs, 1H), 2.92-3.36 (m, 8H), 7.09 (dd, 1H), 7.38-7.58 (m, 4H), 7.74-7.92 (m, 1H), 8.12-8.31 (m, 1H).

### Example 35A32

### 1-(7-Bromo-4-indolyl)-4-(1-cyclohexanecarbonyl-1,2,3,4-tetrahydroquinoline-2-ylmethyl)-piperazine

From 1-(7-bromo-4-indolyl)-piperazine.
Flash chromatography: petroleum ether-EtOAc 50:50. Yield: 59%.
¹H-NMR (δ): 0.79-2.21 (m, 13H), 2.22-2.84 (m, 8H), 2.87-3.24 (m, 4H), 4.99-5.23 (m, 1H), 6.61 (d, 1H), 7.03-7.30 (m, 7H), 8.25-8.47 (m, 1H)

The starting piperazine was prepared as following:
a) 1-*t*-Butoxycarbonyl-4-(7-bromo-4-indolyl)-piperazine (Compound 35A32-A)
   To a solution of 1.53 g of 1-*t*-butoxycarbonyl-4-(4-indolyl)-piperazine in 20 ml of THF was added 0.89 g of NBS. The mixture was stirred for 6 h at r.t., then quenched with water, extracted with EtOAc (2x30ml). The organic layer was washed with water, dried (Na₂SO₄) and evaporated to dryness in vacuo. The residue was purified by flash chromatography (petroleum ether - EtOAc 8:2) to afford the title compound (0.71 g; 37%).
   ¹H-NMR (δ): 1.49 (s, 9H), 3.04-3.34 (m, 4H), 3.55-3.78 (m, 4H), 6.406.58 (m, 1H), 6.59-6.68 (m, 1H), 7.15-7.30 (m, 2H), 8.35 (bs, 1H).
b) 1-(7-Bromo-4-indolyl)-piperazine (Compound 35A32-B)
   The title compound was prepared following the same procedure described for Compound 35A25-B but starting from compound 35A32-A instead of Compound 35A25-A.
   The crude was used without further purification.
   ¹H-NMR (δ): 1.90 (bs, 1H), 3.01-3.24 (m, 8H), 355-3.78 (m, 4H), 6.49 (d, 1H), 6.61 (s, 1H), 7.12-7.30 (m, 2H), 8.35 (bs, 1H)

### Example 35A33

### 1-(1-Cyclohexanecarbonyl-1,2,3,4-tetrahydroquinoline-2-ylmethyl)-4-(3,4-dihydro-2H-benzo[b][1,4]dioxepin-6-yl)-piperazine

From 1-(3,4-dihydro-2*H*-1,5-benzodioxepin-6-yl)-piperazine (*J. Med Chem,* **31**, 1934-1940, (1988)).
Flash chromatography petroleum ether - ethyl acetate 7:3.
¹H-NMR (δ): 0.81-1.89 (m, 11H), 1.91-2.01 (m, 1H), 2.03-2.82 (m, 13H), 2.91-3.11 (m, 4H), 4.16-4.26 (m, 2H), 4.98-5.25 (m, 1H), 6.52-6.71 (m, 2H), 6.81 (t, 1H), 7.06-7.18 (m, 4H).

### Example 35A34

### 1-(2-Chlorobenzyl)-4-(1-cyclohexanecarbonyl-1,2,3,4-tetrahydroquinoline-2-ylmethyl)-piperazine

From 1-(2-chlorobenzyl)-piperazine (CA 2188484)
Eluent: Petroleum Ether-EtOAc 1:1. Yield: 64%.
¹H-NMR (δ): 0.9-2.98 (m, 25H), 3.52 (s, 2H), 4.95-5.12 (m, 1H), 6.807.11 (m, 8H).

### Example 35A35

### 1-(1-Cyclohexanecarbonyl-1,2,3,4-tetrahydroquinoline-2-ylmethyl)-4-(6-methoxy-2-pyridyl)-piperazine

From 1-(6-Methoxy-2-pyridyl)-piperazine (Reignier et al. *Arzneim.Forsch (Drug Res)* **24**, 12 (1974)).
Flash chromatography petroleum ether-ethyl acetate 7:3.
¹H-NMR (δ): 0.8-2.2 (m, 13H), 2.20-2.73 (m, 8H), 3.35-4.05 (m, 4H), 3.793.91 (s, 3H), 4.95-5.12 (m, 1H), 6.05-6.19 (m, 2H), 7.01-7.42 (m, 5H).

### Example 35A36

### 1-(1-Cyclohexanecarbonyl-1,2,3,4-tetrahydroquinoline-2-ylmethyl)-4-(2,5-dichlorobenzyl)-piperazine

From 1-(2,5-dichlorobenzyl)-piperazine (CA 2188484).
Flash chromatography petroleum ether-ethyl acetate 8:2.
¹H-NMR (δ): 0.8-2.20 (m, 13H), 2.30-2.85 (m, 12H), 3.52 (s, 2H), 4.95-5.12 (m, 1H), 7.05 7.30 (m, 6H), 7.45 (m, 1H).

### Example 36

### 1-(4-Indolyl)-4-(1-piperidinocarbonyl-1,2,3,4-tetrahydroquinoline-2-ylmethyl)-piperazine

The title compound was synthesised following the alternative acylation procedure described for the compound of Example 1 (step f) using 1-piperidinocarbonyl chloride instead of cyclohexanecarbonyl chloride and chloroform instead of dichloromethane, and refluxing for 8 hours in the presence of TEA The crude was purified by flash chromatography eluting with petroleum ether - ethyl acetate-2.5N methanolic ammonia 8:2:0.01 to give the title compound (10%).
¹H-NMR (δ): 1.31-2.48 (m, 9H), 2.53-2.91 (m, 7H), 3.08-3.41 (m, 10H), 4.29-4.48 (m, 1H), 6.47-6.63 (m, 2H), 6.80-7.00 (m, 2H), 7.01-7.21 (m, 5H), 8.10-8.28 (bs, 1H)

### Example 37

### 1-[1-(3-Cyanopropionyl)-1,2,3,4-tetrahydroquinoline-2-ylmethyl]-4-(4-indolyl)-piperazine

To a solution of 0.37 g of Compound 27 in 5 ml of 1-methyl-2-pyrrolidinone, was added 0.14 g of sodium cyanide and the mixture was stirred at 130°C for 3 hours, cooled at r.t., poured into H₂O and extracted with EtOAc. The organic layer was washed with water, dried (Na₂SO₄) and evaporated to dryness in vacuo. The residue was purified by flash chromatography (petroleum ether-EtOAc 2:8) to afford the title compound (0.14 g; 36%).
¹H-NMR (δ): 1.38-1.64 (m, 1H), 2.10-3.08 (m, 13H), 3.08-3.32 (m, 4H), 5.01-6.24 (m, 1H), 6.47-6.61 (m, 2H), 7.01-7.31 (m, 5H), 8.10-8.28 (bs, 1H)

### Example 38

### 1-(1-Cyclohexanecarbonyl-8-fluoro-1,2,3,4-tetrahydroquinoline-2-ylmethyl)-4-(4-indolyl)-piperazine

### a) 8-fluoro-2-methylquinoline (Compound 38A)

Compound 3 8A was obtained using the method described for Compound 25A, but using 2-fluoroaniline instead of 4-trifluoromethylaniline. The crude was purified by flash chromatography eluting with petroleum ether - ethyl acetate 7:3, affording 3.5g (80%) of the title compound.
¹H-NMR (δ): 2.81 (s, 3H), 7.28-7.46 (m, 3H), 7.50-7.62 (m, 1H), 8.08 (d, 1H)

### b) 2-Bromomethyl-8-fluoroquinoline (Compound 38B)

The title compound was prepared as described for Compound 5A, starting from Compound 38A instead of 6-fluoro-2-methylquinoline. The crude was purified by flash chromatography eluting with petroleum ether - ethyl acetate 9:1 to give, as a first eluted product, 1.9 g (36%) of the title compound and then 2.16 g of starting material.
¹H-NMR (δ): 4.78 (s, 2H), 7.37-7.72 (m, 4H), 8.22 (d, 1H).

### c) 8-Fluoro-2-[4-(4-indolyl)-1-piperazinylmethyl]-quinoline (Compound 38C)

Compound 38C was obtained in the manner described for Compound 1C (procedure d) but using Compound 38B instead of 2-chloromethylquinoline. The crude was purified by flash chromatography eluting with petroleum ether - ethyl acetate 1:1 to give the title compound (75%).
¹H-NMR (δ): 2.74-2.95 (m, 4H), 3.21-3.42 (m, 4H), 4.02 (s, 2H), 6.49-6.68 (m, 2H), 7.00-7.19 (m, 3H), 7.34-7.52 (m, 2H), 7.61 (d, 1H), 7.80 (d, 1H), 8.12-8.29 (m, 2H).

### d) 1-(8-Fluoro-1,2,3,4-tetrahydroquinoline-2-ylmethyl)-4-(4-indolyl)-piperazine (Compound 38D)

The title compound was prepared as described for Compound 1B (procedure e) starting from Compound 38C instead of Compound 1C. The crude was purified by flash chromatography eluting with petroleum ether - ethyl acetate 1:1 to give the title compound (57%).
¹H-NMR (δ): 1.49-1.75 (m, 1H), 1.87 2.08 (m, 1H), 2.51 (d, 2H), 2.59-3.02 (m, 6H), 3.21-3.60 (m, 5H), 3.73-3.95 (br, 1H), 6.41-6.80 (m, 5H), 7.04-7.22 (m, 3H), 8.088.28 (br, 1H).

### e) 1-(1-Cyclohexanecarbonyl-8-fluoro-1,2,3,4-tetrabydroquinoline-2-ylmethyl)-4-(4-indolyl)-piperazine

The title compound was synthesised following the alternative acylation procedure described for the compound of Example 1 (step f) but starting from Compound 38D instead of Compound 1B. The crude was purified by flash chromatography eluting with toluene - acetone 8:2 affording the title compound (70%).
¹H-NMR (δ): 0.82-1.99 (m, 11H), 2.03-2.25 (m, 1H), 2.28-2.91 (m, 9H), 3.08-3.44 (m, 4H), 4.96-5.22 (m, 1H), 6.45-6.77 (m, 2H), 6.96-7.29 (m, 6H), 8.05-8.26 (br, 1H).

### Example 39

### 1-[1-(3-Acetylaminopropionyl)-1,2,3,4-tetrahydroquinoline-2-ylmethyl]-4-(4-indolyl)-piperazine

A mixture of 0.16 g of the compound of Example 28, 0.058 ml of TEA, 0.03 ml of acetyl chloride and 3.8 ml of CH₂Cl₂ was stirred at 20 to 25°C under nitrogen atmosphere for 2 hour. The solution was washed with a 0.5 N NaOH (1 x 10 ml) and 15 ml of water. The organic layer, dried on anhydrous sodium sulphate, was evaporated to dryness and the residue purified by flash chromatography (EtOAc - 2 N NH₃ sol. in methanol 93:7) to give 0.13 g (75%) of the title compound and the compound of Example 41 (0.027g).
¹H-NMR (δ): 1.40-1.65 (m, 1H), 1.95 (s, 3H), 2.10-2.95 (m, 11H), 3.10-3.30 (m, 4H), 3.30 3.370 (m, 2H), 5.10 (b, 1H), 6.40-6.60 (m, 3H), 7.00-7.25 (m, 7H), 8.20 (s, 1H).

### Example 40

### 1-[1-(3-Carbamoylaminopropionyl)-1,2,3,4-tetrahydroquinoline-2-ylmethyl]-4-(4 indolyl)-piperazine

A mixture of 0.17 g of the compound of Example 28, 0.067 ml of trimethylsilylisocyanate and 4 ml of THF was stirred at 20 to 25°C under nitrogen atmosphere for 2 hours. After overnight resting, the solution was taken up with a 1 N NaOH (1 x 10 ml) and extracted with EtOAc (2x10 ml) which was washed with 10 ml of water. The organic layer, dried on anhydrous sodium sulphate, was evaporated to dryness and the residue purified by flash chromatography (EtOAc - 2 N NH₃ sol. in methanol 92:8) to give 0.13 g (68 %) of the title compound.
¹H-NMR (δ): 1.35-1.65 (m, 1H), 2.10-2.90 (m, 11H), 3.10-3.25 (m, 4H), 3.25-3.65 (m, 2H), 4.35 (b, 1H), 5.10 (b, 1H), 5.35 (b, 1H), 6.45-6.65 (m, 2H), 7.00-7.30 (m, 7H), 8.15 (s, 1H).

### Example 41

### 1-{1-[3-bis(Acetylamino)-propionyl]-1,2,3,4-tetrahydroquinoline-2-ylmethyl}-4-(4-indolyl)-piperazine

The title product was obtained during the synthesis of the compound of Example 39.
¹H-NMR (δ): 1.35-1.65 (m, 1H), 2.05-3.00 (m, 18H), 3.10-3.30 (m, 4H), 3.804.20 (m, 2H), 5.10 (b, 1H), 6.45-6.60 (m, 2H), 7.00-7.30 (m, 7H), 8.15 (s, 1H).

### Example 42

### 1-(6-Chloro-1-cyclohexanecarbonyl-1,2,3,4-tetrahydroquinoline-2-ylmethyl)-4-(4-indolyl)-piperazine

### a) 6-Chloro-2-hydroxymethyl-1,2,3,4-tetrahydroquinoline (Compound 42A)

A mixture of 1.24 g of 6-chloro-1,2,3,4-tetrahydroquinoline-2-carboxylic acid, 30 ml of anhydrous THF and 6 ml of 10 M BH₃.Me₂S in THF was stirred at r.t. for 3 h under nitrogen. After overnight standing at r.t., 5 ml of MeOH was dropped into the mixture, and after 15' was added 5 ml of 2N HCl, The stirred mixture was heated at 60°C for 1 h, then evaporated to dryness *in vacuo.* The residue was treated with 2 N NaOH (30 ml) and extracted with CHCl₃ (2x40 ml). The organic layer was dried (anhydrous Na₂SO₄) and evaporated to dryness *in vacuo*; the residue was purified by flash chromatography (CHCl₃-MeOH 100:1) to afford 0.35 g (35.3%) of Compound 42A as a thick oil.
¹H-NMR (δ): 1.50-2.10 (m, 3H), 2.55-2.95 (m, 2H), 3.30-3.50 (m, 1H), 3.50-3.85 (m, 2H), 3.85-4.70 (b, 1H), 6.45 (d, 1H), 6.80-7.05 (m, 2H)

### b) 6-Chloro-2-iodomethyl-1,2,3,4-tetrahydroquinoline (Compound 42B)

The title compound was synthesized following the procedure described for compound 34C but using as a starting material compound 42A instead of compound 34B.
¹H-NMR (δ): 1.63-2.15 (2m, 2H), 2.55-2.90 (m, 2H), 3.05-3.38 (2m, 2H), 3.38-3.55 (m, 1H), 3.85-4.40 (b, 1H), 6.45 (d, 1H), 6.90-7.00 (m, 2H)

### c) 6-Chloro-2-[4-(4-indolyl)-1-piperazinyl]methyl]-1,2,3,4-tetrahydroquinoline (Compound 42C)

The title compound was synthesized following the procedure described for compound 34D but using as a starting material compound 42B instead of compound 34C.

The residue was purified by flash chromatography (petroleum ether - EtOAc 70:30) to afford Compound 42B (55.8%) as a yellow oil.
¹H-NMR (δ): 1.40-1.75 (m, 1H),1.80-2.05 (m, 1H), 2.35-3.00 (m, 8H), 3.10-3.65 (m, 5H), 4.50-4.95 (b, 1H), 6.35 (d, 1H), 6.50-6.70 (m, 2H), 6.85-7.00 (m, 2H), 7.057.25 (m, 3H), 8.05-8.30 (b, 1H)

### d) 1-(6-Chloro-1-cyclohexanecarbonyl-1,2,3,4-tetrahydroquinoline-2-ylmethyl)-4-(4-indolyl)piperazine

The title compound was synthesised starting from Compound 42C following the procedure described for the of Example 1 (step f). The residue was purified by flash chromatography eluting with petroleum ether - EtOAc gradient from 60 : 40 to 40:60 affording the title compond (71.8%).
¹H-NMR (δ): 0.85-2.00 (m, 12H), 2.00-2.25 (m, 1H), 2.25-2.85 (m, 8H), 3.00-3.40 (m, 4H), 4.85-5.30 (m, 1H), 6.45-6.65 (m, 2H), 6.95-7.25 (m, 6H), 8.15 (b, 1H).

### Example 43

### (R)-1-(1-Cyclohexanecarbonyl-1,2,3,4-tetrahydroquinoline-2-ylmethyl)-4-(1-methyl-4-indolyl)-piperazine

The title compound was prepared as described in Example 15 but using the compound of Example (+)-1 instead of Example 1. Yield: 30%.
¹H-NMR (δ): 0.80-1.99 (m, 11H), 2.05-2.24 (m, 1H), 2.26-2.85 (m, 9H), 3.10-3.35 (m, 4H), 3.76 (s 3H), 5.00-5.25 (m, 1H), 6.45-6.60 (m, 2H), 7.00-7.30 (m, 7H).

### Example 44

### (Z)-1-[1-(4-Hydroxycyclohexanecarbonyl)-1,2,3,4-tetrahydroquinoline-2-ylmethyl]-4-(4-indolyl)-piperazine

### a) (Z)-1-[1-(4-Diphenyltertbutylsilyloxycyclohexanecarbonyl)-1,2,3,4-tetrahydroquinoline-2-ylmethyl]-4-(4-indolyl)-piperazine (Compound 44A)

The title compound was synthesised following the acylation procedure described for the compound of Example 1 (step f) starting from Compound 1B but using cis-4-diphenyltertbutylsilyloxycyclohexanecarbonyl chloride (prepared as described in EP 0352909) instead of cyclohexanecarbonyl chloride. Flash chromatography eluent: petroleum ether - EtOAc 6:4. Yield: 87%.
¹H-NMR (δ): 0.86-1.86 (m, 18H), 1.95-2.78 (m, 10H), 3.11-3.41 (m, 4H), 3.82-4.01 (m, 1H), 5.05-5.28 (m, 1H), 6.48-6.62 (m, 2H), 7.00-7.46 (m, 11H), 7.53-7.71 (m, 6H), 8.02-8.25 (bs 1H).

### b) (Z)-1-[1-(4-Hydroxycyclohexanecarbonyl)-1,2,3,4-tetrahydroquinoline-2-ylmethyl]-4-(4-indolyl)-piperazine

To a solution of compound 44A (0.2 g) in 20 ml of THF was added tetrabutylammonium fluoride and the resulting suspension stirred at r.t for 3 days. Afterwards the solvent was evaporated under vacuum ; the residue was dissolved in CH₂Cl₂ (40 ml) and washed with H₂O (30 ml). The organic layer was dried (Na₂SO₄) and the crude was purified by flash chromatography (CH₂Cl₂ - MeOH 95:5) to give the title compound (63%).
¹H-NMR (δ): 1.10-1.99 (m, 9H), 2.01-2.22 (m, 2H), 2.33-2.88 (m, 9H), 3.12-3.33 (m, 4H), 3.82-4.01 (m, 1H), 5.05-5.29 (m, 1H), 6.48-6.68 (m, 2H), 7.07-7.29 (m, 7H),8.03-.8.29 (bs 1H).

### Example 45

### (E)-1-[1-(4-Hydroxycyclohexanecarbonyl)-1,2,3,4-tetrahydroquinoline-2-ylmethyl]-4-(4-indolyl)-piperazine

### a) (E)-1-[1-(4-Diphenyltertbutylsilyloxycyclohexanecarbonyl)-1,2,3,4-tetrahydroquinoline-2-ylmethyl]-4-(4-indolyl)-piperazine (Compound 45A)

The title compound was synthesised following the same method described for compound 44A but using trans-4-diphenyltertbutylsilyloxycyclohexanecarbonyl chloride (prepared as described in EP 0352909) instead of cis-4-diphenyltertbutylsilyloxycyclohexanecarbonyl chloride. Flash chromatography eluent: petroleum ether - EtOAc 6:4. Yield: 87%.
¹H-NMR (δ): 0.89-1.87 (m, 18H), 1.93-2.75 (m, 10H), 3.14-3.47 (m, 4H), 3.51-3.71 (m, 1H), 5.05-5.28 (m, 1H), 6.50-6.60 (m, 2H), 7.02-7.48 (m, 11H), 7.52-7.70 (m, 6H), 8.03-8.24 (br, 1H).

### b) (E)-1-[1-(4-Hydroxycyclohexanecarbonyl)-1,2,3,4-tetrahydroquinoline-2-ylmethyl]-4-(4-indolyl)-piperazine

The title compound was synthesised following the procedure described for the compound 44 but starting from compound 45A (44%).
¹H-NMR (δ): 1.00-2.05 (m, 9H), 2.07-2.27 (m, 2H), 2.38.-2.88 (m, 9H), 3.15-3.38 (m, 4H), 3.50-3.71 (m, 1H), 5.06-5.32 (m, 1H), 6.51-6.70 (m, 2H), 7.07-7.31 (m, 7H), 8.05-.8.32 (br, 1H).

### Example 46

### 1-(1-Cyclohexanecarbonyl-7-fluoro-1,2,3,4-tetrahydroquinoline-2-ylmethyl)-4-(4-indolyl)-piperazine

### a) 7-Fluoro-2-methylquinoline (Compound 46A)

Compound 46A was obtained using the method described for Compound 25A, but using 3-fluoroaniline instead of 4-trifluoromethylaniline. The crude was purified by flash chromatography eluting with petroleum ether - ethyl acetate 75:25, affording 2.6g (61.1%) of the title compound.
¹H-NMR (δ): 2.74 (s, 3H), 7.17-7.36 (m, 2H), 7.67 (dd, 1H), 7.71-7.84 (m, 1H), 8.06 (d, 1H).

### b) 2-Bromomethyl-7-fluoroquinoline (Compound 46B)

The title compound was prepared as described for Compound 5A, starting from Compound 46A instead of 6-fluoro-2-methylquinoline. The crude was purified by flash chromatography eluting with petroleum ether - ethyl acetate 85:15 to give, as a first eluted product, 1.95 g (61%) of the title compound and then 1.14 g of starting material 46A.
¹H-NMR (δ): 4.71 (s, 2H), 7.32-7.43 (m, 1H), 7.56 (d, 1H), 7.74 (dd, 1H), 7.76-7.87 (m, 1H), 8.18 (d, 1H)

### c) 7-Fluoro-2-[4-(4-indolyl)-1-piperazinylmethyl]-quinoline (Compound 46C)

Compound 46C was obtained in the same way as described for Compound 1C (procedure d) but using Compound 46B instead of 2-chloromethylquinoline. The crude was purified by flash chromatography eluting with petroleum ether - ethyl acetate 6:4 to give the title compound (41 %).
¹H-NMR (δ): 2.68-2.96 (m, 4H), 3.22-3.39 (m, 4H), 3.92 (s, 2H), 6.48-6.66 (m, 2H), 7.02-7.22 (m, 3H), 7.24-7.39 (m, 1H), 7.62-7.88 (m, 3H), 8.09-8.31 (m, 2H).

### d) 1-(7-Fluoro-1,2,3,4-tetrahydroquinoline-2-ylmethyl)-4-(4-indolyl)-piperazine (Compound 46D)

The title compound was prepared as described for Compound 1B (procedure e) starting from Compound 46C instead of Compound 1C. The crude was purified by flash chromatography eluting with petroleum ether - ethyl acetate 7:3 to give the title compound (45%).
¹H-NMR (δ): 1.41-1.68 (m, 1H), 1.86-2.01 (m, 1H), 2.43-2.98 (m, 8H), 3.14-3.57 (m, 5H), 4.51-4.89 (br, 1H), 6.12-6.36 (m, 2H), 6.48-6.68 (m, 2H), 6.81-6.90 (m, 1H), 7.04-7.19 (m, 3H), 8.08-8.23 (br, 1H).

### e) 1-(1-Cyclohexanecarbonyl-7-fluoro-1,2,3,4-tetrahydroquinoline-2-ylmethyl)-4-(4-indolyl)-piperazine

The title compound was synthesised following the alternative acylation procedure described for the compound of Example 1 (step f) but starting from Compound 46D instead of Compound 1B. The crude was purified by flash chromatography eluting with petroleum ether-ethyl acetate 7:3 affording the title compound (57%).
¹H-NMR (δ): 0.91-2.01 (m, 11H), 2.03-2.26 (m, 1H), 2.29-2.89 (m, 9H), 3.11-3.40 (m, 4H), 4.91-5.25 (m, 1H), 6.48-6.68 (m, 2H), 6.82-7.24 (m, 6H), 8.03-8.22 (br, 1H).

### Example 47

### 1-(1-Cyclohexanecarbonyl-6-phenyl-1,2,3,4-tetrahydroquinoline-2-ylmethyl)-4-(4-indolyl)-piperazine

To a solution of 0.053 g of the compound of Example 34 in 2.5 ml of anhydrous 1,2-DME was added 0.008 g of tetrakis(triphenylphosphine)-palladium(0), 0.018g of 97% phenylboric acid and 0.7 ml of K₂CO₃ aq. saturated solution. The mixture was stirred for 12 h under nitrogen and then diluted with H₂O (20 ml) and extracted with EtOAc (3×10 ml). The organic layer was washed with brine, dried (anhydrous Na₂SO₄) and evaporated to dryness *in vacuo.* The residue was purified by flash chromatography (petroleum ether - EtOAc 1 : 1) affording 0.010 g (10%) of the title compound as a thick oil.
¹H-NMR (δ): 0.70-2.05 (m, 11H), 2.05-2.30 (m, 1H), 2.30-2.90(m, 9H), 3.05-3.35 (m, 4H), 4.90-5.35 (m, 1H), 6.45-6.70 (m, 2H), 7.00-7.25 (m, 4H), 7.30-7. 55 (m, 5H), 7.557.70 (m, 2H), 8.15 (b, 1H).

### Example 48

### 1-(1-Cyclohexanecarbonyl-2,3-dihydroindole-2-ylmethyl)-4-(4-indolyl)-piperazine

### a) 2-[4-(4-Indolyl)-1-piperazinylcarbonyl]-indoline (Compound 48A)

The title compound was synthesised following the procedure described for Compound 1D (step f) but using 2-indolinecarboxylic acid instead of 1,2,3,4-tetrahydroquinoline-2-carboxylic acid. The crude solid residue was stirred with Et₂O (30 ml) for 0,5 h at r.t. to afford 0.65 g (93,2%) of the title compound as an ivory solid.
¹H-NMR (δ): 3.05-3.45 (m, 5H), 3.45-3.65 (m, 1H), 3.65-4.00 (m, 8H), 3.054.40 (b, 1H), 4.60-4.80 (m, 1H), 6.50-6.70 (m, 2H), 6.70-6.95 (m, 2H), 6.95-7.25 (m, 5H), 8.20-8.50 (b, 1H).

### b) 1-(2,3-Dihydroindole-2-ylmethyl)-4-(4-indolyl)-piperazine (Compound 48B)

The title compound was synthesised following the procedure described for Compound 1B (step h) but using compound 48A instead of Compound 1D.

The residue was purified by flash chromatography (CHCl₃ - 2 N methanolic ammonia 100 : 1) affording the title compound (11.8%) as a thick oil.
¹H-NMR (δ): 2.30-2.80 (m, 4H), 2.80-3.10 (m, 1H), 3.10-3.50 (m, 7H), 3.90-4.15 (m, 1H), 4.20-5.00 (b, 1H), 6.50-6.80 (m, 4H), 6.90-7.20 (m, 5H), 8.10 (b, 1H)

### c) 1-(1-Cyclohexanecarbonyl-2,3-dihydroindole-2-ylmethyl)-4-(4-indolyl)-piperazine

The title compound was synthesised following the alternative acylation procedure described for the compound of Example 1 (step f) but starting from Compound 48B instead of Compound 1B. The crude was purified by flash chromatography eluting with petroleum ether-ethyl acetate 6:4 affording the title compound (53%).
¹H-NMR (δ):1.00-2.05 (m, 10H), 2.10-2.32 (m, 1H), 2.32-2.95 (m, 5H), 2.95-3.45 (m,7H), 4.40-4.70 (m, 1H), 6.48-6.70 (m, 2H), 6.95-7.30 (m, 7H), 8.15 (b, 1H)

### Example 49

### 1-(1-Cyclohexanecarbonyl-8-methoxy-1,2,3,4-tetrahydroquinoline-2-ylmethyl)-4-(4-indolyl)-piperazine

### a) 1-(8-Hydroxyquinoline-2-ylmethyl)-4-(4-indolyl)-piperazine (Compound 49A)

The title compound was synthesised following the reductive amination procedure described for Compound 35B, but using 2-formyl-8-hydroxyquinoline (commercial) instead of compound 35A. The residue was purified by flash chromatography (petroleum ether - EtOAc- 2.5 N methanolic ammonia 1 : 9: 0.2) affording Compound 49A (74%).
¹H-NMR (δ): 2.71-2.93 (m, 4H), 3.23-3.41 (m, 4H), 3.88-4.03 (m, 2H), 6.50-6.63 (m, 2H), 7.0-7.49 (m, 6H), 7.71 (d, 1H), 8.15 (d, 1H), 8.10-8.45 (bs, 1H)

### b) 1-(8-Methoxyquinolin-2-ylmethyl)-4-(4-indolyl)-piperazine (Compound 49B)

To a mixture of 0.08 g of freshly prepared sodium methoxide in 10 ml of anhydrous THF was added 0.50 g of compound 49A and the resulting solution was stirred for 0.5 hours at r.t.. 0.19 ml of iodomethane was added and the solution was stirred at reflux for 5 hours, cooled to r.t., poured into H₂O and extracted with EtOAc. The organic layer was washed with water, dried (Na₂SO₄) and evaporated to dryness in vacuo to afford the title compound (73%).
¹H-NMR (δ): 2.73-2.90 (m, 4H), 3.21-3.38 (m, 4H), 4.00-4.14 (m, 2H), 6.50-6.65 (m, 2H), 7.00-7.14 (m, 4H), 7.35-7.48 (m, 2H), 7.83 (s, 1H), 8.12 (s, 1H), 8.22-8.34 (bs, 1H)

### c) 1-(8-Methoxy-1,2,3,4-tetrahydroquinoline-2-ylmethyl)-4-(4-indolyl)-piperazine (Compound 49C)

The title compound was synthesised as reported in Example 1 (step e) but using compound 49B instead of Compound 1C. The residue was purified by flash chromatography (petroleum ether - EtOAc- 3:7) affording the title compound (42%).
¹H-NMR (δ): 1.50-1.74 (m, 1H), 1.88-2.05 (m, 1H), 2.42-3.05 (m, 8H), 3.21-3.58 (m, 5H), 3.82 (s, 3H), 5.00 (bs, 1H), 6.50-6.70 (m, 5H), 7.02-7.19 (m, 3H), 8.18 (bis, 1H)

### d) 1-(1-Cyclohexanecarbonyl-8-methoxy-1,2,3,4-tetrahydroquinoline-2-ylmethyl)-4-(4-indolyl)-piperazine

The title compound was synthesised following the alternative acylation procedure described for the compound of Example 1 (step f) but starting from Compound 49C instead of Compound 1B. The crude was purified by flash chromatography eluting with petroleum ether-ethyl acetate 7:3 affording the title compound (57%).
¹H-NMR (δ): 0.75-1.99 (m, 15H), 2.12-3.60 (m, 14H), 4.99-5.24 (m, 1H), 6.43-6.61 (m, 2H), 6.39-6.91 (m, 2H), 7.02-7.21 (m, 4H), 8.20 (bs, 1H)

### Example 50

### 1-(1-Cyclohexanecarbonyl-6-hydroxy-1,2,3,4-tetrahydroquinoline-2-ylmethyl)-4-(4-indolyl)-piperazine

### a) 2-Methyl-6-hydroxyquinoline (Compound 50A)

A suspension of 2-methyl-6-methoxyquinoline (1g) in 48% HBr (20 ml) was refluxed for 8 h. Afterwards, the cooled mixture was poured into 100 ml of water, alkalinised with 32% NaOH and extracted with ethyl acetate (2 x 20 ml). The organic layer was dried on anhydrous sodium sulphate and evaporated to dryness giving 0.83 g of the title compound which was used for the next step without further purification (91%).
¹H-NMR (δ): 1.31-2.05 (br, 1H), 2.75 (s, 3H), 7.02-7.30 (m, 3H), 7.81-7.98 (m, 2H).

### b) 6-tert-Butoxycarbonyloxy-2-methyl-quinoline (Compound 50B)

A solution of Compound 50A (0.83g) and dimethylaminopyridine (0.64g) in THF (40ml) was cooled at 0-5°C; then di-tert-butyldicarbonate (1.2g) was added and the solution stirred at r.t. for 6h. Afterwards, the solvent was evaporated and the crude purified by flash chromatography eluting with petroleum ether-EtOAc 6:4 affording 0.82 g (63%) of the title compound.
¹H-NMR (δ): 1.55 (s, 9H), 2.78 (s, 3H), 7.22-7.34 (m, 1H), 7.51-7.62 (m, 2H), 7.95-8.07 (m 2H).

### c) 2-Bromomethyl-6-tert-butoxycarbonyloxyquinoline (Compound 50C)

The title compound was prepared as described for Compound 5A starting from Compound 50B (0.82 g) instead of 6-fluoro-2-methylquinoline. The crude was purified by flash chromatography eluting with petroleum ether-EtOAc 8:2 to give 0.57g of the title compound (54%).
¹H-NMR (δ): 1.58 (s, 9H), 5.78 (s, 2H), 7.50-7.69 (m, 3H), 8.03-8.19 (m, 2H)

### d) 6-tert-Butoxycarbonyloxy-2-[4-(4-indolyl)-1-piperazinylmethyl]-quinoline (Compound 50D)

This compound was obtained in the same way as described for Compound 1C procedure (d) but using Compound 50C instead of 2-chloromethylquinoline. The crude was purified by flash chromatography eluting with methylene chloride-methanol 95:5 to give the title compound (37%).
¹H-NMR (δ): 1.36-1.71 (m, 9H), 2.71-3.00 (m, 4H), 3.23-3.42 (m, 4H), 3.86-4.02 (m, 2H), 6.49-6.66 (m, 2H), 7.00-7.21 (m, 3H), 7.48-7.71 (m, 3H), 7.98-8.36 (m, 3H).

### e) 1-(6-tert-Butoxycarbonyloxy-1,2,3,4-tetrahydroquinoline-2-ylmethyl)-4-(4-indolyl)-piperazine Compound 50E)

The title compound was prepared as described for Compound 1B (procedure e) starting from Compound 50D instead of Compound 1C. The crude was purified by flash chromatography eluting with dichloromethane-methanol 97:3 to give the title compound (52%).
¹H-NMR (δ): 1.48-1.73 (m, 11H), 1.81-1.98 (m, 1H), 2.42-3.01 (m, 8H), 3.17-3.52 (m, 5H), 6.41-6.53 (m, 3H), 6.65-6.78 (m, 2H), 7.02-7.18 (m, 3H), 8.09-8.24 (br, 1H)

### f) 1-(1-Cyclohexanecarbonyl-6-tert-butoxycarbonyloxy-1,2,3,4-tetrahydroquinoline-2-ylmethyl-4-(4-indolyl)-piperazine (Compound 50F)

The title compound was synthesised following the alternative acylation procedure described for the compound of Example 1 (step f) but starting from Compound 50E instead of Compound 1B. The crude was purified by flash chromatography eluting with petroleum ether-EtOAc 1:1 to give the title compound (47%).
¹H-NMR (δ): 0.81-2.00 (m, 20H), 2.01-2.23 (m, 1H), 2.24-2.82 (m, 9H), 3.07-3.34 (m, 4H), 4.98-5.27 (m, 1H), 6.42-6.61 (m, 2H), 6.92 7.15 (m, 6H), 8.08-8.27 (br, 1H).

### g) 1-(1-Cyclohexanecarbonyl-6-hydroxy-1,2,3,4-tetrahydroquinoline-2-ylmethyl)-4-(4-indolyl)-piperazine

To a cooled solution of Compound 50F (86 mg) in CH₂Cl₂ (4 ml), 0.5 ml of 3.6 N HCl in diethyl ether was added and the resulting mixture heated at reflux for 5h. Then the solvent was removed under vacuum, the residue alkalinised with NaHCO₃ and extracted with CH₂Cl₂ (2x 20 ml). The combined organic layers were dried over Na₂SO₄ and evaporated to dryness. The crude was purified by flash chromatography eluting with CH₂Cl₂-2N NH₃ in MeOH 95:5 to give the title compound (57%).
¹H-NMR (δ): 0.81-1.98 (m, 12H), 1.99-2.20 (m, 1H), 2.22-2.51 (m, 4H),2.52-2.78 (m, 5H), 3.10-3.31 (m, 4H), 5.05-5.25 (m, 1H), 6.45-6.65 (m, 2H), 6.676.75 (m, 2H), 6.92-7.11 (m, 4H), 8.05-8.21 (br, 1H).

### Example 51

### 1-(6-Bromo-1-cyclohexanecarbonyl-1-1,2,3,4-tetrahydroquinoline-2-ylmethyl)-4-(4-fluoro-2-methoxyphenyl)-piperazine

### a) 6-Bromo-2-[4-(4-fluoro-2-methoxyphenyl)-1-piperazinylmethyl]-1,2,3,4-tetrahydroquinoline (Compound 51A)

The title compound was synthesized following the procedure described for compound 34D but using 1-(4-fluoro-2-methoxyphenyl)-piperazine as a starting material instead of 1-(4-indolyl)-piperazine.

The residue was purified by flash chromatography (petroleum ether - EtOAc 70:30) to afford the title compound (44.4%) as a thick oil.
¹H-NMR (δ): 1.55 (s, 1H), 1.80-2.00 (m, 1H), 2.30-4.00 (m, 17H), 6.50-6.80 (m, 3H), 6.80-7.00 (m, 1H), 7.00-7.20 (m, 2H).

### b) 1-(6-Bromo-1-cyclohexanecarbonyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 51 A instead of Compound 1B. The residue was purified by flash chromatography (petroleum ether - EtOAc 80 : 20) affording the title compound (69%) as a thick oil.
¹H-NMR (δ): 0.85-2.00 (m, 11H), 2.00-2.80 (m, 8H), 2.80-3.70 (m, 6H), 3.90 (s, 3H), 4.80 5.20 (m, 1H), 6.45-6.65 (m, 2H), 6.70-7.15 (m, 2H), 7.307.50 (m, 2H),

### Example 52

### 1-(1-Cyclohexanecarbonyl-8-hydroxy-1,2,3,4-tetrahydroquinoline-2-ylmethyl)-4-(4-indolyl)-piperazine

### a) 1-(8-Hydroxy-1,2,3,4-tetrahydroquinoline-2-ylmethyl)-4-(4-indolyl)-piperazine (Compound 52A)

The title compound was synthesised as reported in Example 1 (step e) but using compound 49A instead of Compound 1C. The residue was purified by flash chromatography (petroleum ether - EtOAc 1 : 1) affording 0.15 g (30%) of the title compound.
¹H-NMR (δ): 1.50-2.05 (m, 3H), 2.41-3.09 (m, 8H), 3.12-3.70 (m, 6H), 6.30-6.82 (m, 4H), 7.02-7.24 (m, 4H), 8.15 (bs, 1H).

### b) 1-(8-Cyclohexanecarbonyloxy-1-cyclohexanecarbonyl-1,2,3,4-tetrahydroquinoline-2-ylmethyl)-4-(4-indolyl)-piperazine (Compound 52B)

A solution of 0.14 g of compound 52A, 0.20 ml of cyclohexanecarbonyl chloride and 0.41 ml of triethylamine in 5 ml of toluene was stirred at reflux for 0.5 hours, cooled to r.t.. The mixture was extracted with EtOAc. The organic layer was washed with 1 N NaOH, with H₂O, dried (Na₂SO₄) and evaporated to dryness in vacuo. The crude was purified by flash chromatography (petroleum ether - EtOAc 6:4) to afford 0.17 g (77%) of the title compound.
¹H-NMR (δ): 0.78-2.05 (m, 24H), 2.05-2.90 (m, 8H), 2.97-3.38 (m, 4H), 4.94-5.07 (m, 1H), 6.45-6.62 (m, 2H), 6.85-7.24 (m, 6H), 8.15 (bs, 1H).

### c) 1-(1-Cyclohexanecarbonyl-8-hydroxy-1,2,3,4-tetrahydroquinoline-2-ylmethyl)-4-(4-indolyl)-piperazine

A mixture of 0.17 g of compound 52B, 0.075 g of lithium hydroxide monohydrate in 3 ml of THF and 0.2 ml of H₂O was stirred at r.t. for 12 hours, diluted with H₂O and extracted with EtOAc. The organic layer was washed with water, dried (Na₂SO₄) and evaporated to dryness in vacuo. The crude was purified by flash chromatography (petroleum ether- EtOAc 6 : 4) to afford 0.32 g (65%) of the title compound.
¹H-NMR (δ): 0.80-1.95 (m, 14H), 2.15-2.58 (m, 6H), 2.91-3.48 (m, 6H), 5.38-5.57 (m, 1H), 6.45-6.62 (m, 2H), 6.76 (d, 1H), 6.90 (d, 1H), 7.02-7.20 (m, 4H), 8.15 (bs, 1H).

### Example 53

### 1-(1-Cyclohexanecarbonyl-6-methyl-1,2,3,4-tetrahydroquinoline-2-ylmethyl)-4-(4-fluoro-2-methoxyphenyl)-piperazine

### a) 1-(4-Fluoro-2-methoxyphenyl)-4-(6-methyl-1,2,3,4-tetrahydroquinoline-2-ylcarbonyl)-piperazine (Compound 53A)

The title compound was synthesised following the procedure described for Compound 1D (step g) but using Compound 32B instead of 1,2,3,4-tetrahydroquinoline-2-carboxylic acid and 1-(4-fluoro-2-methoxyphenyl)-piperazine instead of 1-(4-indolyl)-piperazine. The residue was purified by flash chromatography eluting with petroleum ether- EtOAc 6:4 to give of the title compound (69 %).
¹H-NMR (δ): 1.60-1.81 (m, 1H), 2.04-2.10 (m, 1H), 2.11 (s; 3H), 2.66-2.89 (m, 2H), 2.93-3.17 (m, 4H), 3.61-3.98 (m,7H), 4.02-4.26 (m, 1H), 4.28-4.42 (br, 1H), 6.51-6.70 (m, 3H), 6.71-6.94 (m, 3H).

### b) 1-(4-Fluoro-2-methoxy)-4-(6-methyl-1,2,3,4-tetrahydroquinoline-2-ylmethyl)-piperazine (Compound 53B)

The title compound was synthesised following the procedure described for Compound 1B (step h) but using Compound 53A instead of Compound 1D. The residue was purified by flash chromatography eluting with petroleum ether - EtOAc 7:3 to give the title compound (86.5%).
¹H-NMR (δ): 1.45-1.67 (m, 1H), 1.80-1.97 (m, 1H), 2.21 (s, 3H), 2.46 (d, 2H), 2.50-2.62 (m, 2H), 2.63-2.91 (m, 4H), 2.99-3:18 (m, 4H), 3.31-3.52 (m, 1H), 3.87 (s, 3H), 4.71-4.84 (br, 1H), 6.41-6.52 (m, 1H), 6.53-6.70 (m, 2H), 6.756.94 (m, 3H).

### c) 1-(1-Cyclohexanecarbonyl-6-methyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 53B instead of Compound 1B. The crude was purified by flash chromatography eluting with petroleum ether-ethyl acetate 1:1 to give the title compound (80 %).
¹H-NMR (δ): 0.81-1.99, (m, 11H), 2.01-2.19 (m, 1H), 2.23-2.81 (m, 12H), 2.84-3.08 (m, 4H), 3.82 (s, 3H), 4.98-5.17 (m, 1H), 6.49-6.68 (m, 2H), 6.74-7.09 (m, 4H).

### Example 54

### 1-[1-(3-Cyanaminopropionyl-1,2,3,4-tetrahydroquinoline-2-ylmethyl)]-4-(4-indolyl)-piperazine

A mixture of 0.021 g of cyanamide, 0.1 g of K₂CO₃ and 0.75 ml of DMF was stirred for 1.5 h at 80°C, cooled at r.t.; afterwards, 0.04 g of Compound 27A was added with stirring at 80°C for further 6 h. After cooling at r.t., the reaction mixture was diluted with water (7 ml), strirred for 0.5 h and filtered recovering the precipitated solid. This was purifed by flash chromatography (EtOAc - 2 N NH₃ sol. in methanol 96:4) to give 0.025g (56 %) of the title compound.
¹H-NMR (δ): 1.25-1.65 (m, 1H), 2.00-2.85 (m, 11H), 2.85-3.65 (m, 6H), 5.10 (b, 1H), 5.70 (b, 1H), 6.40-6.55 (m, 2H), 6.90-7.25 (m, 7H), 8.10 (s, 1H).

### Example 55

### 1-(5-Chloro-1-cyclohexanecarbonyl-1,2,3,4-tetrahydroquinoline-2-ylmethyl)-4-(4-fluoro-2-methoxyphenyl)-piperazine

### a) 1-Benzoyl-5-chloro-2-cyano-1,2-dihydroquinoline (Compound 55A)

The title compound was prepared as described for compound 32A, but starting from 5-chloroquinoline (WO 01/44247) instead of 6-methylquinoline. (67%).
¹H-NMR (δ): 6.13-6.22 (m, 2H), 6.51 (d, 1H), 6.95 (dd, 1H), 7.18-7.51 (m, 6H).

### b) 5-Chloroquinoline-2-carboxylic acid (Compound 55B)

Compound 55B was obtained in the manner described for compound 32B, but using compound 55A instead of compound 32A. The crude was used without additional purification. (63%).
¹H-NMR (DMSO-d₆, 400 MHz, δ): 6.15-7.01 (bs; 1H), 7.60-7.81 (m, 2H), 7.92-8.18 (m, 2H), 8.48 (d, 1H)

### c) 5-Chloro-1,2,3,4-tetrahydroquinoline-2-carboxylic acid (Compound 55C)

The title compound was prepared as described for Compound 1B (step e) starting from Compound 55B instead of Compound 1D. The crude was used without further purification to give the title compound (78%).
¹H-NMR (DMSO-d₆, 400 MHz, δ): 1.87-2.20 (m, 2H), 2.67-2.88 (m, 2H), 3.99-4.18 (m, 1H), 5.12-5.82 (br, 2H), 6.40 (d, 1H), 6.71 (d, 1H), 6.97 (dd, 1H).

### d) 1-(5-Chloro-1,2,3,4-tetrahydroquinoline-2-ylcarbonyl)-4-(4-fluoro-2-methoxyphenyl)-piperazine (Compound 55D)

The title compound was synthesised following the procedure described for Compound 1D (step g) but using Compound 55C instead of 1,2,3,4-tetrahydroquinoline-2-carboxylic acid. The residue was purified by flash chromatography eluting with petroleum ether - EtOAc 6:4 to give the title compound (79 %).
¹H-NMR (δ): 1.59-1.71 (m, 1H), 2.12-Z.28 (m, 1H), 2.60-2.82 (m, 1H), 2.92-3.12 (m, 5H), 3.55-3.96 (m, 7H), 4.11-4.23 (m, 1H), 4.61-4.74 (br, 1H), 6.50-6.78 (m, 4H), 6.80-7.04 (m, 2H).

### e) 1-(5-chloro-1,2,3,4-tetrahydroquinoline-2-ylmethyl)4-(4-fluoro-2-methoxyphenyl)-piperazine (Compound 55E)

The title compound was synthesised following the procedure described for Compound 1B (step h). The residue was purified by flash chromatography eluting with petroleum ether - EtOAc 1:1 to give the title compound (53 %).
¹H-NMR (δ): 1.40-1.79 (m, 1H), 1.81-2.09 (m, 1H), 2.24-3.64 (m, 13H), 3.84 (s, 3H), 4.55-4.91 (br, 1H),6.31-6.75 (m, 4H), 6.77-7.04 (m, 2H).

### f) 1-(1-Cyclohexanecarbonyl-5-chloro-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 55E instead of Compound 1B. The crude was purified by flash chromatography eluting with petroleum ether - ethyl acetate 65:35 to give the title compound (67 %).
¹H-NMR (δ): 0.76-3.49 (m, 25H), 3.80 (s, 3H), 4.90-5.34 (m, 1H), 6.47-6.63 (m, 2H), 6.80-6.91 (m, 1H), 7.08-7.32 (m, 3H).

### Example 56

### 1-[5-Chloro-1-(4-methoxybenzoyl)-1,2,3,4-tetrahydroquinoline-2-ylmethyl]-4-(4-fluoro-2-methoxyphenyl)-piperazine

The title compound was synthesised following the alternative acylation procedure described for the compound of Example 1 (step f) but starting from Compound 55E instead of Compound 1B, using 4-methoxybenzoyl chloride instead of cyclohexanecarbonyl chloride, toluene instead 1,2-dichlorometane and refluxing for 1 hour. The crude was purified by flash chromatography eluting with petroleum ether - ethyl acetate 6:4 to give the title compound (28 %).
¹H-NMR (δ): 1.94-2.12 (m, 1H), 2.13-2.39 (m, 1H), 2.44-3.08 (m, 12H), 3.79, 3.88 (2s, 6H), 4.89-5.08 (br, 1H), 6.51.-6.66 (m, 3H), 6.72-6.91 (m, 4H), 7.11 (d, 1H), 7.24-7.39 (m, 2H).

### Example 57

### 1-(7-Chloro-1-cyclohexanecarbonyl-1,2,3,4-tetrahydroquinoline-2-ylmethyl)-4-(4-fluoro-2-methoxyphenyl)-piperazine

### a) 2-Bromomethyl-7-chloro-1,2,3,4-tetrahydroquinoline (Compound 57A)

The title compound was prepared as described for Compound 5A but using 7-chloro-2-methylquinoline instead of 6-fluoro-2-methylquinoline. The residue was purified by flash chromatography (petroleum ether - EtOAc gradient from 100 : 5 to 100 : 10) to afford Compound 57A (42%) as a white solid.
¹H-NMR (δ): 4.70 (s, 2H), 7.50 (dd, 1H), 7.58 (d, 1H), 7.75 (d, 1H), 8.10 (d, 1H), 8.18 (d, 1H).

### b) 7-Chloro-2-[4-(4-fluoro-2-methoxyphenyl)-1-piperazinylmethyl]-quinoline (Compound 57B)

The title compound was prepared as described for Compound 1D (step d) but using Compound 57A instead of 2-chloromethylquinoline. The residue was purified by flash chromatography (petroleum ether - EtOAc 70:30) to afford Compound 57B (42.3%) as an orange solid.
¹H-NMR (δ): 2.65-2.85 (m, 4H), 2.90-3.20 (m, 4H), 3.80 (s, 3H), 3.90 (s, 3H), 6.456.65 (m, 2H), 6.75-6.95 (m, 1H), 7.45 (dd, 1H), 7.55-7.80 (m, 2H), 8.00-8.20 (m, 2H).

### c) 1-(7-Chloro-1,2,3,4-tetrahydroquinoline-2-ylmethyl)-4-(4-fluoro-2-methoxyphenyl)-piperazine (Compound 57C)

The title compound was synthesised following the procedure reported for Compound 1B (step e) starting from compound 57B instead Compound 1C. The residue was purified by flash chromatography (petroleum ether - EtOAc 60:40) affording Compound 57C (42%) as an orange oil.
¹H-NMR (δ): 1.32-1.65 (m, 1H), 1.80-2.00 (m, 1H), 2.32-2.65 (m, 4H), 2.65-2.95 (m), 6.80-7.00 (m, 2H).

### d) 1-(7-Chloro-1-cyclohexanecarbonyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 57C instead of Compound 1B. The residue was purified by flash chromatography (CHCl₃-2 N methanolic ammonia 100 : 5) affording the title compound (52.1%) as a thick oil.
¹H-NMR (δ): 0.75-2.20 (m,12H), 2.20-2.80 (m, 9H), 2.84-3.20 (m, 4H), 3.92 (s,3H), 4.80-5.20 (m,1H), 6.50-6.70 (m, 2H), 6.75-6.95 (m, 2H), 7.03-7.25 (m, 2H).

### Example 58

### 3-Benzyl-1-(1-cyclohexanecarbonyl-1,2,3,4-tetrahydroquinoline-2-ylmethyl)-piperidine (upper TLC Rf diastereomer)

The title compound was synthesised using the methodology described in Example 35 using Intermediate 35B and 3-benzylpiperidine (WO 97/23458) instead of 1-(4-fluoro-2-methoxyphenyl)-piperazine. Eluent: EtOAc - MeOH 95:5. Yield: 35%.
¹H-NMR (δ): 0.82-3.48 (m, 28H), 4.88-5.32 (m, 1H), 6.85-7.42 (m, 9H).

### Example 59

### 3-Benzyl-1-(1-cyclohexanecarbonyl-1,2,3,4-tetrahydroquinoline-2-ylmethyl)-piperidine (lower TLC Rf diastereomer)

The title compound was isolated as the lower Rf diastereomer during the purification of Compound 58. Eluent: EtOAc-MeOH 95:5. Yield: 13%.
¹H-NMR (δ): 0.82-1.41 (m, 6H), 1.42-2.01 (m, 11H), 2.17- 3.01 (m, 9H), 3.23-3.52 (m, 2H), 5.02-5.32 (m, 1H), 6.88-7.42 (m, 9H).

### Example 60

The following compounds were synthesised using the methodology described in Example 35 but in a parallel synthesis fashion using dichloromethane instead of chloroform as solvent and using the appropriate basic head instead of 1-(4-fluoro-2-methoxyphenyl)-piperazine. After making alkaline and dilution with CH₂Ch₂, the aqueous layer was removed by filtering on solid phase extraction (SPE) cartridges. The organic layer was evaporated to dryness in vacuo and the crude purified by flash chromatography using conventional techniques or parallel flash chromatography techniques (QUAD3™ from Biotage) (eluent shown):

### Example 60A

### 1-(4-Chloro-2-i-propoxyphenyl)-4-(1-cyclohexanecarbonyl-1,2,3,4-tetrahydroquinoline-2-ylmethyl)-piperazine

From 1-(4-chloro-2-*i*-propoxyphenyl)-piperazine (Martin, G.E. et al., *J*. *Med. Chem.* **32**, 1052-1056, (1989)).
Flash chromatography: petroleum ether - EtOAc 7:3. Yield: 72.7%.
¹H-NMR (δ): 0.85-3.52 (m, 31H), 4.41-4.69 (m, 1H), 5.05-5.23 (m, 1H), 6.80-6.95 (m, 3H), 7.05-7.15 (m, 4H).

### Example 60A1

### 1-(5-Chloro-2-fluorophenyl)-4-(1-cyclohexanecarbonyl-1,2,3,4-tetrahydroquinoline-2-ylmethyl)-piperazine

From 1-(5-chloro-2-fluorophenyl)-piperazine (WO 01/05765).
Flash chromatography eluent: petroleum ether-EtOAc 8:2. Yield: 50%.
¹H-NMR (δ): 0.79-1.99 (m, 11H), 2.01-2.18 (m, 1H), 2.20-2.81 (m, 9H), 2.91-3.18(m, 4H), 4.98-5.21 (m, 1H). 6.77-7.01 (m, 3H), 7.02-7.25 (m, 4H).

### Example 60A2

### 1-[4-(2,1,3-Benzothiadiazolyl))-4-(1-cyclohexanecarbonyl-1,2,3,4-tetrahydroqui-noline-2-ylmethyl)-piperazine

From 1-[4-(2,1,3-benzothiadiazolyl]-piperazine (US 4831031).
Flash chromatography eluent: petroleum ether-EtOAc 1:1. Yield: 55%.
¹H-NMR (δ): 0.75-1.88 (m, 11H), 1.99-2.08 (m, 1H), 2.25-3.01 (m, 9H), 3.31-3.82 (m, 4H), 5.00-5.37 (m, 1H), 6.72 (d, 1H), 7.09-7.32 (m, 4H); 7.44-7.64 (m, 2H).

### Example 60A3

### 1-(1-Cyclohexanecarbonyl-1,2,3,4-tetrahydroquinoline-2-ylmethyl)-4-(2-ethoxy-4-fluorophenyl)-piperazine

From 1-(2-ethoxy-4-fluorophenyl)-piperazine.
Flash chromatography: petroleum ether - EtOAc 6:4. Yield: 44.8%.
¹H-NMR (δ): 0.85-1.87 (m, 13H), 1.88-2.21 (m, 2H), 2.22-2.81 (m, 8H), 2.82-3.21 (m, 4H), 4.08 (q, 2H), 4.95-5.23 (m, 1H), 6.48-6.61 (m, 3H), 6.73-6.91 (m, 1H), 7.03-7.30 (m, 4H).

### Example 60A4

### 1-(1-Cyclohexanecarbonyl-1,2,3,4-tetrahydroquinoline-2-ylmethyl)-4-(2-methoxy-4-hydroxyphenyl)-piperazine

From 1-(4-hydroxy-2-methoxyhenyl)-piperazine.
Flash chromatography: petroleum ether - EtOAc 6:4. Yield: 58%.
¹H-NMR (δ): 0.85-1.87 (m, 13H), 1.88-2.21 (m, 2H), 2.22-2.81 (m, 8H), 2.82-3.21 (m, 4H), 4.08 (q, 2H), 4.95-5.23 (m, 1H), 6.48-6.61 (m, 3H), 6.73-6.91 (m, 1H), 7.03-7.30 (m, 4H).

### Example 60A5

### 1-(1-Cyclohexanecarbonyl-1,2,3,4-tetrahydroquinoline-2-ylmethyl)-4-(7-methoxy-4-indolyl)-piperazine

From 1-(7-methoxy-4-indolyl)-piperazine.
Flash chromatography: petroleum ether - EtOAc - 2 N methanolic ammonia 8:2:0.3.
¹H-NMR (δ□ : 0.80-2.80 (m, 21H), 3.00-3.20 (m, 4H), 3.90 (s, 3H), 5.10 (b, 1H), 6.40-6.55 (m, 3H), 7.00-7.30 (m, 5H), 8.45 (b, 1H).

The starting piperazine was obtained as follows:
a) 4-Amino-7-methoxyindole (Compound 60A5-A)
   A mixture of 0.06 g of 4-nitro-7-methoxyindole (N. Rouè et al, *Heterocycles* **43**, 263-267), 0.003 g of 10% Pd-C and 8 ml of EtOH was hydrogenated in a Parr apparatus at 30 p.s.i. (206910 pascals). The catalyst was filtered off and the solution was evaporated to dryness in vacuo affording the title compound (0.04 g, 80%), which was used in the next step without further purification.
   ¹H-NMR (δ): 1.65-3.70 (b, 2H), 3.85 (s, 3H), 6.30 (d, 1H), 6.406.55 (m, 2H), 7.15 (dd, 1H), 8.35 (b, 1H)
b) 1-(7-Methoxy-4-indolyl)-piperazine (Compound 60A5-B)
   The title compound was prepared as described for Compound 35A13-B starting from Compound 60A5-A instead of Compound 35A13-A. The crude was purified by flash chromatography eluting with CH₂Cl₂ - 2 N methanolic ammonia 90:10 to give the title compound (23%).
   ¹H-NMR (δ): 2.65-3.60 (m, 9H), 3.90 (s, 3H), 6.40-6.60 (m, 3H), 7.15 (dd, 1H), 8.40 (b, 1H).

### Example 60A6

### 1-(1-Cyclohexanecarbonyl-1,2,3,4-tetrahydroquinoline-2-ylmethyl)-4-(2-pyrazinyl)-piperazine

From 1-(2-pyrazinyl)-piperazine (commercial)
Flash chromatography: petroleum ether - EtOAc 7:3. Yield: 44%.
¹H-NMR (δ): 0.80-2.11 (m, 12H), 2.12-3.21 (m, 9H), 3.22-3.76 (m, 9H), 5.05-5.31 (m, 1H), 7.03-7.30 (m, 4H), 7.82 (s, 1H), 8.32 (dd, 2H).

### Example 60A7

### 1-(2-Cyano-4-nitrophenyl)-4-(1-cyclohexanecarbonyl-1,2,3,4-tetrahydroquinoline-2-ylmethyl)-piperazine

From 1-(2-cyano-4-nitrophenyl)-piperazine (CA vol.97, 1982, 109953s)
Flash chromatography: petroleum ether - EtOAc 1:1. Yield: 64%.
¹H-NMR (δ): 0.75-2.01 (m, 11H), 2.11-2.89 (m, 10H), 3.40-3.59 (m, 4H), 5.01-5.21 (m, 1H), 6.94 (d, 1H), 7.03-7.27 (m, 4H), 8.24 (dd, 1H), 8.43 (d, 1H).

### Example 60A8

### 1-[4-(2,1,3-Benzoxadiazolyl)]-4-(1-cyclohexanecarbonyl-1,2,3,4-tetrahydroquino-line-2-ylmethyl)-piperazine

From 1-[4-(2,1,3-benzoxadiazolyl)-piperazine (EP 0189612).
Flash chromatography: petroleum ether- EtOAc 1:1. Yield: 86%.
¹HNMR (δ): 0.81-2.01 (m, 11H), 2.04-2.21 (m, 1H), 2.25-2.87 (m, 9H), 3.41-3.66 (m, 4H), 4.98-5.28 (m, 1H), 6.34 (d, 1H), 6.98-7.48 (m, 6H).

### Example 60A9

### 1-(1-Cyclohexanecarbonyl-1,2,3,4-tetrahydroquinoline-2-ylmethyl)-4-(2-methoxy-5-trifluoromethylphenyl)-piperazine

From 1-(2-methoxy-5-trifluoromethylphenyl)-piperazine (EP 0156443) CA:104, P129918a.
Flash chromatography: petroleum ether-EtOAc 6:4. Yield: 86%.
¹H-NMR (δ): 0.84-2.21 (m, 12H), 2.28-2.87 (m, 9H), 3.92-3.26 (m, 4H), 3.91 (s, 3H), 4.99-3.31 (m, 1H), 6.88 (d, 1H), 7.04-7.35 (m, 6H).

### Example 60A10

### 1-(1-Cyclohexanecarbonyl-1,2,3,4-tetrahydroquinoline-2-ylmethyl)-4-[1-(1,2,3,4-tetrahydronaphthyl)]-piperazine

From 1-(1,2,3,4-tetrahydronaphthyl)-piperazine.
Flash chromatography: petroleum ether- EtOAc 1:1. Yield: 45%.
¹H-NMR (δ): 0.81-2.17 (m, 16H), 2.20-2.92 (m, 15H), 3.65-4.92 (m, 1H), 4.89-5.21 (m, 1H), 7.02-7.28 (m, 7H), 7.57-7.77 (m, 1H).

### Example 60A11

### 1-(7-Chloro-2,3-dihydro-1,4-benzodioxin-5-yl)-4-(1-cyclohexanecarbonyl-1,2,3,4-tetrahydroquinoline-2-ylmethyl)-piperazine

From 1-(7-chloro-2,3-dihydro-1,4-benzodioxin-5-yl)-piperazine (Commercial).
Flash chromatography: CH₂Cl₂- EtOAc 7:3. Yield: 45%.
¹H-NMR (δ): 0.74-2.21 (m, 12H), 2.25-2.88 (m, 8H), 2.90-3.21 (m, 4H), 4.18-4.89 (m, 4H), 4.96-5.38 (m, 1H), 6.43-6.69 (m, 2H), 7.04-7.32 (m, 5H).

### Example 60A12

### 1-(1-Cyclohexanecarbonyl-1,2,3,4-tetrahydroquinoline-2-ylmethyl)-4-(4-pyridyl)-piperazine

From 1-(4-pyridyl)-piperazine (Regneir et al. *Arzneim. Forsch.* **24**, 12, 1974).
Flash chromatography: CH₂Cl₂ - MeOH 9:1. Yield: 79%..
¹H-NMR (δ) : 0.71-2.02 (m, 11H), 2.04-2.81 (m, 10H), 3.35-3.61 (m, 4H), 5.02-5.21 (m, 1H), 6.75 (d, 2H), 6.97-7.31 (m, 4H), 8.17 (d, 2H).

### Example 60A13

### 1-(1-Cyclohexanecarbonyl-1,2,3,4-tetrahydroquinoline-2-ylmethyl)-4-[4-(6,7-dimethoxyquinazolinyl)]-piperazine

From 1-[4-(6,7-dimethoxyquinazolinyl)]-piperazine (CA 70 68419 (1969)
Flash chromatography : EtOAc-MeOH 98:2. Yield: 81%.
¹H-NMR (δ): 0.75-1.89 (m, 11H), 1.91-2.12 (m, 1H), 2.19-3.17 (m, 9H), 3.79-4.24 (m, 10H), 7.07 (s, 1H), 7.14-7.39 (m, 4H), 7.43 (s, 1H), 8.63 (s, 1H).

### Example 61

### 1-(4-Fluoro-2-methoxyphenyl)-4-(1-cyclohexanecarbonyl-6-nitro-1,2,3,4-tetrahydroquinoline-2-ylmethyl)-piperazine

### a) 1-Cyclohexanecarbonyl-2-methoxycarbonyl-6-nitro-1,2,3,4-tetrahydroquinoline (Compound 61A)

Into a solution of 1-cyclohexanecarbonyl-2-methoxycarbonyl-1,2,3,4-tetrahydroquinoline (2.6 g) in acetic anhydride (3.7 ml) a solution of 37% HNO₃ (0.94 ml) and A₂O was dropped. The resulting mixture was stirred at 60°C for 4 h. Afterwards, the solution was poured into 200 ml of water and extracted with ethyl acetate (2 x 60 ml). The organic layer was dried on anhydrous sodium sulphate and evaporated to dryness. The crude was purified by flash chromatography eluting with petroleum ether - EtOAc 7:3 giving 1.8g of the title compound (60%).
¹H-NMR (δ): 0.89-2.10 (m, 11H), 2.49-2.89 (m, 4H), 3.65 (s, 3H), 5.01-5.18 (m, 1H), 7.35 (dd, 1H), 8.03-8.22 (m, 2H).

### b) 2-Hydroxymethyl-6-nitro-1,2,3,4-tetrahydroquinoline (Compound 61B)

To a solution of 0.8 g of Compound 61A in 15 ml of anhydrous THF stirred at 0-5°C was added 1.2 ml of 2 M LiBH₄ in THF; then the mixture was stirred at r.t. for 6 h. Afterwards, the reaction was diluted with an aqueous saturated solution of ammonium chloride (20 ml), extracted with EtOAc (2x50 ml), dried on sodium sulphate and evaporated *in vacuo.* The residue was purified by flash chromatography eluting with CH₂Cl₂- MeOH 95:5 affording 0.3 g of the title compound (62%).
¹H-NMR (δ): 1.48-1.81 (m, 1H), 1.92-2.11 (m, 1H), 2.74-2.94 (m, 2H), 3.45-3.61 (m, 2H), 3.65-3.85 (m, 1H), 4.94-5.41 (bs, 1H), 6.48 (dd, 1H), 7.81-8.01 (m, 2H).

### c) 2-Bromomethyl-6-nitro-1,2,3,4-tetrahydroquinoline (Compound 61C)

A solution of Compound 61B (0.3 g), triphenylphosphine (0.42 g) and CBr₄ (0.86g) in CH₂Cl₂ (40 ml) was stirred at r.t. for 12 h. Then the solvent was evaporated and the crude purified by flash chromatography eluting with CH₂Cl₂- MeOH 95:5 giving 0.24 g of the title compound (60%).
¹H-NMR (δ): 1.65-1.83 (m, 1H), 1.99-2.19 (m, 1H), 2.74-2.94 (m, 2H), 3.18-3.42 (m, 2H), 3.55-3.74 (m, 1H), 6.47 (dd, 1H), 7.83-8.02 (m, 2H).

### d) 1-(4-Fluoro-2-methoxyphenyl)-4-(6-nitro-1,2,3,4-tetrahydroquinoline-2-ylmethyl)-piperazine (Compound 61D).

A mixture of Compound 61C (110 mg), 1-(4-fluoro-2-methoxyphenyl)-piperazine (85 mg) and K₂CO₃ was heated for 30 min at 200°C. Afterwards, the reaction was diluted with H₂O (10 ml) and extracted with EtOAc (2x 20 ml).

The combined organic layers were dried over Na₂SO₄ and evaporated to dryness. The crude was purified by flash chromatography eluting with petroleum ether - EtOAc 1:1 affording 60 mg of the title compound (38%).
¹H-NMR (δ): 1.41-1.71 (m, 1H), 1.91-2.09 (m, 1H), 2.32-3.31 (m, 12H), 3.51-3.73 (m, 1H), 3.81 (s, 3H), 5.52-5.95 (br, 1H), 6.41-6.51 (m, 1H), 6.52-6.68 (m, 2H), 6.83-6.95 (m, 1H), 7.82-7.95 (m, 2H).

### e) 1-(1-Cyclohexanecarbonyl-6-nitro-1,2,3,4-tetrahydroquinoline-2-ylmethyl)-4-(4-indolyl)-piperazine

The title compound was synthesised following the alternative acylation procedure described for the compound of Example 1 (step f) but starting from Compound 61D instead of Compound 1B. The crude was purified by flash chromatography eluting with petroleum ether - EtOAc 1:1 to give the title compound (47%).
¹H-NMR (δ): 0-70-3.12 (m, 23H), 3.13-3.41 (m, 2H), 3.82 (s, 3H), 4.82-5.03 (m, 1H), 6.42-6.71 (m, 1H), 6.72-6.98 (m, 2H), 7.29-7.41 (m, 1H), 8.03-8.21 (m, 2H).

### Example 62

The following compounds were synthesised using the methodology described in Example 35 but in a parallel synthesis fashion using dichloromethane instead of chloroform as solvent and using the appropriate basic head instead of 1-(4-fluoro-2-methoxyphenyl)-piperazine-After making alkaline and dilution with CH₂Cl₂, the aqueous layer was removed by filtering on solid phase extraction (SPE) cartridges. The organic layer was evaporated to dryness in vacuo and the crude purified by flash chromatography using conventional techniques or parallel flash chromatography techniques (QUAD3™ from Biotage) (eluent shown):

### Example 62A

### 1-(1-Cyclohexanecarbonyl-1,2,3,4-tetrahydroquinoline-2-ylmethyl)-4-(1-ethyl-4-indolyl)-piperazine

From 1-(1-ethyl-4-indolyl)-piperazine.
Flash chromatography: petroleum ether-EtOAc 8:2.
¹H-NMR (δ): 0.80-1.88 (m, 14H), 1.89-2.11 (m, 1H), 2.21.-2.95 (m, 9H), 2.96-3.42 (m, 4H), 4.11 (q, 2H), 4.99-5.30 (m, 1H), 6.35-6.61 (m, 2H), 6.95-7.32 (m, 7H).

The starting piperazine was obtained as following:
a) 1-tert-Butoxycarbonyl-4-(1-ethyl-4-indolyl)-piperazine (Compound 62A-A)
   The title compound was prepared as described in Example 35A25-A using ethyl bromide instead of acetyl chloride.
b) 1-(1-Ethyl-4-indolyl)-piperazine (Compound 62A-B)
   The title compound was prepared as described in Example 35A25-B using Compound 62A-A instead of Compound 35A25-A.
   ¹H-NMR (δ): 1.42 (t, 3H), 3.12-3.33 (m, 8H), 4.15 (q, 2H), 4.99-5.30 (m, 1H), 6.38 (dd, 1H), 6.62 (dd, 1H), 6.99-7.20 (m, 3H).

### Example 62A1

### 1-(2-Bromo-5-methoxybenzyl)-4-(1-cyclohexanecarbonyl-1,2,3,4-tetrahydroquino-line-2-ylmethyl)-piperazine

From 1-(2-bromo-5-methoxybenzyl)-piperazine (CA 2188484).
Flash chromatography: petroleum ether - EtOAc 7:3. Yield: 63%.
¹H-NMR (δ): 0.78-1.99 (m, 11H), 2.00-2.21 (m, 1H), 2.22-2.95 (m, 13H), 3.45-3.62 (m, 2H), 3.81 (s, 3H), 4.92-5.21 (m, 1H), 6.61-6.75 (m, 1H), 6.98-7.24 (m, 5H), 7.31-7.48 (m, 1H).

### Example 62A2

### 1-(4-Chloro-2-methoxyphenyl)-4-(1-cyclohexanecarbonyl-1,2,3,4-tetrahydroquinoline-2-ylmethyl)-piperazine

From 1-(4-chloro-2-methoxyphenyl)-piperazine (US 5859014).
Flash chromatography : petroleum ether - EtOAc 8:2. Yield:65%.
¹H-NMR (δ): 0.79-3.42 (m, 25H), 3.87 (s, 3H); 4.88-5.38 (m, 1H), 6.71-6.97 (m, 3H, 7.04-7.31 (m, 4H).

### Example 62A3

### 1-(4-Chloro-2-ethoxyphenyl)-4-(1-cyclohexanecarbonyl-1,2,3,4-tetrahydroquinoline-2-ylmethyl)-piperazine

From 1-(4-chloro-2-ethoxyphenyl)-piperazine.
Flash chromatography : petroleum ether - EtOAc 8:2. Yield: 58%.
¹H-NMR (δ): 0.79-1.18 (m, 11H), 1.19-2.20 (m, 1H), 2.22-2.84 (m, 9H), 2.88-3.24 (m, 4H), 4.05 (q, 2H), 4.98-5.28 (m, 1H), 6.69-6.90 (m, 3H), 7.08-7.38 (m, 4H).

### Example 62A4

### 1-(1-Cyclohexanecarbonyl-1,2,3,4-tetrahydroquinoline-2-ylmethyl)-4-(4-fluoro-2-i-propoxyphenyl)-piperazine

From 1-(4-fluoro-2-*i*-propoxyphenyl)-piperazine (EP 102985).
Flash chromatography: petroleum ether - EtOAc 8:2. Yield: 58%.
¹H-NMR (δ): 0.78-1.92 (m, 16H), 1.93-2.17 (m 2H), 2.18-2.76 (m, 9H), 2.77-3.34 (m, 4H), 4.48-4.62 (m, 1H), 5.03-5.28 (m, 1H), 6.49.-6.63 (m, 2H), 6.78-6.84 (m, 1H), 7.05-7.30 (m, 4H).

### Example 62A5

### 1-(1-Cyclohexanecarbonyl-1,2,3,4-tetrahydroquinoline-2-ylmethyl)-4-(7-methyl-4-indolyl)-piperazine

From 1-(7-methyl-4-indolyl)-piperazine.
Flash chromatography: petroleum ether - EtOAc 7:3. Yield: 54%.
¹H-NMR (δ): 0.81-1.82 (m,11H), 1.83-2.04 (m, 1H), 2.34-2.99 (m, 12H), 3.00-3.42 (m, 4H), 5.01-5.31 (m, 1H), 6.41-6.61 (m, 2H), 6.83-7.93 (m, 1H), 7.05-7.34 (m, 5H), 8.06-8.27 (br, 1H).

The starting piperazine was obtained as following:
a) 4-Amino-7-methylindole (Compound 62A5-A)
   The title compound was synthesised using the methodology described in Example 35A1 using 7-methyl-4-nitroindole (J. Bergman et al, *Tetrahedron* **46**, 6085-6112 (1990)) instead of 2-methyl-4-nitroindole. Yield: 91%.
   ¹H-NMR (δ): 2.46 (s, 3H), 3.69-4.02 (br, 2H), 6.25 (dd, 1H), 6.38 (dd, 1H), 6.75 (dd, 1H), 7.14 (dd 1H), 8.01-8.13 (br, 1H).
b) 1-(7-Methyl-4-indolyl)-piperazine (Compound 62A5-B)
   The title compound was synthesised using the methodology described in Example 35A1 (step b) using Compound 62A5-A instead of Compound 35A1-A. The crude was purified by flash chromatography eluting with CH₂Cl₂ - 2 N methanolic ammonia 9:1 to give the title compound (60%).
   ¹H-NMR (δ): 2.47 (s, 3H), 2.99-3.35 (m, 9H), 6.45-6.52 (m 2H), 6.87 (dd, 1H), 7.21 (dd, 1H), 8.03-8.18 (br, 1H).

### Example 62A6

### 1-(1-Cyclohexanecarbonyl-1,2,3,4-tetrahydroquinoline-2-ylmethyl)-4-(2-methoxyphenoxy)-piperidine

From 4-(2-methoxyphenoxy)-piperidine.
Flash chromatography: petroleum ether - EtOAc 6:4. Yield: 48%.
¹H-NMR (δ): 0.75-3.43 (m, 25H), 3.81 (s, 3H), 4.08-4.32 (m, 1H), 5.06-5.22 (m, 1H), 6.81.7.00 (m, 4H), 7.11-7.28 (m, 4H).

### Example 63

### 1-[6-(5-Acetyl-2-thienyl)-1-cyclohexanecarbonyl-1,2,3,4-tetrahydroquinoline-2-ylmethyl)-4-(4-fluoro-2-methoxyphenyl)-piperazine

The title compound was synthesised following the procedure described for the compound of Example 47 but starting from the Compound of Example 51 instead of the compound of Example 34 and using 5-acetylthiophen-2-boronic acid instead of phenylboronic acid. Flash chromatography: petroleum ether- EtOAc 3:7. Yield: 48%.
¹H-NMR (δ): 0.70-2.25 (m, 12H), 2.55 (s,3H), 2.25-3.20 (m, 13H), 3.80 (s, 3H), 4.85-5.40 (m, 1H), 6.50-6.70 (m, 2H), 6.80-6.95 (m, 1H), 7.05-7.40 (m, 2H), 7.40-7.60 (m, 2H), 7.68 (d,1H).

### Example 64

### 1-[1-Cyclohexanecarbonyl-6-(3,5-dimethylisoxazol-4-yl)-1,2,3,4-tetrahydroquinoline-2-ylmethyl]-4-(4-fluoro-2-methoxyphenyl)-piperazine

The title compound was synthesised following the procedure described for the compound of Example 47 but starting from the Compound of Example 51 instead of the compound of Example 34 and using 3,5-dimethylisoxazole-4-boronic acid instead of phenylboronic acid. Yield: 48%.
¹H-NMR (δ): 0.7.0-2.25 (m, 12H), 2.30 and 2.43 (2s, 6H), 2.25-3.20 (m, 13H), 3.80 (s, 3H), 4.80-5.40 (m, 1H), 6.50-6.70 (m, 2H), 6.75-6.95 (m, 1H), 7.00-7.20 (m, 3H).

### Example 65

### 1-(1-Cyclohexanecarbonyl-7-methyl-1,2,3,4-tetrahydroquinoline-2-ylmethyl)-4-(4-fluoro-2-methoxyphenyl)-piperazine

### a) 1-(4-Fluoro-2-methoxyphenyl)-4-(7-methyl-1,2,3,4-tetrahydroquinoline-2-ylcarbonyl)-piperazine (Compound 65A)

The title compound was synthesised following the procedure described for Compound 1D (step g) but using 7-methyl-1,2,3,4-tetrahydroquinoline-2-carboxylic acid (US 4461896) instead of 1,2,3,4-tetrahydroquinoline-2-carboxylic acid and 1-(4-fluoro-2-methoxyphenyl)-piperazine instead of 1-(4-indolyl)-piperazine. The residue was purified by flash chromatography eluting with CH₂Cl₂- EtOAc 8:2 to giving the title compound (72 %).
¹H-NMR (δ): 1.60-1.85 (m, 1H), 2.05.-2.20 (m, 1H), 2.25 (s, 3H), 2.643.40 (m, 7H), 3.65-4.05 (m, 7H), 4.20-4.35 (m, 1H), 6.50-6.75 (m, 4H), 6.80-7.20 (m, 2H).

### b) 1-(4-Fluoro-2-methoxyphenyl)-4-(7-methyl-1,2,3,4-tetrahydroquinoline-2-ylmethyl)-piperazine (Compound 65B)

The title compound was synthesised following the procedure described for Compound 1B (step h) but using Compound 65A. The residue was purified by flash chromatography eluting with petroleum ether- EtOAc 5:5 to give the title compound (81%).
¹H-NMR (δ): 1.40-1.75 (m, 1H), 1.75-2.00 (m, 1H), 2.20 (s, 3H), 2.353.80 (m, 13H), 3.85 (s, 3H), 4.60 (b, 1H), 6.30-6.50 (m, 2H), 6.50-6.70 (m, 2H), 6.80-6.95 (m, 2H).

### c) 1-(1-Cyclohexanecarbonyl-7-methyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 65B instead of Compound 1B. Yield: 100%.
¹H-NMR (δ) : 0.80-3.30 (m, 28H), 3.80 (s, 3H), 5.08 (b, 1H), 6.50-6.65 (m, 2H), 6.75-7.15 (m, 4H).

### Example 66

### 1-(1-Cyclohexanecarbonyl-4-methyl-1,2,3,4-tetrahydroquinoline-2-ylmethyl)-4-(4-fluoro-2-methoxyphenyl)-piperazine

### a) cis-1-(4-Fluoro-2-methoxyphenyl)-4-(4-methyl-1,2,3,4-tetrahydroquinoline-2-ylcarbonyl)-piperazine (Compound 66A)

The title compound was synthesised following the procedure described for Compound 1D (step g) but using cis-4-methyl-1,2,3,4-tetrahydroquinoline-2-carboxylic acid (obtained by hydrolysis with aq. NaOH in MeOH from the corresponding methyl ester (US 5616586)) instead of 1,2,3,4-tetrahydroquinoline-2-carboxylic acid and 1-(4-fluoro-2-methoxyphenyl)-piperazine instead of 1-(4-indolyl)-piperazine. The residue was purified by flash chromatography eluting with petroleum ether - EtOAc 1:1 to giving the title compound (72 %).
¹H-NMR (δ): 1.30-2.00 (m, 4H), 2.00-2.20 (m, 1H), 2.90-4.50 (m, 14H), 6.55-7.25 (m, 7H).

### b) cis-1-(4-Fluoro-2-methoxyphenyl)-4-(4-methyl-1,2,3,4-tetrahydroquinoline-2-ylmethyl)-piperazine (Compound 66B)

The title compound was synthesised following the procedure described for Compound 1B (step h) but using Compound 66A. The residue was purified by flash chromatography eluting with petroleum ether - EtOAc 6:4 to give the title compound (77 %).
¹H-NMR (δ): 1.20-2.00 (m, 5H), 2.30-3.75 (m, 12H), 3.85 (s, 3H), 4.60 (b,1H), 6.45-6.70 (m, 4H), 6.85-7.00 (m, 2H), 7.10 (d, 1H).

### c) cis-1-(1-Cyclohexanecarbonyl-4-methyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 66B instead of Compound 1B. Yield: 100%.
¹H-NMR (δ): 0.80-3.30 (m, 27H), 3.80 (s, 3H), 5.10 (b, 1H), 6.50-6.65 (m, 2H), 6.75-6.90 (m, 1H), 7.00-7.35 (m, 4H).

### Example 67

### 1-(1-Cyclohexanecarbonyl-8-methyl-1,2,3,4-tetrahydroquinoline-2-ylmethyl)-4-(4-fluoro-2-methoxyphenyl)-piperazine

### a) 2,8-Dimethylquinoline (Compound 67A)

The title compound was prepared as reported for Compound 25A but using 2-methylaniline instead of 4-trifluoromethylaniline. Flash chromatography: petroleum ether - EtOAc 92:8. Yield: 38%.

### b) 8-Methyl-2-formylquinoline (Compound 67B)

A suspension of SeO₂ (6.66 g) and of Compound 67A (3.87 g) in EtOH (40 ml) was refluxed for 24 h. The reaction mixture was cooled to r.t.. The precipitated solid was filtered off, the solvent evaporated under vacuum and the crude was taken up with 2 N HCl (50 ml) and THF (200 ml) and stirred for 1 h. Afterwards, the mixture was diluted with water, alkalinised with NaHCO₃ and extracted with EtOAc. The organic layer was dried (Na₂SO₄) and evaporated to dryness affording the title compound used in the next step without further purification. Yield: 48%.

### c) 1-(4-Fluoro-2-methoxyphenyl)-4-(8-methylquinoline-2-ylmethyl)-piperazine (Compound 67C)

The title compound was synthesised using the same methodology described in Example 35A using Compound 67B instead of Compound 35A-B and 1-(4-fluoro-2-methoxyphenyl)-piperazine. The crude was purified by flash chromatography eluting with petroleum ether-EtOAc 7:3. Yield: 62 %.

### d) 1-(4-Fluoro-2-methoxyphenyl)-4-(8-methyl-1,2,3,4-tetrahydroquinoline-2-ylmethyl)-piperazine (Compound 67D)

The title compound was prepared as described for Compound 1B (procedure e) starting from Compound 67C instead of Compound 1C. The crude was purified by flash chromatography eluting with petroleum ether - EtOAc 7:3 to give the title compound (54%).

### e) 1-(1-Cyclohexanecarbonyl-8-methyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 67D instead of Compound 1B stirring at reflux in toluene. The crude was purified by flash chromatography eluting with petroleum ether - EtOAc 6:4 to give the title compound (80%).

### Example 68A

### 1-(4-Indolyl)-4-[1-(2-pyrrolylcarbonyl)-1,2,3,4-tetrahydroquinoline-2-ylmethyl]-piperazine

To a suspension of 2-pyrrolecarboxylic acid (0.109g) in CH₂Cl₂ (1 ml) was added through a syringe 2 ml of a 2.5 M solution of oxalyl chloride in CH₂Cl₂ followed after 30 min. by 0.020 ml of DMF. After 18 h stirring at r.t., the solution was evaporated (t = 40°C; p = 77000 pascals). To ensure the complete removal of oxalyl chloride the procedure of evaporation was repeated twice after having added CH₂Cl₂. The residue was dissolved in 1.85 ml of CH₂Cl₂ and added to a suspension of Compound 1B (0.084 g) and 4 eq. of polymer supported DIPEA. After 18 h stirring the mixture was filtered to remove the polymer supported DIPEA and the solution so obtained was filtered on cationic exchange resin (Mega Bond Elut®, Bonded Phase SCX), washing with a solution of NH₃ in MeOH (about 3%), evaporating the desired eluted fraction. The residue was purified by flash chromatography eluting with CH₂Cl₂ - MeOH 99.5:0.5 to 90:10 to give the title compound. [M+H]⁺= 440.3
¹H-NMR (DMSO, 400MHz, δ): 1.60-1.75 (m, 1H), 2.15-2.34 (m, 2H), 2.40-2.78 (m, 7H), 3.02-3.17 (m, 4H), 4.85-4.96 (m, 1H), 5.65-5.73 (m, 1H), 5.88-5.94 (br, 1H), 6.31-6.38 (br, 1H), 6.40-6.45 (m, 1H), 6.81-6.86 (br, 1H), 6.90-7.15 (m, 5H) 7.19-7.24 (br, 1H), 7.25-7.31 (br, 1H), 10.92-11.03 (br, 1H), 11.73 (br, 1H).

The following compounds were synthesized in the like manner using the proper carboxylic acid instead of 2-pyrrolecarboxylic acid. When necessary, after the filtration on the resin a further purification by flash chromatography was carried out (eluent: CH₂Cl₂ - MeOH gradient from 99.5:0.5 to 90:10). NMR spectra were registered at 400 MHz in DMSO.

### Example 68A1

### 1-[1-(4-Dimethylaminobenzoyl)-1,2,3,4-tetrahydroquinoline-2-ylmethyl]-4-(4-indolyl)-piperazine

[M+H]⁺= 494.3
¹H-NMR (δ): 1.73-1.85 (m, 1H), 2.15-2.34 (m, 2H), 2.45-2.82 (m, 7H), 2.88 (m, 6H), 3.00 3.17 (m, 4H), 4.73-4.85 (m, 1H), 6.31-6.38 (br, 1H), 6.40 (d, 1H), 6.52 (d, 2H), 6.57-6.63 (m, 1H), 6.85-7.03 (m, 4H), 7.10 (d, 2H), 7.15-7.23 (br, 2H), 10.92-11.03 (br, 1H).

### Example 68A2

### 1-(4-Indolyl)-4-[1-(2-phenylacetyl)-1,2,3,4-tetrahydroquinoline-2-ylmethyl]-piperazine

[M+H]⁺= 465.3

### Example 68A3

### 1-[1-(4-Cyanobenzoyl)-1,2,3,4-tetrahydroquinoline-2-ylmethyl]-4-(4-indolyl)-piperazine

[M+H]⁺= 476.2
¹H-NMR (δ): 1.63-1.75 (m, 1H), 2.16-2.27 (m, 1H), 2.30-2.45 (m, 1H), 2.45-2.82 (m, 7H), 2.88 (m, 6H), 3.00-3.17 (m, 4H), 4.73-4.85 (m, 1H), 6.31-6.38 (br, 1H), 6.40 (d, 1H), 6.52 (d, 2H), 6.57-6.63 (m, 1H), 6.85-7.03 (m, 4H), 7.10 (d, 2H), 7.15-7.23 (br, 2H), 10.9211.03 (br, 1H).

### Example 68A4

### 1-[1-(2-Chlorobenzoyl)-1,2,3,4-tetrahydroquinoline-2-ylmethyl]-4-(4-indolyl)-piperazine

[M+H]⁺ = 485.3
¹H-NMR (δ): 1.51-1.70 (m, 1H), 2.20-2.83 (m, 9H), 2.92-3.16 (m, 4H), 5.04-5.16 (m, 1H), 6.26-6.44 (m, 2H), 6.71-7.60 (m, 11H), 10.92-11.03 (br, 1H).

### Example 68A5

### 1-[1-(4-Chlorobenzoyl)-1,2,3,4-tetrahydroquinoline-2-ylmethyl]-4-(4-indolyl)-piperazine

[M+H]⁺= 485.3
¹H-NMR (δ): 1.61-1.77 (m, 1H), 2.13-2.26 (m, 1H), 2.29-2.81 (m, 8H), 2.97-3.17, (m, 4H), 4.71-4.88 (m, 1H), 6.31-6.35 (m, 1H), 6.37-6.47 (m, 1H), 6.56-6.69 (m, 1H), 6.86-7.08 (m, 4H), 7.15-7.31 (m, 4H), 7.33-7.38 (m, 2H), 10.94-11.01 (br, 1H).

### Example 68A6

### 1-[1-(3-Chlorobenzoyl)-1,2,3,4-tetrahydroquinoline-2-ylmethyl]-4-(4-indolyl)-piperazine

[M+H]⁺= 485.3
¹H-NMR (δ): 1.65-1.76 (m, 1H), 2.17-2.30 (m, 1H), 2.32-2.84 (m, 8H), 3.01-3.16 (m, 4H), 4.74-4.81 (m, 1H), 6.56-6.41 (m, 2H), 6.67-6.78 (m, 1H), 6.96-7.09 (m, 4H), 7.16-7.35 (m, 4H), 7.39-7.49 (m, 2H), 10.95-11.04 (br, 1H).

### Example 68A7

### 1-[1-(5-Benzo[1,3]dioxolylcarbonyl)-1,2,3,4-tetrahydroquinoline-2-ylmethyl]-4-(4-indolyl)-piperazine

[M+H]⁺ = 495.3
¹H-NMR (δ): 1.66-1.82 (m, 1H), 2.16-2.86 (m, 9H), 3.01-3.19 (m, 4H), 4.72-4.88 (m, 1H), 6.01 (s, 2H), 6.31-6.49 (m, 2H), 6.66-6.84 (m, 4H), 6.92-7.12 (m, 4H), 7.18-7.32 (m, 2H), 10.94-11.02 (br, 1H).

### Example 68A8

### 1-(1-Cyclopropylcarbonyl-1,2,3,4-tetrahydroquinoline-2-ylmethyl)-(4-indolyl)-piperazine

[M+H]⁺-415.3
¹H-NMR (δ): 0.76-1.05 (m, 4H),1.45-1.63 (m, 1H), 1.77-1.88 (m, 1H), 2.11-2.20 (m, 1H), 2.24-2.79 (m, 8H), 2.97*-*3.13 (m, 4H), 4.79-4.96 (m, 1H), 6.31-6.49 (m, 2H), 6.86-7.40 (m, 7H), 10.91-11.03 (br, 1H).

### Example 68A9

### 1-(4-Indolyl)-4-[1-(2-methylpzopionyl)-1,2,3,4-tetrahydroquinoline-2-ylmethyl]-piperazine

[M+H]⁺= 417.3
¹H-NMR (δ): 1.14 (d, 6H), 1.37-1.56 (m, 1H), 2.07-2.17 (m, 1H), 2.24-2.71 (m, 8H), 2.96-3.15 (m, 5H), 4.84-4.98 (m, 1H), 6.32-6.44 (m, 2H), 6.91-7.06 (m, 2H), 7.12-7.32 (m, 5H), 10.91-11.03 (br, 1H).

### Example 68A10

### 1-(4-Indolyl)-4-[1-(2-methoxyacetyl)-1,2,3,4-tetrahydroquinoline-2-ylmethyl]-piperazine

[M+H]⁺ = 419.3
¹H-NMR (δ): 1.46-1.64 (m, 1H), 2.11-2.73 (m, 9H), 2.99-3.10 (m, 4H), 3.26 (s, 3H), 3.92 (s, 2H), 4.72-4.88 (m, 1H), 6.29-6.44 (m, 2H), 6.90-7.11 (m, 2H), 7.16-7.25 (m, 4H), 7.31-7.44 (m, 1H), 10.92-11.03 (br, 1H).

### Example 68A11

### 1-(2-Cyclopropylacetyl-1,2,3,4-tetrahydroquinoline-2-ylmethyl)-4-(4-indolyl)piperazine

[M+H]⁺= 429.3
¹H-NMR (δ): 0.21- -0.04 (m, 1H), 0.03-0.11 (m, 1H), 0.25-0..45 (m, 2H), 0.84-0.95 (m, 1H), 1.41-1.58 (m, 1H), 2.06-2.35 (m, 4H), 2.42-2.70 (m, 7H), 3.00-3.14 (m, 4H), 4.81-5.02 (m, 1H), 6.31-6.49 (m, 2H), 6.88-7.02 (m, 2H), 7.11-7.34 (m, 5H), 14.91-11.00 (br, 1H).

### Example 68A12

### 1-(4-Indolyl)-4-[1-(3-methylbutanoyl)-1,2,3,4-tetahydroquinoline-2-ylmethyl]piperazine

[M+H]⁺= 431.3
¹H-NMR (δ): 0.9 (s, 6H), 1.45-1.63 (m, 1H), 1.85-2.48 (m, 8H), 2.55-3.75 (m, 4H), 2.98-3.15 (m, 4H), 4.85-5.04 (m, 1H), 6.31-6.50 (m, 2H), 6.90-7.35 (m, 7H), 10.91-11.03 (br, 1H).

### Example 68A13

### 1-(2-Furoyl-1,2,3,4-tetrahydroquinoline-2-ylmethyl)-4-(4-indolyl)-piperazine

[M+H]⁺= 441.3
¹H-NMR (δ): 1.66-1.82 (m, 1H), 2.20-2.33 (m, 2H), 2.51-2.80 (m, 7H), 2.90-3.15 (m, 4H), 4.75-4.88 (m, 1H), 6.23-6.61 (m, 4H), 6.81-7.15 (m, 5H), 7.17-7.27 (m, 2H), 7.60-6.70 (m, 1H), 10.94-11.02 (br, 1H).

### Example 68A14

### 1-(4-Indolyl)-4-[1-(5-isoxazolylcarbonyl)-1,2,3,4-tetrahydroquinoline-2-ylmethyl]-piperazine

[M+H]⁺= 442.2
¹H-NMR (δ): 1.54-1.73 (m, 1H), 2.25-2.51 (m, 2H), 2.68-2.81 (m, 7H), 3.12-3.31 (m, 4H), 4.80-5.05 (m, 1H), 6.35-6.61 (m, 3H), 6.80.7.31 (m, 8H), 10.96-11.04 (br, 1H).

### Example 68A15

### 1-(4-Indolyl)-4-[1-(3-tetrahydrofurylcarbonyl)-1,2,3,4-tetrahydroquinoline-2-ylmethyl]-piperazine

[M+H]⁺= 445.3

### Example 68A16

### 1-(4-Indolyl)-4-[1-(2-tetrahydrofurylcarbonyl)-1,2,3,4-tetrahydroquinoline-2-ylmethyl]-piperazine

[M+H]⁺= 445.3
¹H-NMR (δ): 1.34-2.41 (m, 9H), 2.51-2.70 (m, 5H), 2.98-3.15 (m, 4H), 3.61-3.82 (m, 2H), 4.53-4.82 (m, 2H), 6.28-6.48 (m, 2H), 6.81-7.03 (m, 2H), 7.11-7.32 (m, 5H), 10.95-11.03 (br, 1H).

### Example 68A17

### 1-[1-(3,3-Dimethylbutanoyl)-1,2,3,4-tetrahydroquinoline-2-ylmethyl]-4-(4-indolyl)-piperazine

[M+H]⁺= 445.4
¹H-NMR (δ): 0.85 (s, 9H), 1.39-1.52 (m, 1H), 2.01-2.12 (m, 1H), 2.202.31 (m, 3H), 2.35-2.48 (m, 2H), 2.51-2.63 (m, 5H), 2.98-3.12 (m, 4H), 4.81-4.97 (m, 1H), 6.28-6.39 (m, 2H), 6.83-7.03 (m, 2H), 7.07-7.31 (m, 5H), 10.89-11.01 (br, 1H).

### Example 68A18

### 1-[1-(2-Acetoxyacetyl)-1,2,3,4-tetrahydroquinoline-2-ylmethyl]-4-(4-indolyl)-piperazine

[M+H]⁺ = 447.3
¹H-NMR (δ): 1.41-1.54 (m, 1H), 2.01 (s, 3H), 2.20-2.45 (m, 3H), 2.48-2.52 (m, 1H), 2.55-2.69 (m, 5H), 3.01-3.17 (m, 4H), 4.68-4.85 (m, 1H), 4.98-5.03 (m, 2H), 6.26-6.45 (m, 2H), 6.85-7.01 (m, 2H), 7.04-7.46 (m, 5H), 10.91-11.03 (br, 1H).

### Example 68A19

### 1-(4-Indolyl)-4-[1-(2-thienylcarbonyl-1,2,3,4-tetrahydroquinoline-2-ylmethyl]-piperazine

[M+H]⁺= 457.2
¹H-NMR (δ): 1.56-1.75 (m, 1H), 2.12-2.25 (m, 1H), 2.27-2.48 (m, 1H), 2.51-2.58 (m, 1H), 2.60-2.77 (m, 6H), 3.02-3.19 (m, 4H), 4.81-4.94 (m, 1H), 6.26-6.48 (m, 2H), 6.81-7.11 (m, 6H), 7.12-7.38 (m, 3H), 7.61-7.70 (m, 1H), 10.98-11.05 (br, 1H).

### Example 68A20

### 1-(4-Indolyl)-4-[1-(3-thienylcarbonyl)-1,2,3,4-tetrahydroquinoline-2-ylmethyl]-piperazine

[M+H]⁺ = 457.2
¹H-NMR (δ): 1.58-1.76(m, 1H), 2.16-2.25 (m, 1H), 2.26-2.47 (m, 1H), 2.52-2.68 (m, 4H), 2.70-2.78 (m, 3H), 3.05-3.21 (m, 4H), 4.76-4.82 (m, 1H), 6.30-6.48 (m, 2H), 6.68-6.81 (m, 2H), 6.84-7.12 (m, 4H), 7.17-7.26 (m, 2H), 7.36-7.40 (m, 1H), 7.58-7.63 (m, 1H), 10.95-11.00 (br, 1H).

### Example 68A21

### 1-[1-(2-Cyclohexylacetyl)-1,2,3,4-tetrahydroquinoline-2-ylmethyl]-4-(4-indolyl)-piperazine [M+H]⁺ = 471.3

### Example 68A22

### 1-(4-Indolyl)-4-[1-(3-trifluoromethylbenzoyl)-1,2,3,4-tetrahydroquinoline-2-ylmethyl]-piperazine

[M+H]⁺= 519.3
¹H-NMR (δ): 1.68-1.78 (m, 1H), 2.38-2.50 (m, 1H), 2.60-2.82 (m, 8H), 3.02-3.17 (m, 4H), 4.75-4.80 (m, 1H), 6.25-6.48 (m, 2H), 6.78-7.16 (m, 3H), 7.19-7.27 (m, 2H), 7.48-7.68 (m, 3H), 7.70-7.77 (m, 2H), 8.16-8.24 (m, 1H), 10.93-11.02 (br, 1H).

### Example 68A23

### 1-(4-Indolyl)-4-[1-(4-trifluoromethylbenzoyl)-1,2,3,4-tetrahydroquinoline-2-ylmethyl]-piperazine

[M+H]⁺= 519.3
¹H-NMR (δ) : 1.60-1.78 (m, 1H), 2.18-2.35 (m, 1H), 2.40-2.51 (m, 1H), 2.61-2.71 (m, 4H), 2.76-2.81 (m, 3H), 3.03-3.17 (m, 4H), 4.77-4.91 (m, 1H), 6.26-6.50 (m, 2H), 6.87-7.13 (m, 5H), 7.18-7.26 (m, 2H), 7.49-7.52 (m, 2H), 7.65-7.73 (m, 2H), 10.95-11.02 (br, 1H).

### Example 68A24

### 1-(4-Indolyl)-4-[1-(3-phenylpropionyl)-1,2,3,4-tetrahydroquinoline-2-ylmethyl]piperazine

[M+H]⁺= 479.3
¹H-NMR (δ): 1.35-1.53 (m, 1H), 2.05-2.37 (m, 4H), 2.51-2.69 (m, 5H), 2.71-2.90 (m, 4H), 2.93-3.15 (m, 4H), 4.73-4.97, (m, 1H), 6.31-6.48 (m, 2H), 6.90-7.35 (m, 12H), 10.90-11.00 (br, 1H).

### Example 68A25

### 1-(4-Indolyl)-4-[1-(2-methoxybenzoyl)-1,2,3,4-tetrahydroquinoline-2-ylmethyl]piperazine

[M+H]⁺= 481.2

### Example 68A26

### 1-(4-Indolyl)-4-[1-(4-methoxybenzoyl)-1,2,3,4-tetrahydroquinoline-2-ylmethyl]piperazine

[M+H]⁺=481.2
¹H-NMR (δ): 1.68-1.87 (m, 1H), 2.17-2.25 (m, 1H), 2.27-2.41 (m, 1H), 2.53-2.69 (m, 4H), 2.71-2.77 (m, 3H), 3.04-3.14 (m, 4H), 3.68 (s, 3H), 4.75-4.85 (m, 1H), 6.28-6.49 (m, 2H), 6.58-6.76 (m, 1H), 6.77-6.80 (m, 2H), 6.81-6.89 (m, 2H), 6.90-7.00 (m, 2H), 7.19-7.26 (m, 4H), 10.98-11.05 (br, 1H).

### Example 68A27

### 1-[1-(4-Fluorophenylacetyl)-1,2,3,4-tetrahydroquinoline-2-ylmethyl]-4-(4-indolyl)-piperazine

[M+H]⁺=483.3
¹H-NMR (δ): 1.35-1.51 (m, 1H), 2.07-2.39 (m, 3H), 2.52-2.69 (m, 4H), 2.98-3.17 (m, 4H), 3.57 (s, 2H), 3.59-3.83 (m, 2H), 4.79-4.93 (m, 1H), 6.27-6.43 (m, 2H), 6.87-7.36 (m, 11H), 10.93-11.01 (br, 1H).

### Example 68A28

### 1-[1-(2,6-Difluorobenzoyl)-1,2,3,4-tetrahydroquinoline-2-ylmethyl]-4-(4-indolyl)-piperazine

[M+H]⁺= 487.2
¹H-NMR (δ): 1.47-1.62 (m, 1H), 2.20-2.28 (m, 1H), 2.29-2.48 (m, 1H), 2.53-2.68 (m, 3H), 2.70-2.77 (m, 4H), 3.03-3.15 (m, 4H), 5.02-5.11 (m, 1H), 6.30-6.45 (m, 2H), 6.75-6.81 (m, 1H), 6.82-7.11 (m, 4H), 7.12-7.31 (m, 5H), 10.94-11.00 (br, 1H).

### Example 68A29

### 1-(4-Indolyl)-4-[1-(2-methoxyphenylacetyl)-1,2,3,4-tetrahydroquinoline-2-ylmethyl]-piperazine

[M+H]⁺= 495.2
¹H-NMR (δ): 1.45-1.58 (m, 1H), 2.15-2.31 (m, 2H), 2.52-2.73 (m, 3H), 3.03-3.15 (m, 4H), 3.61-3.73 (m, 4H), 3.74 (s, 2H), 4.81-4.50 (m, 1H), 6.29-.6.48 (m, 2H), 6.76-7.04 (m, 5H), 7.10-7.28 (m, 6H), 10.94-10.99 (br, 1H).

### Example 68A30

### 1-[1-(4-Chlorophenylacetyl)-1,2,3,4-tetrahydroquinoline-2-ylmethyl]-4-(4-indolyl)-piperazine

[M+H]⁺= 499.3

### Example 68A31

### 1-(4-Indolyl)-4-[1-[2-(4-trifluoromethylphenyl)-acetyl]-1,2,3,4-tetrahydroquinoline]-2-ylmethyl]-piperazine

[M+H]⁺= 533.3

### Example 68A32

### 1-(4-Indolyl)-4-[1-(2-pyridylcarbonyl)-1,2,3,4-tetrahydroquinoline-2-ylmethyl]-piperazine

[M+H]⁺= 452.3

### Example 68A33

### 1-(4-Indolyl)-4-[1-(3-pyridylcarbonyl)-1,2,3,4-tetrahydroquinoline-2-ylmethyl]-piperazine

[M+H]⁺= 452.3

### Example 68A34

### 1-(4-Indolyl)-4-[1-(4-pyridylcarbonyl)-1,2,3,4-tetrahydroquinoline-2-ylmethyl]-piperazine

[M+H]⁺= 452.3

### Example 68A35

### 1-[1-(3,5-Dimethyl-4-isoxazolylecarbonyl)-1,2,3,4-tetrahydroquinoline-2-ylmethyl]-4-(4-indolyl)-piperazine

[M+H]⁺ = 470.3

### Example 68A36

### 1-[1-(2,2-Dimethylpropionyl)-1,2,3,4-tetrahydroquinoline-2-ylmethyl]-4-(4-indolyl)-piperazine

[M+H]⁺= 431.3
¹H-NMR (δ): 1.21 (s, 9H), 1.43-1.50 (m, 1H), 2.20-2.28 (m, 1H), 2.31-2.41 (m, 2H), 2.42-2.51 (m, 2H), 2.60-2.73 (m, 4H), 3.03-3.17 (m, 4H), 4.95-5.01 (m, 1H), 6.25-6.48 (m, 2H), 6.82-7.02 (m, 3H), 7.18-7.28 (m, 4H), 10.95-11.01 (br, 1H).

### Example 68A37

### 1-(4-Indolyl)-4-[1-[2-(3-pyridyl)-acetyl]-1,2,3,4-tetrahydroquinoline-2-ylmethyl]-piperazine

[M+H]⁺= 466.3

### Example 68A38

### 1-[1-(1-Acetyl-4-piperidinylcarbonyl)-1,2,3,4-tetrahydroquinoline-2-ylmethyl]-4-(4-indolyl)-piperazine

[M+H]⁺= 500.3

### Example 68A39

### 1-(4-Indolyl)-4-[1-[2-(2-thienylacetyl)]-1,2,3,4-tetrahydroquinoline-2-ylmethyl]-piperazine

[M+H]⁺= 471.2

### Example 68A40

### 1-(4-Indolyl)-4-[1-[2-(3-thienylacetyl)]-1,2,3,4-tetrahydroquinoline-2-ylmethyl]-piperazine

[M+H]⁺= 471.3

### Example 68A41

### 1-(4-Indolyl)-4-[1-[3-(3-pyridylpropionyl)]-1,2,3,4-tetrahydroquinoline-2-ylmethyl]-piperazine

[M+H]⁺= 480.3

### Example 68A42

### 1-(4-Indolyl)-4-[1-(2-phenoxyacetyl)-1,2,3,4-tetrahydroquinoline-2-ylmethyl]-piperazine

[M+H]⁺= 481.3

### Example 68A43

### 1-(4-Indolyl)-4-[1-[2-(4-methoxyphenylacetyl)]-1,2,3,4-tetrahydroquinoline-2-ylmethyl]-piperazine

[M+H]⁺= 495.3

### Example 68A44

### 1-(4-Indolyl)-4-[1-(2-phenylmethoxyacetyl)-1,2,3,4-tetrahydroquinoline-2-ylmethyl]-piperazine

[M+H]⁺= 495.3

### Example 68A45

### 1-[1-[2-(2-Chlorophenylacetyl)]-1,2,3,4-tetrahydroquinoline-2-ylmethyl]-4-(4-indolyl)-piperazine

[M+H]⁺= 499.2

### Example 68A46

### 1-{1-[2-(N-Benzoyl-N-methyl)-aminoacetyl]-1,2,3,4-tetrahydroquinoline-2-ylmethyl}-4-(4-indolyl)-piperazine

[M+H]⁺ = 522.3

### Example 68A47

### 1-(4-Indolyl)-4-[1-(1-methyl-3-indolylcarbonyl)-1,2,3,4-tetrahydroquinoline-2-ylmethyl]-piperazine

[M+H]⁺ = 504.3

### Example 68A48

### 1-[1-[2-(4-Dimethylaminophenylacetyl)]-1,2,3,4-tetrahydroquinoline-2-ylmethyl]-4-(4-indolyl)-piperazine

[M+H]⁺= 508.3

### Example 68A49

### 1-[1-(5-Benzo[1,3]dioxolylacetyl)-1,2,3,4-tetrahydroquinoline-2-ylmethyl]-4-(4-indolyl)-piperazine

[M+H]⁺= 509.3

### Example 68A50

### 1-[1-[2-(2-Chlorophenoxyacetyl)-1,2,3,4-tetrahydroquinoline-2-ylmethyl]-4-(4-indolyl)-piperazine

[M+H]⁺= 515.3

### Example 68A51

### 1-[1-[2-(4-Chlorophenoxyacetyl)-1,2,3,4-tetrahydroquinoline-2-ylmethyl]-4-(4-indolyl)-piperazine

[M+H]⁺= 515.3

### Example 68A52

### 1-(4-Indolyl)-4-[1-(2-morpholino-5-pyridylcarbonyl]-1,3,3,4-tetrahydroquinoline-2-ylmethyl]-piperazine

[M+H]⁺= 537.3

### Example 68A53

### 1-(4-Indolyl)-4-[1-(5-methyl-4-isoxazolylcarbonyl)-1,2,3,4-tetrahydroquinoline-2-ylmethyl]-piperazine

[M+H]⁺= 456.2

### Example 68A54

### 1-(4-Indolyl)-4-[1-(5-oxo-2-pyrrolidinylacetyl)-1,2,3,4-tetrahydroquinoline-2-ylmethyl]-piperazine

[M+H]⁺= 472.3

### Example 68A55

### 1-(4-Indolyl)-4-[1-(2-methyl-5-piperidinosulphonyl-3-furoyl)-1,2,3,4-tetrahydroquinoline-2-ylmethyl]-piperazine

### Example 68A56

### 1-(4-Indolyl)-4-[1-(2-methyl-5-morpholinosulphonyl-3-furoyl)-1,2,3,4-tetrahydroquinoline-2-ylmethyl]-piperazine

### Example 68A57

### 1-(4-Indolyl)-4-{1-[1-(p-tolylsulphonyl)-3-pyrrolylcarbonyl]-1,2,3,4-tetrahydroquinoline-2-ylmethyl}-piperazine

### Example 68A58

### 1-(4-Indolyl)-4-[1-(2-methyl-5-dimethylsulphamoyl-3-furoyl)-1,2,3,4-tetrahydroquinoline-2-ylmethyl]-piperazine

[M+H]⁺= 562

### Example 68A59

### 1-(4-Indolyl)-4-{1-[2-methyl-5-(1-pyrrolidinylsulphamoyl)-3-furoyl)-1,2,3,4-tetrahydroquinoline-2-ylmethyl]-piperazine

[M+H]⁺= 588

### Example 68A60

### 1-[(1-Adamantylcarbonyl)-1,2,3,4-tetrahydroquinoline-2-ylmethyl]-4-(4-indolyl)-piperazine

### Example 68A61

### 1-(4-Indolyl)-4-[1-(3-phenoxypropionyl)-1,2,3,4-tetrahydroquinoline-2-ylmethyl]-piperazine

### Example 68A62

### 1-(4-Indolyl)-1-[1-(4-phenoxybutanoyl)-1,2,3,4-tetrahydroquinoline-2-ylmethyl]-piperazine

### Example 68A63

### 1-(4-Indolyl)-4-[1-(2-fluorophenylacetyl)-1,2,3,4-tetrahydroquinoline-2-ylmethyl]-piperazine

### Example 68A64

### 1-(4-Indolyl)-4-[1-(2-trifluoromethyl-phenylacetyl)-1,2,3,4-tetrahydroquinoline-2-ylmethyl]-piperazine

### Example 68A65

### 1-[1-(2-Bicyclo [2.2.2] octylcarbonyl)-1,2,3,4-tetrahydroquinoline-2-ylmethyl]-4-(4-indolyl)-piperazine

### Example 68A66

### 1-(4-Indolyl)-1-[1-(4-phenylbutanoyl)-1,2,3,4-tetrahydroquinoline-2-ylmethyl]-piperazine

### Example 68A67

### 1-(4-Indolyl)-4-[1-(3-methoxybenzoyl)-1,2,3,4-tetrahydroquinoline-2-ylmethyl]-piperazine

### Example 68A68

### 1-[1-(4-Hydroaybenzoyl)-1,2,3,4-tetrahydroquinoline-2-ylmethyl]-4-(4-indolyl)-piperazine

### Example 68A69

### 1-[1-(4-Ethoxybenzoyl)-1,2,3,4-tetrahydroquinoline-2-ylmethyl]-4-(4-indolyl)-piperazine

### Example 68A70

### 1-(4-Indolyl)-4-[1-(4-methylbenzoyl)-1,2,3,4-tetrahydroquinoline-2-ylmethyl]-piperazine

### Example 68A71

### 1-[1-[(N-Benzyl-N-methanesulphonyl)-2-aminoacetyl]-1,2,3,4-tetrahydroquinoline-2-ylmethyl]-4-(4-indolyl)-piperazine

### Example 68A72

### 1-(4-Indolyl)-4-[1-(2-(S)-5-oxopyrrolidinylcarbonyl)-1,2,3,4-tetrahydroquinoline-2-ylmethyl]-piperazine

[M+H]⁺= 458.2

### Example 68A73

### 1-(4-Indolyl)-4-[1-(2-(R)-5-oxopyrrolidinylcarbonyl)-1,2,3,4-tetrahydroquinoline-2-ylmethyl]-piperazine

[M+H]⁺= 458

### Example 69

### General method

To a solution of Compound 1B was added a solution of the appropriate amine (1 eq.) in anhydrous THF (5 ml); then via a syringe was added, under magnetic stirring at r.t. in a nitrogen atmosphere, the isocyanate as such or disssolved in about 1 ml of anhydrous THF. The reaction mixture was reacted at r.t. or at reflux for 20-50 h, then was filtered on a cationic resin (Mega Bond Elut®, Bonded Phase SCX) washing with CH₂Cl₂ - MeOH 1:1 to remove the not alkaline impurities followed by a 3% solution of NH₃ in MeOH to recover the title products. When necessary a further purification by flash chromatography was carried out (eluent: CH₂Cl₂- MeOH gradient from 99.5:0.5 to 90:10).

The following compounds were synthesised according to the above procedure:

### Example 69A

### 1-(4-Indolyl)-4-[1-(2-methylanilinocarbonyl)-1,2,3,4-tetrahydroquinoline-2-ylmethyl]-piperazine

[M+H]⁺= 480.2

### Example 69A1

### 1-(4-Indolyl)-4-[1-(3-methylanilinocarbonyl)-1,2,3,4-tetrahydroquinoline-2-ylmethyl]-piperazine

[M+H]⁺= 480.2

### Example 69A2

### 1-[1-(4-Fluoroanilinocarbonyl)-1,2,3,4-tetrahydroquinoline-2-ylmethyl)-4-(4-indolyl)-piperazine

[M+H]⁺= 484.3

### Example 69A3

### 1-(4-Indolyl)-4-[1-(4-methylanilinocarbonyl)-1,2,3,4-tetrahydroquinoline-2-ylmethyl]-piperazine

[M+H]⁺= 480.3

### Example 69A4

### 1-(4-Indolyl)-4-[1-((1S)-1-phenylethylaminocarbonyl)-1,2,3,4-tetrahydroquinoline-2-ylmethyl]-piperazine

[M+H]⁺= 494.4

### Example 69A5

### 1-(4-Indolyl)-4-[1-((1R)-1-phenylethylaminocarbonyl)-1,2,3,4-tetrahydroquinoline-2-ylmethyl)-piperazine (diastereoisomer A)

[M+H]⁺= 494.4

### Example 69A6

### 1-(4-Indolyl)-4-[1-((1R)-1-phenylethylaminocarbonyl)-1,2,3,4-tetrahydroquinoline-2-ylmethyl]-piperazine (1:1 diastereoisomers mixture)

[M+H]⁺ = 494.3

### Example 69A7

### 1-(4-Indolyl)-4-[1-(1-methylethylaminocarbonyl)-1,2,3,4-tetrahydroquinoline-2-ylmethyl]-piperazine

[M+H]⁺= 432.3

### Example 69A8

### 1-(4-Indolyl)-4-[1-methylaminothiocarbonyl-1,2,3,4-tetrahydroquinoline-2-ylmethyl)-piperazine

[M+H]⁺= 420.2

### Example 69A9

### 1-(1-Benzylaminocarbonyl-1,2,3,4-tetrahydroquinoline-2-ylmethyl)-4-(4-indolyl)-piperazine

[M+H]⁺= 480.3

### Example 69A10

### 1-(4-Indolyl)-4-[1-(2-phenylethylaminocarbonyl)-1,2,3,4-tetrahydroquinoline-2-ylmethyl]-piperazine

[M+H]⁺= 494.3

### Example 69A11

### 1-(4-Indolyl)-4-(1-pentylaminocarbonyl-1,2,3,4-tetrahydroquinoline-2-ylmethyl]-piperazine

[M+H]⁺= 460.3

### Example 69A12

### 1-[1-(1,1-Dimethylethylaminocarbonyl-1,2,3,4-tetrahydroquinoline-2-ylmethyl]-4-(4-indolyl)-piperazine

[M+H]⁺= 446.3

### Example 69A13

### 1-(4-Indolyl)-4-[(4-methoxyphenylaminocarbonyl)-1,2,3,4-tetrahydroquinoline-2-ylmethyl]-piperazine

### Example 69A14

### 1-(4-Indolyl)-4-[1-(4-methoxyphenylmethylaminocarbonyl)-1,2,3,4-tetrahydroquinoline-2-ylmethyl]-piperazine

### Example 70

Sulphonyl chloride was dissolved in 1.85 ml of CH₂Cl₂ and added to a suspension of Compound 1B and 4 eq. of polymer supported DIPEA. After 18 h stirring, the mixture was filtered to remove the polymer supported DIPEA and the solution so obtained was filtered on cationic exchange resin (Mega Bond Elut®, Bonded Phase SCX), washing with a solution of NH₃ in MeOH (about 3%), evaporating the desired eluted fraction. The residue was purified by flash chromatography eluting with CH₂Cl₂ - MeOH gradient from 99.5:0.5 to 90:10 to give the title compound.

The following compounds were synthesised according to the above procedure:

### Example 70A

### 1-(4-Indolyl)-4-[1-phenylsulphonyl-1,2,3,4-tetrahydroquinoline-2-ylmethyl]-piperazine

[M+H]⁺= 487.2

### Example 70A1

### 1-(4-Indolyl)-4-[1-(2-thienylsulphonyl)-1,2,3,4-tetrahydroquinoline-2-ylmethyl]-piperazine

[M+H]⁺= 493.7

### Example 70A2

### 1-(4-Indolyl)-4-[1-(4-methoxyphenylsulphonyl)-1,2,3,4-tetrahydroquinoline-2-ylmethyl]-piperazine

### Example 70A3

### 1-(4-Indolyl)-4-[1-(4-methylphenylsulphonyl)-1,2,3,4-tetrahydroquinoline-2-ylmethyl]-piperazine

### Example 70A4

### 1-[1-(4-Fluorophenylsulphonyl)-1,2,3,4-tetrahydroquinoline-2-ylmethyl]-4-(4-indolyl)-piperazine

### Example 70A5

### 1-[1-(4-Cyanophenylsulphonyl)-1,2,3,4-tetrahydroquinoline-2-ylmethyl]-4-(4-indolyl)-piperazine

### Example 70A6

### 1-(4-Indolyl)-4-[1-benzylsulphonyl-1,2,3,4-tetrahydroquinoline-2-ylmethyl]-piperazine

[M+H]⁺= 501.2

### Example 71

This procedure is useful for the acylation of Compound 1B with acids containing the *tert*butoxycarbonyl moiety.

To a solution of compound 1B (100 mg, 1 eq.), of the appropriate acid (1.5 eq.) and of 4 DMAP (10.5 mg, 0.3 eq.) in anhydrous CH₂Cl₂ (5 ml) was added DCC (870 µl of a 1 M solution, 3 eq.) via a syringe, stirring at r.t. under nitrogen atmosphere. After at least 18 h, the dicycloheaylurea by-product precipitated. The reaction mixture was dissolved in MeOH and the solution was eluted through a cationic resin (Mega Bond Elut®, Bonded Phase SCX) washing with a solution of NH₃ in MeOH (about 3%). When necessary a further purification was carried out by flash chromatography (CH₂Cl₂ - MeOH gradient from 99.5 : 0.5 to 90 : 10).

By the above procedure the following compounds of Example 71 were synthesized:

### Example 71A

### 1-(1-tert-Butoxycarbonylaminoacetyl-1,2,3,4-tetrahydroquinoline-2-ylmethyl)-4-(4-indolyl)-piperazine

[M+H]⁺ = 504.5

### Example 71A1

### 1-[1-(3-tert-Butoxycarbonylamino-propionyl)-1,2,3,4-tetrahydroquinoline-2-ylmethyl]-4-(4-indolyl)-piperazine

[M+H]⁺= 518.3

### Example 71A2

### 1-[1-(1-tert-Butoxycarbonyl-3-indolyl-acetyl)-1,2,3,4-tetrahydroquinoline-2-ylmethyl]-4-(4-indolyl]-piperazine

### Example 71A3

### 1-[1-(1-tert-Butoxycarbonyl-4-piperidylcarbonyl)-1,2,3,4-tetrahydroquinoline-2-ylmethyl]-4-(4-indolyl)-piperazine

### Example 71A4

### 1-[1-(1-tert-Butoxycarbonyl-2R-pyrrolidin-2-ylcarbonyl)-1,2,3,4-tetrahydroquinoline-2-ylmethyl]-4-(4-indolyl)-piperazine

### Example 71A5

### 1-[1-(1-tert-Butoxycarbonyl-2S-pyrrolidin-2-ylcarbonyl)-1,2,3,4-tetrahydroquinoline-2-ylmethyl]-4-(4-indolyl)-piperazine

### Example 72

### General method:

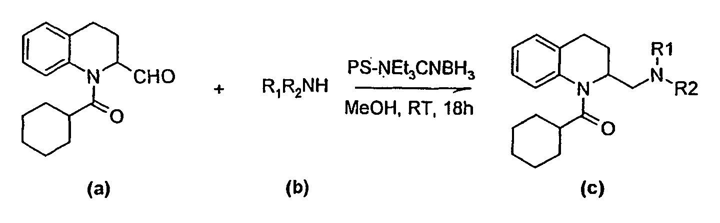

A solution of the appropriate amine (1.2 eq.) in MeOH (1.5 ml) and a solution of Compound 35A-B (100 mg, 1 eq.) in MeOH (1.5 ml) were sequentially added via a syringe to polymer supported NEt₃CNBH₃ (317 mg, 2 eq., 2.32 mmol/g) under nitrogen atmosphere. After stirring for 18 h at r.t., the polymer supported NEt₃CNBH₃ was removed by filtration and the filtrate was purified on a cationic exchange resin (Mega Bond Elut®, Bonded Phase SCX) washing with CH₂Cl₂ - MeOH 1:1 to remove the not alkaline impurities; then with a 3% solution of NH₃ in MeOH to yield the wished products. The solvent was evaporated and the residue dissolved in CH₂Cl₂ (5 ml) and taken up with polymer supported NCO (250 mg, 1.51 mmol/g). After 2 h the resin was removed by filtration and the solvent evaporated. When necessary a further purification was carried out by flash chromatography (CH₂Cl₂ - MeOH gradient from 99 : 1 to 90 : 10).

By the above procedure the following compounds of Example 72 were synthesized

### Example 72A

### 1-(1-Cyclohexanecarbonyl-1,2,3,4-tetrahydroquinolin-2-ylmethyl)-2-(α-hydroxybenzyl)-piperidine

### Example 72A1

### 1-(1-Cyclohexanecarbonyl-1,2,3,4-tetrahydroquinolin-2-ylmethyl)-4-(2,6-dimethylphenyl)-piperidine

### Example 72A2

### 2-(4-Chlorophenyl)-1-(1-cyclohexanecarbonyl-1,2,3,4-tetrahydroquinolin-2-ylmethyl)-piperidine

### Example 72A3

### 1-(1-Cyclohexanecarbonyl-1,2,3,4-tetrahydroquinolin-2-ylmethyl)-4-phenylpiperidine

### Example 72A4

### 4-Benzoyl-1-(1-Cyclohexanecarbonyl-1,2,3,4-tetrahydroquinolin-2-ylmethyl)-piperidine

### Example 72A5

### 1-(1-Cyclohexanecarbonyl-1,2,3,4-tetrahydroquinolin-2-ylmethyl)-4-(2-nitrophenyl)-piperazine

### Example 72A6

### 1-(1-Cyclohexanecarbonyl-1,2,3,4-tetrahydroquinolin-2-ylmethyl)-4-(3-trifluoromethyl-2-pyridyl)-piperazine

### Example 72A7

### 1-(1-Cyclohexanecarbonyl-1,2,3,4-tetrahydroquinolin-2-ylmethyl)-4-(4-fluoro-2-nitrophenyl)-piperazine

### Example 72A8

### 1-(1-Cydohexanecarbonyl-1,2,3,4-tetrahydroquinolin-2-ylmethyl)-4-(2,3-dichlorophenyl)-piperazine

### Example 72A9

### 1-(1-Cyclohexanecarbonyl-1,2,3,4-tetrahydroquinolin-2-ylmethyl)-4-(3-cyano-2-pyridyl)-piperazine

### Example 72A10

### 1-(1-Cyclohexanecarbonyl-1,2,3,4-tetrahydroquinolin-2-ylmethyl)-4-(3,4-dichlorophenyl)-piperazine

### Example 72A11

### 1-(1-Cyclohexanecarbonyl-1,2,3,4-tetrahydroquinolin-2-ylmethyl)-4-(2-ethylphenyl)-piperazine

### Example 72A12

### 1-(4-Chloro-3-trifluoromethylphenyl)-4-(1-cyclohexanecarbonyl-1,2,3,4-tetrahydroquinolin-2-ylmethyl)-piperazine

### Example 72A13

### 1-(2-Cyanophenyl)-4-(1-cyclohexanecarbonyl-1,2,3,4-tetrahydroquinolin-2-ylmethyl)-piperazine

### Example 72A14

### 1-(1-Cyclohexanecarbonyl-1,2,3,4-tetrahydroquinolin-2-ylmethyl)-4-(2,4-dimethoxyphenyl)-piperazine

### Example 72A15

### 1-(1-Cydohexanecarbonyl-1,2,3,4-tetrahydroquinolin-2-ylmethyl)-4-(2-methylthiophenyl)-piperazine

### Example 72A16

### 1-(1-Cyclohexanecarbonyl-1,2,3,4-tetrahydroquinolin-2-ylmethyl)-4-(3,4-dimethoxyphenyl)-piperazine

### Example 72A17

### 1-(1-Cyclohexanecarbonyl-1,2,3,4-tetrahydroquinolin-2-ylmethyl)-4-(3-hydroxyphenyl)-piperazine

### Example 72A18

### 1-(1-Cyclohexanecarbonyl-1,2,3,4-tetrahydroquinolin-2-ylmethyl)-4-(2,4-difluorophenyl)-piperazine

### Example 72A19

### 1-(5-Chloro-2-methoxyphenyl)-4-(1-cyclohexanecarbonyl-1,2,3,4-tetrahydroquinolin-2-ylmethyl)-piperazine

### Example 72A20

### 1-(1-Cyclohexanecarbonyl-1,2,3,4-tetrahydroquinolin-2-ylmethyl)-4-(6-methyl-2-pyridyl)-piperazine

### Example 72A21

### 1-(1-Cyclohexanecarbonyl-1,2,3,4-tetrahydroquinolin-2-ylmethyl)-4-(4-methylphenyl)-piperazine

### Example 72A22

### 1-(1-Cyclohexanecarbonyl-1,2,3,4-tetrahydroquinolin-2-ylmethyl)-4-(4-trifluoromethylphenyl)-piperazine

### Example 72A23

### 1-(3-Chlorophenyl)-4-(1-cyclohexanecarbonyl-1,2,3,4-tetrahydroquinolin-2-ylmethyl)-piperazine

### Example 72A24

### 1-(2-Chlorophenyl)-4-(1-cyclohexanecarbonyl-1,2,3,4-tetrahydroquinolin-2-ylmethyl)-piperazine

### Example 72A25

### 1-(1-Cyclohexanecarbonyl-1,2,3,4-tetrahydroquinolin-2-ylmethyl)-4-(3-methoxyphenyl)-piperazine

### Example 72A26

### 1-(1-Cyclohexanecarbonyl-1,2,3,4-tetrahydroquinolin-2-ylmethyl)-4-(4-fluorophenyl)-piperazine

[M+H]⁺= 436.2

### Example 72A27

### 1-(4-Acetylphenyl)-4-(1-cyclohexanecarbonyl-1,2,3,4-tetrahydroquinolin-2-ylmethyl)-piperazine

[M+H]⁺= 460.2

### Example 72A28

### 1-(1-Cyclohexanecarbonyl-1,2,3,4-tetrahydroquinolin-2-ylmethyl)-4-(4-hydroxyphenyl)-piperazine

[M+H]⁺ = 434.03

### Example 72A29

### 1-(5-Chloro-2-methylphenyl)-4-(1-cyclohexanecarbonyl-1,2,3,4-tetrahydroquinolin-2-ylmethyl)-piperazine

[M+H]⁺= 466.2

### Example 72A30

### 1-(1-Cyclohexanecarbonyl-1,2,3,4-tetrahydroquinolin-2-ylmethyl)-4-(3-methylphenyl)-piperazine

[M+H]⁺= 432

### Example 72A31

### 1-(1-Cyclohexanecarbonyl-1,2,3,4-tetrahydroquinolin-2-ylmethyl)-4-(2-nitro-4-trifluoromethylphenyl)-piperazine

[M+H]⁺= 531

### Example 72A32

### 1-(1-Cyclohexanecarbonyl-1,2,3,4-tetrahydroquinolin-2-ylmethyl)-4-(5-trifluoromethyl-2-pyridyl)-piperazine

[M+H]⁺= 487

### Example 72A33

### 1-(1-Cyclohexanecarbonyl-1,2,3,4-tetrahydroquinolin-2-ylmethyl)-4-(3-chloro-5-trifluoromethyl-2-pyridyl)-piperazine

[M+H]⁺= 521

### Example 72A34

### 1-(5-Cyano-2-pyridyl)-4-(1-cyclohexanecarbonyl-1,2,3,4-tetrahydroquinolin-2-ylmethyl)-piperazine

[M+H]⁺= 444

### Example 72A35

### 1-(1-Cyclohexanecarbonyl-1,2,3,4-tetrahydroquinolin-2-ylmethyl)-4-(2-methyl-4-quinolinyl)-piperazine

[M+H]⁺= 483.05

### Example 72A3b

### 1-(1-Cyclohexanecarbonyl-1,2,3,4-tetrahydroquinolin-2-ylmethyl)-4-benzyl-piperidine

[M+H]⁺= 431

### Example 72A37

### 1-(Benzofuran-3-yl)-4-(1-Cyclohexanecarbonyl-1,2,3,4-tetrahydroquinolin-2-ylmethyl)-piperidine

[M+H]⁺= 457

### Example 72A38

### 1-(1-Cyclohexanecarbonyl-1,2,3,4-tetrahydroquinolin-2-ylmethyl)-4-(4-fluorophenyl)-piperidine

[M+H]⁺= 435.2

### Example 72A39

### 1-(1-Cyclohexanecarbonyl-1,2,3,4-tetrahydroquinolin-2-ylmethyl)-4-(3-fluorophenyl)-piperidine

[M+H]⁺= 435.2

### Example 72A40

### 1-(1-Cyclohexanecarbonyl-1,2,3,4-tetrahydroquinolin-2-ylmethyl)-4-(2-fluorophenyl)-piperidine

[M+H]⁺= 435.2

### Example 72A41

### 4-(4-Chlorophenyl)-1-(1-cyclohexanecarbonyl-1,2,3,4-tetrahydroquinolin-2-ylmethyl)-piperidine

[M+H]⁺= 451.2

### Example 72A42

### 1-(1-Cyclohexanecarbonyl-1,2,3,4-tetrahydroquinolin-2-ylmethyl)-4-(4-trifluoromethylphenyl)-piperidine

[M+H]⁺= 485.2

### Example 72A43

### 1-(1-Cyclohexanecarbonyl-1,2,3,4-tetrahydroquinolin-2-ylmethyl)-4-(3-trifluoromethylphenyl)-piperidine

[M+H]⁺= 485

### Example 72A44

### 1-(1-Cyclohexanecarbonyl-1,2,3,4-tetrahydroquinolin-2-ylmethyl)-4-[5-(2-furyl)-2H-3-pyrazolyl]-piperidine

[M+H]⁺= 473

### Example 72A45

### 1-(1-Cyclohexanecarbonyl-1,2,3,4-tetrahydroquinolin-2-ylmethyl)-4-(4-methoxyphenyl)-piperidine

[M+H]⁺= 447

### Example 72A46

### 1-(1-Cyclohexanecarbonyl-1,2,3,4-tetrahydroquinolin-2-ylmethyl)-4-(3-methoxyphenyl)-piperidine

[M+H]⁺= 447

### Example 72A47

### 1-(1-Cyclohexanecarbonyl-1,2,3,4-tetrahydroquinolin-2-ylmethyl)-4-(4-fluorobenzoyl)-piperidine

[M+H]⁺= 463

### Example 72A48

### 1-(1-Cyclohexanecarbonyl-1,2,3,4-tetrahydroquinolin-2-ylmethyl)-4-benzylureido-piperidine

[M+H]⁺= 489.2

### Example 72A49

### 1-(1-Cyclohexanecarbonyl-1,2,3,4-tetrahydroquinolin-2-ylmethyl)-2-(4-methoxybenzyl)-piperidine

[M+H]⁺= 461

### Example 72A50

### 1-(1-Cyclohexanecarbonyl-1,2,3,4-tetrahydroquinolin-2-ylmethyl)-2-(4-fluorobenzyl)-piperidine

[M+H]⁺= 449

### Example 72A51

### 1-(1-Cyclohexanecarbonyl-1,2,3,4-tetrahydroquinolin-2-ylmethyl)-4-(2,3-dichlorophenyl)-piperidine

[M+H]⁺ = 485.2

### Example 72A52

### 1-(1-Cyclohexanecarbonyl-1,2,3,4-tetrahydroquinolin-2-ylmethyl)-4-(2,6-difluorophenyl)-piperidine

[M+H]⁺ = 453.2

### Example 72A53

### 4-(2-Chloro-6-fluorophenyl)-1-(1-cyclohexanecarbonyl-1,2,3,4-tetrahydroquinolin-2-ylmethyl)-piperidine

[M+H]⁺= 469.2

### Example 72A54

### 1-(1-Cyclohexanecarbonyl-1,2,3,4-tetrahydroquinolin-2-ylmethyl)-4-(2,5-dimethylphenyl)-piperidine

[M+H]⁺= 445.2

### Example 72A55

### 1-(1-Cyclohexanecarbonyl-1,2,3,4-tetrahydroquinolin-2-ylmethyl)-4-(3-methylphenyl)-piperidine

[M+H]⁺= 431.2

### Example 72A56

### 1-(1-Cyclohexanecarbonyl-1,2,3,4-tetrahydroquinolin-2-ylmethyl)-4-(2-methylphenyl)-piperidine

[M+H]⁺= 431.2

### Example 72A57

### 1-(1-Cyclohexanecarbonyl-1,2,3,4-tetrahydroquinolin-2-ylmethyl)-4-(3-fluoro-2-methylphenyl)-piperidine

[M+H]⁺= 449.2

### Example 72A58

### 1-(1-Cyclohexanecarbonyl-1,2,3,4-tetrahydroquinolin-2-ylmethyl)-4-(3,5-difluorophenyl)-piperidine

[M+H]⁺= 453.2

### Example 72A59

### 1-(1-Cyclohexanecarbonyl-1,2,3,4-tetrahydroquinolin-2-ylmethyl)-4-(2,5-difluorophenyl)-piperidine

[M+H]⁺ = 453.2

### Example 72A60

### 1-(1-Cyclohexanecarbonyl-1,2,3,4-tetrahydroquinolin-2-ylmethyl)-4-(3,5-dimethylphenyl)-piperidine

[M+H]⁺ = 445.2

### Example 72A61

### 4-(3-Bromophenyl)-1-(1-cyclohexanecarbonyl-1,2,3,4-tetrahydroquinolin-2-ylmethyl)-piperidine

[M+H]⁺= 495.1

### Example 72A62

### 4-(2-Bromophenyl)-1-(1-cyclohexanecarbonyl-1,2,3,4-tetrahydroquinolin-2-ylmethyl)-piperidine

[M+H]⁺= 495.1

### Example 72A63

### 4-(4-Butoxyphenyl)-1-(1-cyclohexanecarbonyl-1,2,3,4-tetrahydroquinolin-2-ylmethyl)-piperidine

[M+H]⁺= 489.2

### Example 72A64

### 1-(1-Cyclohexanecarbonyl-1,2,3,4-tetahydroquinolin-2-ylmethyl)-4-(2-fluorophenyl)-piperazine

[M+H]⁺= 436.2

### Example 72A65

### 1-(1-Cyclohexanecarbonyl-1,2,3,4-tetrahydroquinolin-2-ylmethyl)-4-(6-chloro-2-pyridyl)-piperazine

[M+H]⁺= 453.1

### Example 72A66

### 1-(1-Cyclohexanecarbonyl-1,2,3,4-tetrahydroquinolin-2-ylmethyl)-4-(3,5-dichloro-4-pyridyl)-piperazine

[M+H]⁺= 487.1

### Example 72A67

### 1-(4-Aminophenyl)-4-(1-cyclohexanecarbonyl-1,2,3,4-tetrahydroquinolin-2-ylmethyl)-piperazine

[M+H]⁺= 433.2

### Example 72A68

### 4-(3-Chlorophenyl)-1-(1-cyclohexanecarbonyl-1,2,3,4-tetahydroquinolin-2-ylmethyl)-piperidine

[M+H]⁺= 451.2

### Example 72A69

### 4-(2-Chlorophenyl)-1-(1-cyclohexanecarbonyl-1,2,3,4-tetrahydroquinolin-2-ylmethyl)-piperidine

[M+H]⁺ = 451.2

### Example 72A70

### 1-(1-Cyclohexanecarbonyl-1,2,3,4-tetrahydroquinolin-2-ylmethyl)-2-(2-pyridyl)-pyrrolidine

[M+H]⁺ = 404.1.

### Example 72A71

### 1-(1-Cyclohexanecarbonyl-1,2,3,4-tetrahydroquinolin-2-ylmethyl)-2-(2-thienyl)-pyrrolidine

[M+H]⁺= 409.1

### Example 72A72

### 1-(1-Cyclohexanecarbonyl-1,2,3,4-tetrahydroquinolin-2-ylmethyl)-2-phenylpyrrolidine

[M+H]⁺= 403.2

### Example 72A73

### 1-(1-Cyclohexanecarbonyl-1,2,3,4-tetrahydroquinolin-2-ylmethyl)-2-(2,4-dimethoxyphenyl)-pyrrolidine

M+H]⁺= 463.2

### Example 72A74

### 1-(1-Cyclohexanecarbonyl-1,2,3,4-tetrahydroquinolin-2-ylmethyl)-2-(3-pyridyl)-pyrrolidine

[M+H]⁺= 404.1

### Example 72A75

### 1-(1-Cyclohexanecarbonyl-1,2,3,4-tetrahydroquinolin-2-ylmethyl)-2-benzylpyrrolidine

[M+H]⁺= 417.2

### Example 72A76

### 1-(1-Cyclohexanecarbonyl-1,2,3,4-tetrahydroquinolin-2-ylmethyl)-4-(3,5-dimethoxyphenyl)-piperazine

[M+H]⁺= 478.2

### Example 72A77

### 1-(1-Cyclohexanecarbonyl-1,2,3,4-tetrahydroquinolin-2-ylmethyl)-2-(2-furyl)-pyrrolidine

[M+H]⁺= 393.1

### Example 72A78

### 1-(1-Cyclohexanecarbonyl-1,2,3,4-tetrahydroquinolin-2-ylmethyl)-2-(4-methoxybenzyl)-pyrrolidine

[M+H]⁺= 447.2

### Example 72A79

### 1-(1-Cyclohexanecarbonyl-1,2,3,4-tetrahydroquinolin-2-ylmethyl)-2-(4-chlorophenyl)-pyrrolidine

[M+H]⁺= 437.2

### Example 72A80

### 1-(1-Cyclohexanecarbonyl-1,2,3,4-tetrahydroquinolin-2-ylmethyl)-2-(2-indolyl)-pyrrolidine

[M+H]⁺= 442.2

### Example 72A81

### 1-(1-Cyclohexanecarbonyl-1,2,3,4-tetrahydroquinolin-2-ylmethyl)-4-(2-trifluoromethylphenyl)-piperidine

[M+H]⁺= 485.2

### Example 72A82

### 1-Cyclohexanecarbonyl-2-{N-[2-(2-methoxyphenoxy)-ethyl]-N-methyl-aminomethyl}-1,2,3,4-tetrahydroquinoline

[M+H]⁺= 437.1

### Example 72A83

### 1-Cyclohexanecarbonyl-2-[N-(2,3-dihydro-benzo[1,4]dioxin-2-ylmethyl)-N-methylaminomethyl]-1,2,3,4-tetrahydroquinoline

[M+H]⁺= 435.2

### Example 72A84

### 1-Cyclohexanecarbonyl-2-{N-[2-(2-chlorophenoxy)-ethyl]-N-methyl-aminomethyl}-1,2,3,4-tetrahydroquinoline

[M+H]⁺= 441.1

### Example 72A85

### 1-Cyclohexanecarbonyl-2-{N-[2-(2-trifluoromethoxyphenoxy)-ethyl]-N-methylaminomethyl}-1,2,3,4-tetrahydroquinoline

[M+H]⁺= 505.1

### Example 72A86

### 1-Cyclohexanecarbonyl-2-{N-[2-(4-indolyloxy)-ethyl]-N-methyl-aminomethyl}-1,2,3,4-tetrahydroquinoline

[M+H]⁺= 446.2

### Example 72A87

### 1-Cyclohexanecarbonyl-2-{N-[2-(8-quinolyloxy)-ethyl]-N-methyl-aminomethyl}-1,2,3,4-tetrahydroquinoline

[M+H]⁺= 458.1

### Example 72A88

### 1-(4-Chloro-2-nitrophenyl)-4-(1-cyclohexanecarbonyl-1,2,3,4-tetrahydroquinolin-2-ylmethyl)-piperazine

### Example 72A89

### 1-(1-Cyclohexanecarbonyl-1,2,3,4-tetrahydroquinolin-2-ylmethyl)-4-(2-trifluoromethyl-4-quinolinyl)-piperazine

### Example 72A90

### 4-(1-Benzimidazolyl)-1-(1-cyclohexanecarbonyl-1,2,3,4-tetrahydroquinolin-2-ylmethyl)-piperidine

### Example 72A91

### 1-(1-Cyclohexanecarbonyl-1,2,3,4-tetrahydroquinolin-2-ylmethyl)-2-(4-methoxyphenyl)-piperidine

### Example 72A92

### 1-(-Cylohexanecarbonyl-1,2,3,4-tetahydroquinolin-2-ylmethyl)-4-(2,6-dichlorophenyl)-piperidine

### Example 72A93

### 1-(1-Cyclohexanecarbonyl-1,2,3,4-tetrahydroquinolin-2-ylmethyl)-4-(2-pyridyl)-piperazine

### Example 72A94

### 1-(1-Cyclohexanecarb onyl-1,2,3,4-tetrahydroquinolin-2-ylmethyl)-4-(4-quinolyl)-piperazine

### Example 72A95

### 1-(1-Cyclohexanecarbonyl-1,2,3,4-tetrahydroquinolin-2-ylmethyl)-4-(7-methoxy-4-quinolyl)-piperazine

### Example 73

The Compounds of this Example were prepared from Compound 53B instead of Compound 1B following the acylation / sulphonylation procedures described in Example 68A, 69, 70.

### Example 73A

### 1-(4-Fluoro-2-methoxyphenyl)-4-[1-(2-methylanilinocarbonyl)-1,2,3,4-tetrahydroquinolin-6-methyl-2-ylmethyl]-piperazine

### Example 73A1

### 1-(4-Fluoro-2-methoxyphenyl)-4-[1-(4-methylanilinocarbonyl)-1,2,3,4-tetrahydroquinolin-6-methyl-2-ylmethyl]-piperazine

### Example 73A2

### 1-[1-(t-Butylcarbamoyl)-1,2,3,4-tetrahydroquinolin-6-methyl-2-ylmethyl]-4-(4-fluoro-2-methoxyphenyl)-piperazine

### Example 73A3

### 1-(4-Fluoro-2-methoxyphenyl)-4-(1-pentylaminocarbonyl-1,2,3,4-tetrahydroquinolin-6-methyl-2-ylmethyl)-piperazine

### Example 73A4

### 1-(4-Fluoro-2-methoxyphenyl)-4-[1-(2-thienylacetyl)-1,2,3,4-tetrahydroquinolin-6-methyl-2-ylmethyl]-piperazine

### Example 73A5

### 1-(4-Fluoro-2-methoxyphenyl)-4-[1-(3-thienylacetyl)-1,2,3,4-tetrahydroquinolin-6-methyl-2-ylmethyl]-piperazine

### Example 73A6

### 1-(1-Cyclohexylacetyl-1,2,3,4-tetrahydroquinolin-6-methyl-2-ylmethyl)-4-(4-fluoro-2-methoxyphenyl)-piperazine

### Example 73A7

### 1-[1-(4-Cyanobenzoyl)-1,2,3,4-tetrahydroquinolin-6-methyl-2-ylmethyl]-4-(4-fluoro-2-methoxyphenyl)-piperazine

### Example 73A8

### 1-(4-Fluoro-2-methoxyphenyl)-4-[1-(4-methoxybenzoyl)-1,2,3,4-tetrahydroquinolin-6-methyl-2-ylmethyl)-piperazine

### Example 73A9

### 1-[1-(4-Chlorobenzoyl)-1,2,3,4-tetrahydroquinolin-6-methyl-2-ylmethyl]-4-(4-fluoro-2-methoxyphenyl)-piperazine

### Example 73A10

### 1-[1-(Benzo[1,3]dioxol-5-ylcarbonyl)-1,2,3,4-tetahydroquinolin-6-methyl-2-ylmethyl]-4-(4-fluoro-2-methoxyphenyl)-piperazine

### Example 73A11

### 1-[1-(2-Chlorophenylacetyl)-1,2,3,4-tetrahydroquinolin-6-methyl-2-ylmethyl]-4-(4-fluoro-2-methoxyphenyl)-piperazine

### Example 73A12

### 1-(4-Fluoro-2-methoxyphenyl)-4-[1-(1-methyl-3-indolylcarbonyl)-1,2,3,4-tetrahydroquinolin-6-methyl-2-ylmethyl]-piperazine

### Example 73A13

### 1-(1-Cycloheptanecarbonyl-1,2,3,4-tetrahydroquinolin-6-methyl-2-ylmethyl)-4-(4-fluoro-2-methoxyphenyl)-piperazine

### Example 73A14

### 1-(4-Fluoro-2-methoxyphenyl)-4-(1-phenylsulphonyl-1,2,3,4-tetrahydroquinolin-6-methyl-2-ylmethyl)-piperazine

### Example 73A15

### 1-(4-Fluoro-2-methoxyphenyl)4-(1-benzylsulphonyl-1,2,3,4-tetrahydroquinolin-6-methyl-3-ylmethyl)-piperazine

### Example 73A16

### 1-(4-Fluoro-2-methoxyphenyl)-4-[1-(4-methoxyphenylsulphonyl)-1,2,3,4-tetrahydroquinolin-6-methyl-2-ylmethyl]-piperazine

### Example 73A17

### 1-(4-Fluoro-2-methoxyphenyl)-4-[1-(4-methylphenylsulphonyl)-1,2,3,4-tetrahydroquinolin-6-methyl-2-ylmethyl]-piperazine

### Example 73A18

### 1-[1-(4-Cyanophenylsulphonyl)-1,2,3,4-tetrahydroquinolin-6-methyl-2-ylmethyl]-4-(4-fluoro-2-methoxyphenyl)-piperazine

### Example 73A19

### 1-[1-(4-Fluorophenylsulphonyl)-1,2,3,4-tetrahydroquinolin-6-methyl-2-ylmethyl]-4-(4-fluoro-2-methoxyphenyl)-piperazine

### Example 74

The Compounds of this Example were prepared from Compound 74A-B instead of Compound 1B following the acylation / sulphonylation procedures described in Example 68A, 69, 70. Compound 74A-B was prepared as follows:
a) 4-(4-Fluoro-2-methoxyphenyl)-1-(2-quinolylmethyl)-piperazine (Compound 74A-A)
   Compound 74A-A was obtained in the manner described for Compound 1C (procedure d) but using 1-(4-fluoro-2-methoxyphenyl)-piperazine instead of 1-(4-indolyl)-piperazine. The crude was purified by flash chromatography eluting with ethyl acetate to give the title compound (82%).
   ¹H-NMR (δ): 2.71-3.39 (m, 8H), 3.85 (s, 3H), 3.93-4.29 (m, 1H), 6.51-6.72 (m, 2H), 6.80-6.97 (m, 1H), 7.48-7.62 (m, 1H), 7.65-7.96 (m, 3H), 8.10 (d, 1H), 8.21 (d, 1H).
b) 1-(4-Fluoro-2-methoxyphenyl)-4-(1,2,3,4-tetrahydroquinoline-2-ylmethyl)-piperazine (Compound 74A-B)
   The title compound was prepared as described for Compound 1B (procedure e) starting from Compound 74A-A instead of Compound 1C. The crude was purified by flash chromatography eluting with petroleum ether-ethyl acetate 7:3 to give the title compound (74%).
   ¹H-NMR (δ): 1.49-1.70 (m; 1H), 1.77-2.01 (m; 1H), 2.31-3.27 (m; 12H), 3.36-3.62 (m; 1H), 3.82 (s, 3H), 4.57-4.89 (bs; 1H), 6.48-6.71 (m; 4H), 6.81-7.04 (m; 3H).

### Example 74A

### 1-(4-Fluoro-2-methoxyphenyl)-4-[1-(3-methylbutanoyl)-1,2,3,4-tetrahydroquinolin-2-ylmethyl]-piperazine

### Example 74A1

### 1-(3,3-Dimethylbutanoyl-1,2,3,4-tetrahydroquinolin-2-ylmethyl)-4-(4-fluoro-2-methoxyhenyl)-piperazine

### Example 74A2

### 1-(1-Cyclopropylacetyl-1,2,3,4-tetrahydroquinolin-2-ylmethyl)-4-(4-fluoro-2-methoxyhenyl)-piperazine

### Example 74A3

### 1-(4-Fluoro-2-methoxyphenyl)-4-[1-(2-pyrrolylcarbonyl)-1,2,3,4-tetrahydroquinolin-2-ylmethyl]-piperazine

### Example 74A4

### 1-(4-Fluoro-2-methoxyphenyl)-4-[1-(2-furoyl)-1,2,3,4-tetrahydroquinolin-2-ylmethyl]-piperazine

### Example 74A5

### 1-(4-Fluoro-2-methoxyphenyl)-4-[1-(4-methoxybenzoyl)-1,2,3,4-tetrahydroquinolin-2-ylmethy]-piperazine

### Example 74A6

### 1-[1-(4-Cyanobenzoyl)-1,2,3,4-tetrahydroquinolin-2-ylmethy]-4-(4-fluoro-2-methoxyphenyl)-piperazine

### Example 74A7

### 1-[1-(1-Acetyl-4-piperidinylcarbonyl)-1,2,3,4-tetrahydroquinolin-2-ylmethy]-4-(4-fluoro-2-methoxyphenyl)-piperazine

### Example 74A8

### 1-(4-Fluoro-2-methoxyphenyl)-4-[1-(2-thienylacetyl)-1,2,3,4-tetrahydxoquinolin-2-ylmethyl]-piperazine

### Example 74A9

### 1-(4-Fluoro-2-methoxyphenyl)-4-[1-(3-thienylacetyl)-12,3,4-tetahydroquinolin-2-ylmethyl]-piperazine

### Example 74A10

### 1-(1-Cyclobutylcarbonyl-1,2,3,4-tetrahydroquinolin-2-ylmethy)-4-(4-fluoro-2-methoxyphenyl)-piperazine

### Example 74A11

### 1-(4-Fluoro-2-methoxyphenyl)-4-[1-(4-phenoxybutanoyl)-1,2,3,4-tetahydroquinolin-2-ylmethyl]-piperazine

### Example 74A12

### 1-(4-Fluoro-2-methoxyphenyl)-4-(1-phenylsulphonyl-1,2,3,4-tetrahydroquinolin-2-ylmethyl)-piperazine

### Example 74A13

### 1-(1-Benzylsulphonyl-1,2,3,4-tetahydroquinolin-2-ylmethyl)-4-(4-fluoro-2-methoxyphenyl)-piperazine

### Example 74A14

### 1-(4-Fluoro-2-methoxyphenyl)-4-[1-(2-methylanilinocarbonyl)-1,2,3,4-tetrahydroquinolin-2-ylmethyl]-piperazine

### Example 74A15

### 1-[1-(t-Butylcarbamoyl)-1,2,3,4-tetrahydroquinolin-2-ylmethy]-4-(4-fluoro-2-methoxyphenyl)-piperazine

### Example 75

The Compounds of this Example were prepared from Compound 21B instead of Compound 1B following the acylation / sulphonylation procedures described in Example 68A, 69, 70.

### Example 75A

### 1-(1-Isoquinolinyl)-4-[1-(3-methylbutanoyl)-1,2,3,4-tetrahydroquinolin-2-ylmethyl]-piperazine

### Example 75A1

### 1-[1-(3,3-Dimethylbutanoyl)-1,2,3,4-tetrahydroquinolin-2-ylmethyl]-4-(1-isoquinolinyl)-piperazine

### Example 75A2

### 1-[1-(2-Cyclopropylacetyl)-1,2,3,4-tetrahydroquinolin-2-ylmethyl]-4-(1-isoquinolinyl)-piperazine

### Example 75A3

### 1-(1-Isoquinolinyl)-4-[1-(2-pyrrolycarbonyl)-1,2,3,4-tetrahydroquinolin-2-ylmethyl]-piperazine

### Example 75A4

### 1-[1-(2-Furoyl)-1,2,3,4-tetrahydroquinolin-2-ylmethyl]-4-(1-isoquinolinyl)-piperazine

### Example 75A5

### 1-(1-Isoquinolinyl)-4-[1-(4-methoxybenzoyl)-1,2,3,4-tetrahydroquinolin-2-ylmethyl]-piperazine

### Example 75A6

### 1-[1-(4-Cyanobenzoyl)-1,2,3,4-tetahydroquinolin-2-ylmethyl]-4-(1-isoquinolinyl)-piperazine

### Example 75A7

### 1-[1-(1-Acetyl-4-piperidylcarbonyl)-1,2,3,4-tetrahydroquinolin-2-ylmethyl]-4-(1-isoquinolinyl)-piperazine

### Example 75A8

### 1-(1-Isoquinolinyl)-4-[1-(2-thienylacetyl)-1,2,3,4-tetrahydroquinolin-2-ylmethyl]-piperazine

### Example 75A9

### 1-(1-Isoquinolinyl)-4-[1-(3-thienylacetyl)-1,2,3,4-tetrahydroquinolin-2-ylmethyl]-piperazine

### Example 75A10

### 1-(1-Cyclobutanecarbonyl-1,2,3,4-tetrahydroquinolin 2-ylmethyl)-4-(1-isoquinolinyl)-piperazine

### Example 75A11

### 1-(1-Isoquinolinyl)-4-[1-(4-phenoxybutanoyl)-1,2,3,4-tetahydroquinolin-2-ylmethyl]-piperazine

### Example 75A12

### 1-(1-Isoquinolinyl)-4-(1-phenylsulphonyl-1,2,3,4-tetrahydroquinolin-2-ylmethyl)-piperazine

### Example 75A13

### 1-(1-Benzylsulphonyl-1,2,3,4-tetrahydroquinolin-2-ylmethyl)-4-(1-Isoquinolinyl)-piperazine

### Example 75A14

### 1-(1-Isoquinolinyl)-4-[1-(2-methylanilinocarbonyl)-1,2,3,4-tetrahydroquinolin-2-ylmethyl]-piperazine

### Example 75A15

### 1-[1-(t-Butylcarbamoyl)-1,2,3,4-tetrahydroquinolin-2-ylmethy]-4-(1-Isoquinolinyl)-piperazine

### Example 76

The Compounds of this Example were prepared from Compound 76A-B instead of Compound 1B following the acylation / sulphonylation procedures described in Example 68A, 69, 70. Compound 76A-B was prepared as follows:
a) 1-(2,3-Dihydrobenzo[1,4]dioxin-5-yl)-4-(2-quinolinylmethyl)-piperazine (Compound 76A-A)
   Compound 74A-A was obtained in the manner described for Compound 1C (procedure d) but using 1-(2,3-dihydrobenzo[1,4]dioxin-5-yl)-piperazine instead of 1-(4-indolyl)-piperazine. The crude was purified by flash chromatography eluting with ethyl acetate to give the title compound (82%).
b) 1-(2,3-Dihydrobenzo[1,4]dioxin-5-yl)-4-(1,2,3,4-tetrahydroquinoline-2-ylmethyl)-piperazine (Compound 76A-B)
   The title compound was prepared as described for Compound 1B (procedure e) starting from Compound 76A-A instead of Compound 1C. The crude was purified by flash chromatography eluting with petroleum ether-ethyl acetate 7:3 to give the title compound (74%).

### Example 76A

### 1-(2,3-Dihydrobenzo[1,4]dioxin-5-yl)-4-[1-(3-methylbutanoyl)-1,2,3,4-tetrahydroquinolin-2-ylmethyl]-piperazine

### Example 76A1

### 1-[1-(2-Cyclopropylacetyl)-1,2,3,4-tetrahydroquinolin-2-ylmethyl]-4-(2,3-dihydrobenzo[1,4]dioxin-5-yl)-piperazine

### Example 76A2

### 1-(2,3-Dihydrobenzo[1,4]dioxin-5-yl)-4-[1-(2-pyrrolylcarbonyl)-1,2,3,4-tetrahydroquinolin-2-ylmethyl]-piperazine

### Example 76A3

### 1-(2,3-Dihydrobenzo[1,4]dioxin-5-yl)-4-[1-(2-furoyl)-1,2,3,4-tetrahydroquinolin-2-ylmethyl]-piperazine

### Example 76A4

### 1-(2,3-Dihydrobenzo[1,4]dioxin-5-yl)-4-[1-(4-methoxybenzoyl)-1,2,3,4-tetrahydroquinolin-2-ylmethyl]-piperazine

### Example 76A5

### 1-[1-(4-Cyanobenzoyl)-1,2,3,4-tetrahydroquinolin-2-ylmethyl]-4-(2,3-dihydrobenzo[1,4]dioxin-5-yl)-piperazine

### Example 76A6

### 1-(2,3-Dihydrobenzo[1,4]dioacin-5-yl)-4-[1-(2-thienylacetyl)-1,2,3,4-tetrahydroquinolin-2-ylmethyl]-piperazine

### Example 76A7

### 1-(2,3-Dihydrobenzo[1,4]dioxin-5-yl)-4-[1-(3-thienylacetyl)-1,2,3,4-tetrahydroquinolin-2-ylmethyl]-piperazine

### Example 76A8

### 1-(1-Cyclobutanecarbonyl-1,2,3,4-tetrahydroquinolin-2-ylmethyl)-4-(2,3-dihydrobenzo[1,4]dioxin-5-yl)-piperazine

### Example 76A9

### 1-(2,3-Dihydrobenzo [1,4]dioxin-5-yl)-4-[1-(4-phenoxybutanoyl)-1,2,3,4-tetrahydroquinolin-2-ylmethyl]-piperazine

### Example 76A10

### 1-(2,3-Dihydrobenzo[1,4]dioxin-5-yl)-4-(1-phenylsulphonyl-1,2,3,4-tetrahydroquinolin-2-ylmethyl]-piperazine

### Example 76A11

### 1-(1-Benzylsulphonyl-1,2,3,4-tetrahydroquinolin-2-ylmethyl)-4-(2,3-dihydrobenzo[1,4]dioxin-5-yl)-piperazine

### Example 76A12

### 1-(3,3-Dihydrobenzo[1,4]dioxin-5-yl)-4-[1-(2-methylanilinocarbonyl)-1,2,3,4-tetrahydroquinolin-2-ylmethyl]-piperazine

### Example 76A13

### 1-[1-(t-Butylcarbamoyl)-1,2,3,4-tetrahydroquinolin-2-ylmethy]-4-(2,3-dihydrobenzo[1,4]dioxin-5-yl)-piperazine

### Example 76A14

### 1-(2,3-Dihydrobenzo[1,4]dioxin-5-yl)-4-[1-(3,3-dimethylbutanoyl)-1,2,3,4-tetrahydroquinolin-2-ylmethyl]-piperazine

### Example 76A15

### 1-[1-(1-Acetyl-4-piperidylecarbonyl)-1,2,3,4-tetrahydroquinolin-2-ylmethyl]-4-(2,3-dihydrobenzo[1,4]dioxin-5-yl)-piperazine

### Example 76A16

### 1-(2,3-Dihydrobenzo[1,4]dioxin-5-yl)-4-[1-(2,2-dimethylbutanoyl)-1,2,3,4-tetrahydroquinolin-2-ylmethyl]-piperazine

[M+H]⁺= 464.42

### Example 76A17

### 1-(2,3-Dihydrobenzo[1,4]dioxin-5-yl)-4-[1-(1-trifluoromethylcyclopropanecarbonyl)-1,2,3,4-tetrahydroquinolin-2-ylmethyl]-piperazine

[M+H]⁺= 502.27

### Example 76A18

### 1-[1-(Bicyclo[2.2.2]oct-2-ylcarbonyl)-1,2,3,4-tetrahydroquinolin-2-ylmethyl]-4-(2,3-dihydrobenzo[1,4]dioxin-5-yl)-piperazine

[M+H]⁺= 502.40

### Example 76A19

### 1-(-Cyclopentanecarbonyl-1,2,3,4-tetrahydroquinolin-2-ylmethyl)-4-(2,3-dihydrobenzo[1,4]dioxin-5-yl)-piperazine

[M+H]⁺= 462.33

### Example 76A20

### 1-(2,3-Dihydrobenzo[1,4]dioxin-5-yl)-4-[1-(4-fluorobenzoyl)-1,2,3,4-tetrahydroquinolin-2-ylmethyl]-piperazine

[M+H]⁺= 488.30

### Example 76A21

### 1-[1-(Benzo[1,3]dioxol-5-ylcarbonyl)-1,2,3,4-tetrahydroquinolin-2-ylmethyl]-4-(2,3-dihydrobenzo [1,4]dioxin-5-yl)-piperazine

[M+H]⁺= 514.29

### Example 76A22

### 1-(2,3-Dihydrobenzo[1,4]dioxin-5-yl)-4-[1-(3-phenylpropenoyl)-1,2,3,4-tetrahydroquinolin-2-ylmethyl]-piperazine

[M+H]⁺= 496.31

### Example 76A23

### 1-(2,3-Dihydrobenzo[1,4] dioxin-5-yl)-4-[1-[3-(4-fluorophenyl)-propenoyl]-1,2,3,4-tetrahydroquinolin-2-ylmethyl]-piperazine

[M+H]⁺= 514.34

### Example 76A24

### 1-(2,3-Dihydrobenzo[1,4]dioxin-5-yl)-4-[1-(3-methyl-2-thienylcarbonyl)-1,2,3,4-tetrahydroquinolin-2-ylmethyl]-piperazine

[M+H]⁺= 490.28

### Example 76A25

### 1-(2,3-Dihydrobenzo[1,4]dioxin-5-yl)-4-[1-(3-furoyl)-1,2,3,4-tetrahydroquinolin-2-ylmethyl]-piperazine

[M+H]⁺= 460.28

### Example 76A26

### 1-(2,3-Dihydrobenzo[1,4]dioxin-5-yl)-4-[1-(2-methyl-3-furoyl)-1,2,3,4-tetrahydroquinolin-2-ylmethyl]-piperazine

[M+H]⁺= 474.29

### Example 76A27

### 1-(2,3-Dihydrobenzo[1,4]dioxin-5-yl)-4-[1-[5-methyl-4-(1,2,4-triazol-1-ylmethyl)-2-furoyl)-1,2,3,4-tetrahydroquinolin-2-ylmethyl]-piperazine

[M+H]⁺= 555.31

### Example 76A28

### 1-(2,3-Dihydrobenzo[1,4]dioxin-5-yl)-4-[1-(5-methyl-4-isoxazolecarbonyl)-1,2,3,4-tetrahydroquinolin-2-ylmethyl]-piperazine

[M+H]⁺= 475.28

### Example 76A29

### 1-[1-(4-Acetyl-2-pyrrolecarbonyl)-1,2,3,4-tetrahydroquinolin-2-ylmethyl]-4-(2,3-dihydrobenzo[1,4]dioxin-5-yl)-piperazine

[M+H]⁺= 501.32

### Example 77

The Compounds of this Example were prepared from Compound 77A-B instead of Compound 1B following the acylation / sulphonylation procedures described in Example 68A, 69, 70. Compound 77A-B was prepared as follows:
a) 2-{N-[2-(2-Methoxyphenoxy)-ethyl]-N-methyl-aminomethyl}-quinoline (Compound 77A-A
   Compound 77A-A was obtained in the manner described for Compound 1C (procedure d) but using 1-(2-methylamino-ethoxy)-2-methoxybenzene instead of 1-(4-indolyl)-piperazine. The crude was purified by flash chromatography eluting with ethyl acetate to give the title compound (82%).
b) 2-{N-[2-(2-Methoxyphenoxy)-ethyl]-N-methyl-aminomethyl}-1,2,3,4-tetrahydroquinoline (Compound 77A-B)
   The title compound was prepared as described for Compound 1B (procedure e) starting from Compound 77A-A instead of Compound 1C. The crude was purified by flash chromatography eluting with petroleum ether-ethyl acetate 7:3 to give the title compound (74%).

### Example 77A

### 2-{N-[2-(2-Methoxyphenoxy)-ethyl]-N-methyl-aminomethyl}-1-(3-methylbutanoyl)-1,2,3,4-tetrahydroquinoline

### Example 77A1

### 1-(3,3-Dimethylbutanoyl)-2-{N-[2-(2-methoxyphenoxy)-ethyl]-N-methyl-aminomethyl}-1,2,3,4-tetrahydroquinoline

### Example 77A2

### 1-Cyclopropylacetyl-2-{N-[2-(2-methoxyphenoxy)-ethyl]-N-methyl-aminomethyl}-1,2,3,4-tetrahydroquinoline

### Example 77A3

### 2- {N-[2-(2-Methoxyphenoxy)-ethyl]-N-methyl-aminomethyl}-1-(2-pyrrolylcarbonyl)-1,2,3,4-tetrahydroquinoline

### Example 77A4

### 1-(2-Furoyl)-2-{N-[2-(2-methoxyphenoxy)-ethyl]-N-methyl-aminomethyl}-1,2,3,4-tetrahydroquinoline

### Example 77A5

### 1-(4-Methoxybenzoyl)-2-{N-[2-(2-methoxyphenoxy)-ethyl]-N-methyl-aminomethyl}-1,2,3,4-tetrahydroquinoline

### Example 77A6

### 1-(1-Acetyl-4-piperidylcarbonyl)-2-{N-[2-(2-methoxyphenoxy)-ethyl]-N-methylaminomethyl}-1,2,3,4-tetrahydroquinoline

### Example 77A7

### 2-{N-[2-(2-Methoxyphenoxy)-ethyl]-N-methyl-aminomethyl}-1-(2-thienylacetyl)-1,2,3,4-tetrahydroquinoline

### Example 77A8

### 2-{N-[2-(2-Methoxyphenoxy)-ethyl]-N-methyl-aminomethyl}-1-(3-thienylacetyl)-1,2,3,4-tetrahydroquinoline

### Example 77A9

### 1-Cyclobutanecarbonyl-2-{N-[2-(2-methoxyphenoxy)-ethyl]-N-methyl-aminomethyl}-1,2,3,4-tetrahydroquinoline

### Example 77A10

### 2-{N-[2-(2-Methoxyphenoxy)-ethyl]-N-methyl-aminomethyl}-1-(4-phenoxybutanoyl)-1,2,3,4-tetrahydroquinoline

### Example 77A11

### 2-{N-[2-(2-Methoxyphenoxy)-ethyl]-N-methyl-aminomethyl}-1-phenylsulphonyl-1,2,3,4-tetrahydroquinoline

### Example 77A12

### 1-Benzylsulphonyl-2-{N-[2-(2-methoxyphenoxy)-ethyl]-N-methyl-aminomethyl}-1,2,3,4-tetrahydroquinoline

### Example 77A13

### 2-{N-[2-(2-Methoxyphenoxy)-ethyl]-N-methyl-aminomethyl}-1-(2-methylanilinocarbonyl)-1,2,3,4-tetrahydroquinoline

### Example 77A14

### 1-(t-Butylcarbamoyl)-2-{N-[2-(2-methoxyphenoxy)-ethyl]-N-methyl-aminomethyl}-1,2,3,4-tetrahydroquinoline

### Example 78

### 1-(1-Cydohexanecarbonyl-5-fluoro-1,2,3,4-tetrahydroquinoline-2-ylmethyl)-4-(4-fluoro-2-methoxyphenyl)-piperazine

### a) 1-Benzoyl-2-cyano-5-fluoro-1,2-dihydroquinoline (Compound 78A)

The title compound was prepared as described for compound 32A, but starting from 5-fluoroquinoline (WO 01/44247) instead of 6-methylquinoline. (46%)
¹H-NMR (δ): 6.13-6.22 (m, 1H), 6.41 (d, 1H), 6.79-7.02 (m, 2H), 7.18-7.37 (m, 7H).

### b) 2-Carboxy-5-fluoroquinoline (Compound 78B)

Compound 78B was obtained in the same manner as described for compound 32B, but using compound 78A instead of compound 32A. The crude was used in the next step without additional purification. (54%).
¹H-NMR (DMSO-d₆, δ): 6.15-7.17 (br, 1H), 7.29-7.48 (m, 1H), 7.61-7.80 (m, 1H), 7.90 (d, 1H), 8.04 (d, 1H), 8.87 (d, 1H).

### c) 2-Carboxy-5-fluoro-1,2,3,4-tetrahydroquinoline (Compound 78C)

The title compound was prepared as described for Compound 1B (step e) starting from Compound 78B instead of Compound 1D. The crude was treated with 37% HCl and taken up with MeCN (5 ml) to afford, after cooling to 0°C and filtration, Compound 78C (62%).
¹H-NMR (DMSO-d₆, δ): 1.87-2.08 (m, 2H), 2.34-2.52 (m, 1H), 2.61-2.78 (m, 1H), 3.86 4.06 (m, 1H), 6.30 (t, 1H), 6.39 (d, 1H), 6.77-6.97 (m, 1H), 6.99-7.62 (br, 1H)

### d) 1-(4-Fluoro-2-methoxyphenyl)-4-(5-fluoro-1,2,3,4-tetrahydroquinoline-2-ylcarbonyl)-piperazine (Compound 78D)

The title compound was synthesised following the procedure described for Compound 1D (step g) but using Compound 78C instead of 1,2,3,4-tetrahydroquinoline-2-carboxylic acid. The residue was purified by flash chromatography eluting with petroleum ether - EtOAc 6:4 to give the title compound (48 %).
¹H-NMR (δ): 1.59-1.70 (m, 1H), 2.12-2.31 (m, 1H), 2.54-2.85 (m, 1H), 2.89-3.17 (m, 5H), 3.61-3.98 (m, 7H), 4.11-4.79 (m, 1H), 4.61-4.74 (br, 1H), 6.31-6.50 (m, 2H), 6.55-6.73 (m, 2H), 6.79-7.05 (m, 2H).

### e) 1-(4-Fluoro-2-methoyphenyl)-4-(5-fluoro-1,2,3 4-tetrahydroquinoline-2-ylmethyl)-piperazine (Compound 78E)

The title compound was synthesised following the procedure described for Compound 1B (step h), but using Compound 78D instead of Compound 1D. The residue was purified by flash chromatography eluting with petroleum ether - EtOAc 55:45 to give the title compound (73%).
¹H-NMR (δ): 1.40-1.67 (m, 1H), 1.81-2.04 (m, 1H), 2.39-2.94 (m, 8H), 3.32-3.52 (m, 1H), 3.84 (s, 3H), 4.70-4.91 (br, 1H),6.24-6.41 (m, 2H), 6.54-6.68 (m, 2H), 6.70-6.99 (m, 2H).

### f) 1-(1-Cyclohexanecarbonyl-5-fluoro-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 78E instead of Compound 1B. The crude was purified by flash chromatography eluting with petroleum ether - ethyl acetate 7:3 to give the title compound (88 %).
¹H-NMR (δ): 0.77-2.02 (m, 11H), 2.08-2.48 (m, 4H), 2.51-3.06 (m, 10H), 3.81 (s, 3 H), 4.96-5.17 (m, 1H), 6.51-6.67 (m, 2H), 6.74-7.03 (m, 2H), 7.09-7.19 (m, 2H).

### Example 79

### 1-(1-Cyclohexanecarbonyl-1,2,3,4-tetrahydroquinoline-2-ylmethyl)-4-(2-triluoromethanesulphonyloxyphenyl)-piperazine.

### Example 80

### 1-(1-Cyclohexanecarbonyl-1,2,3,4-tetrahydroquinoline-2-ylmethyl)-4-(1-isopropyl-4-indolyl)-piperazine

### a) 1-(1-Isopropyl-4-indolyl)-piperazine (Compound 80A)

This compound was prepared as described in Example 35A25-A and B using isopropylbromide instead of acetyl chloride in step a. Yield: 75% overall.
¹H-NMR (δ): 1.51 (d, 6H), 2.78-3.42 (m, 9H), 4.52-4.76 (m, 1H), 6.48 (dd, 1H), 6.61 (dd, 1H), 7.02-7.21 (m, 3H).

### b) 1-(1-Cyclohexanecarbonyl-1,2,3,4-tetrahydroquinoline-2-ylmethyl)-4-(1-isopropyl-4-indolyl)-piperazine

The title compound was synthesised using the methodology described in Example 35 using Compound 35A-B and 1-(1-isopropyl-4-indolyl)-piperazine instead of 1-(4-fluoro-2-methoxyphenyl)-piperazine. Eluent: petroleum ether - EtOAc 7:3. Yield: 66%.
¹H-NMR (δ): 0.82-1.87 (m, 16H), 1.88-2.04 (m, 1H), 2.05-2.23 (m, 1H), 2.31-2.76 (m, 9H), 3.11-3.30 (m, 4H), 4.51-4.70 (m, 1H), 5.02-5.23 (m, 1H), 6.41-6.60 (m., 1H), 6.957.22 (m, 7H).

### Example 81

### 1-(6-tert-Butoxycarbonylamino-1-cyclohexanecarbonyl-1,2,3,4-tetrahydroquinoline-2-ylmethyl)-4-(4-fluoro-2-methoxyphenyl)-piperazine

### a) 6-tert-Butoxycarbonylamino-2-methylquinoline (Compound 81A)

The title compound was synthesised using the methodology described for Compound 50B but using 6-amino-2-methylquinoline instead of Compound 50A. The crude was purified by flash chromatography eluting with CH₂Cl₂ - MeOH 9:1 to give the title compound (85%).
¹H-NMR (δ): 1.53 (s, 9H), 2.74 (s, 3H), 6.63-6.75 (br, 1H), 7.23 (dd, 1H), 7.42 (dd, 1H), 7.84-8.09 (m, 3H).

### b) 6-tert-Butoxycarbonylamino-2-formyl-quinoline (Compound 81B)

To a suspension of SeO₂ (0.33g) in dioxane (10 ml) a solution of Compound 81 A (0.5g) in 10 ml of the same solvent was added dropwise and the resulting mixture refluxed for 4 h. Afterwards, the precipitated solid was filtered off, the solvent evaporated under vacuum and the crude used for the next step without further purification. (95%).
¹H-NMR (DMSO-d₆, δ): 1.50 (s, 9H), 7.78 (dd, 1H), 7.89 (dd, 1H), 8.11 (dd, 1H), 8.32 (dd, 1H), 8.42 (dd, 1H), 9.90 (s, 1H), 10.03 (s, 1H).

### c) 1-(6-tert-Butoxycarbonylaminoquinoline-2-ylmethyl)-4-(-4-fluoro-2-methoxyphenyl)-piperazine (Compound 81C)

The title compound was synthesised using the same methodology described in Example 35A using Compound 81B instead of Compound 35A-B and 1-(4-fluoro-2-methoxyphenyl)-piperazine. The crude was purified by flash chromatography eluting with CH₂Cl₂ - MeOH 95:5 to give the title compound (57%).
¹H-NMR (δ): 1.50 (s, 9H), 2.82-3.31 (m, 8H), 3.75 (s, 3H), 3.78-4.21 (m, 2H), 6.50-6.62 (m, 2H), 6.63-6.78 (m, 1H), 6.80-6.92 (m, 1H), 7.51 (dd, 1H), 7.70-7.89 (m, 1H), 7.91-8.21 (m, 3H).

### d) 1-6-tert-Butoxycarbonylamino-1,2,3,4-tetrahydroquinoline-2-ylmethyl)-4-(4-fluoro-2-methoxyphenyl)-piperazine (Compound 81D)

The title compound was prepared as described for Compound 1B (procedure e) starting from Compound 81C instead of Compound 1C. The crude was purified by flash chromatography eluting with petroleum ether - EtOAc 1:1 to give the title compound (65%).
¹H-NMR (δ): 1.40-1.68 (m, 10H), 1.81-1.99 (m, 1H), 2.42-2.61 (m, 3H), 2.62-2.87 (m, 4H), 2.88-3.15 (m, 4H), 3.31-3.51 (m, 1H), 3.83 (s,3H), 6.08-6.20 (m, 1H), 6.42 (dd, 1H), 6.47-6.55 (m, 2H), 6.81-6.95 (m, 2H), 7.02-7.08 (br, 1H).

### f) 1-(6-tert-Butoxycarbonylamino-1-cyclohexanecarbonyl-1,2,3,4-tetrahydroquinoline-2-ylmethyl-4-(4-fluoro-2-methoxyphenyl)-piperazine

The title compound was synthesised following the alternative acylation procedure described for the compound of Example 1 (step f) but starting from Compound 81D instead of Compound 1B. The crude was purified by flash chromatography eluting with petroleum ether - EtOAc 6:4 to give the title compound (80%).
¹H-NMR (δ): 0.78-1.95 (m, 20H), 1.96-2.19 (m, 1H), 2.20-2.75 (m, 9H), 2.76-3.08 (m, 4H), 3.82 (s, 3H), 5.02-5.22 (m, 1H), 6.40-6.51 (m, 1H),6.52-6.68 (m, 2H), 6.75-6.85 (m, 1H), 6.97-7.19 (m, 2H), 7.30-7.41 (m, 1H).

### Example 82

### 1-(1-Cyclohexanecarbonyl-1,2,3,4-tetrahydroquinoline-2-ylmethyl)-4-(1-methoxymethyl-4-indolyl)-piperazine

### a) 1-(1-Methoxymethyl-4-indolyl)-piperazine (Compound 82A)

The starting piperazine was prepared as described in Example 35A25 A and B but using methoxymethyl chloride instead of acetyl chloride in step A.
¹H-NMR (δ): 2.02-2.11 (br, 1H), 3.07-3.17 (m, 4H), 3.18-3.37 (m, 7H), 5.42 (s, 2H), 6.45 (dd,1H), 6.65 (dd, 1H), 7.02-7.23 (m, 3H).

### b) 1-(1-Cyclohexanecarbonyl-1,2,3,4-tetrahydroquinoline-2-ylmethyl)-4-(1-methoxymethyl-4-indolyl)-piperazine

The title compound was synthesised using the methodology described in Example 35 using Intermediate 35A-B and 1-(1-methoxymethyl-4-indolyl)-piperazine instead of 1-(4-fluoro-2-methoxyphenyl)-piperazine.
Eluent: petroleum ether-EtOAc 1:1. Yield: 63%.
¹H-NMR (δ): 0.81-1.97 (m, 11H), 1.98-2.14 (m, 1H), 2.25-2.78 (m, 9H), 3.103.28 (m, 7H), 5.03-5.27 (m, 1H), 5.43 (s, 2H), 6.41-6.73 (m., 2H), 7.03-7.29 (m, 7H).

### Example 83

### 1-(1-Cyclohexanecarbonyl-2-methyl-1,2,3,4-tetrahydroquinoline-2-ylmethyl)-4-(4-fluoro-2-methoxyphenyl)-piperazine

### a) 2-Bromomethyl-2-methyl-1,2,3,4-tetrahydroquinoline (Compound 83A)

A mixture of 0.24 g of 2-hydroxymethyl-2-methyl-1,2,3,4-tetrahydroquinoline (*Bioorg. Med. Chem. Lett.* **5**, 1527-1532, (1995)), 0.55 g of CBr₄, 0.44 g of Ph₃P and 8 ml of CH₂Cl₂ was stirred at r.t. for 2 h. After evaporation to dryness, the residue was purified by flash chromatography (petroleum ether - ethyl acetate 95:5) to give the title compound (0.225 g, 69%).
¹H-NMR (δ):1.35 (s, 3H), 1.70-1.90 (m, 1H), 1.90-2.10 (m, 1H),2.70-2.85 (m, 2H), 3.45 (d, 2H), 4.00-5.85 (b, 1H), 6.55-6.80 (m, 2H), 6.95-7.10 (m, 2H)

### b) 1-(2-Methyl-1,2,3,4-tetrahydroquinoline-2-ylmethyl)-4-(4-fluoro-2-methoxyphenyl)-piperazine (Compound 83B)

The title compound was synthesized following the procedure described for compound 34D but using as a starting material compound 83A instead of compound 34C.

The residue was purified by flash chromatography (petroleum ether - EtOAc 6:4) to afford the title compound (74%).
¹H-NMR (δ): 1.20 (s, 3H), 1.40-1.90 (m, 2H), 2.30-3.50 (m, 11H), 3.50-3.90 (m, 4H), 4.15 (b, 0.6 H), 6.25 (b, 0.4 H), 6.40-7.10 (m, 7H)

### c) 1-(1-Cyclohexanecarbonyl-2-methyl-1,2,3,4-tetrahydroquinoline-2-ylmethyl)-4-(4-fluoro-2-methoxyphenyl)-piperazine

The title compound was synthesised following the alternative acylation procedure described for the compound of Example 1 (step f) but starting from Compound 83B instead of Compound 1B. The residue was purified by flash chromatography eluting with toluene - EtOAc 7:3 then with CH₂Cl₂ affording the title compond (12%)
¹H-NMR (δ) : 0.80-3.00 (m, 28H), 3.80 (s, 3H), 6.50-6.65 (m, 2H), 6.65-6.85 (m, 2H), 7.00 7.20 (m, 3H)

### Example 84

### 1-(1-Cyclopentylcarbonyl-6-methyl-1,2,3,4-tetrahydroquinoline-2-ylmethyl]-4-(4-fluoro-2-methoxyphenyl)-piperazine

The title compound was synthesised following the alternative acylation procedure described for the compound of Example 1 (step f) but starting from Compound 53B instead of Compound 1B and using cyclopentanecarbonyl chloride instead of cyclohexanecarbonyl chloride. The crude was purified by flash chromatography eluting with petroleum ether - ethyl acetate 6:4 to afford the title compound (78%).
¹H-NMR (δ):1.31-2.11 (m, 10H), 2.15-3.48 (m, 16H), 3.82 (s, 3H), 5.03-5.26 (m, 1H), 6.50-6.68 (m, 2H), 6.77-6.98 (m, 1H), 6.99-7.21 (m, 3H).

### Example 85

### 1-(6-Amino-1-cyclohexanecarbonyl-1,2,3,4-tetrahydroquinoline-2-ylmethyl)-4-(4-fluoro-2-methoxyphenyl)-piperazine

To a cooled solution of the compound of Example 81 (0.24 g) in MeOH (10 ml) 1.2 ml of 1.8 N HCl in diethyl ether was added and the resulting mixture heated at reflux for 5h. Then the solvent was removed under vacuum, the residue alkalinised with aq. NaHCO₃ and extracted with CH₂Cl₂ (2x 20 ml). The combined organic layers were dried over Na₂SO₄ and evaporated to dryness. The crude was purified by flash chromatography eluting with CH₂Cl₂ - 2 N methanolic ammonia 95:5 to give the title compound (72%).
¹H-NMR (δ): 0.82-3.35 (m, 26H), 3.52-3.85 (m, 1H), 3.83 (s, 3H), 5.07-5.29 (m, 1H), 6.45-6.68 (m, 3H), 6.81-7.25 (m, 3H).

### Example 86

The following compounds were prepared using the method reported for Example 35A.

### Example 86A

### 1-(1-Cyclohexanecarbonyl-1,2,3,4-tetrahydroquinoline-2-ylmethyl)-4-(2-methoxy-4-methylphenyl)-piperazine

¹H-NMR (δ) : 0.80-3.60 (m, 28H), 3.80 (s, 3H), 5.10 (b, 1H), 6.60-6.85 (m, 3H), 7.10-7.25 (m,4H)

### Example 86A1

### 1-(Benzo[1,3]dioxol-5-ylmethyl)-4-(1-cyclohexanecarbonyl-1,2,3,4-tetrahydroquinoline-2-ylmethyl)-piperazine

¹H-NMR (δ): 0.80-2.80 (m, 25H), 3.45 (s, 2H), 5.05 (b, 1H), 5.90 (s, 2H), 6.70-6.90 (m, 3H), 6.95-7.25 (m, 4H)

### Example 86A2

### 1-Cyclohexanecarbonyl-2-{N-[2-(2-methoxyphenoxy)-ethyl]-aminomethyl}-1,2,3,4-tetrahydroquinoline

### Example 86A3

### 1-Cyclohexanecarbonyl-2-{N-[3-(2-methoxyphenoxy)-propyl]-N-methyl-aminomethyl}-1,2,3,4-tetrahydroquinoline

### Example 86A4

### 4-Cyano-1-(1-cyclohexanecarbonyl-1,2,3,4-tetrahydroquinolin-2-ylmethyl)-4-phenylpiperidine

### Example 86A5

### 4-Benzyl-1-(1-cyclohexanecarbonyl-1,2,3,4-tetrahydroquinolin-2-ylmethyl)-4-hydroxy-piperidine

### Example 86A6

### 4-Benzyloxy-1-(1-cyclohexanecarbonyl-1,2,3,4-tetrahydroquinolin-2-ylmethyl)-piperidine

### Example 86A7

### 4-Benzoylamino-1-(1-cyclohexanecarbonyl-1,2,3,4-tetrahydroquinolin-2-ylmethyl)-piperidine

### Example 86A8

### 4-Acetyl-1-(1-cyclohexanecarbony-1,2,3,4-tetrahydroquinolin-2-ylmethyl)-4-phenylpiperidine

### Example 86A9

### 1-(Benzo[1,3]dioxol-4-yl)-4-(1-cyclohexanecarbonyl-1,2,3,4-tetrahydroquinoline-2-ylmethyl)-piperazine

### Example 86A10

### 2-{N-[2-(3-Chlorophenoxy)-ethyl]-N-methyl-aminomethyl}-1-cyclohexanecarbonyl-1,2,3,4-tetrahydroquinoline

### Example 86A11

### 2-{N-[2-(4-Chlorophenoxy)-ethyl]-N-methyl-aminomethyl}-1-cyclohexanecarbonyl-1,2,3,4-tetrahydroquinoline

### Example 86A12

### 2-{N-Benzyl-N-[2-(2-methoxyphenoxy)-ethyl]-aminomethyl}-1-cyclohexanecarbonyl-1,2,3,4-tetrahydroquinoline

### Example 86A13

### 1-Cyclohexanecarbonyl-2-[N-methyl-N-(3-phenylpropyl)-aminomethyl]-1,2,3,4-tetrahydroquinoline

### Example 86A14

### 1-(4-Cyano-2-trifluoromethoxyphenyl)-4-(1-cyaohexanecarbonyl-1,2,3,4-tetrahydroquinoline-2-ylmethyl)-piperazine

### Example 86A15

### 1-(1-Cyclohexanecarbonyl-1,2,3,4-tetrahydroquinoline-2-ylmethyl)-4-(4-fluoro-2-methoxy-anilino)-piperidine

### Example 86A16

### 1-(3H-1,2,3-Benzotriazol-4-yl)-4-(1-cyclohexanecarbonyl-1,2,3,4-tetrahydroquinoline-2-ylmethyl)-piperazine

### Example 86A17

### 1-(1H-1,3-Benzodiazol-4-yl)-4-(1-cyclohexanecarbonyl-1,3,3,4-tetrahydroquinoline-2-ylmethyl)-piperazine

### Example 86A18

### 1-Cyclohexanecarbonyl-2-{N-[2-(2-pyridyloxy)-ethyl]-aminomethyl}-1,2,3,4-tetrahydroquinoline

### Example 86A19

### 1-Cyclohexanecarbonyl-2-{N-methyl-N-[2-(2-pyridyloxy)-ethyl]-aminomethyl}-1,2,3,4-tetrahydroquinoline

### Example 86A20

### 1-Cyclohexanecarbonyl-2-{N-methyl-N-[3-(2-methoxyphenyl)-propyl]-aminomethyl}-1,2,3,4-tetrahydroquinoline

### Example 86A21

### 4-Benzoyl-1-(1-cyclohexanecarbonyl-1,2,3,4-tetrahydroquinolin-2-ylmethyl)-piperazine

### Example 86A22

### 1-(1-Cyclohexanecarbonyl-1,2,3,4-tetrahydroquinoline-2-ylmethyl)-3-oxo-4-phenylpiperazine

### Example 87

### 1-(4-Fluoro-2-methoxyphenyl)-4-(1-hexanoyl-1,2,3,4-tetrahydroquinoline-2-ylmethyl)-piperazine

The title compound was synthesised following the alternative acylation procedure described for the compound of Example 1 (step f) but starting from Compound 74A-B instead of Compound 1B and using hexanoyl chloride instead of cyclohexanecarbonyl chloride. The crude was purified by flash chromatography eluting with petroleum ether-ethyl acetate 65:35 to give the title compound (75%).
¹H-NMR (δ): 0.71-1.79 (m, 10H), 2.03-2.79 (m, 14H), 2.82-3.10 (m, 4H), 3.32 (s, 3H), 4.96-5.28 (m, 1H); 6.48-6.67 (m, 2H), 6.75-6.91 (m, 1H), 7.04-7.25 (m, 4H).

### Example 88

### 1-(2,3-Dihydrobenzo[1,4]dioxin-5-yl)-4-[1-pentafluoropropionyl-1,2,3,4-tetrahydroquinoline-2-ylmethyl]-piperazine

The title compound was prepared by acylating Compound 76A-B with pentafluoropropionyl chloride.

### Example 89

### 1-(2,3-Dihydrobenzo[1,4]dioxin-5-yl)-4-[1-(3,3,3-trifluoropropionyl)-1,2,3,4-tetrahydroquinoline-2-ylmethyl]-piperazine

The title compound was prepared by acylating Compound 76A-B with 3,3,3-trifluoropropionyl chloride.

### Example 90

### (Z)-1-(2,3-Dihydrobenzo[1,4]dioxin-5-yl)-4-[1-(4-hydroxy-cyclohexanecarbonyl)-1,2,3,4-tetrahydroquinoline-2-ylmethyl]-piperazine

The title compound was prepared according to the method described in Example 44, but using Compound 76A-B in place of Compound 1B.

### Example 91

### 1-(1-Cyclohexanecarbonyl-6-fluoro-1,2,3,4-tetrahydroquinoline-2-ylmethyl)-4-(4-fluoro-2-methoxyphenyl)-piperazine

The title compound was prepared according to the method described in Example 78, but starting from 6-fluoroquinoline instead of 5-fluoroquinoline.

### Example 92: Radioligand binding to recombinant 5-HT_{1A} receptors

### A. Method:

Genomic clone G-21 coding for the human 5HT_{1A}-serotonergic receptor was stably transfected in a human cell line (HeLa). HeLa cells were grown as monolayers in Dulbecco's modified Eagle medium (DMEM), containing 10% foetal bovine serum, gentamicin (10 mg/ml) and 5% carbon dioxide, at 37°C. The cells were detached from the growth flask at 95% confluence by a cell scraper and were lysed in cold 5 mM Tris and 5 mM EDTA buffer (pH 7.4). The homogenates were centrifuged at 40000 xg x 20 minutes and the pellets were resuspended in a small volume of cold 5 mM Tris and 5 mM EDTA buffer (pH 7.4) and immediately frozen and stored at -70°C until use. On the day of experiment, the cell membranes were resuspended in incubation buffer: 50 mM Tris HCl (pH 7.4), 2.5 mM MgCl₂, 10 mM pargyline (Fargin et al., Nature 335, 358-360, 1988). The membranes were incubated in a final volume of 1 ml for 30 minutes at 30°C with 1 nM [³H]8-OH-DPAT, in the absence or presence of the test compounds. Non-specific binding was determined in the presence of 10 µM 5-HT. Incubation was stopped by addition of cold Tris-HCl buffer and rapid filtration through a 0.2%-polyethyleneimine-pretreated Whatman-GF/B or Schleicher-&-Schuell-GF52 filter.

### B. Results

The affinity of the tested compounds was evaluated as inhibition of specific binding of the radioligand to 5-HT_{1A} receptors (IC₅₀) by using the non-linear curve-fitting program Allfit (De Lean et al., *Am. J. Physiol.* **235**, E97-E102 (1978). The IC₅₀ value was converted to an affinity constant (Ki) by the equation of Cheng & Prusoff (Cheng Y. C., Prusoff W. H., *Biochem. Pharmacol.* **22**, 3099-3108 (1973)). The results reported in Table 1 show that the compounds of the invention tested have a high affinity for the 5-HT_{1A} receptor.

**TABLE 1**

| **Binding affinity for 5HT**_{**1A**} **receptors** *Data are expressed as Ki (nM)* | |
|---|---|
| **Compound** | **Affinity** |
| 1 | 3.3 |
| 1 (+) | 0.2 |
| 2 | 0.3 |
| 3 | 8.4 |
| 4 | 0.7 |
| 5 | 10.7 |
| 7 | 31.2 |
| 8 | 10.3 |
| 9 | 9.6 |
| 11 | 40.8 |
| 12 | 47.5 |
| 14 | 82.0 |
| 15 | 8.9 |
| 18 | 2.8 |
| 20 | 38.6 |
| 24A | 12.9 |
| 24A1 | 2.6 |
| 24A3 | 5.4 |
| 24A4 | 9.3 |
| 26 | 47.9 |
| 31 | 12.8 |
| 32 | 2.8 |
| 33 | 47.7 |
| 34 | 18.9 |
| 35A9 | 39.6 |
| 35A12 | 9.5 |
| 35A13 | 4.0 |
| 35A18 | 3.0 |
| 35A19 | 5.2 |
| 35A21 | 18.2 |
| 35A23 | 3.1 |
| 35A24 | 11.6 |
| 35A25 | 10.1 |
| 35A26 | 3.1 |
| 35A29 | 31.1 |
| 35A32 | 20.0 |
| 35A34 | 12.3 |
| 35A35 | 6.2 |
| 35A36 | 2.4 |
| 36 | 15.6 |
| 38 | 17.3 |
| 43 | 5.0 |
| 44 | 30.0 |
| 46 | 11.2 |
| 48 | 17.7 |
| 50 | 41.0 |
| 53 | 24.2 |
| 54 | 51.0 |
| 55 | 2.4 |
| 56 | 3.6 |
| 57 | 2.3 |
| 59 | 45.7 |
| 60A | 10.1 |
| 60A1 | 8.1 |
| 60A4 | 18.5 |
| 60A8 | 12.2 |
| 60A9 | 6.5 |
| 60A10 | 31.6 |
| 60A11 | 10.6 |
| 62A | 43.4 |
| 62A2 | 15.6 |
| 62A3 | 5.7 |
| 62A5 | 31.2 |
| 63 | 42.9 |
| 64 | 23.9 |
| 67 | 29.4 |
| 68A | 9.2 |
| 68A1 | 23.9 |
| 68A2 | 13.9 |
| 68A3 | 7.1 |
| 68A4 | 4.7 |
| 68A5 | 4.0 |
| 68A6 | 14.2 |
| 68A9 | 24.6 |
| 68A11 | 6.8 |
| 68A12 | 2.6 |
| 68A13 | 5.3 |
| 68A19 | 4.1 |
| 68A20 | 30.7 |
| 68A23 | 42.8 |
| 68A24 | 32.9 |
| 68A28 | 34.4 |
| 68A30 | 23.8 |
| 68A31 | 41.8 |
| 68A32 | 19.4 |
| 68A33 | 13.3 |
| 68A34 | 24.9 |
| 68A36 | 10.7 |
| 68A38 | 24.4 |
| 68A39 | 6.8 |
| 68A40 | 7.5 |
| 68A41 | 49.1 |
| 68A42 | 19.8 |
| 68A43 | 42.7 |
| 68A44 | 20.7 |
| 68A45 | 8.6 |
| 68A46 | 34.9 |
| 68A47 | 17.1 |
| 68A49 | 18.7 |
| 68A50 | 27.3 |
| 68A51 | 15.6 |
| 68A52 | 18.3 |
| 68A54 | 41.4 |
| 68A55 | 14.3 |
| 68A56 | 26.8 |
| 68A57 | 33.0 |
| 68A59 | 46.5 |
| 68A61 | 19.7 |
| 68A63 | 8.7 |
| 68A64 | 13.4 |
| 68A66 | 19.6 |
| 68A67 | 9.1 |
| 68A68 | 7.5 |
| 68A69 | 4.2 |
| 68A70 | 5.3 |
| 68A71 | 23.5 |
| 68A73 | 17.9 |
| 68A74 | 41.4 |
| 69A | 7.3 |
| 69A1 | 17.5 |
| 69A2 | 24.0 |
| 69A3 | 9.3 |
| 69A5 | 15.7 |
| 69A7 | 36.0 |
| 69A8 | 39.5 |
| 69A9 | 25.5 |
| 69A10 | 13.5 |
| 69A11 | 10.4 |
| 69A12 | 2.1 |
| 69A13 | 21.3 |
| 70A | 7.8 |
| 70A1 | 18.8 |
| 70A2 | 18.8 |
| 70A3 | 15.2 |
| 70A4 | 20.9 |
| 70A5 | 40.3 |
| 70A6 | 4.7 |
| 71A | 35.7 |
| 71A1 | 15.2 |
| 71A2 | 92.0 |
| 71A3 | 40.6 |
| 71A4 | 45.9 |
| 71A5 | 45.0 |
| 72A3 | 47.2 |
| 72A6 | 3.2 |
| 72A7 | 4.6 |
| 72A8 | 31.6 |
| 72A11 | 7.7 |
| 72A13 | 7:8 |
| 72A17 | 38.5 |
| 72A18 | 11 |
| 72A20 | 5.4 |
| 72A23 | 24.6 |
| 72A25 | 15.3 |
| 72A26 | 19.6 |
| 72A29 | 28.1 |
| 72A30 | 10.9 |
| 72A39 | 39.1 |
| 72A40 | 19.8 |
| 72A51 | 15.0 |
| 72A53 | 6.1 |
| 72A56 | 10.1 |
| 72A57 | 6.4 |
| 72A65 | 15.5 |
| 72A66 | 10.2 |
| 72A68 | 38.6 |
| 72A69 | 3.9 |
| 72A81 | 25.5 |
| 72A84 | 15.7 |
| 72A85 | 3.6 |
| 72A86 | 4.3 |
| 72A87 | 3.9 |
| 72A89 | 34.7 |
| 72A93 | 7.8 |
| 72A94 | 10.0 |
| 73A6 | 18.1 |
| 73A13 | 8.1 |
| 74A | 17.6 |
| 74A1 | 2.0 |
| 74A5 | 2.0 |
| 74A8 | 5.9 |
| 74A9 | 38.3 |
| 75A | 3.2 |
| 75A2 | 10.1 |
| 75A3 | 14.0 |
| 75A4 | 14.6 |
| 75A5 | 2.6 |
| 75A6 | 4.3 |
| 75A7 | 5.4 |
| 75A8 | 17.0 |
| 75A9 | 16.1 |
| 75A10 | 26.4 |
| 75A11 | 17.1 |
| 75A15 | 27.7 |
| 76A1 | 5.4 |
| 76A2 | 5.1 |
| 76A3 | 5.0 |
| 76A4 | 3.9 |
| 76A5 | 12.4 |
| 76A6 | 5.5 |
| 76A7 | 8.5 |
| 76A8 | 19.2 |
| 76A10 | 21.5 |
| 76A11 | 4.5 |
| 76A12 | 8.0 |
| 76A13 | 2.3 |
| 76A15 | 8.1 |
| 77A1 | 24.2 |
| 77A5 | 13.1 |
| 79 | 4.5 |
| 85 | 41.2 |
| 86A | 18.2 |

### Example 93: Effects on rhythmic bladder-voiding contractions induced by bladder filling in anaesthetised rats

### A. Method:

Female Sprague-Dawley rats weighing 225-275 g (Crl: CDo Br, Charles River Italia) were used. The animals were housed with free access to food and water and maintained on a forced 12-hour alternating light-dark cycle at 22-24°C for at least one week, except during the experiment. The activity on rhythmic bladder voiding contractions was evaluated according to the method of Dray (Dray J., *Pharmacol. Methods,* **13**:157, 1985), with some modifications as in Guarneri (Guarneri, *Pharmacol. Res.* **27**:173, 1993). Briefly, the rats were anaesthetised by subcutaneous injection of 1.25 g/kg (5 ml/kg) urethane, after which the urinary bladder was catheterised via the urethra using PE 50 polyethylene tubing filled with physiological saline. The catheter was tied in place with a ligature around the external urethral orifice and was connected to conventional pressure transducers (Statham P23 ID/P23 XL). The intravesical pressure was displayed continuously on a chart recorder (Battaglia Rangoni KV 135 with DCI/TI amplifier). The bladder was then filled via the recording catheter by incremental volumes of warm (37°C) saline until reflex bladder-voiding contractions occurred (usually 0.8-1.5 ml). For intravenous injection of bioactive compounds, PE 50 polyethylene tubing filled with physiological saline was inserted into the jugular vein.

From the cystometrogram, the number of contractions recorded 15 minutes before (basal values) and after treatment, as well as the mean amplitude of these contractions (mean height of the peaks in mmHg), was evaluated.

Since most compounds produced an effect that was relatively rapid in onset and led to a complete cessation of bladder contractions, bioactivity was conveniently estimated by measuring the duration of bladder quiescence (i.e. the length of the time during which no contractions occurred). The number of tested animals showing a reduction in the number of contractions higher than 30% of that observed in the basal period was recorded too.

To compare the potency of the tested compounds for inhibiting the bladder voiding contractions, equieffective doses which resulted in the disappearance of contractions for a time of 10 minutes (ED)₁₀ₘᵢₙ) were computed by means of linear regression using the least square method. The extrapolated doses which induced a reduction in the number of contractions greater than 30% in 50% of the treated rats (ED₅₀) were evaluated by the method of Bliss (Bliss C. I., *Quart J. Pharm. Pharmacol*. **11**, 192-216, 1938).

### B. Results

The rapid distension of the urinary bladder in urethane-anaesthetised rats produced a series of rhythmic bladder-voiding contractions whose characteristics have been described (Maggi et al., *Brain Res.* 380:83, 1986; Maggi et al., *J*. *Pharmacol. Exp. Ther*., **230**: 500, 1984).

The frequency of these contractions is related to the sensory afferent arm of reflex micturition and to the integrity of the micturition centre, while their amplitude depends on the function of the reflex efferent arm. In this model system, compounds that act mainly on the central nervous system (such as morphine) cause a block in voiding contraction, whereas drugs that act at the level of the detrusor muscle, such as oxybutynin, lower the amplitude of the bladder contractions.

The results obtained after administration of prior-art compounds and compounds of the invention are shown in Table 2.

The compounds of the invention were clearly superior to the reference standards, particularly with regard to the ED₅₀ values which are indicators of urinary frequency.

Oxybutynin only decreased the amplitude of the contractions in a dose-related manner, with an ED₅₀ value (the extrapolated dose inducing a 30% reduction of amplitude of the contractions in 50% of treated rats) of 240 µg/kg. That is, oxybutynin does not cause cessation of bladder contractions. This amplitude-reduction effect characteristic of oxybutynin, which can potentially cause lower bladder contractility and the undesirable retention of residual urine in the bladder after micturition, is not a characteristic of the compounds of the invention.

**TABLE 2**

| **Effects on rhythmic bladder-voiding contractions after intravenous administration** | | | |
|---|---|---|---|
| Data represent the ED₁₀ₘᵢₙ values (the extrapolated dose inducing 10 minutes of disappearance of the contractions), the ED₅₀ values (the extrapolated doses inducing a reduction of the number of contractions > 30% in 50% of treated rats) (frequency), and the ED₅₀ values (the extrapolated doses inducing a 30% reduction of amplitude of the contractions in 50% of treated rats) (amplitude). | | | |

| **Compound** | **ED**_{**10min**} **µg/kg** | **ED**_{**50**} **(frequency) µg/kg** | **ED**_{**50**} **(amplitude) µg/kg** |
|---|---|---|---|
| Ex. 1 | 107 | 64 | n.a. |
| Ex. (+)-1 | 72 | 24 | n.a. |
| Ex. 2 | 383 | 172 | n.a. |
| Ex.3 | 80 | 20 | n.a. |
| Flavoxate | >10000 | 2648 | n.a. |
| Oxybutynin | 7770 | > 10000 | 240 |
| n.a. = not active; no significant reduction of the height of the peaks | | | |

### Example 94: Effect on cystometric parameters in conscious rats after oral administration

### A. Method:

Male Sprague-Dawley rats [Crl: CD° (SD) BR] of 300-400 g supplied by Charles River Italia were used. The animals were housed with free access to food and water and maintained on a forced 12-hour-light/12-hour-dark cycle at 22-24°C of temperature, except during the experiment. To quantify urodynamic parameters in conscious rats, cystometrographic studies were performed according to the procedure previously reported (Guarneri et al., Pharmacol. Res. 24: 175, 1991).

Briefly, the rats were anaesthetised by intraperitoneal administration of 3 ml/kg of Equithensin solution (pentobarbital 30 mg/kg and chloral hydrate 125 mg/kg) and placed in a supine position. An approximately-10-mm-long midline incision was made in the shaved and cleaned abdominal wall. The urinary bladder was gently freed from adhering tissues, emptied and then cannulated via an incision in the bladder, body, using a polyethylene cannula (0.58-mm internal diameter, 0.96-mm external diameter) which was permanently sutured with silk thread. The cannula was exteriorised through a subcutaneous tunnel in the retroscapular area, where it was connected to a plastic adapter in order to avoid the risk of removal by the animal. For drug testing, the rats were utilised one day after implantation.

On the day of the experiment, the rats were placed in modified Bollman cages, i.e. restraining cages that were large enough to permit the rats to adopt a normal crouched posture, but narrow enough to prevent turning around. After a stabilisation period of about 20 minutes, the free tip of the bladder cannula was connected through a T-shaped tube to a pressure transducer (Statham P23XL) and to a peristaltic pump (Gilson minipuls 2) for continuos infusion of a warm (37°C) saline solution into the urinary bladder, at a constant rate of 0.1 ml/minute. The intraluminal-pressure signal during infusion of saline into the bladder was continuously recorded on a polygraph (Rectigraph-8K San-ei with BM614/2 amplifier from Biomedica Mangoni) and, from the cystometrogram, two urodynamic parameters were evaluated: bladder volume capacity (BVC) and micturition pressure (MP). BVC (in ml) is defined as the volume of saline infused into the bladder necessary to induce detrusor contraction followed by micturition. MP (in mmHg) is defined as the maximal intravesical pressure caused by contraction during micturition. Basal BVC and MP values were evaluated as mean of the values observed in the cystometrograms recorded in an initial period of 30-60 minutes. At this point in the assay, the infusion was interrupted and the test compounds were administered orally by a stomach tube. The bladder infusion restarted and changes in BVC and MP were evaluated from the mean values obtained in the cystometrograms observed during 1, 2, 3, 4 and 5 hours after treatment. The compounds were administered in a volume of 2 ml/kg and groups of control animals received the same amount of vehicle (0.5% methocel in water) orally.

### Statistical analysis

All data were expressed as mean ± standard error. Time-course data were analysed by S.A.S./STAT software, version 6.12. The difference between vehicle and active-treatment effect was evaluated by: General Linear Model Procedure - Repeated Measures Analysis of Variance: Univariate test of Hypotheses for within Subject Effects - Analysis of Variance of Contrast Variables. In the figures, data were reported as % changes *versus* the basal values.

### B. Results:

The time course of the effects of the administered doses of the tested compounds is shown in Figures 1 and 2. The compound of Example 1 administered at 3 mg/kg p.o. proved effective in increasing the bladder volume capacity without significant effects on micturition pressure (Figure 1).

On the contrary, the oral administration of 3 mg/kg of oxybutynin induced a slight, not-significant increase of BVC in comparison with the control group. Moreover this dose greatly reduced the micturition pressure values and the differences from control animals were at all times statistically significant (Figure 2).

### Example 95: Inhibition of stereotypy (rhythmic forepaw treading) induced by 8OH-DPAT in rats (post-synaptic antagonism)

### A. Method:

The inhibitory effect of 5-HT_{1A}-receptor antagonists on stereotyped forepaw treading induced in rats by subcutaneous injection of 8-OH-DPAT was evaluated by the method of Tricklebank (Tricklebank et al., *Eur. J. Pharmacol.,* **117**: 15, 1985) with minor modifications as described below.

Male Sprague-Dawley rats [Crl: CD° (SD) BR] weighing 150-175 g from Charles River Italia were used. The animals were housed with free access to food and water and maintained on a forced 12-hour-light/12-hour-dark cycle at 22-24°C of temperature. On the day of the experiment, the rats were placed singly in clear plastic containers, 10-15 minutes before administration of the vehicle or compounds to be tested. For evaluation of antagonistic activity after oral administration, the compounds were administered 0.5, 1 and 4 hours before induction of stereotypy by 8-OH-DPAT (1 mg/kg subcutaneously). Observation sessions lasted 30 seconds and began 3 minutes after 8-OH-DPAT treatment and were repeated every 3 minutes over a period of 15 minutes.

The appearance of the symptom induced by postsynaptic stimulation of 5-HT_{1A} receptors was noted, and the intensity was scored using an intensity scale in which: 0 = absent, 1 = equivocal, 2 = present and 3 = intense. Behavioural scores for treated rats were accumulated throughout the observation time (5 observation periods) and expressed as mean values of 4 rats/dose. Change in mean values of treated animals in comparison with control (vehicle) group, expressed as per-cent inhibition, was used in order to quantify the antagonistic activity.

### B. Results:

The results are shown in Table 3. These results demonstrate that the compound of Example 1, after oral administration of a dose of 1 mg/kg, exhibits significant and long-lasting post-synaptic 5-HT_{1A}-receptor antagonist activity. The results obtained after oral administration of 1 mg/kg of Example 1 enantiomers are reported in Table 3 too. The (+)-enantiomer exhibits significant and complete inhibition of the symptom up to 4 hours after administration, whereas the same dose of the (-)-enantiomer proved devoid of activity. Also the other compounds tested, after oral administration of a dose of 10 mg/kg, exhibited significant and long-lasting post-synaptic 5-HT_{1A}-receptor antagonist activity.

**TABLE 3**

| **Inhibition of forepaw treading induced by 8-OH-DPAT in rats (post-synaptic antagonism)** | | | | |
|---|---|---|---|---|
| **Compound** | **Dose (mg/kg p.o.)** | **% Inhibition of forepaw treading** | | |
| | | **0.5 h** | **1 h** | **4 h** |
| Ex. 1 | 1 | 91 | 91 | 96 |
| Ex. (+) 1 | 1 | 100 | 100 | 98 |
| Ex. (-) 1 | 1 | 19 | 5 | 9 |
| 18 | 10 | 98 | 92 | 92 |
| 24A1 | 10 | 100 | 100 | 100 |
| 24A3 | 10 | 100 | 93 | 100 |
| 35A13 | 10 | n.t. | 95 | 100 |
| n.t. = not tested | | | | |

## Claims

1. A compound having the general formula I 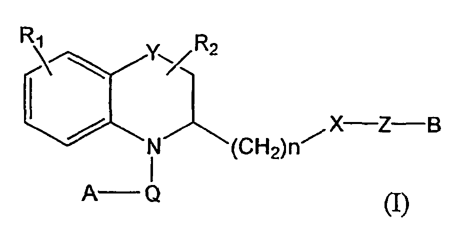 wherein
R₁ represents one or more substituents selected from hydrogen and halogen atoms and hydroxy, alkyl, haloalkyl, alkoxy, haloalkoxy, nitro, phenyl, substituted phenyl, heterocycle, substituted heterocycle and NR₃R₄ groups wherein each of R₃ and R₄ independently represents a hydrogen atom or an alkyl, acyl or alkoxycarbonyl group;
R₂ represents one or two substituents selected from hydrogen atoms and alkyl groups;
Y represents a CH₂ group or a bond;
Q represents a carbonyl, thiocarbonyl or sulphonyl group;
A represents an alkyl, alkenyl, cycloalkyl, cycloalkenyl, aryl, heterocyclic, alkylamino, dialkylamino, arylamino or arylalkylamino group, each of which is optionally substituted;
n is 1 or 2;
X represents an amino moiety selected from 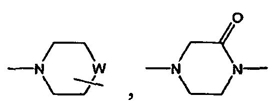 and -N(R₅)-CH₂- wherein R₅ represents a hydrogen atom or a methyl or benzyl group and W represents a bond, a nitrogen atom or a CH, CH₂ C(CN) C(OH) or C(COCH₃) group (when W represents a nitrogen atom or a CH, C(CN) C(OH) or C(COCH₃) group, the group Z-B attaches to the W ring atom);
when X represents a 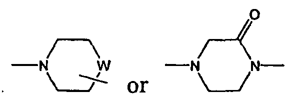 group, Z represents a bond, an oxygen or sulphur atom or a CH₂, CR₂CH₂, CO, CHOH, OCH₂, NH, NHCO or NHCONHCH₂ group, when X represents a -N(R₅)-CH₂- group, Z represents a CH₂CH₂ or CH₂O group or Z and B together represent a 2,3-dihydro-benzo[1,4]dioxin-2-yl group; and
except as aforesaid, B represents a monocyclic or bicyclic aryl group or a monocyclic or bicyclic heterocycle, each of which is optionally substituted;
or an enantiomer, diastereomer, N-oxide, crystalline form, hydrate, solvate or pharmaceutically acceptable salt of such a compound.

2. A compound according to claim 1 in which R₁ represents a hydrogen atom.

3. A compound according to claim 1 or claim 2 in which R₂ represents a hydrogen atom.

4. A compound according to any of claims 1 to 3 in which Y represents a CH₂ group.

5. A compound according to any of claims 1 to 4 in which Q represents a carbonyl group.

6. A compound according to any of claims 1 to 5 in which n is 1.

7. A compound according to any of claims 1 to 6 in which X represents a 1,4-piperazin-di-yl group.

8. A compound according to claim 7 in which Z represents a bond.

9. A compound according to any of claims 1 to 8 in which A represents a cyclohexyl group.

10. A compound according to any of claims 1 to 9 in which B represents a monocyclic aryl group or a bicyclic heterocycle, each of which is optionally substituted.

11. A compound according to claim 10 in which B represents a 4-indolyl or 2,3-dihydrobenzo[1,4]dioxin-5-yl group.

12. A compound according to claim 10 in which B represents a phenyl group substitited with one or more substituents selected from fluorine and chlorine atoms and methoxy, trifluoromethoxy and 2,2,2-trifluoroethoxy groups.

13. A compound according to claim 12 in which B represents a 2-methoxyphenyl or 4-fluoro-2-methoxyphenyl group.

14. A compound according to claim 1, which compound is one the following:
1-(1-cyclohexanecarbonyl-1,2,3,4-tetrahydroquinoline-2-ylmethyl)-4-(4-indolyl)-piperazine,
(+)-1-(1-cyclohexanecarbonyl-1,2,3,4-tetrahydroquinoline-2-ylmethyl)-4-(4-indolyl)-piperazine,
(-)-1-(1-cyclohexanecarbonyl-1,2,3,4-tetrahydroquinoline-2-ylmethyl)-4-(4-indolyl)-piperazine,
1-(1-cyclohexanecarbonyl-1,2,3,4-tetrahydroquinoline-2-ylmethyl)-4-(2-methoxyphenyl)-piperazine,
1-[2-(1-cydohexanecarbonyl-1,2,3,4-tetrahydroquinoline-2-yl)-ethyl]-4-(2-methoxyphenyl)-piperazine,
1-(1-cyclohexanecarbonyl-1,2,3,4-tetrahydroquinoline-2-ylmethyl)-4-[2-(2,2,2-trifluoroethoxy)-phenyl]-piperazine,
1-(1-cyclohexanecarbonyl-6-fluoro-1,2,3,4-tetrahydroquinoline-2-ylmethyl)-4-[2-(2,2,2,-trifluoroethoxy)-phenyl]-piperazine,
1-[1-(2-ethylbutanoyl)-1,2,3,4-tetrahydroquinoline-2-ylmethyl]-4-[2-(2,2,2-trifluoroethoxy)-phenyl]-piperazine,
1-[1-(3-methoxypropionyl)-1,2,3,4-tetrahydroquinoline-2-ylmethyl]-4-[2-(2,2,2-trifluoroethoxy)-phenyl]-piperazine,
1-[1-(3-benzyloxypropionyl)-1,2,3,4-tetrahydroquinoline-2-ylmethyl]-4-[2-(2,2,2-trifluoroethoxy)-phenyl]-piperazine,
1-[1-(3-benzyloaypropionyl)-1,2,3,4-tetrahydroquinoline-2-ylmethyl]-4-(4-indolyl)-piperazine,
1-[1-(3-hydroxypropionyl)-1,2,3,4-tetrahydroquinoline-2-ylmethyl]-4-[2-(2,2,2-trifluoroethoxy)-phenyl]-piperazine,
1-[1-(3-methoxypropionyl)-1,2,3,4-tetrahydroquinoline-2-ylmethyl]-4-(4-indolyl)-piperazine,
1-[1-(3-isopropoxypropionyl)-1,2,3,4-tetrahydroquinoline-2-ylmethyl]-4-(4-indolyl)-piperazine,
1-[1-acetyl-1,2,3,4-tetrahydroquinoline-2-ylmethyl]-4-(4-indolyl)-piperazine,
1-[1-(4-morpholinocarbonyl)-1,2,3,4-tetrahydroquinoline-2-ylmethyl]-4-(4-indolyl)-piperazine,
1-(1-cyclohexanecarbonyl-1,2,3,4-tetrahydroquinoline-2-ylmethyl)-4-(1-methyl-4-indolyl)-piperazine,
1-(1-cyclohexanecarbonyl-6-methoxy-1,2,3,4-tetrahydroquinoline-2-ylmethyl)-4-(4-indolyl)-piperazine,
1-[1-(3-hydroxypropionyl)-1,2,3,4-tetrahydroquinoline-2-ylmethyl]-4-(4-indolyl)-piperazine,
1-(1-cyclohexanecarbonyl-6-fluoro-1,2,3,4-tetrahydroquinoline-2-ylmethyl)-4-(4-indolyl)-piperazine,
1-(1-dimethylaminocarbonyl-1,2,3,4-tetrahydroquinoline-2-ylmethyl)-4-(4-indolyl)-piperazine,
1-(1-ethylaminocarbonyl-1,2,3,4-tetrahydroquinoline-2-ylmethyl)-4-(4-indolyl)-piperazine,
1-(1-cyclohexanecarbonyl-1,2,3,4-tetrahydroquinoline-2-ylmethyl)-4-(1-isoquinolinyl)-piperazine,
1-(1-cyclohexanecarbonyl-1,2,3,4-tetrahydroquinoline-2-ylmethyl)-4-(2-methoxyphenyl)-piperidine,
1-(7-benzofuranyl)-4-(1-cyclohexanecarbonyl-1,2,3,4-tetrahydroquinoline-2-ylmethyl)-piperazine,
1-(2-ethylbutanoyl-1,2,3,4-tetrahydroquinoline-2-ylmethyl)-4-(4-indolyl)-piperazine,
1-(1-cyclohex-3-enylcarbonyl-1,2,3,4-tetrahydroquinoline-2-ylmethyl)-4-(4-indolyl)-piperazine,
1-(1-cycloheptanecarbonyl-1,2,3,4-tetrahydroquinoline-2-ylmethyl)-4-(4-indolyl)-piperazine,
1-(1-cyclopentanecarbonyl-1,2,3,4-tetrahydroquinoline-2-ylmethyl)-4-(4-indolyl)-piperazine,
1-(1-benzoyl-1,2,3,4-tetrahydroquinoline-2-ylmethyl)-4-(4-indolyl)-piperazine
1-(4-indolyl)-4-(1-pentanoyl-1,2,3,4-tetrahydroquinoline-2-ylmethyl)-piperazine
1-(1-Cyclohexanecarbonyl-6-trifluoromethyl-1,2,3,4-tetrahydroquinol ine-2-ylmethyl)-4-(4-indolyl)-piperazine,
1-(1-cyclohexanecarbonyl-6-trifluoromethoxy- 1,2,3,4-tetrahydroquinoline-2-ylmethyl)-4-(4-indolyl)-piperazine,
1-[1-(3-benzylaminopropionyl)-1,2,3,4-tetrahydroquinoline-2-ylmethyl]-4-(4-indolyl)-piperazine,
1-(3-aminopropionyl-1,2,3,4-tetrahydroquinoline-2-ylmethyl]-4-(4-indolylppiperazine,
1-(4-indolyl)-4-[1-(3-methylaminopropionyl-1,2,3,4-tetrahydroquinoline-2-ylmethyl]-piperazine,
1-[1-(3-dimethylaminopropionyl)-1,2,3,4-tetrahydroquinoline-2-ylmethyl)-4-(4-indolyl)-piperazine,
1-(4-indolyl)-4-(-anilinocarbonyl-1,2,3,4-tetrahydroquinoline-2-ylmethyl)-piperazine,
1-(1-cyclohexanecarbonyl-6-methyl-1,2,3,4-tetrahydroquinoline-2-ylmethyl]-4-(4-indolyl)-piperazine,
1-(4-indolyl)-4-(1-pyrrolidinecarbonyl-1,2,3,4-tetrahydroquinoline-2-ylmethyl)-piperazine,
1-(6-bromo-1-cyclohexanecarbonyl-1,2,3,4-tetrahydroquinoline-2-ylmethyl)-4-(4-indolyl)-piperazine,
1-(1-cyclohexanecarbonyl-1,2,3,4-tetrahydroquinoline-2-ylmethyl)-4-(4-fluoro-2-methoxyphenyl)-piperazine,
1-(1-cyclohexanecarbonyl-1,2,3,4-tetrahydroquinoline-2-ylmethyl)-4-(2-methyl-4-indolyl)-piperazine,
1-(1-cyclohexanecarbonyl-1,2,3,4-tetrahydroquinoline-2-ylmethyl)-4-phenylpiperazine,
1-(1-cyclohexanecarbonyl-1,2,3,4-tetrahydroquinoline-2-ylmethyl)-4-(2-ethoxyphenyl)-piperazine,
1-(1-cyclohexanecarbonyl-1,2,3,4-tetrahydroquinoline-2-ylmethyl)-4-(2,5-dimethoxyphenyl)-piperazine,
1-(1-cyclohexanecarbonyl-1,2,3,4-tetrahydroquinoline-2-ylmethyl)-4-(2,3-dihydrobenzofuran-7-yl)-piperazine,
1-(1-cyclohexanecarbonyl-1,2,3,4-tetrahydroquinoline-2-ylmethyl)-4-(5-fluoro-2-methoxyphenyl)-piperazine,
1-(1-cyclohexanecarbonyl-1,2,3,4-tetrahydroquinoline-2-ylmethyl)-4-(2-methylphenyl)-piperazine,
1-(1-cyclohexanecarbonyl-1,2,3,4-tetrahydroquinoline-2-ylmethyl)-4-(2-trifluoromethylphenyl)-piperazine,
1-(1-cyclohexanecarbonyl-1,2,3,4-tetrahydroquinoline-2-ylmethyl)-4-(2,4-dichlorophenyl)-piperazine,
1-(1-cyclohexanecarbonyl-1,2,3,4-tetrahydroquinoline-2-ylmethyl)-4-(2-hydroxyphenyl)-piperazine,
1-(1-cyclohexanecarbonyl-1,2,3,4-tetrahydroquinoline-2-ylmethyl)-4-(2-isopropoxyphenyl)-piperazine,
1-(1-cyclohexanecarbonyl-1,2,3,4-tetrahydroquinoline-2-ylmethyl)-4-(2-fluoro-5-methylphenyl)-piperazine,
1-(1-cyclohexanecarbonyl-1,2,3,4-tetrahydroquinoline-2-ylmethyl)-4-(2,3-dimethyl-4-indolyl)-piperazine,
1-(1-cyclohexanecarbonyl-1,2,3,4-tetrahydroquinoline-2-ylmethyl)-4-(2-chloro-5-fluorophenyl)-piperazine,
1-(1-cyclohexanecarbonyl-1,2,3,4-tetrahydroquinoline-2-ylmethyl)-4-(5-fluoro-2-methylphenyl)-piperazine,
1-[(2,3-dihydro-1,4-benzodioxin-5-yl)]-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(2-trifluoromethoxyphenyl)-piperazine,
1-(1-cyclohexanecarbonyl-1,2,3,4-tetrahydroquinoline-2-ylmethyl)-4-(4-fluoro-2-methylphenyl)-piperazine,
1-(1-cyclohexanecarbonyl-1,2,3,4-tetrahydroquinoline-2-ylmethyl)-2,5-dichlorophenyl)-piperazine,
1-(-cyclohexanecarbonyl-1,2,3,4-tetrahydroquinoline-2-ylmethyl)-4-[4-fluoro-2-(2,2,2-trifluoroethoxyphenyl)]-piperazine,
1-(1-cyclohexanecarbonyl-1,2,3,4-tetrahydroquinoline-2-ylmethyl)-4-(2-pyrimidinyl)-piperazine,
1-(1-cyclohexanecarbonyl-1,2,3,4-tetrahydroquinoline-2-ylmethyl)-4-(8-quinolinyl)-piperazine,
1-(5-chloro-2-cyanophenyl)-4-(1-cyclohexanecarbonyl-1,2,3,4-tetrahydroquinoline-2-ylmethyl)-piperazine,
1-(5-cyano-2-methoxyphenyl)-4-(1-cyclohexanecarbonyl-1,2,3,4-tetrahydroquinoline-2-ylmethyl)-piperazine
1-(1-acetyl-4-indolyl)-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(7-indolyl)-piperazine,
1-(3-cyano-4-indolyl)-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(2,4-difluorobenzyl)-piperazine,
1-(2-bromobenzyl)-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(2,5-difluorobenzyl)-piperazine,
1-(1-cyclohexanecarbonyl-1,2,3,4-tetrahydroquinoline-2-ylmethyl)-4-(1-naphthyl)-piperazine,
1-(7-bromo-4-indolyl)-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(3,4-dihydro-2H-benzo[b] [1,4] dioxepin-6-yl)-piperazine,
1-(2-chlorobenzyl)-4-(1-cyclohexanecarbonyl-1,2,3,4-tetrahydroquinoline-2-ylmethyl)-piperazine,
1-(1-cydohexanecarbonyl-1,2,3,4-tetrahydroquinoline-2-ylmethyl)-4-(6-methoxy-2-pyridyl)-piperazine,
1-(1-cyclohexanecarbonyl-1,2,3,4-tetrahydroquinoline-2-ylmethyl)-4-(2,5-dichlorobenzyl)-piperazine,
1-(4-indolyl)-4-(1-piperidinocarbonyl-1,2,3,4-tetrahydroquinoline-2-ylmethyl)-piperazine,
1-[1-(3-cyanopropionyl)-1,2,3,4-tetrahydroquinoline-2-ylmethyl]-4-(4-indolyl)-piperazine,
1-(1-cyclohexanecarbonyl-8-fluoro-1,2,3,4-tetrahydroquinoline-2-ylmethyl)-4-(4-indolyl)-piperazine,
1-[1-(3-acetylaminopropionyl)-1,2,3,4-tetrahydroquinoline-2-ylmethyl]-4-(4-indolyl)-piperazine,
1-[1-(3-carbamoylaminopropionyl)-1,2,3,4-tetrahydroquinoline-2-ylmethyl]-4-(4-indolyl)-piperazine,
1-{1-[3-bis(acetylamino)-propionyl]-1,2,3,4-tetrahydroquinoline-2-ylmethyl}-4-(4-indolyl)-piperazine,
1-(6-chloro-1-cyclohexanecarbonyl-1,2,3,4-tetrahydroquinoline-2-ylmethyl)-4-(4-indolyl)-piperazine,
(R)-1-(1-cyclohexanecarbonyl-1,2,3,4-tetrahydroquinoline-2-ylmethyl)-4-(1-methyl-4-indolyl)-piperazine,
(Z)-1-[1-(4-hydroxycyclohexanecarbonyl)-1,2,3,4-tetrahydroquinoline-2-ylmethyl]-4-(4-indolyl)-piperazine,
(E)-1-[1-(4-hydroxycyclohexanecarbonyl)-1,2,3,4-tetrahydroquinoline-2-ylmethyl]-4-(4-indolyl)-piperazine,
1-(1-cyclohexanecarbonyl-7-fluoro-1,2,3,4-tetrahydroquinoline-2-ylmethyl)-4-(4-indolyl)-piperazine,
1-(1-cyclohexanecarbonyl-6-phenyl-1,2,3,4-tetrahydroquinoline-2-ylmethyl)-4-(4-indolyl)-piperazine,
1-(1-cyclohexanecarbonyl-2,3-dihydroindole-2-ylmethyl)-4-(4-indolyl)-piperazine,
1-(1-cyclohexanecarbonyl-8-methoxy-1,2,3,4-tetrahydroquinoline-2-ylmethyl)-4-(4-indolyl)-piperazine,
1-(1-cyclohexanecarbonyl-6-hydrohy-1,2,3,4-tetrahydroquinoline-2-ylmethyl)-4-(4-indolyl)-piperazine,
1-(6-bromo-1-cyclohexanecarbonyl-1,2,3,4-tetrahydroquinoline-2-ylmethyl)-4-(4-fluoro-2-methoxyphenyl)-piperazine,
1-(1-cyclohexanecarbonyl-8-hydroxy-1,2,3,4-tetrahydroquinoline-2-ylmethyl)-4-(4-indolyl)-piperazine,
1-(1-cyclohexanecarbonyl-6-methyl-1,2,3,4-tetrahydroquinoline-2-ylmethyl)-4-(4-fluoro-2-methoxyphenyl)-piperazine,
1-[1-(3-cyanaminopropionyl-1,2,3,4-tetrahydroquinoline-2-ylmethyl)]-4-(4-indolyl)-piperazine,
1-(5-chloro-1-cyclohexanecarbonyl-1,2,3,4-tetrahydroquinoline-2-ylmethyl)-4-(4-fluoro-2-methoxyphenyl)-piperazine,
1-[5-chloro-1-(4-methoxybenzoyl)-1,2,3,4-tetrahydroquinoline-2-ylmethyl]-4-(4-fluoro-2-methoxyphenyl)-piperazine,
1-(7-chloro-1-cyclohexanecarbonyl-1,2,3,4-tetrahydroquinoline-2-ylmethyl)-4-(4-fluoro-2-methoxyphenyl)-piperazine,
3-benzyl-1-(1-cyclohexanecarbonyl-1,2,3,4-tetrahydroquinoline-2-ylmethyl)-piperidine (upper TLC Rf diastereomer),
3-benzyl-1-(1-cyclohexanecarbonyl-1,2,3,4-tetrahydroquinoline-2-ylmethyl)-piperidine (lower TLC Rf diastereomer),
1-(4-chloro-2-*i*-propoxyphenyl)-4-(1-cyclohexanecarbonyl-1,2,3,4-tetrahydroquinoline-2-ylmethyl)-piperazine,
1-(5-chloro-2-fluorophenyl)-4-(1-cyclohexanecarbonyl-1,2,3,4-tetrahydroquinoline-2-ylmethyl)-piperazine,
1-[4-(2,1,3-benzothiadiazolyl)]-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(2-ethoxy-4-fluorophenyl)-piperazine,
1-(1-cyclohexanecarbonyl-1,2,3,4-tetrahydroquinoline-2-ylmethyl)-4-(2-methoxy-4-hydroxyphenyl)-piperazine,
1-(1-cyclohexanecarbonyl-1,2,3,4-tetrahydroquinoline-2-ylmethyl)-4-(7-methoxy-4-indolyl)-piperazine,
1-(1cyclohexanecarbonyl-1,2,3,4-tetrahydroquinoline-2-ylmethyl)-4-(2-pyrazinyl)-piperazine,
1-(2-cyano-4-nitrophenyl)-4-(1-cyclohexanecarbonyl-1,2,3,4-tetrahydroquinoline-2-ylmethyl)-piperazine,
1-[4-(2,1,3-benzoxadiazolyl)]-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(2-methoxy-5-trifluoromethylphenyl)-piperazine,
1-(1-cyclohexanecarbonyl-1,2,3,4-tetrahydroquinoline-2-ylmethyl)-4-[1-(1,2,3,4-tetrahydronaphthyl)]-piperazine,
1-(7-chloro-2,3-dihydro-1,4-benzodioxin-5-yl)-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(4-pyridyl)-piperazine,
1-(1-cyclohexanecarbonyl-1,2,3,4-tetrahydroquinoline-2-ylmethyl)-4-[4-(6,7-dimethoxyquinazolinyl)]-piperazine,
1-(4-fluoro-2-methoxyphenyl)-4-(1-cyclohexanecarbonyl-6-nitro-1,2,3,4-tetrahydroquinoline-2-ylmethyl)-piperazine,
1-(1-cyclohexanecarbonyl-1,2,3,4-tetrahydroquinoline-2-ylmethyl)-4-(1-ethyl-4-indolyl)-piperazine,
1-(2-bromo-5-methoxybenzyl)-4-(1-cyclohexanecarbonyl-1,2,3,4-tetrahydroquino-line-2-ylmethyl)-piperazine,
1-(4-chloro-2-methoxyphenyl)-4-(1-cyclohexanecarbonyl-1,2,3,4-tetrahydroquinoline-2-ylmethyl)-piperazine,
1-(4-chloro-2-ethoxyphenyl)-4-(1-cyclohexanecarbonyl-1,2,3,4-tetrahydroquinoline-2-ylmethyl)-piperazine,
1-(1-cyclohexanecarbonyl-1,2,3,4-tetrahydroquinoline-2-ylmethyl)-4-(4-fluoro-2-*i*-propoxyphenyl)-piperazine,
1-(1-cyclohexanecarbonyl- 1,2,3,4-tetrahydroquinoline-2-ylmethyl)-4-(7-methyl-4-indolyl)-piperazine,
1-(1-cyclohexanecarbonyl-1,2,3,4-tetrahydroquinoline-2-ylmethyl)-4-(2-methoxy-phenoxy)-piperidine,
1-[6-(5-acetyl-2-thienyl)-1-cyclohexanecarbonyl-1,2,3,4-tetrahydroquinoline-2-ylmethyl)-4-(4-fluoro-2-methoxyphenyl)-piperazine,
1-[1-cyclohexanecarbonyl-6-(3,5-dimethylisoxazol-4-yl)-1,2,3,4-tetrahydroquinoline-2-ylmethyl]-4-(4-fluoro-2-methoxyphenyl)-piperazine,
1-(1-cyclohexanecarbonyl-7-methyl-1,2,3,4-tetrahydroquinoline-2-ylmethyl)-4-(4-fluoro-2-methoxyphenyl)-piperazine,
1-(1-cyclohexanecarbonyl-4-methy 1-1,2,3,4-tetrahydroquinoline-2-ylmethyl)-4-(4-fluoro-2-methoxyphenyl)-piperazine,
1-(1-cyclohexanecarbonyl-8-methyl-1,2,3,4-tetrahydroquinoline-2-ylmethyl)-4-(4-fluoro-2-methoxyphenyl)-piperazine,
1-(4-indolyl)-4-[1-(2-pyrrolylcarbonyl)-1,2,3,4-tetrahydroquinoline-2-ylmethyl]-piperazine,
1-[1-(4-dimethylaminobenzoyl)-1,2,3,4-tetrahydroquinoline-2-ylmethyl]-4-(4-indolyl)-piperazine,
1-(4-indolyl)-4-[1-(2-phenylacetyl)-1,2,3,4-tetrahydroquinoline-2-ylmethyl]-piperazine,
1-[1-(4-cyanobenzoyl)-1,2,3,4-tetrahydroquinoline-2-ylmethyl]-4-(4-indolyl)-piperazine,
1-[1-(2-chlorobenzoyl)-1,2,3,4-tetrahydroquinoline-2-ylmethyl]-4-(4-indolyl)-piperazine,
1-[1-(4-chlorobenzoyl)-1,2,3,4-tetrahydroquinoline-2-ylmethyl]-4-(4-indolyl)-piperazine,
1-[1-(3-chlorobenzoyl)-1,2,3,4-tetrahydroquinoline-2-ylmethyl]-4-(4-indolyl)-piperazine,
1-[1-(5-benzo[1,3]dioxolylcarbonyl)-1,2,3,4-tetrahydroquinoline-2-ylmethyl]-4-(4-indolyl)-piperazine,
1-(1-cyclopropylcarbonyl-1,2,3,4-tetrahydroquinoline-2-ylmethyl)-4-(4-indolyl)-piperazine,
1-(4-indolyl)-4-[1-(2-methylpropionyl)-1,2,3,4-tetrahydroquinoline-2-ylmethyl]-piperazine,
1-(4-Indolyl)-4-[1-(2-methoxyacetyl)-1,2,3,4-tetrahydroquinoline-2-ylmethyl]-piperazine,
1-(2-cyclopropylacetyl-1,2,3,4-tetrahydroquinoline-2-ylmethyl)-4-(4-indolyl)piperazine,
1-(4-indolyl)-4-[1-(3-methylbutanoyl)-1,2,3,4-tetrahydroquinoline-2-ylmethyl]piperazine,
1-(2-furoyl-1,2,3,4-tetrahydroquinoline-2-ylmethyl)-4-(4-indolyl)-piperazine,
1-(4-indolyl)-4-[1-(5-isoxazolylcarbonyl)-1,2,3,4-tetrahydroquinoline-2-ylmethyl]-piperazine,
1-(4-indolyl)-4-[1-(3-tetrahydrofurylcarbonyl)-1,2,3,4-tetrahydroquinoline-2-ylmethyl]-piperazine,
1-(4-indolyl)-4-[1-(2-tetrahydrofurylcarbonyl)-1,2,3,4-tetrahydroquinoline-2-ylmethyl]-piperazine,
1-[1-(3,3-dimethylbutanoyl)-1,2,3,4-tetrahydroquinoline-2-ylmethyl]-4-(4-indolyl)-piperazine,
1-[1-(2-Acetoxyacetyl)-1,2,3,4-tetrahydroquinoline-2-ylmethyl]-4-(4-indolyl)-piperazine,
1-(4-indolyl)-4-[1-(2-thienylcarbonyl-1,2,3,4-tetrahydroquinoline-2-ylmethyl]-piperazine,
1-(4-indolyl)-4-[1-(3-thienylcarbonyl)-1,2,3,4-tetrahydroquinoline-2-ylmethyl]-piperazine,
1-[1-(2-cyclohexylacetyl)-1,2,3,4-tetrahydroquinoline-2-ylmethyl]-4-(4-indolyl)-piperazine,
1-(4-indolyl)-4- [1-(3-trifluoromethylbenzoyl)-1,2,3,4-tetrahydroquinoline-2-ylmethyl]-piperazine,
1-(4-indolyl)-4-[1-(4-trifluoromethylbenzoyl)-1,2,3,4-tetrahydroquinoline-2-ylmethyl]-piperazine,
1-(4-indolyl)-4-[1-(3-phenylpropionyl)-1,2,3,4-tetrahydroquinoline-2-ylmethyl]piperazine,
1-(4-indolyl)-4-[1-(2-methoxybenzoyl)-1,2,3,4-tetrahydroquinoline-2-ylmethyl]piperazine,
1-(4-indolyl)-4-[1-(4-methoxybenzoyl)-1,2,3,4-tetrahydroquinoline-2-ylmethyl]piperazine,
1-[1-(4-fluorophenylacetyl)-1,2,3,4-tetrahydroquinoline-2-ylmethyl]-4-(4-indolyl)-piperazine,
1-[1-(2,6-difluorobenzoyl)-1,2,3,4-tetrahydroquinoline-2-ylmethyl]-4-(4-indolyl)-piperazine,
1-(4-indolyl)-4-[1-(2-methoxyphenylacetyl)-1,2,3,4-tetrahydroquinoline-2-ylmethyl]-piperazine,
1-[1-(4-chlorophenylacetyl)-1,2,3,4-tetrahydroquinoline-2-ylmethyl]-4-(4-indolyl)-piperazine,
1-(4-indolyl)-4-[1-[2-(4-trifluoromethylphenyl)-acetyl]-1,2,3,4-tetrahydroquinoline]-2-ylmethyl]-piperazine,
1-(4-indolyl)-4-[1-(2-pyridylcarbonyl)-1,2,3,4-tetrahydroquinoline-2-ylmethyl]-piperazine
1-(4-indolyl)-4-[1-(3-pyridylcarbonyl)-1,2,3,4-tetrahydroquinoline-2-ylmethyl]-piperazine
1-(4-indolyl)-4-[1-(4-pyridylcarbonyl)-1,2,3,4-tetrahydroquinoline-2-ylmethyl]-piperazine
1-[1-(3,5-dimethyl-4-isoxazolylcarbonyl)-1,2,3,4-tetrahydroquinoline-2-ylmethyl]-4-(4-indolyl)-piperazine,
1-[1-(2,2-dimethylpropionyl)-1,2,3,4-tetrahydroquinoline-2-ylmethyl]-4-(4-indolyl)-piperazine,
1-(4-indolyl)-4-[1-[2-(3-pyridyl)-acetyl]-1,2,3,4-tetrahydroquinoline-2-ylmethyl]-piperazine,
1-[-(1-acetyl-4-piperidinylcarbonyl)-1,2,3,4-tetrahydroquinoline-2-ylmethyl]-4-(4-indolyl)-piperazine,
1-(4-indolyl)-4-[1-[2-(2-thienylacetyl)]-1,2,3,4-tetrahydroquinoline-2-ylmethyl]-piperazine,
1-(4-indolyl)-4-[1-[2-(3-thienylacetyl)]-1,2,3,4-tetrahydroquinoline-2-ylmethyl]-piperazine,
1-(4-indolyl)-4-[1-[3-(3-pyridylpropionyl)]-1,2,3,4-tetrahydroquinoline-2-ylmethyl]-piperazine,
1-(4-indolyl)-4-[1-(2-phenoxyacetyl)-1,2,3,4-tetrahydroquinoline-2-ylmethyl]-piperazine,
1-(4-indolyl)-4-[1-[2-(4-methoxyphenylacetyl)]-1,2,3,4-tetrahydroquinoline-2-ylmethyl]-piperazine,
1-(4-indolyl)-4-[1-(2-phenylmethoxyacetyl)-1,2,3,4-tetrahydroquinoline-2-ylmethyl]-piperazine,
1-[1-[2-(2-chlorophenylacetyl)]-1,2,3,4-tetrahydroquinoline-2-ylmethyl]-4-(4-indolyl)-piperazine,
1-{ 1-[2-(N-benzoyl-N-methyl)-aminoacetyl]-1,2,3,4-tetrahydroquinoline-2-ylmethyl}-4-(4-indolyl)-piperazine,
1-(4-indolyl)-4-[1-(1-methyl-3-indolylcarbonyl)-1,2,3,4-tetrahydroquinoline-2-ylmethyl]-piperazine,
1-[1-[2-(4-dimethylaminophenylacetyl)]-1,2,3,4-tetrahydroquinoline-2-ylmethyl]-4-(4-indolyl)-piperazine,
1-[1-(5-benzo[1,3]dioxolylacetyl)-1,2,3,4-tetrahydroquinoline-2-ylmethyl]-4-(4-indolyl)-piperazine,
1-[1-[2-(2-chlorophenoxyacetyl)-1,2,3,4-tetrahydroquinoline-2-ylmethyl]-4-(4-indolyl)-piperazine,
1-[1-[2-(4-chlorophenoxyacetyl)-1,2,3,4-tetrahydroquinoline-2-ylmethyl]-4-(4-indolyl)-piperazine,
1-(4-indolyl)-4-[1-(2-morpholino-5-pyridylcarbonyl]-1,2,3,4-tetrahydroquinoline-2-ylmethyl]-piperazine,
1-(4-indolyl)-4-[1-(5-methyl-4-isoxazolylcarbonyl)-1,2,3,4-tetrahydroquinoline-2-ylmethyl]-piperazine,
1-(4-indolyl)-4-[1-(5-oxo-2-pyrrolidinylacetyl)-1,2,3,4-tetrahydroquinoline-2-ylmethyl]-piperazine,
1-(4-indolyl)-4-[1-(2-methyl-5-piperidinosulphonyl-3-furoyl)-1,2,3,4-tetrahydroquinoline-2-ylmethyl]-piperazine,
1-(4-indolyl)-4-[1-(2-methyl-5-morpholinosulphonyl-3-furoyl)-1,2,3,4-tetrahydroquinoline-2-ylmethyl]-piperazine,
1-(4-indolyl)-4-{1-[1-(p-tolylsulphonyl)-3-pyrrolylcarbonyl]-1,2,3,4-tetrahydroquinoline-2-ylmethyl}-piperazine
1-(4-indolyl)-4-[1-(2-methyl-5-dimethylsulphamoyl-3-furoyl)-1,2,3,4-tetrahydroquinoline-2-ylmethyl]-piperazine,
1-(4-indolyl)-4-{1-[2-methyl-5-(1-pyrrolidinylsulphamoyl)-3-furoyl)-1,2,3,4-tetrahydroquinoline-2-ylmethyl]-piperazine,
1-[(1-adamantylcarbonyl)-1,2,3,4-tetrahydroquinoline-2-ylmethyl]-4-(4-indolyl)-piperazine,
1-(4-indolyl)-4-[1-(3-phenoxypropionyl)-1,2,3,4-tetrahydroquinoline-2-ylmethyl]-piperazine,
1-(4-indolyl)-1-[1-(4-phenoxybutanoyl)-1,2,3,4-tetrahydroquinoline-2-ylmethyl]-piperazine,
1-(4-indolyl)-4-[1-(2-fluorophenylacetyl)-1,2,3,4-tetrahydroquinoline-2-ylmethyl]-piperazine,
1-(4-indolyl)-4-[1-(2-trifluoromethyl-phenylacetyl)-1,2,3,4-tetrahydroquinoline-2-ylmethyl]-piperazine,
1-[1-(2-bicyclo[2.2.2]octylcarbonyl)-1,2,3,4-tetrahydroquinoline-2-ylmethyl]-4-(4-indolyl)-piperazine,
1-(4-indolyl)-1-[1-(4-phenylbutanoyl)-1,2,3,4-tetrahydroquinoline-2-ylmethyl]-piperazine,
1-(4-indolyl)-4-[1-(3-methoxybenzoyl)-1,2,3,4-tetrahydroquinoline-2-ylmethyl]-piperazine,
1-[1-(4-hydroxybenzoyl)-1,2,3,4-tetrahydroquinoline-2-ylmethyl]-4-(4-indolyl)-piperazine,
1-[1-(4-ethoxybenzoyl)-1,2,3,4-tetrahydroquinoline-2-ylmethyl]-4-(4-indolyl)-piperazine,
1-(4-indolyl)-4-[1-(4-methylbenzoyl)-1,2,3,4-tetrahydroquinoline-2-ylmethyl]-piperazine,
1-[1-[(N-benzyl-N-methanesulphonyl)-2-aminoacetyl]-1,2,3,4-tetrahydroquinoline-2-ylmethyl]-4-(4-indolyl)-piperazine,
1-(4-indolyl)-4-[1-(2-(S)-5-oxopyrrolidinylcarbonyl)-1,2,3,4-tetrahydroquinoline-2-ylmethyll-piperazine,
1-(4-indolyl)-4-[1-(2-(R)-5-oxopyrrolidinylcarbonyl)-1,2,3,4-tetrahydroquinoline-2-ylmethyl]-piperazine,
1-(4-indolyl)-4-[1-(2-methylanilinocarbonyl)-1,2,3,4-tetrahydroquinoline-2-ylmethyl]-piperazine,
1-(4-indolyl)-4-[1-(3-methylanilinocarbonyl)-1,2,3,4-tetrahydroquinoline-2-ylmethyl]-piperazine,
1-[1-(4-fluoroanilinocarbonyl)-1,2,3,4-tetrahydroquinoline-2-ylmethyl)-4-(4-indolyl)-piperazine,
1-(4-indolyl)-4-[1-(4-methylanilinocarbonyl)-1,2,3,4-tetrahydroquinoline-2-ylmethyl]-piperazine,
1-(4-indolyl)-4-[1-((1S)-1-phenylethylaminocarbonyl)-1,2,3,4-tetrahydroquinoline-2-ylmethyl]-piperazine,
1-(4-indolyl)-4-[1-((1R)-1-phenylethylaminocarbonyl)-1,2,3,4-tetrahydroquinoline-2-ylmethyl)-piperazine (diastereoisomer A),
1-(4-Indolyl)-4-[1-((1R)-1-phenylethylaminocarbonyl)-1,2,3,4-tetrahydroquinoline-2-ylmethyl]-piperazine (1:1 diastereoisomers mixture),
1-(4-indolyl)-4-[1-(1-methylethylaminocarbonyl)-1,2,3,4-tetrahydroquinoline-2-ylmethyl]-piperazine,
1-(4-indolyl)-4-[1-methylaminothiocarbonyl-1,2,3,4-tetrahydroquinoline-2-ylmethyl)-piperazine,
1-(1-benzylaminocarbonyl-1,2,3,4-tetrahydroquinoline-2-ylmethyl)-4-(4-indolyl)-piperazine,
1-(4-indolyl)-4-[1-(2-phenylethylaminocarbonyl)-1,2,3,4-tetrahydroquinoline-2-ylmethyl]-piperazine,
1-(4-indolyl)-4-(1-pentylaminocarbonyl-1,2,3,4-tetrahydroquinoline-2-ylmethyl]-piperazine,
1-[1-(1,1-dimethylethylaminocarbonyl-1,2,3,4-tetrahydroquinoline-2-ylmethyl]-4-(4-indolyl)-piperazine,
1-(4-indolyl)-4-[(4-methoxyphenylaminocarbonyl)-1,2,3,4-tetrahydroquinoline-2-ylmethyl]-piperazine,
1-(4-indolyl)-4-[(4-methoxyphenylmethylaminocarbonyl)-1,2,3,4-tetrahydroquinoline-2-ylmethyl]-piperazine,
1-(4-indolyl)-4-[1-phenylsulphonyl-1,2,3,4-tetrahydroquinoline-2-ylmethyl]-piperazine,
1-(4-indolyl)-4-[1-(2-thienylsulphonyl)-1,2,3,4-tetrahydroquinoline-2-ylmethyl]-piperazine,
1-(4-indolyl)-4-[1-(4-methoxyphenylsulphonyl)-1,2,3,4-tetrahydroquinoline-2-ylmethyl]-piperazine,
1-(4-indolyl)-4-[1-(4-methylphenylsulphonyl)-1,2,3,4-tetrahydroquinoline-2-ylmethyl]-piperazine,
1-[1-(4-fluorophenylsulphonyl)-1,2,3,4-tetrahydroquinoline-2-ylmethyl]-4-(4-indolyl)-piperazine,
1-[1-(4-cyanophenylsulphonyl)-1,2,3,4-tetrahydroquinoline-2-ylmethyl]-4-(4-indolyl)-piperazine,
1-(4-indolyl)-4-[1-benzylsulphonyl-1,2,3,4-tetrahydroquinoline-2-ylmethyl]-piperazine,
1-(1-*tert*-butoxycarbonylaminoacetyl-1,2,3,4-tetrahydroquinoline-2-ylmethyl)-4-(4-indolyl)-piperazine,
1-[1-(3-*tert*-butoxycarbonylamino-propionyl)-1,2,3,4-tetrahydroquinoline-2-ylmethyl]-4-(4-indolyl)-piperazine,
1-[1-(1-*tert*-butoxycarbonyl-3-indolyl-acetyl)-1,2,3,4-tetrahydroquinoline-2-ylmethyl]-4-(4-indolyl]-piperazine,
1-[-(1-tert-butoxycarbonyl-4-piperidylcarbonyl)-1,2,3,4-tetrahydroquinoline-2-ylmethyl]-4-(4-indolyl)-piperazine,
1-[1-(1-tert-butoxycarbonyl-2R-pynolidin-2-ylcarbonyl)-1,2,3,4-tetrahydroquinoline-2-ylmethyl]-4-(4-indolyl)-piperazine,
1-[1-(1-tert-butoxycarbonyl-2S-pyrrolidin-2-ylcarbonyl)-1,2,3,4-tetrahydroquinoline-2-ylmethyl]-4-(4-indolyl)-piperazine,
1-(1-cyclohexanecarbonyl-1,2,3,4-tetrahydroquinolin-2-ylmethyl)-2-(α-hydroxybenzyl)-piperidine,
1-(1-cyclohexanecarbonyl-1,2,3,4-tetrahydroquinolin-2-ylmethyl)-4-(2,6-dimethylphenyl)-piperidine,
2-(4-chlorophenyl)-1-(1-cydohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-4-phenylpiperidine,
4-benzoyl-1-(1-Cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-4-(2-nitrophnyl)-piperazine,
1-(1-cyclohexanecarbonyl-1,2,3,4-tetrahydroquinolin-2-ylmethyl)-4-(3-trifluoromethyl-2-pyridyl)-piperazine,
1-(-cyclohexanecarbonyl-1,2,3,4-tetrahydroquinolin-2-ylmethyl)-4-(4-fluoro-2-nitrophenyl)-piperazine,
1-(1-cydohexanecarbonyl-1,2,3,4-tetrahydroquinolin-2-ylmethyl)-4-(2,3-dichlorophenyl)-piperazine,
1-(1-cyclohexanecarbonyl-1,2,3,4-tetrahydroquinolin-2-yhnethyl)-4-(3-cyano-2-pyridyl)-piperazine,
1-(1-cyclohexanecarbonyl-1,2,3,4-tetrahydroquinolin-2-ylmethyl)-4-(3,4-dichlorophenyl)-piperazine,
1-(1-cyclohexanecarbonyl-1,2,3,4-tetrahydroquinolin-2-ylmethyl)-4-(2-ethylphenyl)-piperazine,
1-(4-chloro-3-trifluoromethylphenyl)-4-(1-cyclohexanecarbonyl-1,2,3,4-tetrahydroquinolin-2-ylmethyl)-piperazine,
1-(2-cyanophenyl)-4-(1-cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(2,4-dimethoxyphenyl)-piperazine,
1-(1-cyclohexanecarbonyl-1,2,3,4-tetrahydroquinolin-2-ylmethyl)-4-(2-methylthiophenyl)-piperazine,
1-(1-cyclohexanecarbonyl-1,2,3,4-tetrahydroquinolin-2-ylmethyl)-4-(3,4-dimethoxyphenyl)-piperazine,
1-(1-cyclohexanecarbonyl-1,2,3,4-tetrahydroquinolin-2-ylmethyl)-4-(3-hydroxyphenyl)-piperazine,
1-(1-cyclohexanecarbonyl-1,2,3,4-tetrahydroquinolin-2-ylmethyl)-4-(2,4-difluorophenyl)-piperazine,
1-(5-chloro-2-methoxyphenyl)-4-(1-cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(6-methyl-2-pyridyl)-piperazine;
1-(1-cyclohexanecarbonyl-1,2,3,4-tetrahydroquinolin-2-ylmethyl)-4-(4-methylphenyl)-piperazine,
1-(1-cyclohexanecarbonyl-1,2,3,4-tetrahydroquinolin-2-ylmethyl)-4-(4-trifluoromethylphenyl)-piperazine,
1-(3-chlorophenyl)-4-(1-cyclohexanecarbonyl-1,2,3,4-tetrahydroquinolin-2-ylmethyl)-piperazine,
1-(2-chlorophenyl)-4-(1--cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(3-methoxyphenyl)-piperazine,
1-(1-cyclohexanecarbonyl-1,2,3,4-tetrahydroquinolin-2-ylmethyl)-4-(4-fluorophenyl)-piperazine,
1-(4-acetylphenyl)-4-(1-cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(4-hydroxyphenyl)-piperazine,
1-(5-chloro-2-methylphenyl)-4-(1-cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(3-methylphenyl)-piperazine,
1-(1-cyclohexanecarbonyl-1,2,3,4-tetrahydroquinolin-2-ylmethyl)-4-(2-nitro-4-trifluorometlrylphenyl)-piperazine,
1-(1-cyclohexanecarbonyl-1,2,3,4-tetrahydroquinolin-2-ylmethyl)-4-(5-trifluoromethyl-2-pyridyl)-piperazine,
1-(1-cyclohexanecarbonyl-1,2,3,4-tetrahydroquinolin-2-ylmethyl)-4-(3-chloro-5-trifluoromethyl-2-pyridyl)-piperazine,
1-(5-cyano-2-pyridyl)-4-(1-cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(2-methyl-4-quinolinyl)-piperazine,
1-(1-cyclohexanecarbonyl-1,2,3,4-tetrahydroquinolin-2-ylmethyl)-4-benzyl-piperidine,
1-(benzofuran-3-yl)-4-(1-Cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-4-(4-fluorophenyl)-piperidine,
1-(1-cyclohexanecarbonyl-1,2,3,4-tetrahydroquinolin-2-ylmethyl)-4-(3-fluorophenyl)-piperidine,
1-(1-cyclohexanecarbonyl-1,2,3,4-tetrahydroquinolin-2-ylmethyl)-4-(2-fluorophenyl)-piperidine,
4-(4-chlorophenyl)-1-(1-cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-4-(4-trifluoromethylphenyl)-piperidine,
1-(1-cyclohexanecarbonyl-1,2,3,4-tetrahydroquinolin-2-ylmethyl)-4-(3-trifluoromethylphenyl)-piperidine,
1-(1-cyclohexanecarbonyl-1,2,3,4-tetrahydroquinolin-2-ylmethyl)-4-[5-(2-furyl)-2H-3-pyrazolyl]-piperidine,
1-(1-cyclohexanecarbonyl-1,2,3,4-tetrahydroquinolin-2-ylmethyl)-4-(4-methoxyphenyl)-piperidine,
1-(1-cyclohexanecarbonyl-1,2,3,4-tetrahydroquinolin-2-ylmethyl)-4-(3-methoxyphenyl)-piperidine,
1-(1-cyclohexanecarbonyl-1,2,3,4-tetrahydroquinolin-2-ylmethyl)-4-(4-fluorobenzoyl)-piperidine,
1-(1-Cyclohexanecarbonyl-1,2,3,4-tetrahydroquinolin-2-ylmethyl)-4-benzylureido-piperidine,
1-(1-cyclohexanecarbonyl-1,2,3,4-tetrahydroquinolin-2-ylmethyl)-2-(4-methoxybenzyl)-piperidine,
1-(1-cyclohexanecarbonyl-1,2,3,4-tetrahydroquinolin-2-ylmethyl)-2-(4-fluorobenzyl)-piperidine,
1-(1-cyclohexanecarbonyl-1,2,3,4-tetrahydroquinolin-2-ylmethyl)-4-(2,3-dichlorophenyl)-piperidine,
1-(1-cyclohesanecarbonyl-1,2,3,4-tetrahydroquinolin-2-ylmethyl)-4-(2,6-difluorophenyl)-piperidine,
4-(2-chloro-6-fluorophenyl)-1-(1-cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-4-(2,5-dimethylphenyl)-piperidine,
1-(1-cyclohexanecarbonyl-1,2,3,4-tetrahydroquinolin-2-ylmethyl)-4-(3-methylphenyl)-piperidine,
1-(1-cyclohexanecarbonyl-1,2,3, 4-tetrahydroquinolin-2-ylmethyl)-4-(2-methylphenyl)-piperidine,
1-(1-cyclohexanecarbonyl-1,2,3, 4-tetrahydroquinolin-2-ylmethyl)-4-(3-fluoro-2-methylphenyl)-piperidine,
1-(1-cyclohexanecarbonyl-1,2,3,4-tetrahydroquinolin-2-ylmethyl)-4-(3,5-difluorophenyl)-piperidine,
1-(1-cyclohexanecarbonyl-1,2,3,4-tetrahydroquinolin-2-ylmethyl)-4-(2,5-difluorophenyl)-piperidine,
1-(1-cyclohexanecarbonyl-1,2,3,4-tetrahydroquinolin-2-ylmethyl)-4-(3,5-dimethylphenyl)-piperidine,
4-(3-bromophenyl)-1-(1-cyclohexanecarbonyl-1,2,3,4-tetrahydroquinolin-2-ylmethyl)-piperidine,
4-(2-bromophenyl)-1-(1-cyclohexanecarbonyl-1,2,3,4-tetrahydroquinolin-2-ylmethyl)-piperidine,
4-(4-butoxyphenyl)-1-(1-cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-4-(2-fluorophenyl)-piperazine,
1-(1-cyclohexanecarbonyl-1,2,3,4-tetrahydroquinolin-2-ylmethyl)-4-(6-chloro-2-pyridyl)-piperazine,
1-(1-cyclohexanecarbonyl-1,2,3,4-tetrahydroquinolin-2-ylmethyl)-4-(3,5-dichloro-4-pyridyl)-piperazine,
1-(4-aminophenyl)-4-(1-cyclohexanecarbonyl-1,2,3,4-tetrahydroquinolin-2-ylmethyl)-piperazine,
4-(3-chlorophenyl)-1-(1-cyclohexanecarbonyl-1,2,3,4-tetrahydroquinolin-2-ylmethyl)-piperidine,
4-(2-chlorophenyl)-1-(1-cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-2-(2-pyridyl)-pyrrolidine,
1-(1-cyclohexanecarbonyl-1,2,3,4-tetrahydroquinolin-2-ylmethyl)-2-(2-thienyl)-pyrrolidine,
1-(1-cyclohexanecarbonyl-1,2,3,4-tetrahydroquinolin-2-ylmethyl)-2-phenylpyrrolidine,
1-(1-cyclohexanecarbonyl-1,2,3,4-tetrahydroquinolin-2-ylmethyl)-2-(2,4-dimethoxyphenyl)-pyrrolidine,
1-(1-cyclohexanecarbonyl-1,2,3,4-tetrahydroquinolin-2-ylmethyl)-2-(3-pyridyl)-pyrrolidine,
1-(1-cyclohexanecarbonyl-1,2,3,4-tetrahydroquinolin-2-ylmethyl)-2-benzylpyrrolidine,
1-(1-cyclohexanecarbonyl-1,2,3,4-tetrahydroquinolin-2-ylmethyl)-4-(3,5-dimethoxyphenyl)-piperazine,
1-(1-cyclohexanecarbonyl-1,2,3,4-tetrahydroquinolin-2-ylmethyl)-2-(2-furyl)-pyrrolidine,
1-(1cyclohexanecarbonyl-1,2,3,4-tetrahydroquinolin-2-ylmethyl)-2-(4-methoxybenzyl)-pyrrolidine,
1-(1-cyclohexanecarbonyl-1,2,3,4-tetrahydroquinolin-2-ylmethyl)-2-(4-chlorophenyl)-pyrrolidine,
1-(1-cyclohexanecarbonyl-1,2,3,4-tetrahydroquinolin-2-ylmethyl)-2-(2-indolyl)-pyrrolidine,
1-(1-cyclohexanecarbonyl-1,2,3,4-tetrahydroquinolin-2-ylmethyl)-4-(2-trifluoromethylphenyl)-piperidine,
1-cyclohexanecarbonyl-2-{N-[2-(2-methoxyphenoxy)-ethyl]-N-methyl-aminomethyl}-1,2,3,4-tetrahydroquinoline,
1-cyclohexanecarbonyl-2-[N-(2,3-dihydro-benzo[1,4]dioxin-2-ylmethyl)-N-methylaminomethyl]-1,2,3,4-tetrahydroquinoline,
1-cyclohexanecarbonyl-2-{N-[2-(2-chlorophenoxy)-ethyl]-N-methyl-aminomethyl}-1,2,3,4-tetrahydroquinoline,
1-cyclohexanecarbonyl-2-{N-[2-(2-trifluoromethoxyphenoxy)-ethyl]-N-methylaminomethyl}-1,2,3,4-tetrahydroquinoline,
1-cyclohexanecarbonyl-2-{N-[2-(4-indolyloxy)-ethyl]-N-methyl-aminomethyl}-1,2,3,4-tetrahydroquinoline,
1-cyclohexanecarbonyl-2-{N-[2-(8-quinolyloxy)-ethyl]-N-methyl-aminomethyl}-1,2,3,4-tetrahydroquinoline,
1-(4-chloro-2-nitrophenyl)-4-(1-cyclohexanecarbonyl-1,2,3,4-tetrahydroquinolin-2-ylmethyl)-piperazine,
1-(1-cyclohexanecarbonyl-1,2,3,4-tetrahydroquinolin-2-ylmethyl)-4-(2-trifluoromethyl-4-quinolinyl)-piperazine,
4-(1-bemimidazolyl)-1-(1-cyclohexanecarbonyl-1,2,3,4-tetrahydroquinolin-2-ylmethyl)-piperidine,
1-(1-cyclohexanecarbonyl-1,2,3,4-tetrahydroquinolin-2-ylmethyl)-2-(4-methoxyphenyl)-piperidine,
1-(1-cyclohexanecarbonyl-1,2,3,4-tetrahydroquinolin-2-ylmethyl)-4-(2,6-dichlorophenyl)-piperidine,
1-(1-cyclohexanecarbonyl-1,2,3,4-tetrahydroquinolin-2-ylmethyl)-4-(2-pyridyl)-piperazine,
1-(1-cyclohexanecarbonyl-1,2,3,4-tetrahydroquinolin-2-ylmethyl)-4-(4-quinolyl)-piperazine,
1-(1-cyclohexanecarbonyl-1,2,3,4-tetrahydroquinolin-2-ylmethyl)-4-(7-methoxy-4-quinolyl)-piperazine,
1-(4-fluoro-2-methoxyphenyl)-4-[1-(2-methylanilinocarbonyl)-1,2,3,4-tetrahydroquinolin-6-methyl-2-ylmethyl]-piperazine,
1-(4-fluoro-2-methoxyphenyl)-4-[1-(4-methylanilinocarbonyl)-1,2,3,4-tetrahydroquinolin-6-methyl-2-ylmethyl]-piperazine,
1-[1-(t-butylcarbamoyl)-1,2,3,4-tetrahydroquinolin-6-methyl-2-ylmethyl]-4-(4-fluoro-2-methoxyphenyl)-piperazine,
1-(4-fluoro-2-methoxyphenyl)-4-(1-pentylaminocarbonyl-1,2,3,4-tetrahydroquinolin-6-methyl-2-ylmethyl)-piperazine,
1-(4-fluoro-2-methoxyphenyl)-4-[(2-thienylacetyl)-1,2,3,4-tetrahydroquinolin-6-methyl-2-ylmethyl]-piperazine,
1-(4-fluoro-2-methoxyphenyl)-4-[(3-thienylacetyl)-1,2,3,4-tetrahydroquinolin-6-methyl-2-ylmethyl]-piperazine,
1-(1-cyclohexylacetyl-1,2,3,4-tetrahydroquinolin-6-methyl-2-ylmethyl)-4-(4-fluoro-2-methoxyphenyl)-piperazine,
1-[1-(4-cyanobenzoyl)-1,2,3,4-tetrahydroquinolin-6-methyl-2-ylmethyl]-4-(4-fluoro-2-methoxyphenyl)-piperazine,
1-(4-fluoro-2-methoxyphenyl)-4-[1-(4-methoxybenzoyl)-1,2,3,4-tetrahydroquinolin-6-methyl-2-ylmethyl)-piperazine,
1-[1-(4-chlorobenzoyl)-1,2,3,4-tetrahydroquinolin-6-methyl-2-ylmethyl]-4-(4-fluoro-2-methoxyphenyl)-piperazine,
1-[1-(benzo[1,3]dioxol-5-ylcarbonyl)-1,2,3,4-tetrahydroquinolin-6-methyl-2-ylmethyl]-4-(4-fluoro-2-methoxyphenyl)-piperazine,
1-[1-(2-chlorophenylacetyl)-1,2,3,4-tetrahydroquinolin-6-methyl-2-ylmethyl]-4-(4-fluoro-2-methoayphenyl)-piperazine,
1-(4-fluoro-2-methoayphenyl)-4-[1-(1-methyl-3-indolylcarbonyl)-1,2,3,4-tetrahydroquinolin-6-methyl-2-ylmethyl]-piperazine,
1-(1-cycloheptanecarbonyl-1,2,3,4-tetrahydroquinolin-6-methyl-2-ylmethyl)-4-(4-fluoro-2-methoxyphenyl)-piperazine,
1-(4-fluoro-2-methoxyphenyl)-4-(1-phenylsulphonyl-1,2,3,4-tetrahydroquinolin-6-methyl-2-ylmethyl)-piperazine,
1-(4-fluoro-2-methoxyphenyl)-4-(1-benzylsulphonyl-1,2,3,4-tetrahydroquinolin-6-methyl-2-ylmethyl)-piperazine,
1-(4-fluoro-2-methoxyphenyl)-4-[1-(4-methoxyphenylsulphonyl)-1,2,3,4-tetrahydroquinolin-6-methyl-2-ylmethyl]-piperazine,
1-(4-fluoro-2-methoxyphenyl)-4-[1-(4-methylphenylsulphonyl)-1,2,3,4-tetrahydroquinolin-6-methyl-2-ylmethyl]-piperazine,
1-[1-(4-cyanophenylsulphonyl)-1,2,3,4-tetrahydroquinolin-6-methyl-2-ylmethyl]-4-(4-fluoro-2-methoxyphenyl)-piperazine,
1-[1-(4-fluorophenylsulphonyl)-1,2,3,4-tetrahydroquinolin-6-methyl-2-ylmethyl]-4-(4-fluoro-2-methoxyphenyl)-piperazine,
1-(4-fluoro-2-methoxyphenyl)-4-[1-(3-methylbutanoyl)-1,2,3,4-tetrahydroquinolin-2-ylmethyl]-piperazine,
1-(3,3-dimethylbutanoyl-1,2,3,4-tetrahydroquinolin-2-ylmethyl)-4-(4-fluoro-2-methoxyhenyl)-piperazine,
1-(1-cyclopropylacetyl-1,2,3,4-tetrahydroquinolin-2-ylmethyl)-4-(4-fluoro-2-methoxyhenyl)-piperazine,
1-(4-fluoro-2-methoxyphenyl)-4-[1-(2-pyrrolylcarbonyl)-1,2,3,4-tetrahydroquinolin-2-ylmethyl]-piperazine,
1-(4-fluoro-2-methoxophenyl)-4-[1-(2-furoyl)-1,2,3,4-tetrahydroquinolin-2-ylmethyl]-piperazine,
1-(4-fluoro-2-methoxyphenyl)-4-[1-(4-methoxybenzoyl)-1,2,3,4-tetrahydroquinolin-2-ylmethy]-piperazine,
1-[1-(4-cyanobenzoyl)-1,2,3,4-tetrahydroquinolin-2-ylmethy]-4-(4-fluoro-2-methoxyphenyl)-piperazine,
1-[1-(1-acetyl-4-piperidinylcarbonyl)-1,2,3,4-tetrahydroquinolin-2-ylmethy]-4-(4-fluoro-2-methoxyphenyl)-piperazine,
1-(4-fluoro-2-methoxyphenyl)-4-[1-(2-thienylacetyl)-1,2,3,4-tetrahydroquinolin-2-ylmethyl]-piperazine,
1-(4-fluoro-2-methoxyphenyl)-4-[1-(3-thienylacetyl)-1,2,3,4-tetrahydroquinolin-2-ylmethyl]-piperazine,
1-(1-cyclobutylcarbonyl-1,2,3,4-tetrahydroquinolin-2-ylmethyl-4-(4-fluoro-2-methoxyphenyl)-piperazine,
1-(4-fluoro-2-methoxyphenyl)-4-[1-(4-phenoxybutanoyl)-1,2,3,4-tetrahydroquinolin-2-ylmethyl]-piperazine,
1-(4-fluoro-2-methoxyphenyl)-4-(1-phenylsulphonyl-1,2,3,4-tetrahydroquinolin-2-ylmethyl)-piperazine,
1-(1-benzylsulphonyl-1,2,3,4-tetrahydroquinolin-2-ylmethyl)-4-(4-fluoro-2-methoxyphenyl)-piperazine,
1-(4-fluoro-2-methoxyphenyl)-4-[1-(2-methylanilinocarbonyl)-1,2,3,4-tetrahydroquino]in-2-ylmethyl]-piperazine,
1-[1-(t-butylcarbamoyl)-1,2,3,4-tetrahydroquinolin-2-ylmethy]-4-(4-fluoro-2-methoxyphenyl)-piperazine,
1-(1-isoquinolinyl)-4-[1-(3-methylbutanoyl)-1,2,3,4-tetrahydroquinolin-2-ylmethyl]-piperazine,
1-[1-(3,3-dimethylbutanoyl)-1,2,3,4-tetrahydroquinolin-2-ylmethyl]-4-(1-isoquinolinyl)-piperazine,
1-[1-(2-cyclopropylacetyl)-1,2,3,4-tetrahydroquinolin-2-ylmethyl]-4-(1-isoqmnolinyl)-piperazine,
1-(1-isoquinolinyl)-4-[1-(2-pyrrolylcarbonyl)-1,2,3,4-tetrahydroquinolin-2-ylmethyl]-piperazine,
1-[1-(2-furoyl)-1,2,3,4-tetrahydroquinolin-2-ylmethyl]-4-(1-isoquinolinyl)-piperazine,
1-(1-isoquinolinyl)-4-[1-(4-methoxybenzoyl)-1,2,3,4-tetrahydroquinolin-2-ylmethyl]- piperazine,
1-[1-(4-cyanobenzoyl)-1,2,3,4-tetrahydroquinolin-2-ylmethyl]-4-(1-isoquinolinyl)-piperazine,
1-[1-(1-acetyl-4-piperidylcarbonyl)-1,2,3,4-tetrahydroquinolin-2-yhnethyl]-4-(1-isoquinolinyl)-piperazine,
1-(1-isoquinolinyl)-4-[1-(2-thienylacetyl)-1,2,3,4-tetrahydroquinolin-2-ylmethyl]-piperazine,
1-(1-isoquinolinyl)-4-[1-(3-thienylacetyl)-1,2,3,4-tetrahydroquinolin-2-ylmethyl]-piperazine,
1-(1-cyclobutanecarbonyl-1,2,3,4-tetrahydroquinolin-2-ylmethyl)-4-(1-isoquinolinyl)-piperazine,
1-(1-isoquinolinyl)-4-[1-(4-phenoxybutanoyl)-1,2,3,4-tetrahydroquinolin-2-ylmethyl]-piperazine,
1-(1-isoquinolinyl)-4-(1-phenylsulphonyl-1,2,3,4-tetrahydroquinolin-2-ylmethyl)-piperazine,
1-(1-benzylsulphonyl-1,2,3,4-tetrahydroquinolin-2-ylmethyl)-4-(1-Isoquinolinyl)-piperazine,
1-(1-isoquinolinyl)-4-[1-(2-methylanilinocarbonyl)-1,2,3,4-tetrahydroquinolin-2-ylmethyl]-piperazine,
1-[1-(t-butylcarbamoyl)-1,2,3,4-tetrahydroquinolin-2-ylmethy]-4-(1-Isoquinolinyl)-piperazine,
1-(2,3-dihydrobenzo[1,4]dioxin-5-yl)-4-[1-(3-methylbutanoyl)-1,2,3,4-tetrahydroquinolin-2-ylmethyl]-piperazine,
4-[1-(2-cyclopropylacetyl)-1,2,3,4-tetrahydroquinolin-2-ylmethyl]-1-(2,3-dihydrobenzo[1,4]dioxin-5-yl)-piperazine,
1-(2,3-dihydrobenzo[1,4]dioxin-5-yl)-4-[1-(2-pyrrolylcarbonyl)-1,2,3,4-tetrahydroquinolin-2-ylmethyl]-piperazine,
1-(2,3-dihydrobenzo[1,4]dioxin-5-yl)-4-[1-(2-furoyl)-1,2,3,4-tetrahydroquinolin-2-ylmethyl]-piperazine,
1-(2,3-dihydrobenzo[1,4]dioxin-5-yl)-4-[1-(4-methoxybenzoyl)-1,2,3,4-tetrahydroquinolin-2-ylmethyl]-piperazine,
4-[1-(4-cyanobenzoyl)-1,2,3,4-tetrahydroquinolin-2-ylmethyl]-1-(2,3-dihydrobenzo[1,4]dioxin-5-yl)-piperazine,
1-(2,3-dihydrobenzo[1,4]dioxin-5-yl)-4-[1-(2-thienylacetyl)-1,2,3,4-tetrahydroquinolin-2-ylmethyl]-piperazine,
1-(2,3-dihydrobenzo[1,4]dioxin-5-yl)-4-[1-(3-thienylacetyl)-1,2,3,4-tetrahydroquinolin-2-ylmethyl]-piperazine,
4-(1-cyclobutanecarbonyl-1,2,3,4-tetrahydroquinolin-2-ylmethyl)-1-(2,3-dihy drobenzo [1,4] dioxin-5-yl)-piperazine,
1-(2,3-dihydrobenzo[1,4]dioxin-5-yl)-4-[1-(4-phenoxybutanoyl)-1,2,3,4-tetrahydroquinolin-3-ylmethyl]-piperazine,
1-(2,3-dihydrobenzo[1,4]dioxin-5-yl)-4-(1-phenylsulphonyl-1,2,3,4-tetrahydroquinolin-2-ylmethyl]-piperazine,
1-(1-benzylsulphonyl-1,2,3,4-tetrahydroquinolin-2-ylmethyl)-4-(2,3-dihydrobenzo[1,4]dioxin-5-yl)-piperazine,
1-(2,3-dihydrobenzo[1,4]dioxin-5-yl)-4-[1-(2-methylanilinocarbonyl)-1,2,3,4-tetrahydroquinolin-2-ylmethyl]-piperazine,
1-[1-(t-butylcarbarnoyl)-1,2,3,4-tetrahydroquinolin-2-ylmethy]-4-(2,3-dihydrobenzo [1,4]dioxin-5-yl)-piperazine,
1-(2,3-dihydrobenzo[1,4]dioxin-5-yl)-4-[1-(3,3-dimethylbutanoyl)-1,2,3,4-tetrahydroquinolin-2-ylmethyl]-piperazine,
4-[1-(1-acetyl-4-piperidylecarbonyl)-1,2,3,4-tetrahydroquinolin-2-ylmethyl]-1-(2,3-dihydrobenzo[1,4]dioxin-5-yl)-piperazine,
1-(2,3-dihydrobenzo[1,4]dioxin-5-yl)-4-[1-(2,2-dimethylbutanoyl)-1,2,3,4-tetrahydroquinolin-2-ylmethyl]-piperazine,
1-(2,3-dihydrobenzo[1,4]dioxin-5-yl)-4-[1-(1-trifluoromethylcyclopropanecarbonyl)-1,2,3,4-tetrahydroquinolin-2-ylmethyl]-piperazine,
1-[1-(bicyclo[2.2.2]oct-2-ylcarbonyl)-1,2,3,4-tetrahydroquinolin-2-ylmethyl]-4-(2,3-dihydrobenzo[1,4]dioxin-5-yl)-piperazine,
1-(1-cyclopentanecarbonyl-1,2,3,4-tetrahydroquinolin-2-ylmethyl)-4-(2,3-dihydrobenzo[1,4]dioxin-5-yl)-piperazine,
1-(2,3-dihydrobenzo[1,4]dioxin-5-yl)-4-[1-(4-fluorobenzoyl)-1,2,3,4-tetrahydroquinolin-2-ylmethyl]-piperazine,
1-[1-(benzo[1,3]dioxol-5-ylcarbonyl)-1,2,3,4-tetrahydroquinolin-2-ylmethyl]-4-(2,3-dihydrobenzo[1,4]dioxin-5-yl)-piperazine,
1-(2,3-dihydrobenzo[1,4]dioxin-5-yl)-4-[1-(3-phenylpropenoyl)-1,2,3,4-tetrahydroquinolin-2-ylmethyl]-piperazine,
1-(2,3-dihydrobenzo[1,4]dioxin-5-yl)-4-[1-[3-(4-fluorophenyl)-propenoyl]-1,2,3,4-tetrahydroquinolin-2-ylmethyl]-piperazine,
1-(2,3-dihydrobenzo[1,4]dioxin-5-yl)-4-[1-(3-methyl-2-thienylcarbonyl)-1,2,3,4-tetrahydroquinolin-2-ylmethyl]-piperazine,
1-(2,3-dihydrobenzo[1,4]dioxin-5-yl)-4-[1-(3-furoyl)-1,2,3,4-tetrahydroquinolin-2-ylmethyl]-piperazine,
1-(2,3-dihydrobenzo[1,4]dioxin-5-yl)-4-[1-(2-methyl-3-furoyl)-1,2,3,4-tetrahydroquinolin-2-ylmethyl]-piperazine,
1-(2,3-dihydrobenzo[1,4]dioxin-5-yl)-4-[1-[5-methyl-4-(1,2,4-triazol-1-ylmethyl)-2-furoyl)-1,2,3,4-tetrahydroquinolin-2-ylmethyl]-piperazine,
1-(2,3-dihydrobenzo[1,4]dioxin-5-yl)-4-[1-(5-methyl-4-isoxazolecarbonyl)-1,2,3,4-tetrahydroquinolin-2-ylinethyl]-piperazine,
1-[1-(4-acetyl-2-pyrrolecarbonyl)-1,2,3,4-tetrahydroquinolin-2-ylmethyl]-4-(2,3-dihydrobenzo[1,4]dioxin-5-yl)-piperazine,
2-{N-[2-(2-methoxyphenoxy)-ethyl]-N-methyl-aminomethyl}-1-(3-methylbutanoyl)-1,2,3,4-tetrahydroquinoline,
1-(3,3-dimethylbutanoyl)-2-{N-[2-(2-methoxyphenoxy)-ethyl]-N-methyl-aminomethyl}-1,2,3,4-tetrahydroquinoline,
1-cyclopropylacetyl-2-{N-[2-(2-methoxyphenoxy)-ethyl]-N-methyl-aminomethyl}-1,2,3,4-tetrahydroquinoline,
2-{N-[2-(2-methoxyphenoxy)-ethyl]-N-methyl-aminomethyl}-1-(2-pyrrolylcarbonyl)-1,2,3,4-tetrahydroquinoline,
1-(2-furoyl)-2-{N-[2-(2-methoxyphenoxy)-ethyl]-N-methyl-aminomethyl}-1,2,3,4-tetrahydroquinoline,
1-(4-methoxybenzoyl)-2-{N-[2-(2-methoxyphenoxy)-ethyl]-N-methyl-aminomethyl}-1,2,3,4-tetrahydroquinoline,
1-(1-acetyl-4-piperidylcarbonyl)-2-{N-[2-(2-methoxyphenoxy)-ethyl]-N-methylaminomethyl}-1,2,3,4-tetrahydroquinoline,
2-{N-[2-(2-methoxyphenoxy)-ethyl]-N-methyl-aminomethyl}-1-(2-thienylacetyl)-1,2,3,4-tetrahydroquinoline,
2-{N-[2-(2-methoxyphenoxy)-ethyl]-N-methyl-aminomethyl}-1-(3-thienylacetyl)-1,2,3,4-tetrahydroquinoline,
1-cylobutanecarbonyl-2-{N-[2-(2-methoxyphenoxy)-ethyl]-N-methyl-aminomethyl}-1,2,3,4-tetrahydroquinoline,
2-{N-[2-(2-methoxyphenoxy)-ethyl]-N-methyl-aminomethyl}-1-(4-phenoxybutanoyl)-1,2,3,4-tetrahydroquinoline,
2-{N-[2-(2-methoxyphenoxy)-ethyl]-N-methyl-aminomethyl}-1-phenylsulphonyl-1,2,3,4-tetrahydroquinoline,
1-benzylsulphonyl-2-{N-[2-(2-methoxyphenoxy)-ethyl]-N-methyl-aminomethyl}-1,2,3,4-tetrahydroquinoline,
2-{N-[2-(2-methoxyphenoay)-ethyl]-N-methyl-aminomethyl}-1-(2-methylanilinocarbonyl)-1,2,3,4-tetrahydroquinoline,
1-(t-butylcarbamoyl)-2-{N-[2-(2-methoxyphenoxy)-ethyl]-N-methyl-aminomethyl}-1,2,3,4-tetrahydroquinoline
1-(1-cyclohexanecarbonyl-5-fluoro-1,2,3,4-tetrahydroquinoline-2-ylmethyl)-4-(4-fluoro-2-methoxyphenyl)-piperazine,
1-(1-cyclohexanecarbonyl-1,2,3,4-tetrahydroquinoline-2-ylmethyl)-4-(2-triluoromethanesulphonyloxyphenyl)-piperazine,
1-(1-cyclohexanecarbonyl-1,2,3,4-tetrahydroquinoline-2-ylmethyl)-4-(1-isopropyl-4-indolyl)-piperazine,
1-(6-*tert*-butoxycarbonylamino-1-cyclohexanecarbonyl-1,2,3,4-tetrahydroquinoline-2-ylmethyl)-4-(4-fluoro-2-methoxyphenyl)-piperazine,
1-(1-cyclohexanecarbonyl-1,2,3,4-tetrahydroquinoline-2-ylmethyl)-4-(1-methoxymethyl-4-indolyl)-piperazine,
1-(1-cyclohexanecarbonyl-2-methyl-1,2,3,4-tetrahydroquinoline-2-ylmethyl)-4-(4-fluoro-2-methoxyphenyl)-piperazine,
1-(1-cyclopentylcarbonyl-6-methyl-1,2,3,4-tetrahydroquinoline-2-ylmethyl]-4-(4-fluoro-2-methoxyphenyl)-piperazine,
1-(6-amino-1-cyclohexanecarbonyl-1,2,3,4-tetrahydroquinoline-2-ylmethyl)-4-(4-fluoro-2-methoxyphenyl)-piperazine,
1-(1-cyclohexanecarbonyl-1,2,3,4-tetrahydroquinoline-2-ylmethyl)-4-(2-methoxy-4-methylphenyl)-piperazine,
1-(4-benzo[1,3]dioxol-5-ylmethyl)-4-(1-cyclohexanecarbonyl-1,2,3,4-tetrahydroquinoline-2-ylmethyl)-piperazine,
1-cyclohexanecarbonyl-2-{N-[3-(2-methoxyphenoxy)-propyl]-aminomethyl}-1,2,3,4-tetrahydroquinoline,
1-cyclohexanecarbonyl-2-{N-[2-(2-methoxyphenoxy)-ethyl]-N-methyl-aminomethyl}-1,2,3,4-tetrahydroquinoline,
4-cyano-1-(1-cyclohexanecarbonyl-1,2,3,4-tetrahydroquinolin-2-ylmethyl)-4-phenylpiperidine,
4-benzyl-1-(-cyclohexanecarbonyl-1,2,3,4-tetrahydroquinolin-2-ylmethyl)-4-hydroxypiperidine,
4-benzyloxy-1-(1-cyclohexanecarbonyl-1,2,3,4-tetrahydroquinolin-2-ylmethyl)-piperidine,
4-benzoylamino-1-(1-cyclohexanecarbonyl-1,2,3,4-tetrahydroquinolin-2-ylmethyl)-piperidine,
4-acetyl-1-(1-cyclohexanecarbonyl-1,2,3,4-tetrahydroquinolin-2-ylmethyl)-4-phenylpiperidine,
1-(benzo[1,3]dioxol-4-yl)-4-(1-cyclohexanecarbonyl-1,2,3,4-tetrahydroquinoline-2-ylmethyl)-piperazine,
2-{N-[2-(3-chlorophenoxy)-ethyl]-N-methyl-aminomethyl}-1-cyclohexanecarbonyl-1,2,3,4-tetrahydroquinoline,
2-{N-[2-(4-chlorophenoxy)-ethyl]-N-methyl-aminomethyl}-1-cyclohexanecarbonyl-1,2,3,4-tetrahydroquinoline,
2-{N-benzyl-N-[2-(2-methoxyphenoxy)-ethyl]-aminomethyl}-1-cyclohexanecarbonyl-1,2,3,4-tetrahydroquinoline,
1-cyclohexanecarbonyl-2-[N-methyl-N-(3-phenylpropyl)-aminomethyl]-1,2,3,4-tetrahydroquinoline,
1-(4-cyano-2-trifluoromethoxyphenyl)-4-(1-cyclohexanecarbonyl-1,2,3,4-tetrahydroquinoline-2-ylmethyl)-piperazine,
1-(1-cylohexanecarbonyl-1,2,3,4-tetrahydroquinoline-2-ylmethyl)-4-(4-fluoro-2-methoxy-anilino)-piperidine,
1-(3H-1,2,3-benzotriazol-4-yl)-4-(1-cydohexanecarbonyl-1,2,3,4-tetrahydroquinoline-2-ylmethyl)-piperazine,
1-(1H-1,3-benzodiazol-4-yl)-4-(1-cyclohexanecarbonyl-1,2,3,4-tetrahydroquinoline-2-ylmethyl)-piperazine,
1-cyclohexanecarbonyl-2-{N-[2-(2-pyridyloxy)-ethyl]-aminomethyl}-1,2,3,4-tetrahydroquinoline,
1-cyclohexanecarbonyl-2-{N-methyl-N-[2-(2-pyridyloxy)-ethyl]-aminomethyl}-1,2,3,4-tetrahydroquinoline,
1-cyclohexanecarbonyl-2-{N-methyl-N-[3-(2-methoxyphenyl)-propyl]-aminomethyl}-1,2,3,4-tetrahydroquinoline,
4-benzoyl-1-(1-cyclohexanecarbonyl-1,2,3,4-tetrahydroqumolin-2-ylmethyl)-piperazine,
1-(1-cyclohexanecarbonyl-1,2,3,4-tetrahydroquinoline-2-ylmethyl)-3-oxo-4-phenylpiperazine,
1-(4-fluoro-2-methoxyphenyl)-4-(1-hexanoyl-1,2,3,4-tetrahydroquinoline-2-ylmethyl)-piperazine,
1-(2,3-dihydrobenzo[1,4]dioxin-5-yl)-4-[1-pentafluoropropionyl-1,2,3,4-tetrahydroquinoline-2-ylmethyl]-piperazine,
1-(2,3-dihydrobenzo[1,4]dioxin-5-yl)-4-[1-(3,3,3-trifluoropropionyl)-1,2,3,4-tetrahydroquinoline-2-ylmethyl]-piperazine,
(Z)-1-(2,3-dihydrobenzo[1,4]dioxin-5-yl)-4-[1-(4-hydroxy-cyclohexanecarbonyl)-1,2,3,4-tetrahydroquinoline-2-ylmethyl]-piperazine, and
1-(1-cyclohexanecarbonyl-6-fluoro-1,2,3,4-tetrahydroquinoline-2-ylmethyl)-4-(4-fluoro-2-methoxyphenyl)-piperazine.

15. A pharmaceutical composition comprising a compound according to any of claims 1 to 14, or an enantiomer, diastereomer, N-oxide, crystalline form, hydrate, solvate or pharmaceutically acceptable salt of such a compound, in admixture with a pharmaceutically acceptable diluent or carrier.

## Patentansprüche

1. Verbindung mit der allgemeinen Formel I 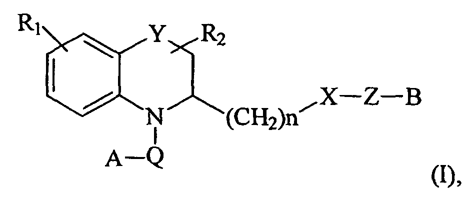 wobei
R₁ für einen oder mehrere Substituenten, ausgewählt aus Wasserstoff- und Halogenatomen und Hydroxy-, Alkyl-, Haloalkyl-, Alkoxy-, Haloalkoxy-, Nitro-, Phenyl-, substituierten Phenylgruppen, heterocyclischen Gruppen, substituierten heterocyclischen Gruppen und NR₃R₄- Gruppen steht, wobei jedes von R₃ und R₄ unabhängig für ein Wasserstoffatom oder eine Alkyl-, Acyl- oder Alkoxycarbonylgruppe steht,
R₂ für ein oder zwei Substituenten, ausgewählt aus Wasserstoffatomen und Alkylgruppen steht,
Y für eine CH₂- Gruppe oder eine Bindung steht,
Q für eine Carbonyl- Thiocarbonyl oder Sulphonylgruppe steht,
A für eine Alkyl-, Alkenyl-, Cycloalkyl-, Cycloalkenyl-, Aryl-, Alkylamino-, Dialkylamino-, Arylamino-, oder Arylalkylaminogruppe oder eine heterocyclische Gruppe, die jeweils gegebenenfalls substituiert sein kann, steht,
n 1 oder 2 ist,
X für einen Aminorest, ausgewählt aus 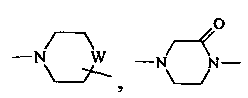 und -N(R₅)-CH₂- steht, wobei R₅ für ein Wasserstoffatom oder eine Methyl- oder Benzylgruppe steht und W für eine Bindung, ein Stickstoffatom oder eine CH, CH₂,C(CN), C(OH) oder C(COCH₃)- Gruppe steht (wenn W für ein Stickstoffatom oder eine CH, C(CN), C(OH) oder C(COCH₃)- Gruppe steht; ist die Gruppe Z-B an das W-Ringatom gebunden;
Wenn X für eine aus 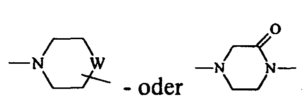 -Gruppe steht, steht Z für eine Bindung, ein Sauerstoff- oder Schwefelatom, oder eine CH_{2,}CH₂CH_{2,}CO, CHOH, OCH₂, NH, NHCO oder NHCONHCH₂- Gruppe, wenn X für eine -N(R₅)-CH₂-Gruppe steht, steht Z für eine CH₂CH₂ oder CH₂O Gruppe oder Z und B zusammen stehen für eine 2,3-Dihydro-benzo[1,4]dioxin-2-yl Gruppe und
ausgenommen des zuvor gesagten steht B für eine monozyklische oder bizyklische Arylgruppe oder ein monozyklischer oder bizyklischer Heterozyklus, wobei jeder dieser gegebenenfalls substituiert sein kann
oder ein Enantiomer, Diastereomer, N-Oxid, eine kristalline Form, ein Hydrat, Solvat oder pharmazeutisch akzeptables Salz einer solchen Verbindung.

2. Verbindung nach Anspruch 1, **dadurch gekennzeichnet, dass** R₁ für ein Wasserstoffatom steht.

3. Verbindung nach Anspruch 1 oder Anspruch 2, **dadurch gekennzeichnet, dass** R₂ für ein Wasserstoffatom steht.

4. Verbindung nach einem der Ansprüche 1 bis 3,**dadurch gekennzeichnet, dass** Y für eine CH₂- Gruppe steht.

5. Verbindung nach einem der Ansprüche 1 bis 4,**dadurch gekennzeichnet, dass** Q für eine Carbonylgruppe steht.

6. Verbindung nach einem der Ansprüche 1 bis 5, **dadurch gekennzeichnet, dass** n 1 ist.

7. Verbindung nach einem der Ansprüche 1 bis 6, **dadurch gekennzeichnet, dass** X für eine 1,4-Piperazin-di-yl- Gruppe steht.

8. Verbindung nach Anspruch 7, **dadurch gekennzeichnet, dass** Z für eine Bindung steht.

9. Verbindung nach einem der Ansprüche 1 bis 8, **dadurch gekennzeichnet, dass** A für eine Cyclohexylgruppe steht.

10. Verbindung nach einem der Ansprüche 1 bis 9, **dadurch gekennzeichnet, dass** B für eine monozyklische Arylgruppe oder einen bizyklischen Heterozyklus steht, wobei jeder davon gegebenenfalls substituiert sein kann.

11. Verbindung nach Anspruch 10, **dadurch gekennzeichnet, dass** B für eine 4-Indolyl- oder 2,3-Dihydrobenzo[1,4]dioxin-5-ylgruppe steht.

12. Verbindung nach Anspruch 10, **dadurch gekennzeichnet, dass** B für eine Phenylgruppe steht, die mit einem oder mehreren Substituenten, ausgewählt aus Fluor- und Chloratomen, Methoxy- Trifluormethoxy und 2,2,2-Trifluorethoxygruppen substituiert ist.

13. Verbindung nach Anspruch 12,**dadurch gekennzeichnet, dass** B für eine 2-Methoxyphenyl- oder 4-Fluor-2-methoxyphenylgruppe steht.

14. Verbindung nach Anspruch 1,**dadurch gekennzeichnet, dass** sie eine der Folgenden ist:
1-(1-Cyclohexancarbonyl-1,2,3,4-tetrahydrochinolin-2-ylmethyl)-4-(4-indolyl)-piperazin,
(+)-1-(1-Cyclohexancarbonyl-1,2,3,4-tetrahydrochinolin-2-ylmethyl)-4-(4-indolyl)-piperazin,
(-)-1-(1-Cyclohexancarbonyl-1,2,3,4-tetrahydrochinolin-2-ylmethyl)-4-(4-indolyl)-piperazin,
1-(1-Cyclohexancarbonyl-1,2,3,4-tetrahydrochinolin-2-ylmethyl)-4-(2-methoxyphenyl)-piperazin,
1-[2-(1-Cyclohexancarbonyl-1,2,3,4-tetrahydrochinolin-2-yl)-ethyl]-4-(2-methoxyphenyl)-piperazin,
1-(1-Cyclohexancarbonyl-1,2,3,4-tetrahydrochinolin-2-ylmethyl)-4-[2-(2,2,2-trifluorethoxy)-phenyl]-piperazin,
1-(1-Cyclohexancarbonyl-6-fluor-1,2,3,4-tetrahydrochinolin-2-ylmethyl)-4-[2-(2,2,2-trifluorethoxy)-phenyl]-piperazin,
1-[1-(2-Ethylbutanoyl)-1,2,3,4-tetrahydrochinolin-2-ylmethyl]-4-[2-(2,2,2-trifluorethoxy)-phenyl]-piperazin,
1-[1-(3-Methoxypropionyl)-1,2,3,4-tetrahydrochinolin-2-ylmethyl]-4-[2-(2,2,2-trifluorethoxy)-phenyl]-piperazin,
1-[1-(3-Benzyloxypropionyl)-1,2,3,4-tetrahydrochinolin-2-ylmethyl]-4-[2-(2,2,2-trifluor-ethoxy)-phenyl]-piperazin,
1-[1-(3-Benzyloxypropionyl)-1,2,3,4-tetrahydrochinolin-2-ylmethyl]-4-(4-indolyl)-piperazin,
1-[1-(3-Hydroxypropionyl)-1,2,3,4-tetrahydrochinolin-2-ylmethyl]-4-[2-(2,2,2-trifluorethoxy)-phenyl]-piperazin,
1-[1-(3-Methoxypropionyl)-1,2,3,4-tetrahydrochinolin-2-ylmethyl]-4-(4-indolyl)-piperazin,
1-[1-(3-Isopropoxypropionyl)-1,2,3,4-tetrahydrochinolin-2-ylmethyl]-4-(4-indolyl)-piperazin,
1-[1-Acetyl-1,2,3,4-tetrahydrochinolin-2-ylmethyl]-4-(4-indolyl)-piperazin,
1-[1-(4-Morpholincarbonyl)-1,2,3,4-tetrahydrochinolin-2-ylmethyl]-4-(4-indolyl)-piperazin,
1-(1-Cyclohexanearbonyl-1,2,3,4-tetrahydrochinolin-2-ylmethyl)-4-(1-methyl-4-indolyl)-piperazin,
1-(1-Cyclohexancarbonyl-6-methoxy-1,2,3,4-tetrahydrochinolin-2-ylmethyl)-4-(4-indolyl)-piperazin,
1-[1-(3-Hydroxypropionyl)-1,2,3,4-tetrahydrochinolin-2-ylmethyl]-4-(4-indolyl)-piperazin,
1-(1-Cyclohexancarbonyl-6-fluor-1,2,3,4-tetrahydrochinolin-2-ylmethyl)-4-(4-indolyl)-piperazin,
1-(1-Dimethylaminocarbonyl-1,2,3,4-tetrahydrochinolin-2-ylmethyl)-4-(4-indolyl)-piperazin,
1-(1-Ethylaminocarbonyl-1,2,3,4-tetrahydrochinolin-2-ylmethyl)-4-(4-indolyl)-piperazin,
1-(1-Cyclohexancarbonyl-1,2,3,4-tetrahydrochinolin-2-ylmethyl)-4-(1-isochinolinyl)-piperazin,
1-(1-Cyclohexancarbonyl-1,2,3,4-tetrahydrochinolin-2-ylmethyl)-4-(2-methoxyphenyl)-piperidin,
1-(7-Benzofuranyl)-4-(1-cyclohexancarbonyl-1,2,3,4-tetrahydrochinolin-2-ylmethyl)-piperazin,
1-(2-Ethylbutanoyl-1,2,3,4-tetrahydrochinolin-2-ylmethyl)-4-(4-indolyl)-piperazin,
1-(1-Cyclohex-3-enylcarbonyl-1,2,3,4-tetrahydrochinolin-2-ylmethyl)-4-(4-indolyl)-piperazin,
1-(1-Cycloheptancarbonyl-1,2,3,4-tetrahydrochinolin-2-ylmethyl)-4-(4-indolyl)-piperazin,
1-(1-Cyclopentancarbonyl-1,2,3,4-tetrahydrochinolin-2-ylmethyl)-4-(4-indolyl)-piperazin,
1-(1-Benzoyl-1,2,3,4-tetrahydrochinolin-2-ylmethyl)-4-(4-indolyl)-piperazin
1-(4-Indolyl)-4-(1-pentanoyl-1,2,3,4-tetrahydrochinolin-2-ylmethyl)-piperazin
1-(1-Cyclohexancarbonyl-6-trifluormethyl-1,2,3,4-tetrahydrochinolin-2-ylmethyl)-4-(4-indolyl)-piperazin,
1-(1-Cyclohexancarbonyl-6-trifluormethoxy-1,2,3,4-tetrahydrochinolin-2-ylmethyl)-4-(4-indolyl)-piperazin,
1-[1-(3-Benzylaminopropionyl)-1,2,3,4-tetrahydrochinolin-2-ylmethyl]-4-(4-indolyl)-piperazin,
1-(3-aminopropionyl-1,2,3,4-tetrahydrochinolin-2-ylmethyl]-4-(4-indolyl)-piperazin,
1-(4-Indolyl)-4-[1-(3-methylaminopropionyl-1,2,3,4-tetrahydrochinolin-2-ylmethyl]-piperazin,
1-[1-(3-Dimethylaminopropionyl)-1,2,3,4-tetrahydrochinolin-2-ylmethyl]-4-(4-indolyl)-piperazin,
1-(4-Indolyl)-4-(1-anilincarbonyl-1,2,3,4-tetrahydrochinolin-2-ylmethyl)-piperazin,
1-(1-Cyclohexancarbonyl-6-methyl-1,2,3,4-tetrahydrochinolin-2-ylmethyl)-4-(4-indolyl)-piperazin,
1-(4-Indolyl)-4-(1-pyrrolidincarbonyl-1,2,3,4-tetrahydrochinolin-2-ylmethyl)-piperazin,
1-(6-Brom-1-cyclohexancarbonyl-1,2,3,4-tetrahydrochinolin-2-ylmethyl)-4-(4-indolyl)-piperazin,
1-(1-Cyclohexancarbonyl-1,2,3,4-tetrahydrochinolin-2-ylmethyl)-4-(4-fluor-2-methoxyphenyl)-piperazin,
1-(1-Cyclohexancarbonyl-1,2,3,4-tetrahydrochinolin-2-ylmethyl)-4-(2-methyl-4-indolyl)-piperazin,
1-(1-Cyclohexancarbonyl-1,2,3,4-tetrahydrochinolin-2-ylmethyl)-4-phenylpiperazin,
1-(1-Cyclohexancarbonyl-1,2,3,4-tetrahydrochinolin-2-ylmethyl)-4-(2-ethoxyphenyl)-piperazin,
1-(1-Cyclohexancarbonyl-1,2,3,4-tetrahydrochinolin-2-ylmethyl)-4-(2,5-dimethoxyphenyl)-piperazin,
1-(1-Cyclohexancarbonyl-1,2,3,4-tetrahydrochinolin-2-ylmethyl)-4-(2,3-dihydrobenzofuran-7-yl)-piperazin,
1-(1-Cyclohexancarbonyl-1,2,3,4-tetrahydrochinolin-2-ylmethyl)-4-(5-fluor-2-methoxyphenyl)-piperazin,
1-(1-Cyclohexancarbonyl-1,2,3,4-tetrahydrochinolin-2-ylmethyl)-4-(2-methylphenyl)-piperazin,
1-(1-Cyclohexancarbonyl-1,2,3,4-tetrahydrochinolin-2-ylmethyl)-4-(2-trifluormethylphenyl)-piperazin,
1-(1-Cyclohexancarbonyl-1,2,3,4-tetrahydrochinolin-2-ylmethyl)-4-(2,4-dichlorphenyl)-piperazin,
1-(1-Cyclohexancarbonyl-1,2,3,4-tetrahydrochinolin-2-ylmethyl)-4-(2-hydroxyphenyl)-piperazin,
1-(1-Cyclohexancarbonyl-1,2,3,4-tetrahydrochinolin-2-ylmethyl)-4-(2-isopropoxyphenyl)-piperazin,
1-(1-Cyclohexancarbonyl-1,2,3,4-tetrahydrochinolin-2-ylmethyl)-4-(2-fluor-5-methylphenyl)-piperazin,
1-(1-Cyclohexancarbonyl-1,2,3,4-tetrahydrochinolin-2-ylmethyl)-4-(2,3-dimethyl-4-indolyl)-piperazin,
1-(1-Cyclohexancarbonyl-1,2,3,4-tetrahydrochinolin-2-ylmethyl)-4-(2-chlor-5-fluorphenyl)-piperazin,
1-(1-Cyclohexancarbonyl-1,2,3,4-tetrahydrochinolin-2-ylmethyl)-4-(5-fluor-2-methylphenyl)-piperazin,
1-[(2,3-Dihydro-1,4-benzodioxin-5-yl)]-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(2-trifluormethoxyphenyl)-piperazin,
1-(1-Cyclohexancarbonyl-1,2,3,4-tetrahydrochinolin-2-ylmethyl)-4-(4-fluor-2-methylphenyl)-piperazin,
1-(1-Cyclohexancarbonyl-1,2,3,4-tetrahydrochinolin-2-ylmethyl)-2,5-dichlorphenyl)-piperazin,
1-(1-Cyclohexancarbonyl-1,2,3,4-tetrahydrochinolin-2-ylmethyl)-4-[4-fluor-2-(2,2,2-trifluorethoxyphenyl)]-piperazin,
1-(1-Cyclohexancarbonyl-1,2,3,4-tetrahydrochinolin-2-ylmethyl)-4-(2-pyrimidinyl)-piperazin,
1-(1-Cyclohexancarbonyl-1,2,3,4-tetrahydrochinolin-2-ylmethyl)-4-(8-chinolinyl)-piperazin,
1-(5-Chlor-2-cyanophenyl)-4-(1-cyclohexancarbonyl-1,2,3,4-tetrahydrochinolin-2-ylmethyl)-piperazin,
1-(5-Cyano-2-methoxyphenyl)-4-(1-cyclohexancarbonyl-1,2,3,4-tetrahydrochinolin-2-ylmethyl)-piperazin,
1-(1-Acetyl-4-indolyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(7-indolyl)-piperazin,
1-(3-Cyano-4-indolyl)-4-(1-cyclohexancarbonyl-1,2,3,4-tetrahydrochinolin-2-ylmethyl)-piperazin,
1-(1-Cyclohexanecarbonyl-1,2,3,4-tetrahydrochinolin-2-ylmethyl)-4-(2,4-difluorbenzyl)-piperazin,
1-(2-Brombenzyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(2,5-difluorbenzyl)-piperazin,
1-(1-Cyclohexancarbonyl-1,2,3,4-tetrahydrochinolin-2-ylmethyl)-4-(1-naphthyl)-piperazin,
1-(7-Brom-4-indolyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(3,4-dihydro-2H-benzo [b] [1,4] dioxepin-6-yl)-piperazin,
1-(2-Chlorbenzyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(6-methoxy-2-pyridyl)-piperazin,
1-(1-Cyclohexancarbonyl-1,2,3,4-tetrahydrochinolin-2-ylmethyl)-4-(2,5-dichlorbenzyl)-piperazin,
1-(4-Indolyl)-4-(1-piperidinocarbonyl-1,2,3,4-tetrahydrochinolin-2-ylmethyl)-piperazin,
1-[1-(3-Cyanopropionyl)-1,2,3,4-tetrahydrochinolin-2-ylmethyl]-4-(4-indolyl)-piperazin,
1-(1-Cyclohexancarbonyl-8-fluor-1,2,3,4-tetrahydrochinolin-2-ylmethyl)-4-(4-indolyl)-piperazin,
1-[1-(3-Acetylaminopropionyl)-1,2,3,4-tetrahydrochinolin-2-ylmethyl]-4-(4-indolyl)-piperazin,
1-[1-(3-Carbamoylaminopropionyl)-1,2,3,4-tetrahydrochinolin-2-ylmethyl]-4-(4-indolyl)-piperazin,
1-{1-[3-Bis (acetylamino)-propionyl]-1,2,3,4-tetrahydrochinolin-2-ylmethyl}-4-(4-indolyl)-piperazin,
1-(6-Chlor-1-cyclohexancarbonyl-1,2,3,4-tetrahydrochinolin-2-ylmethyl)-4-(4-indolyl)-piperazin,
(R)-1-(1-Cyclohexancarbonyl-1,2,3,4-tetrahydrochinolin-2-ylmethyl)-4-(1-methyl-4-indolyl)-piperazin,
(Z)-1-[1-(4-Hydroxycyclohexancarbonyl)-1,2,3,4-tetrahydrochinolin-2-ylmethyl]-4-(4-indolyl)-piperazin,
(E)-1-[1-(4-Hydroxycyclohexancarbonyl)-1,2,3,4-tetrahydrochinolin-2-ylmethyl]-4-(4-indolyl)-piperazin,
1-(1-Cyclohexancarbonyl-7-fluor-1,2,3,4-tetrahydrochinolin-2-ylmethyl)-4-(4-indolyl)-piperazin,
1-(1-Cyclohexancarbonyl-6-phenyl-1,2,3,4-tetrahydrochinolin-2-ylmethyl)-4-(4-indolyl)-piperazin,
1-(1-Cyclohexancarbonyl-2,3-dihydroindol-2-ylmethyl)-4-(4-indolyl)-piperazin,
1-(1-Cyclohexancarbonyl-8-methoxy-1,2,3,4-tetrahydrochinolin-2-ylmethyl)-4-(4-indolyl)-piperazin,
1-(1-Cyclohexancarbonyl-6-hydroxy-1,2,3,4-tetrahydrochinolin-2-ylmethyl)-4-(4-indolyl)-piperazin,
1-(6-Brom-1-cyclohexancarbonyl-1,2,3,4-tetrahydrochinolin-2-ylmethyl)-4-(4-fluor-2-methoxyphenyl)-piperazin,
1-(1-Cyclohexancarbonyl-8-hydroxy-1,2,3,4-tetrahydrochinolin-2-ylmethyl)-4-(4-indolyl)-piperazin,
1-(1-Cyclohexancarbonyl-6-methyl-1,2,3,4-tetrahydrochinolin-2-ylmethyl)-4-(4-fluor-2-methoxyphenyl)-piperazin,
1-[1-(3-Cyanaminopropionyl-1,2,3,4-tetrahydrochinolin-2-ylmethyl)]-4-(4-indolyl)-piperazin,
1-(5-Chlor-1-cyclohexancarbonyl-1,2,3,4-tetrahydrochinolin-2-ylmethyl)-4-(4-fluor-2-methoxyphenyl)-piperazin,
1-[5-Chlor-1-(4-methoxybenzoyl)-1,2,3,4-tetrahydrochinolin-2-ylmethyl]-4-(4-fluor-2-methoxyphenyl)-piperazin,
1-(7-Chlor-1-cyclohexancarbonyl-1,2,3,4-tetrahydrochinolin-2-ylmethyl)-4-(4-fluor-2-methoxyphenyl)-piperazin,
3-Benzyl-1-(1-cyclohexancarbonyl-1,2,3,4-tetrahydrochinolin-2-ylmethyl)-piperidin (oberes TLC Rf Diastereomer),
3-Benzyl-1-(1-cyclohexancarbonyl-1,2,3,4-tetrahydrochinolin-2-ylmethyl)-piperidin (unteres TLC Rf Diastereomer),
1-(4-Chlor-2-i-propoxyphenyl)-4-(1-cyclohexancarbonyl-1,2,3,4-tetrahydrochinolin-2-ylmethyl)-piperazin,
1-(5-Chlor-2-fluorphenyl)-4-(1-cyclohexancarbonyl-1,2,3,4-tetrahydrochinolin-2-ylmethyl)-piperazin,
1-[4-(2,1,3-Benzothiadiazolyl)]-4-(1-cyclohexancarbonyl-1,2,3,4-tetrahydrochinolin-2-ylmethyl)-piperazin,
1-(1-cyclohexancarbonyl-1,2,3,4-tetrahydrochinolin-2-ylmethyl)-4-(2-ethoxy-4-fluorphenyl)-piperazin,
1-(1-Cyclohexancarbonyl-1,2,3,4-tetrahydrochinolin-2-ylmethyl)-4-(2-methoxy-4-hydroxyphenyl)-piperazin,
1-(1-Cyclohexancarbonyl-1,2,3,4-tetrahydrochinolin-2-ylmethyl)-4-(7-methoxy-4-indolyl)-piperazin,
1-(1-Cyclohexancarbonyl-1,2,3,4-tetrahydrochinolin-2-ylmethyl)-4-(2-pyrazinyl)-piperazin,
1-(2-Cyano-4-nitrophenyl)-4-(1-cyclohexancarbonyl-1,2,3,4-tetrahydrochinolin-2-ylmethyl)-piperazin,
1-[4-(2,1,3-Benzoxadiazolyl)]-4-(1-cyclohexancarbonyl-1, 2,3,4-tetrahydrochinolin-2-ylmethyl)-piperazin,
1-(1-Cyclohexancarbonyl-1,2,3,4-tetrahydrochinolin-2-ylmethyl)-4-(2-methoxy-5-trifluormethylphenyl)-piperazin,
1-(1-Cyclohexancarbonyl-1,2,3,4-tetrahydrochinolin-2-ylmethyl)-4-[1-(1,2,3,4-tetrahydronaphthyl)]-piperazin,
1-(7-Chlor-2,3-dihydro-1,4-benzodioxin-5-yl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(4-pyridyl)-piperazin,
1-(1-Cyclohexancarbonyl-1,2,3,4-tetrahydrochinolin-2-ylmethyl)-4-[4-(6, 7-dimethoxychinazolinyl)]-piperazin,
1-(4-Fluor-2-methoxyphenyl)-4-(1-cyclohexancarbonyl-6-nitro-1,2,3,4-tetrahydrochinolin-2-ylmethyl)-piperazin,
1-(1-Cyclohexancarbonyl-1,2,3,4-tetrahydrochinolin-2-ylmethyl)-4-(1-ethyl-4-indolyl)-piperazin,
1-(2-Brom-5-methoxybenzyl)-4-(1-cyclohexancarbonyl-1,2,3,4-tetrahydrochinoline-2-ylmethyl)-piperazin,
1-(4-Chlor-2-methoxyphenyl)-4-(1-cyclohexancarbonyl-1,2,3,4-tetrahydrochinolin-2-ylmethyl)-piperazin,
1-(4-Chlor-2-ethoxyphenyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(4-fluor-2-i-propoxyphenyl)-piperazin,
1-(1-Cyclohexancarbonyl-1,2,3,4-tetrahydrochinolin-2-ylmethyl)-4-(7-methyl-4-indolyl)-piperazin,
1-(1-Cyclohexancarbonyl-1,2,3,4-tetrahydrochinolin-2-ylmethyl)-4-(2-methoxyphenoxy)-piperidin,
1-[6-(5-Acetyl-2-thienyl)-1-cyclolhexancarbonyl-1,2,3,4-tetrahydrochinolin-2-ylmethyl)-4-(4-fluor-2-methoxyphenyl)-piperazin,
1- [1-Cyclohexancarbonyl-6-(3,5-dimethylisoxazol-4-yl)-1,2,3,4-tetrahydrochinolin-2-ylmethyl]-4-(4-fluor-2-methoxyphenyl)-piperazin,
1-(1-Cyclohexancarbonyl-7-methyl-1,2,3,4-tetrahydrochinolin-2-ylmethyl)-4-(4-fluor-2-methoxyphenyl)-piperazin,
1-(1-Cyclohexancarbonyl-4-methyl-1,2,3,4-tetrahydrochinolin-2-ylmethyl)-4-(4-fluor-2-methoxyphenyl)-piperazin,
1-(1-Cyclohexancarbonyl-8-methyl-1,2,3,4-tetrahydrochinolin-2-ylmethyl)-4-(4-fluor-2-methoxyphenyl)-piperazin,
1-(4-Indolyl)-4-[1-(2-pyrrolylcarbonyl)-1,2,3,4-tetrahydrochinolin-2-ylmethyl]-piperazin,
1-[1-(4-Dimethylaminobenzoyl)-1,2,3,4-tetrahydrochinolin-2-ylmethyl]-4-(4-indolyl)-piperazin,
1-(4-Indolyl)-4-[1-(2-phenylacetyl)-1,2,3,4 tetrahydrochinolin-2-ylmethyl] piperazin,
1-[1-(4-Cyanobenzoyl) 1,2,3,4 tetrahydrochinolin-2-ylmethyl]-4-(4-indolyl)-piperazin,
1-[1-(2-Chlorbenzoyl) 1,2,3,4 tetrahydrochinolin-2-ylmethyl]-4-(4-indolyl)-piperazin,
1-[1-(4-Chlorbenzoyl) 1,2,3,4 tetrahydrochinolin-2-ylmethyl]-4-(4-indolyl)-piperazin,
1-[1-(3-Chlorbenzoyl) 1,2,3,4 tetrahydrochinolin-2-ylmethyl]-4-(4-indolyl)-piperazin,
1-[1-(5-Benzo[1,3]dioxolylcarbonyl) 1,2,3,4 tetrahydrochinolin-2-ylmethyl]-4-(4-indolyl)-piperazin,
1-(1-Cyclopropylcarbonyl-1,2,3,4 tetrahydrochinolin-2-ylmethyl)-4-(4-indolyl)-piperazin,
1-(4-Indolyl)-4-[1-(2-methylpropionyl)-1,2,3,4 tetrahydrochinolin-2-ylmethyl]-piperazin,
1-(4-Indolyl)-4-[1-(2-methoxyacetyl)-1,2,3,4 tetrahydrochinolin-2-ylmethyl]-piperazin,
1-(2-Cyclopropylacetyl-1,2,3,4-tetrahydrochinolin-2-ylmethyl)-4-(4-indolyl)piperazin,
1-(4-Indolyl)-4-[1-(3-methylbutanoyl)-1,2,3,4-tetrahydrochinolin-2-ylmethyl] piperazin,
1-(2-Furoyl-1,2,3,4-tetrahydrochinolin-2-ylmethyl)-4-(4-indolyl)-piperazin,
1-(4-Indolyl)-4-[1-(5-isoxazolylcarbonyl)-1,2,3,4-tetrahydrochinolin-2-ylmethyl]-piperazin,
1-(4-Indolyl)-4-[1-(3-tetrahydrofurylcarbonyl)-1,2,3,4-tetrahydrochinolin-2-ylmethyl]-piperazin,
1-(4-Indolyl)-4-[1-(2-tetrahydrofurylcarbonyl)-1,2,3,4-tetrahydrochinolin-2-ylmethyl]-piperazin,
1-[1-(3,3-Dimethylbutanoyl)-1,2,3,4-tetrahydrochinolin-2-ylmethyl]-4-(4-indolyl)-piperazin,
1-[1-(2-Acetoxyacetyl)-1,2,3,4-tetrahydrochinolin-2-ylmethyl]-4-(4-indolyl)-piperazin,
1-(4-Indolyl)-4-[1-(2-thienylcarbonyl)-1,2,3,4-tetrahydrochinolin-2-ylmethyl]-piperazin,
1-(4-Indolyl)-4- [1-(3-thienylcarbonyl)-1,2,3,4-tetrahydrochinolin-2-ylmethyl]-piperazin,
1-[1-(2-Cyclohexylacetyl)-1,2,3,4-tetrahydrochinolin-2-ylmethyl]-4-(4-indolyl)-piperazin,
1-(4-Indolyl)-4-[1-(3-trifluormethylbenzoyl)-1,2,3,4-tetrahydrochinolin-2-ylmethyl]-piperazin,
1-(4-Indolyl)-4-[1-(4-trifluormethylbenzoyl)-1,2,3,4-tetrahydrochinolin-2-ylmethyl]-piperazin,
1-(4-Indolyl)-4-[1-(3-phenylpropionyl)-1,2,3,4-tetrahydrochinolin-2-ylmethyl] piperazin,
1-(4-Indolyl)-4-[1-(2-methoxybenzoyl)-1,2,3,4-tetrahydrochinolin-2-ylmethyl] piperazin,
1-(4-Indolyl)-4-[1-(4-methoxybenzoyl)-1,2,3,4-tetrahydrochinolin-2-ylmethyl] piperazin,
1-[1-(4-Fluorphenylacetyl)-1,2,3,4-tetrahydrochinolin-2-ylmethyl]-4-(4-indolyl)-piperazin,
1-[1-(2,6-Difluorbenzoyl)-1,2,3,4-tetrahydrochinolin-2-ylmethyl]-4-(4-indolyl)piperazin,
1-(4-Indolyl)-4-[1-(2-methoxyphenylacetyl)-1,2,3,4-tetrahydrochinolin-2-ylmethyl] piperazin,
1-[1-(4-Chlorphenylacetyl)-1,2,3,4-tetrahydrochinolin-2-ylmethyl]-4-(4-indolyl)-piperazin,
1-(4-Indolyl)-4-[1-[2-(4-trifluormethylphenyl)-acetyl]-1,2,3,4-tetrahydrochinolin] -2-ylmethyl]-piperazin,
1-(4-Indolyl)-4-[1-(2-pyridylcarbonyl)-1,2,3,4-tetrahydrochinolin-2-ylmethyl]-piperazin
1-(4-Indolyl)-4-[1-(3-pyridylcarbonyl)-1,2,3,4-tetrahydrochinolin-2-ylmethyl]-piperazin
1-(4-Indolyl)-4-[1-(4-pyridylcarbonyl)-1,2,3,4-tetrahydrochinolin-2-ylmethyl]-piperazin
1-[1-(3,5-Dimethyl-4-isoxazolylcarbonyl)-1,2,3,4-tetrahydrochinolin-2-ylmethyl]-4-(4-indolyl)-piperazin,
1-[1-(2,2-Dimethylpropionyl)-1,2,3,4-tetrahydrochinolin-2-ylmethyl]-4-(4-indolyl)-piperazin,
1-(4-Indolyl)-4-[1-[2-(3-pyridyl)-acetyl]-1,2,3,4-tetrahydrochinolin-2-ylmethyl]-piperazin,
1-[1-(1-Acetyl-4-piperidinylcarbonyl)-1,2,3,4-tetrahydrochinolin-2-ylmethyl]-4-(4-indolyl)-piperazin,
1-(4-Indolyl)-4-[1-[2-(2-thienylacetyl)]-1,2,3,4-tetrahydrochinolin-2-ylmethyl]-piperazin,
1-(4-Indolyl)-4-[1-[2-(3-thienylacetyl)]-1,2,3,4-tetrahydrochinolin-2-ylmethyl]-piperazin,
1-(4-Indolyl)-4-[1-[3-(3-pyridylpropionyl)]-1,2,3,4-tetrahydrochinolin-2-ylmethyl]-piperazin,
1-(4-Indolyl)-4-[1-(2-phenoxyacetyl)-1,2,3,4-tetrahydrochinolin-2-ylmethyl]-piperazin,
1-(4-Indolyl)-4-[1-[2-(4-methoxyphenyl acetyl)]-1,2,3,4-tetrahydrochinolin-2-ylmethyl]-piperazin,
1-(4-Indolyl)-4-[1-(2-phenylmethoxyacetyl)-1,2,3,4-tetrahydrochinolin-2-ylmethyl]-piperazin,
1-[1-[2-(2-Chlorphenylacetyl)]-1,2,3,4-tetrahydrochinolin-2-ylmethyl]-4-(4-indolyl)-piperazin,
1-{1-[2-(N-Benzoyl-N-methyl)-aminoacetyl]-1,2,3,4-tetrahydrochinolin-2-ylmethyl }-4-(4-indolyl)-piperazin,
1-(4-Indolyl)-4-[1-(1-methyl-3-indolylcarbonyl)-1,2,3,4-tetrahydrochinolin-2-ylmethyl]-piperazin,
1-[1-[2-(4-Dimethylaminophenylacetyl)]-1,2,3,4-tetrahydrochinolin-2-ylmethyl]-4-(4-indolyl)-piperazin,
1-[1-(5-Benzo[1,3]dioxolylacetyl)-1,2,3,4-tetrahydrochinolin-2-ylmethyl]-4-(4-indolyl)-piperazin,
1-[1-[2-(2-Chlorphenoxyacetyl)-1,2,3,4-tetrahydrochinolin-2-ylmethyl]-4-(4-indolyl)-piperazin,
1-[1-[2-(4-Chlorphenoxyacetyl)-1,2,3,4-tetrahydrochinolin-2-ylmethyl]-4-(4-indolyl)-piperazin,
1-(4-Indolyl)-4-[1-(2-morpholino-5-pyridylcarbonyl]-1,2,3,4-tetrahydrochinolin-2-ylmethyl]-piperazin,
1-(4-Indolyl)-4-[1-(5-methyl-4-isoxazolylcarbonyl)-1,2,3,4-tetrahydrochinolin-2-ylmethyl]-piperazin,
1-(4-Indolyl)-4-[1-(5-oxo-2-pyrrolidinylacetyl)-1,2,3,4-tetrahydrochinolin-2-ylmethyl]-piperazin,
1-(4-Indolyl)-4-[1-(2-methyl-5-piperidinosulphonyl-3-furoyl)-1,2,3,4-tetrahydrochinolin-2-ylmethyl]-piperazin,
1-(4-Indolyl)-4-[1-(2-methyl-5-morpholinosulphonyl-3-furoyl)-1,2,3,4-tetrahydrochinolin-2-ylmethyl]-piperazin,
1-(4-Indolyl)-4-{1-[1-(p-tolylsulphonyl)-3-pyrrolylcarbonyl]-1,2,3,4-tetrahydrochinolin-2-ylmethyl}-piperazin,
1-(4-Indolyl)-4-[1-(2-methyl-5-dimethylsulphamoyl-3-furoyl)-1,2,3,4-tetrahydrochinolin-2-ylmethyl]-piperazin,
1-(4-Indolyl)-4-{1-[2-methyl-5-(1-pyrrolidinylsulphamoyl)-3-furoyl)-1,2,3,4-tetrahydrochinolin-2-ylmethyl]-piperazin,
1- [(1-Adamantylcarbonyl)-1,2,3,4-tetrahydrochinolin-2-ylmethyl]-4-(4-indolyl)-piperazin,
1-(4-Indolyl)-4-[1-(3-phenoxypropionyl)-1,2,3,4-tetrahydrochinolin-2-ylmethyl]-piperazin,
1-(4-Indolyl)-1-[1-(4-phenoxybutanoyl)-1,2,3,4-tetrahydrochinolin-2-ylmethyl]- piperazin,
1-(4-Indolyl)-4-[1-(2-fluorphenylacetyl)-1,2,3,4-tetrahydrochinolin-2-ylmethyl] piperazin,
1-(4-Indolyl)-4-[1-(2-trifluormethyl-phenylacetyl)-1,2,3,4-tetrahydrochinolin-2-ylmethyl]-piperazin,
1- [1-(2-Bicyclo [2.2. 2]octylcarbonyl)-1,2,3,4-tetrahydrochinolin-2-ylmethyl]-4-(4-indolyl)-piperazin,
1-(4-Indolyl)-1-[1-(4-phenylbutanoyl)-1,2,3,4-tetrahydrochinolin-2-ylmethyl]-piperazin,
1-(4-Indolyl)-4-[1-(3-methoxybenzoyl)-1,2,3,4-tetrahydrochinolin-2-ylmethyl]-piperazin,
1-[1-(4-Hydroxybenzoyl)-1,2,3,4-tetrahydrochinolin-2-ylmethyl]-4-(4-indolyl)-piperazin,
1- [1-(4-Ethoxybenzoyl)-1,2,3,4-tetrahydrochinolin-2-ylmethyl]-4-(4-indolyl)-piperazin,
1-(4-Indolyl)-4-[1-(4-methylbenzoyl)-1,2,3,4-tetrahydrochinolin-2-ylmethyl]- piperazin,
1-[1-[(N-Benzyl-N-methansulphonyl)-2-aminoacetyl]-1,2,3,4-tetrahydrochinolin-2-ylmethyl]-4-(4-indolyl)-piperazin,
1-(4-Indolyl)-4-[1-(2-(S)-5-oxopyrrolidinylcarbonyl)-1,2,3,4-tetrahydrochinolin-2-ylmethyl]-piperazin,
1-(4-Indolyl)-4-[1-(2-(R)-5-oxopyrrolidinylcarbonyl)-1,2,3,4-tetrahydrochinolin-2-ylmethyl]-piperazin,
1-(4-Indolyl)-4-[1-(2-methylanilinocarbonyl)-1,2,3,4-tetrahydrochinolin-2-ylmethyl]-piperazin,
1-(4-Indolyl)-4-[1-(3-methylanilinocarbonyl)-1,2,3,4-tetrahydrochinolin-2-ylmethyl]-piperazin,
1-[1-(4-Fluoranilinocarbonyl)-1,2,3,4-tetrahydrochinolin-2-ylmethyl)-4-(4-indolyl)-piperazin,
1-(4-Indolyl)-4-[1-(4-methylanilinocarbonyl)-1,2,3,4-tetrahydrochinolin-2-ylmethyl]-piperazin,
1-(4-Indolyl)-4-[1-((1 S)-1-phenylethylaminocarbonyl)-1,2,3,4-tetrahydrochinolin-2-ylmethyl]-piperazin,
1-(4-Indolyl)-4-[1-((1R)-1-phenylethylaminocarbonyl)-1,2,3,4-tetrahydrochinolin-2-ylmethyl)-piperazin (Diastereoisomer A),
1-(4-Indolyl)-4-[1-((1 R)-1-phenylethylaminocarbonyl)-1,2,3,4-tetrahydrochinolin-2-ylmethyl] piperazin (1:1 Diastereoisomerenmischung),
1-(4-Indolyl)-4-[1-(1-methylethylaminocarbonyl)-1,2,3,4-tetrahydrochinolin-2-ylmethyl]-piperazin,
1-(4-Indolyl)-4-[1-methylaminothiocarbonyl-1,2,3,4-tetrahydrochinolin-2-ylmethyl)piperazin,
1-(1-Benzylaminocarbonyl-1,2,3,4-tetrahydrochinolin-2-ylmethyl)-4-(4-indolyl)-piperazin,
1-(4-Indolyl)-4-[1-(2-phenylethylaminocarbonyl)-1,2,3,4-tetrahydrochinolin-2-ylmethyl]-piperazin,
1-(4-Indolyl)-4-(1-pentylaminocarbonyl-1,2,3,4-tetrahydrochinolin-2-ylmethyl]-piperazin,
1-[1-(1,1-Dimethylethylaminocarbonyl-1,2,3,4-tetrahydrochinolin-2-ylmethyl]-4-(4-indolyl)-piperazin,
1-(4-Indolyl)-4-[(4-methoxyplienylaminocarbonyl)-1,2,3,4-tetrahydrochinolin-2-ylmethyl]-piperazin,
1-(4-Indolyl)-4-[(4-methoxyphenylmethylaminocarbonyl)-1,2,3,4-tetrahydrochinolin-2-ylmethyl]-piperazin,
1-(4-Indolyl)-4-[1-phenylsulphonyl-1,2,3,4-tetrahydrochinolin-2-ylmethyl]-piperazin,
1-(4-Indolyl)-4-[1-(2-thienylsulphonyl)-1,2,3,4-tetrahydrochinolin-2-ylmethyl]-piperazin,
1-(4-Indolyl)-4-[1-(4-methoxyphenylsulphonyl)-1,2,3,4-tetrahydrochinolin-2-ylmethyl]-piperazin,
1-(4-Indolyl)-4-[1-(4-methylphenylsulphonyl)-1,2,3,4-tetrahydrochinolin-2-ylmethyl]-piperazin,
1-[1-(4-Fluorphenylsulphonyl)-1,2,3,4-tetrahydrochinolin-2-ylmethyl]-4-(4-indolyl)-piperazin,
1-[1-(4-Cyanophenylsulphonyl)-1,2,3,4-tetrahydrochinolin-2-ylmethyl]-4-(4-indolyl)- piperazin,
1-(4-Indolyl)-4-[1-benzylsulphonyl-1,2,3,4-tetrahydrochinolin-2-ylmethyl]-piperazin,
1-(1-tert-Butoxyvarbonylaminoacetyl-1,2,3,4-tetrahydrochinolin-2-ylmethyl)-4-(4-indolyl)-piperazin,
1-[1-(3-tert-Butoxycarbonylamino-propionyl)-1,2,3,4-tetrahydrochinolin-2-ylmethyl]-4-(4-indolyl)-piperazin,
1-[1-(1-tert-Butoxycarbonyl-3-indolyl-acetyl)-1,2,3,4-tetrahydrochinolin-2-ylmethyl]-4-(4-indolyl]-piperazin,
1- [1-(1-tert-Butoxycarbonyl-4-piperidylcarbonyl)-1,2,3,4-tetrahydrochinolin-2-ylmethyl]-4-(4-indolyl)-piperazin,
1-[1-(1-tert-Butoxycarbonyl-2R-pyrrolidin-2-ylcarbonyl)-1,2,3,4-tetrahydrochinolin-2-ylmethyl]-4-(4-indolyl)-piperazin,
1-[1-(1-tert-Butoxycarbonyl-2S-pyrrolidin-2-ylcarbonyl)-1,2,3,4-tetrahydrochinolin-2-ylmethyl]-4-(4-indolyl)-piperazin,
1-(1-Cyclohexancarbonyl-1,2,3,4-tetrahydrochinolin-2-ylmethyl)-2-(a-hydroxybenzyl)-piperidin,
1-(1-Cyclohexancarbonyl-1,2,3,4-tetrahydrochinolin-2-ylmethyl)-4-(2,6-dimethylphenyl)-piperidin,
2-(4-Chlorphenyl)-1-(-cyclohexanearbonyl-1,2,3,4-tetrahydrochinolin-2-ylmethyl)-piperidin,
1-(1-Cyclohexancarbonyl-1,2,3,4-tetrahydrochinolin-2-ylmethyl)-4-phenylpiperidin,
4-Benzoyl-1-(1-Cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-4-(2-nitrophenyl)-piperazin,
1-(1-Cyclohexancarbonyl-1,2,3,4-tetrahydrochinolin-2-ylmethyl)-4-(3-trifluormethyl-2-pyridyl)-piperazin,
1-(1-Cyclohexancarbonyl-1,2,3,4-tetrahydrochinolin-2-ylmethyl)-4-(4-fluor-2-nitrophenyl)-piperazin,
1-(1-Cyclohexancarbonyl-1,2,3,4-tetrahydrochinolin-2-ylmethyl)-4-(2,3-dichlorphenyl)-piperazin,
1-(1-Cyclohexancarbonyl-1,2,3,4-tetrahydrochinolin-2-ylmethyl)-4-(3-cyano-2-pyridyl)-piperazin,
1-(1-Cyclohexancarbonyl-1,2,3,4-tetrahydrochinolin-2-ylmethyl)-4-(3,4-dichlorphenyl)-piperazin,
1-(1-Cyclohexancarbonyl-1,2,3,4-tetrahydrochinolin-2-ylmethyl)-4-(2-ethylphenyl)-piperazin,
1-(4-Chlor-3-trifluormethylphenyl)-4-(1-cyclohexancarbonyl-1,2,3,4-tetrahydrochinolin-2-ylmethyl)-piperazin,
1-(2-Cyanophenyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(2,4-dimethoxyphenyl)-piperazin,
1-(1-Cyclohexancarbonyl-1,2,3,4-tetrahydrochinolin-2-ylmethyl)-4-(2-methylthiophenyl)-piperazin,
1-(1-Cyclohexancarbonyl-1,2,3,4-tetrahydrochinolin-2-ylmethyl)-4-(3,4-dimethoxyphenyl)-piperazin,
1-(1-Cyclohexancarbonyl-1,2,3,4-tetrahydrochinolin-2-ylmethyl)-4-(3-hydroxyphenyl)-piperazin,
1-(1-Cyclohexancarbonyl-1,2,3,4-tetrahydrochinolin-2-ylmethyl)-4-(2,4-difluorphenyl)-piperazin,
1-(5-Chlor-2-methoxyphenyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(6-methyl-2-pyridyl)-piperazin,
1-(1-Cyclohexancarbonyl-1,2,3,4-tetrahydrochinolin-2-ylmethyl)-4-(4-methylphenyl)-piperazin,
1-(1-Cyclohexancarbonyl-1,2,3,4-tetrahydrochinolin-2-ylmethyl)-4-(4-trifluormethylphenyl)-piperazin,
1-(3-Chlorphenyl)-4-(1-cyclohexancarbonyl-1,2,3,4-tetrahydroqumolm-2-ylmethyl)-piperazin,
1-(2-Chlorphenyl)-4-(1-cyclohexancabonyl-1,2,3,4-tetrahydrochinolin-2-ylmethyl)-piperazin,
1-(1-Cyclohexancarbonyl-1,2,3,4-tetrahydrochinolin-2-ylmethyl)-4-(3-methoxyphenyl)-piperazin,
1-(1-Cyclohexancarbonyl-1,2,3,4-tetrahydrochinolin-2-ylmethyl)-4-(4-fluorphenyl)-piperazin,
1-(4-Acetylphenyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(4-hydroxyphenyl)-piperazin,
1-(5-Chlor-2-methylphenyl)-4-(1-cyclohexancarbonyl-1,2,3,4-tetrahydrochinolin-2-ylmethyl)-piperazin,
1-(1-Cyclohexanearbonyl-1,2,3,4-tetrahydrochinolin-2-ylmethyl)-4-(3-methylphenyl)-piperazin,
1-(1-Cyclohexancarbonyl-1,2,3,4-tetrahydrochinolin-2-ylmethyl)-4-(2-nitro-4-trifluormethylphenyl)-piperazin,
1-(1-Cyclohexancarbonyl-1,2,3,4-tetrahydrochinolin-2-ylmethyl)-4-(5-trifluormethyl-2-pyridyl)-piperazin,
1-(1-Cyclohexancarbonyl-1,2,3,4-tetrahydrochinolin-2-ylmethyl)-4-(3-chlor-5-trifluormethyl-2-pyridyl)-piperazin,
1-(5-Cyano-2-pyridyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(2-methyl-4-chinolinyl)-piperazin,
1-(1-Cyclohexancarbonyl-1,2,3,4-tetrahydrochinolin-2-ylmethyl)-4-benzyl-piperidin,
1-(Benzofuran-3-yl)-4-(1-Cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-4-(4-fluorphenyl)-piperidin,
1-(1-Cyclohexancarbonyl-1,2,3,4-tetrahydrochinolin-2-ylmethyl)-4-(3-fluorphenyl)-piperidin,
1-(1-Cyclohexancarbonyl-1,2,3,4-tetrahydrochinolin-2-ylmethyl)-4-(2-fluorphenyl)-piperidin,
4-(4-Chlorphenyl)-1-(1-cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-4-(4-trifluormethylphenyl)-piperidin,
1-(1-Cyclohexancarbonyl-1,2,3,4-tetrahydrochinolin-2-ylmethyl)-4-(3-trifluormethylphenyl) piperidin
1-(1-Cyclohexancarbonyl-1,2,3,4-tetrahydrochinolin-2-ylmethyl)-4-[5-(2-furyl]-2H-3-pyrazolyl]-piperidin,
1-(1-Cyclohexancarbonyl-1,2,3,4-tetrahydrochinolin-2-ylmethyl)-4-(4-methoxyphenyl)-piperidin,
1-(1-Cyclohexancarbonyl-1,2,3,4-tetrahydrochinolin-2-ylmethyl)-4-(3-methoxyphenyl)-piperidin,
1-(1-Cyclohexancarbonyl-1,2,3,4-tetrahydrochinolin-2-ylmethyl)-4-(4-fluorbenzoyl)-piperidin,
1-(1-Cyclohexancarbonyl-1,2,3,4-tetrahydrochinolin-2-ylmethyl)-4-benzylureido-piperidin,
1-(1-Cyclohexancarbonyl-1,2,3,4-tetrahydrochinolin-2-ylmethyl)-2-(4-methoxybenzyl)-piperidin,
1-(1-Cyclohexancarbonyl-1,2,3,4-tetrahydrochinolin-2-ylmethyl)-2-(4-fluorbenzyl)-piperidin,
1-(1-Cyclohexancarbonyl-1,2,3,4-tetrahydrochinolin-2-ylmethyl)-4-(2,3-dichlorphenyl)-piperidin,
1-(1-Cyclohexancarbonyl-1,2,3,4-tetrahydrochinolin-2-ylmethyl)-4-(2,6-difluorphenyl)-piperidin,
4-(2-Chlor-6-fluorphenyl)-1-(1-cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-4-(2,5-dimethylphenyl)-piperidin,
1-(1-Cyclohexancarbonyl-1,2,3,4-tetrahydrochinolin-2-ylmethyl)-4-(3-methylphenyl)-piperidin,
1-(1-Cyclohexancarbonyl-1,2,3,4-tetrahydrochinolin-2-ylmethyl)-4-(2-methylphenyl)-piperidin,
1-(1-Cyclohexancarbonyl-1,2,3,4-tetrahydrochinolin-2-ylmethyl)-4-(3-fluor-2-methylphenyl)-piperidin,
1-(1-Cyclohexancarbonyl-1,2,3,4-tetrahydrochinolin-2-ylmethyl)-4-(3,5-difluorphenyl)-piperidin,
1-(1-Cyclohexancarbonyl-1,2,3,4-tetrahydrochinolin-2-ylmethyl)-4-(2,5-difluorphenyl)-piperidin,
1-(1-Cyclohexancarbonyl-1,2,3,4-tetrahydrochinolin-2-ylmethyl)-4-(3,5-dimethylphenyl)-piperidin,
4-(3-Bromphenyl)-1-(1-cyclohexancarbonyl-1,2,3,4-tetrahydrochinolin-2-ylmethyl)-piperidin,
4-(2-Bromphenyl)-1-(1-cyclohexancarbonyl-1,2,3,4-tetrahydrochinolin-2-ylmethyl)-piperidin,
4-(4-Butoxyphenyl)-1-(1-cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-4-(2-fluorphenyl)-piperazin,
1-(1-Cyclohexancarbonyl-1,2,3,4-tetrahydrochinolin-2-ylmethyl)-4-(6-chlor-2-pyridyl)-piperazin,
1-(1-Cyclohexancarbonyl-1,2,3,4-tetrahydrochinolin-2-ylmethyl)-4-(3,5-dichlor-4-pyridyl)-piperazin,
1-(4-Aminophenyl)-4-(1-cyclohexancarbonyl-1,2,3,4-tetrahydrochinolin-2-ylmethyl)-piperazin,
4-(3-Chlorphenyl)-1-(1-cyclohexancarbonyl-1,2,3,4-tetrahydrochinolin-2-ylmethyl)-piperidin,
4-(2-Chlorphenyl)-1-(1-cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-2-(2-pyridyl)- pyrrolidin,
1-(1-Cyclohexancarbonyl-1,2,3,4-tetrahydrochinolin-2-ylmethyl)-2-(2-thienyl)- pyrrolidin,
1-(1-Cyclohexancarbonyl-1,2,3,4-tetrahydrochinolin-2-ylmethyl)-2-phenylpyrrolidin,
1-(1-Cyclohexancarbonyl-1,2,3,4-tetrahydrochinolin-2-ylmethyl)-2-(2,4-dimethoxyphenyl)-pyrrolidin,
1-(1-Cyclohexancarbonyl-1,2,3,4-tetrahydrochinolin-2-ylmethyl)-2-(3-pyridyl)- pyrrolidin,
1-(1-Cyclohexancarbonyl-1,2,3,4-tetrahydrochinolin-2-ylmethyl)-2-benzylpyrrolidin,
1-(1-Cyclohexancarbonyl-1,2,3,4-tetrahydrochinolin-2-ylmethyl)-4-(3,5-dimethoxyphenyl)-piperazin,
1-(1-Cyclohexancarbonyl-1,2,3,4-tetrahydrochinolin-2-ylmethyl)-2-(2-furyl)-pyrrolidin,
1-(1-Cyclohexancarbonyl-1,2,3,4-tetrahydrochinolin-2-ylmethyl)-2-(4-methoxybenzyl)-pyrrolidin,
1-(1-Cyclohexancarbonyl-1,2,3,4-tetrahydrochinolin-2-ylmethyl)-2-(4-chlorphenyl)-pyrrolidin,
1-(1-Cyclohexancarbonyl-1,2,3,4-tetrahydrochinolin-2-ylmethyl)-2-(2-indolyl)-pyrrolidin,
1-(1-Cyclohexancarbonyl-1,2,3,4-tetrahydrochinolin-2-ylmethyl)-4-(2-trifluormethylphenyl)-piperidin,
1-Cyclohexancarbonyl-2-{N-[2-(2-methoxyphenoxy)-ethyl]-N-methylaminomethyl}-1,2,3,4-tetrahydrochinolin,
1-Cyclohexancarbonyl-2-[N-(2,3-dihydro-benzo[1,4] dioxin-2-ylmethyl)-N-methyl-aminomethyl]-1,2,3,4-tetrahydrochinolin,
1-Cyclohexancarbonyl-2-{N-[2-(2-chlorphenoxy)-ethyl]-N-methylaminomethyl}-1,2,3,4-tetrahydrochinolin,
1-Cyclohexancarbonyl-2-{N-[2-(2-trifluormethoxyphenoxy)-ethyl]-N-methylaminomethyl}-1,2,3,4-tetrahydrochinolin,
1-Cyclohexancarbonyl-2-{N-[2-(4-indolyloxy)-ethyl]-N-methyl-aminomethyl}-1,2,3,4-tetrahydrochinolin,
1-Cyclohexancarbonyl-2-{N-[2-(8-chinolyloxy)-ethyl]-N-methyl-aminomethyl}-1,2,3,4-tetrahydrochinolin,
1-(4-Chlor-2-nitrophenyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(2-trifluormethyl-4-chinolyl)-piperazin,
4-(1-Benzimidazolyl)-1-(1-cyclohexancarbonyl-1,2,3,4-tetrahydrochinolin-2-ylmethyl)-piperidin,
1-(1-Cyclohexancarbonyl-1,2,3,4-tetrahydrochinolin-2-ylmethyl)-2-(4-methoxyphenyl)-piperidin,
1-(1-Cyclohexancarbonyl-1,2,3,4-tetrahydrochinolin-2-ylmethyl)-4-(2,6-dichlorphenyl)-piperidin,
1-(1-Cyclohexancarbonyl-1,2,3,4-tetrahydrochinolin-2-ylmethyl)-4-(2-piridyl)-piperazin,
1-(1-Cyclohexancarbonyl-1,2,3,4-tetrahydrochinolin-2-ylmethyl)-4-(4-chinolyl)-piperazin,
1-(1-Cyclohexancarbonyl-1,2,3,4-tetrahydrochinolin-2-ylmethyl)-4-(7-methoxy-4-chinolyl)-piperazin,
1-(4-Fluor-2-methoxyphenyl)-4-[1-(2-methylanilinocarbonyl)-1,2,3,4-tetrahydrochinolin-6-methyl-2-ylmethyl]-piperazin,
1-(4-Fluor-2-methoxyphenyl)-4-[1-(4-methylanilinocarbonyl)-1,2,3,4-tetrahydrochinolin-6-methyl-2-ylmethyl]-piperazin,
1-[1-(t-Butylcarbamoyl)-1,2,3,4-tetrahydrochinolin-6-methyl-2-ylmethyl]-4-(4-fluor-2-methoxyphenyl)-piperazin,
1-(4-Fluor-2-methoxyphenyl)-4-(1-pentylaminocarbonyl-1,2,3,4-tetrahydrochinolin-6-methyl-2-ylmethyl)-piperazin,
1-(4-Fluor-2-methoxyphenyl)-4-[(2-thienylacetyl)-1,2,3,4-tetrahydrochinolin-6-methyl-2-ylmethyl]-piperazin,
1-(4-Fluor-2-methoxyphenyl)-4-[(3-thienylacetyl)-1,2,3,4-tetrahydrochinolin-6-methyl-2-ylmethyl]-piperazin,
1-(1-Cyclohexylacetyl-1,2,3,4-tetrahydrochinolin-6-methyl-2-ylmethyl)-4-(4-fluor-2-methoxyphenyl)-piperazin,
1-[1-(4-Cyanobenzoyl)-1,2,3,4-tekahydrochinolin-6-methyl-2-ylmethyl]-4-(4-fluor-2-methoxyphenyl)-piperazin,
1-(4-Fluor-2-methoxyphenyl)-4-[1-(4-methoxybenzoyl)-1,2,3,4-tetrahydrochinolin-6-methyl-2-ylmethyl)-piperazin,
1-[1-(4-Chlorbenzoyl)-1,2,3,4-tekahydrochinolin-6-methyl-2-ylmethyl]-4-(4-fluor-2-methoxyphenyl)-piperazin,
1-[1-(Benzo [1,3]dioxol-5-ylcarbonyl)-1,2,3,4-tetrahydrochinolin-6-methyl-2-ylmethyl]-4-(4- fluor-2-methoxyphenyl)-piperazin,
1-[1-(2-Chlorphenylacetyl)-1,2,3,4-tetrahydrochinolin-6-methyl-2-ylmethyl]-4-(4-fluor-2-methoxyphenyl)-piperazin,
1-(4-Fluor-2-methoxyphenyl)-4-[1-(1-methyl-3-indolylcarbonyl)-1,2,3,4-tetrahydrochinolin-6-methyl-2-ylmethyl]-piperazin,
1-(1-Cycloheptancarbonyl-1,2,3,4-tetrahydrochinolin-6-methyl-2-ylmethyl)-4-(4-fluor-2-methoxyphenyl)-piperazin,
1-(4-Fluor-2-methoxyphenyl)-4-(1-phenylsulphonyl-1,2,3,4-tetrahydrochinolin-6-methyl-2-ylmethyl)-piperazin,
1-(4-Fluor-2-methoxyphenyl)-4-(1-benzylsulphonyl-1,2,3,4-tetrahydrochinolin-6-methyl-2-ylmethyl)-piperazin,
1-(4-Fluor-2-methoxyphenyl)-4-[1-(4-methoxyphenylsulphonyl)-1,2,3,4-tetrahydrochinolin-6-methyl-2-ylmethyl]-piperazin,
1-(4-Fluor-2-methoxyphenyl)-4-[1-(4-methylphenylsulphonyl)-1,2,3,4-tetrahydrochinolin-6-methyl-2-ylmethyl)-piperazin,
1-[1-(4-Cyanophenylsulphonyl)-1,2,3,4-tetrahydrochinolin-6-methyl-2-ylmethyl]-4-(4-fluor-2-methoxyphenyl)-piperazin,
1-[1-(4-Fluorphenylsulphonyl)-1,2,3,4-tetrahydrochinolin-6-methyl-2-ylmethyl]-4-(4-fluor-2-methoxyphenyl)-piperazin,
1-(4-Fluor-2-methoxyphenyl)-4-[1-(3-methylbutanoyl)-1,2,3,4-tetrahydrochinolin-2-ylmethyl]-piperazin,
1-(3,3-Dimethylbutanoyl-1,2,3,4-tetrahydrochinolin-2-ylmethyl)-4-(4-fluor-2-methoxyphenyl)-piperazin,
1-(1-Cyclopropylacetyl-1,2,3,4-tetrahydrochinolin-2-ylmethyl)-4-(4-fluor-2-methoxyphenyl)-piperazin,
1-(4-Fluor-2-methoxyphenyl)-4-[1-(2-pyrrolylcarbonyl)-1,2,3,4-tetrahydrochinolin-2-ylmethyl]-piperazin,
1-(4-Fluor-2-methoxyphenyl)-4-[1-(2-furoyl)-1,2,3,4-tetrahydrochinolin-2-ylmethyl]-piperazin,
1-(4-Fluor-2-methoxyphenyl)-4-[1-(4-methoxybenzoyl)-1,2,3,4-tetrahydrochinolin-2-ylmethyl]-piperazin,
1-[1-(4-Cyanobnezoyl)-1,2,3,4-tetrahydrochinolin-2-ylmethyl]-4-(4-fluor-2-methoxyphenyl)-piperazin,
1-[1-(1-acetyl-4-piperidinylcarbonyl)-1,2,3,4-tetrahydrochinolin-2-ylmethyl]-4-(4-fluor-2-methoxyphenyl)-piperazin,
1-(4-Fluor-2-methoxyphenyl)-4-[1-(2-thienylacetyl)-1,2,3,4-tetrahydrochinolin-2-ylmethyl]-piperazin,
1-(4-Fluor-2-methoxyphenyl)-4-[1-(3-thienylacetyl)-1,2,3,4-tetrahydrochinolin-2-ylmethyl]-piperazin,
1-(1-Cyclobutylcarbonyl-1,2,3,4-tetrahydrochinolin-2-ylmethy)-4-(4-fluor-2-methoxyphenyl)-piperazin,
1-(4-Fluor-2-methoxyphenyl)-4-[1-(4-phenoxybutanoyl)-1,2,3,4-tetrahydrochinolin-2-ylmethyl]-piperazin,
1-(4-Fluor-2-methoxyphenyl)-4-(1-phenylsulphonyl-1,2,3,4-tetrahydrochinolin-2-ylmethyl)-piperazin,
1-(1-Benzylsulphonyl-1,2,3,4-tetrahydrochinolin-2-ylmethyl)-4-(4-fluor-2-methoxyphenyl)-piperazin,
1-(4-Fluor-2-methoxyphetyl)-4-[1-(2-methylanilinocarbonyl)-1,2,3,4-tetrahydrochinolin-2-ylmethyl]-piperazin,
1-[1-(t-Butylcarbamoyl)-1,2,3,4-tetrahydrochinolin-2-ylmethy]-4-(4-fluor-2-methoxyphenyl)-piperazin,
1-(1-Isochinolinyl)-4-[1-(3-methylbutanoyl)-1,2,3,4-tetrahydrochinolin-2-ylmethyl]-piperazin,
1-[1-(3,3-Dimethylbutanoyl)-1,2,3,4-tetrahydrochinolin-2-ylmethyl]-4-(1-isochinolinyl)-piperazin,
1-[1-(2-Cyclopropylacetyl)-1,2,3,4-tetrahydrochinolin-2-ylmethyl]-4-(1-isochinolinyl)-piperazin,
1-(1-Isochinolinyl)-4-[1-(2-pyrrolylcarbonyl)-1,2,3,4-tetrahydrochinolin-2-ylmethyl]-piperazin,
1-[1-(2-Furoyl)-1,2,3,4-tetrahydrochinolin-2-ylmethyl]-4-(1-isochinolinyl)-piperazin,
1-(1-Isochinolinyl)-4-[1-(4-methoxybenzoyl)-1,2,3,4-tetrahydrochinolin-2-ylmethyl]-piperazin,
1-[1-(4-Cyanobenzoyl)-1,2,3,4-tetrahydrochinolin-2-ylmethyl]-4-(1-isochinolinyl)-piperazin,
1-[1-(1-Acetyl-4-piperidylcarbonyl)-1,2,3,4-tetrahydrochinolin-2-ylmethyl]-4-(1-isochinolinyl)-piperazin,
1-(1-Isochinolinyl)-4-[1-(2-thienylacetyl)-1,2,3,4-tetrahydrochinolin-2-ylmethyl]-piperazin,
1-(1-Isochinolinyl)-4-[1-(3-thienylacetyl)-1,2,3,4-tetrahydrochinolin-2-ylmethyl]- piperazin,
1-(1-Cyclobutancarbonyl-1,2,3,4-tetrahydrochinolin-2-ylmethyl)-4-(1-isochinolinyl)-piperazin,
1-(1-Isochinolinyl)-4-[1-(4-phenoxybutanoyl)-1,2,3,4-tetrahydrochinolin-2-ylmethyl]-piperazin,
1-(1-Isochinolinyl)-4-(1-phenylsulphonyl-1,2,3,4-tetrahydrochinolin-2-ylmethyl)-piperazin,
1-(1-Benzylsulphonyl-1,2,3,4-tetrahydrochinolin-2-ylmethyl)-4-(1-Isochinolinyl)-piperazin,
1-(1-Isochinolinyl)-4-[1-(2-methylanilinocarbonyl)-1,2,3,4-tetrahydrochinolin-2-ylmethyl]-piperazin,
1-[1-(t-Butylcarbamoyl)-1,2,3,4-tetrahydrochinolin-2-ylmethyl]-4-(1-Isochinolinyl)-piperazin,
1-(2,3-Dihydrobenzo [1,4] dioxin-5-yl)-4-[1-(3-methylbutanoyl)-1,2,3,4-tetrahydrochinolin-2-ylmethyl]-piperazin,
4-[1-(2-Cyclopropylacetyl)-1,2,3,4-tetrahydrochinolin-2-ylmethyl]-1-(2,3-dihydrobenzo [1,4] dioxin-5-yl)-piperazin,
1-(2,3-Dihydrobenzo[1,4] dioxin-5-yl)-4-[1-(2-pyrrolylcarbonyl)-1,2,3,4-tetrahydrochinolin-2-ylmethyl]-piperazin,
1-(2,3-Dihydrobenzo[1,4] dioxin-5-yl)-4-[1-(2-furoyl)-1,2,3,4-tetrahydrochinolin-2-ylmethyl]-piperazin,
1-(2,3-Dihydrobenzo[1,4]dioxin-5-yl)-4-[1-(4-methoxybenzoyl)-1,2,3,4-tetrahydrochinolin- 2-ylmethyl]-piperazin,
4-[1-(4-Cyanobenzoyl)-1,2,3,4-tetrahydrochinolin-2-ylmethyl]-1-(2,3-dihydrobenzo [1,4] dioxin-5-yl)-piperazin,
1-(2,3-Dihydrobenzo[1,4]dioxan-5-yl)-4-[1-[2-thienylacetyl)-1,2,3,4-tetrahydrochinolin-2-ylmethyl]-piperazin,
1-(2,3-Dihydrobenzo[1,4] dioxan-5-yl)-4-[1-[3-thienylacetyl)-1,2,3,4-tetrahydrochinolin-2-ylmethyl]-piperazin,
4-(1-Cyclobutancarbonyl-1,2,3,4-tetrahydrochinolin-2-ylmethyl)-1-(2,3-dihydrobenzo [1,4] dioxin-5-yl)-piperazin,
1-(2,3-Dihydrobenzo[1,4]dioxin-5-yl)-4-[1-(4-phenoxybutanoyl)-1,2,3,4-tetrahydrochinolin-2-ylmethyl]-piperazin,
1-(2,3-Dihydrobenzo[1,4]dioxin-5-yl)-4-(1-phenylsulphonyl-1,2,3,4-tetrahydrochinolin-2-ylmethyl]-piperazin,
1-(1-Benzylsulphonyl-1,2,3,4-tetrahydrochinolin-2-ylmethyl)-4-(2,3-dihydrobenzo [1,4] dioxin-5-yl)-piperazin,
1-(2,3-Dihydrobenzo [1,4] dioxin-5-yl)-4-[1-(2-methylanilincarbonyl)-1,2,3,4-tetrahydrochinolin-2-ylmethyl]-piperazin,
1-[1-(t-Butylcarbamoyl)-1,2,3,4- tetrahydrochinolin-2-ylmethyl]-4-(2,3-dihydrobenzo [1,4] dioxin-5-yl)-piperazin,
1-(2,3-Dihydrobenzo [1,4] dioxin-5-yl)-4-[1-(3,3-dimethylbutanoyl)-1,2,3,4-tetrahydrochinolin-2-ylmethyl]-piperazin,
4-[1(1-Acetyl-4-piperidylcarbonyl)-1,2,3,4-tetrahydrochinolin-2-ylmethyl]-1- (2,3-dihydrobenzo [1,4] dioxin-5-yl)-piperazin,
1-(2,3-Dihydrobenzo [1,4] dioxin-5-yl)-4-[1-(2,2-dimethylbutanoyl)-1,2,3,4-tetrahydrochinolin-2-ylmethyl]-piperazin,
1-(2,3-Dihydrobenzo[1,4] dioxin-5-yl)-4-[1-(1-trifluormethylcyclopropancarbonyl) -1,2,3,4-tetrahydrochinolin-2-ylmethyl]-piperazin,
1-[1(Bicyclo[2.2.2]oct-2-ylcarbonyl)-1,2,3,4-tetrahydrochinolin-2-ylmethyl]-4-(2,3-dihydrobenzo [1,4] dioxin-5-yl)-piperazin,
1-(1-Cyclopentancarbonyl)-1,2,3,4-tetrahydrochinolin-2-ylmethyl]-4-(2,3-dihydrobenzo [1,4] dioxin-5-yl)-piperazin,
1-(2,3-Dihydrobenzo [1,4] dioxin-5-yl)-4-[1-(4-fluorbenzoyl)-1,2,3,4-tetrahydrochinolin-2-ylmethyl]-piperazin,
1-[1(Benzo[1,3]dioxol-5-ylcarbonyl)-1,2,3,4-tetrahydrochinolin-2-ylmethyl]-4-(2,3-dihydrobenzo [1,4] dioxin-5-yl)-piperazin,
1-(2,3-dihydrobenzo [1,4] dioxin-5-yl)-4-[1-(3-phenylpropenoyl)-1,2,3,4-tetrahydrochinolin-2-ylmethyl]-piperazin,
1-(2,3-Dihydrobenzo[1,4]dioxin-5-yl)-4-[1-[3-(4-fluorphenyl)-propenoyl]-1,2,3,4-tetrahydrochinolin-2-ylmethyl]-piperazin,
1-(2,3-Dihydrobenzo [1,4] dioxin-5-yl)-4-[1-(3-methyl-2-thienylcarbonyl)-1,2,3,4-tetrahydrochinolin-2-ylmetliyl]-piperazin,
1-(2,3-Dihydrobenzo[1,4] dioxin-5-yl)-4-[1-(3-furoyl)-1,2,3,4-tetrahydrochinolin-2-ylmethyl]-piperazin,
1-(2,3-Dihydrobenzo [1,4] dioxin-5-yl)-4-[1-(2-methyl-3-furoyl)-1,2,3,4-tetrahydrochinolin-2-ylmethyl]-piperazin,
1-(2,3-Dihydrobenzo[1,4]dioxin-5-yl)-4-[1-[5-methyl-4-(1,2,4-triazol-1-ylmethyl)-2-furoyl)-1,2,3,4-tetrahydrochinolin-2-ylmethyl]-piperazin,
1-(2,3-Dihydrobenzo[1,4]dioxin-5-yl)-4-[1-(5-methyl-4-isoxazolcarbonyl)-1,2,3,4-tetrahydrochinolin-2-ylmethyl]-piperazin,
1-[1-(4-Acetyl-2-pyrrolcarbonyl)-1,2,3,4-tetrahydrochinolin-2-lymethyl]-4(2,3-dihydrobenzo[1,4]dioxin-5-yl)-piperazin
2-{N-[2-(2-Methoxyphenoxy)-ethyl]-N-methyl-aminomethyl}-1-(3-methylbutanoyl)-1,2,3,4-tetrahydrochinolin,
1-(3,3-Dimethylbutanoyl)-2-{N-[2-(2methoxyphenoxy)-ethyl]-N-methylaminomethyl}-1,2,3,4-tetrahydrochinolin,
1-Cyclopropylacetyl-2-{N-[2-(2methoxyphenoxy)-ethyl]-N-methyl-aminomethyl}-1,2,3,4-tetrahydrochinolin,
2-{N-[2-(2-Methoxyphenoxy)-ethyl]-N-methyl-aminomethyl}-1-(2-pyrrolylcarbonyl)-1,2,3,4-tetrahydrochinolin,
1-(2-Furoyl)-2-{N-[2-(2-methoxyphenoxy)-ethyl]-N-methyl-aminomethyl}-1,2,3,4-tetrahydrochinolin,
1-(4-Methoxybezoyl)-2-{N-[2-(2-methoxyphenoxy)-ethyl]-N-methylaminomethyl}-1,2,3,4-tetrahydrochinolin,
1-(1-Acetyl-4-piperidylcarbonyl)-2-{N-[2-(2-methoxyphenoxy)-ethyl]-N-methylaminomethyl}-1,2,3,4-tetrahydrochinolin,
2-{N-[2-(2-Methoxyphenoxy)-ethyl]-N-methyl-aminomethyl}-1-(2-thienylacetyl)-1,2,3,4-tetrahydrochinolin,
2-{N-[2-(2-Methoxyphenoxy)-ethyl]-N-methyl-aminomethyl}-1-(3-thienylacetyl)-1,2,3,4-tetrahydrochinolin,
1-Cyclobutancarbonyl-2-{N-[2-(2-methoxyphenoxy)-ethyl]-N-methylaminomethyl}-1,2,3,4-tetrahydrochinolin,
2-{N-[2-(2-Methoxyphenoxy)-ethyl]-N-methyl-aminomethyl}-1-(4-phenoxybutanoyl)- 1,2,3,4-tetrahydrochinolin,
2-{N-[2-(2-Methoxyphenoxy)-ethyl]-N-methyl-aminomethyl}-1-phenylsulphonyl-1,2,3,4- tetraliydrochinolin,
1-Benzylsulphonyl-2-{N-[2-(2-methoxyphenoxy)-ethyl] N-methyl-aminomethyl}-1,2,3,4- tetrahydrochinolin,
2- {N-[2-(2-Methoxyphenoxy)-ethyl]-N-methyl-aminomethyl}-1-(2-methylanilinocarbonyl)- 1,2,3,4-tetrahydrochinolin,
1-(t-Butylcarbamoyl)-2- {N-[2-(2-methoxyphenoxy)-ethyl]-N-methylaminomethyl}-1,2,3,4- tetrahydrochinolin,
1-(1-Cyclohexancarbonyl-5-fluor-1,2,3,4-tetrahydrochinolin-2-ylmethyl)-4-(4-fluor-2- methoxyphenyl)-piperazin,
1-(1-Cyclohexancarbonyl-1,2,3,4-tetrahydrochinolin-2-ylmethyl)-4-(2-triluoromethansulphonyloxyphenyl)-piperazin,
1-(1-Cyclohexancarbonyl-1,2,3,4-tetrahydrochinolin-2-ylmethyl)-4-(1-isopropyl-4-indolyl)-piperazin,
1-(6-tert-Butoxycarbonylamino-1-cyclohexancarbonyl-1,2,3,4-tetrahydrochinolin-2-ylmethyl)-4-(4-fluor-2-methoxyphenyl)-piperazin,
1-(1-Cyclohexancarbonyl-1,2,3,4-tetrahydrochinolin-2-ylmethyl)-4-(1-methoxymethyl-4-indolyl)-piperazin,
1-(1-cyclohexancarbonyl-2-methyl-1,2,3,4-tetrahydrochinolin-2-ylmethyl)-4-(4-fluor-2-methoxyphenyl)-piperazin,
1-(1-Cyclopentylcarbonyl-6-methyl-1,2,3,4-tetrahydrochinolin-2-ylmethyl]-4-(4-fluor-2-methoxyphenyl)-piperazin,
1-(6-Amino-1-cyclohexancarbonyl-1,2,3,4-tetrahydrochinolin-2-ylmethyl)-4-(4-fluor-2-methoxyphenyl)-piperazin,
1-(1-Cyclohexancarbonyl-1,2,3,4-tetrahydrochinolin-2-ylmethyl)-4-(2-methoxy-4-methylphenyl)-piperazin,
1-(4-Benzo [1,3] dioxol-5-ylmethyl)-4-(1-cyclohexancarbonyl-1,2,3,4-tetrahydrochinolin- 2-ylmethyl)-piperazin,
1-Cyclohexancarbonyl-2- {N- [3-(2-methoxyphenoxy)-propyl]-aminomethyl}-1, 2,3,4- tetrahydrochinolin,
1-Cyclohexancarbonyl-2- {N-[2-(2-methoxyphenoxy)-ethyl]-N-methylaminomethyl}- 1,2,3,4-tetrahydrochinolin,
4-Cyano-1-(1-cyclohexancarbonyl-1,2,3,4-tetrahydrochinolin-2-ylmethyl)-4-phenyl- piperidin,
4-Benzyl-1-(1-cyclohexancarbonyl-1,2,3,4-tetrahydrochinolin-2-ylmetliyl)-4-hydroxy- piperidin,
4-Benzyloxy-1-(1-cyclohexancarbonyl-1,2,3,4-tetrahydrochinolin-2-ylmethyl)-piperidin,
4-Benzoyl amino-1-(1-cyclohexancarbonyl-1,2,3,4-tetrahydrochinolin-2-ylmethyl)-piperidin,
4-Acetyl-1-(1-cyclohexancarbonyl-1,2,3,4-tetrahydrochinolin-2-ylmethyl)-4-phenyl- piperidin,
1-(Benzo[1,3]dioxol-4-yl)-4-(1-cyclohexancarbonyl-1,2,3,4-tetrahydrochinolin-2-ylmethyl)-piperazin,
2-{N-[2-(3-Chlorphenoxy)-ethyl]-N-methyl-aminomethyl}-1 cyclohexancarbonyl-1,2,3,4- tetrahydrochinolin,
2- {N- [2-(4-Chlorphenoxy)-ethyl]-N-methyl-aminomethyl}-1-cyclohexancarbonyl-1,2,3,4- tetrahydrochinolin,
2- {N-Benzyl-N-[2-(2-methoxyphenoxy)-ethyl]-aminomethyl}-1-cyclohexancarbonyl- 1,2,3,4-tetrahydrochinolin,
1-Cyclohexancarbonyl-2-[N-methyl-N-(3-phenylpropyl)-aminomethyl]-1,2,3,4-tetrahydrochinolin,
1-(4-Cyano-2-trifluormethoxyphenyl)-4-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-4-(4-fluor-2-methoxy-anilino)-piperidin,
1-(3H-1,2,3-Benzotriazol-4-yl)-4-(1-cyclohexancarbonyl-1,2,3,4-tetrahydrochinolin-2- ylmethyl)-piperazin,
1-(1H-1,3-Benzodiazol-4-yl)-4-(1-cyclohexancarbonyl-1,2,3,4-tetrahydrochinolin-2-ylmethyl)-piperazin,
1-Cyclohexancarbonyl-2-{N-[2-(2-pyridyloxy)-ethyl]-aminomethyl}-1,2,3,4-tetrahydrochinolin,
1-Cyclohexancarbonyl-2-{N-methyl-N-[2-(2-pyridyloxy)-ethyl]-aminomethyl}-1,2,3,4- tetrahydrochinolin,
1-Cyclohexancarbonyl-2-{N-methyl-N- [3-(2-methoxyphenyl)-propyl]-aminomethyl}- 1,2,3,4-tetrahydrochinolin,
4-Benzoyl-1-(1-cyclohexancarbonyl-1,2,3,4-tetrahydrochinolin-2-ylmethyl)-piperazin,
1-(1-Cyclohexancarbonyl-1,2,3,4-tetrahydrochinolin-2-ylmethyl)-3-oxo-4-phenylpiperazin,
1-(4-Fluor-2-methoxyphenyl)-4-(1-hexanoyl-1,2,3,4-tetrahydrochinolin-2-ylmethyl)- piperazin,
1-(2,3-Dihydrobenzo[1,4]dioxin-5-yl)-4- [1-pentafluorpropionyl-1,2,3,4-tetrahydrochinolin-2-ylmethyl]-piperazin,
1-(2,3-Dihydrobenzo [1,4] dioxin-5-yl)-4-[1-(3,3,3-trifluorpropionyl)-1,2,3,4-tetrahydrochinolin-2-ylmethyl]-piperazin,
(Z)-1-(2,3-Dihydrobenzo[1,4]dioxin-5-yl)-4-[1-(4-hydroxy-cyclohexancarbonyl)-1,2,3,4-tetrahydrochinolin-2-ylmethyl]-piperazin, und
1-(-Cyclohexancarbonyl-6-fluor-1,2,3,4-tetrahydrochinolin-2-ylmethyl)-4-(4-fluor-2- methoxyphenyl)-piperazin.

15. Pharmazeutische Zusammensetzung, aufweisend eine Verbindung nach einem der Ansprüche 1 bis 14 oder ein Enantiomer, Diastereomer, N-Oxid, eine kristalline Form, ein Hydrat, Solvat oder pharmazeutisch zulässiges Salz einer solchen Verbindung, in Beimischung mit einem pharmazeutisch zulässigen Verdünnungsmittel oder Träger.

## Revendications

1. Un composé ayant la formule générale I 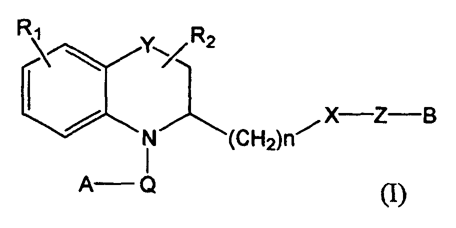 où
R₁ représente un ou plusieurs substituants choisis parmi les atomes d'hydrogène et d'halogène et les groupes hydroxy, alkyle, haloalkyle, alcoxy, haloalcoxy, nitro, phényle, phényle substitué, hétérocycle, hétérocycle substitué et NR₃R₄ où chacun de R₃ et R₄ représente indépendamment l'un de l'autre un atome d'hydrogène ou un groupe alkyle, acyle or alkoxycarbonyle;
R₂ représente un ou deux substituants choisis parmi l'atome d'hydrogène et un groupe alkyle;
Y représente un groupe CH₂ ou une liaison;
Q représente un groupe carbonyle, thiocarbonyle ou sulfonyle;
A représente un groupe alkyle, alcényle, cycloalkyle, cycloalcényle, aryle, hétérocyclique, alkylamino, dialkylamino, arylamino ou aralkylamino, chacun desquels est facultativement substitué;
n est 1 ou 2;
X représente un fragment amino choisi parmi 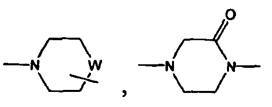 et -N(R₅)-CH₂- où R₅ représente un atome d'hydrogène ou un groupe méthyle or benzyle et W représente une liaision, un atome azote ou un groupe CH, CH₂, C(CN) C(OH) ou C(COCH₃) (quand W représente un atome d'azote ou un groupe CH, C(CN) C(OH) ou C(COCH₃), le groupe Z-B s'attache à l'atome cyclique W);
quand X représente un groupe 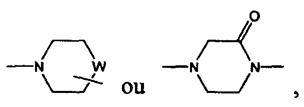 Z représente une liaison, un atome d'oxygène ou de soufre ou un groupe CH₂, CH₂CH₂, CO, CHOH, OCH₂, NH, NHCO ou NHCONHCH₂,
quand X représente un groupe -N(R₅)-CH₂-, Z représente un groupe CH₂CH₂ ou CH₂O ou Z et B représentent ensemble un groupe 2,3-dihydro-benzo[1,4]dioxin-2-yl; et
à l'exception de ce qui est indiqué ci-dessus, B représente un groupe aryle monocyclique ou bicyclique ou un hétérocycle monocyclique ou bicyclique, chacun desquels étant facultativement substitué ;
ou un énantiomère, diastéréomères, N-oxyde, forme cristalline, hydrate, solvate ou un sel pharmaceutiquement acceptable de ce composé.

2. Une composé selon la revendication 1 dans lequel R₁ représente un atome d'hydrogène.

3. Un composé selon la revendication 1 ou la revendication 2 dans lequel R₂ représente un atome d'hydrogène.

4. Un composé selon l'une quelconque des revendications 1 à 3 dans lequel Y représente un groupe CH₂.

5. Un composé selon l'une quelconque des revendications 1 à 4 dans lequel Q représente un groupe carbonyle.

6. Un composé selon l'une quelconque des revendications 1 à 5 dans lequel n est 1.

7. Un composé selon l'une quelconque des revendications 1 à 6 où X représente un groupe 1,4-piperazine-di-yl.

8. Un composé selon la revendication 7 dans lequel Z représente une liaison.

9. Un composé selon l'une quelconque des revendications 1 à 8 dans lequel A représente un groupe cyclohexyle.

10. Un composé selon l'une quelconque des revendications 1 à 9 dans lequel B représente un groupe monocyclique aryle ou hétérocycle bicyclique, chacun desquels pouvant facultativement être substitué.

11. Un composé selon la revendication 10 dans lequel B représente un groupe 4-indolyle ou 2,3-dihydrobenzo[1,4]dioxine-5-yl.

12. Un composé selon la revendication 10 dans lequel B représente un groupe phényle substitué avec un ou plusieurs substituants choisis parmi les atomes de fluor ou de chlore et les groupes méthoxy, trifluorométhoxy et 2,2,2-trifluoroéthoxy.

13. Un composé selon la revendication 12 dans lequel B représente un groupe 2-méthoxyphényle ou 4-fluoro-2-méthoxyphényle.

14. Un composé selon la revendication 1, ce composé étant l'un des composés suivants :
1-(1-cyclohexanecarbonyle-1,2,3,4-tétrahydroquinoline-2-ylméthyle)-4-(4-indolyle)-pipérazine,
(+)-1-(1-cyclohexanecarbonyle-1,2,3,4-tétrahydroquinoline-2-ylméthyle)-4-(4-indolyle)-pipérazine,
(-)-1-(1-cyclohexanecarbonyle-1,2,3,4-tétrahydroquinoline-2-ylméthyle)-4-(4-indolyle)-pipérazine,
1-(1-cyclohexanecarbonyle-1,2,3,4-tétrahydroquinoline-2-ylméthyle)-4-(2-méthoxyphényle)-pipérazine,
1-[2-(1-cyclohexanecarbonyle-1,2,3,4-tétrahydroquinoline-2-yl)-éthyle]-4-(2-méthoxyphényle)-pipérazine,
1-(1-cyclohexanecarbonyle-1,2,3,4-tétrahydroquinoline-2-ylméthyle)-4-[2-(2,2,2-trifluoroéthoxy)-phényle]-pipérazine,
1-(1-cyclohexanecarbonyle-6-fluoro-1,2,3,4-tétrahydroquinoline-2-ylméthyle)-4-[2-(2,2,2-trifluoroéthoxy)-phényle]-pipérazine,
1-[1-(2-éthylbutanoyle)-1,2,3,4-tétrahydroquinoline-2-ylméthyle]-4-[2-(2,2,2-trifluoroéthoxy)-phényle]-pipérazine,
1-[1-(3-méthoxypropionyle)-1,2,3,4-tétrahydroquinoline-2-ylméthyle]-4-[2-(2,2,2-trifluoroéthoxy)-phényle]-pipérazine,
1-[1-(3-benzyloxypropionyle)-1,2,3,4-tétrahydroquinoline-2-ylméthyle]-4-[2-(2,2,2-trifluoroéthoxy)-phényle]-pipérazine,
1-[1-(3-benzyloxypropionyle)-1,2,3,4-tétrahydroquinoline-2-ylméthyle]-4-(4-indolyle)-pipérazine,
1-[1-(3-hydroxypropionyle)-1,2,3,4-tétrahydroquinoline-2-ylméthyle]-4-[2-(2,2,2-trifluoroéthoxy)-phényle]-pipérazine,
1-[1-(3-méthoxypropionyle)-1,2,3,4-tétrahydroquinoline-2-ylméthyle]-4-(4-indolyle)-pipérazine,
1-[1-(3-isopropoxypropionyle)-1,2,3,4-tétrahydroquinoline-2-ylméthyle]-4-(4-indolyle)-pipérazine,
1-[1-acétyle-1,2,3,4-tétrahydroquinoline-2-ylméthyle]-4-(4-indolyle)-pipérazine,
1-[1-(4-morpholinocarbonyle)-1,2,3,4-tétrahydroquinoline-2-ylméthyle]-4-(4-indolyle)-pipérazine,
1-(1-cyclohexanecarbonyle-1,2,3,4-tétrahydroquinoline-2-ylméthyle)-4-(1-méthyle-4-indolyle)-pipérazine,
1-(1-cyclohexanecarbonyle-6-méthoxy-1,2,3,4-tétrahydroquinoline-2-ylméthyle)-4-(4-indolyle)-pipérazine,
1-[1-(3-hydroxypropionyle)-1,2,3,4-tétrahydroquinoline-2-ylméthyle]-4-(4-indolyle)-pipérazine,
1-(1-cyclohexanecarbonyle-6-fluoro-1,2,3,4-tétrahydroquinoline-2-ylméthyle)-4-(4-indolyle)-pipérazine,
1-(1-diméthylaminocarbonyle-1,2,3,4-tétrahydroquinoline-2-ylméthyle)-4-(4-indolyle)-pipérazine,
1-(1-éthylaminocarbonyle-1,2,3,4-tétrahydroquinoline-2-ylméthyle)-4-(4-indolyle)-pipérazine,
1-(1-cyclohexanecarbonyle-1,2,3,4-tétrahydroquinoline-2-ylméthyle)-4-(1-isoquinolinyle)-pipérazine,
1-(1-cyclohexanecarbonyle-1,2,3,4-tétrahydroquinoline-2-ylméthyle)-4-(2-méthoxyphényle)-pipéridine,
1-(7-benzofuranyle)-4-(1-cyclohexanecarbonyle-1,2,3,4-tétrahydroquinoline-2-ylméthyle)-pipérazine,
1-(2-éthylbutanoyle-1,2,3,4-tétrahydroquinoline-2-ylméthyle)-4-(4-indolyle)-pipérazine,
1-(-cyclohex-3-enylcarbonyle-1,2,3,4-tétrahydroquinoline-2-ylméthyle)-4-(4-indolyle)-pipérazine,
1-(1-cycloheptanecarbonyle-1,2,3,4-tétrahydroquinoline-2-ylméthyle)-4-(4-indolyle)-pipérazine,
1-(1-cyclopentanecarbonyle-1,2,3,4-tétrahydroquinoline-2-ylméthyle)-4-(4-indolyle)-pipérazine,
1-(1-benzoyle-1,2,3,4-tétrahydroquinoline-2-ylméthyle)-4-(4-indolyle)-pipérazine
1-(4-indolyle)-4-(1-pentanoyle-1,2,3,4-tétrahydroquinoline-2-ylméthyle)-pipérazine
1-(1-Cyclohexanecarbonyle-6-trifluorométhyle-1,2,3,4-tétrahydroquinoline-2-ylméthyle)-4-(4-indolyle)-pipérazine,
1-(1-cyclohexanecarbonyl-6-trifluorométhoxy-1,2,3,4-tétrahydroquinoline-2-ylméthyle)-4-(4-indolyle)-pipérazine,
1-[1-(3-benzylaminopropionyle)-1,2,3,4-tétrahydroquinoline-2-ylméthyle]-4-(4-indolyle)-pipérazine,
1-(3-aminopropionyle-1,2,3,4-tétrahydroquinoline-2-ylméthyle]-4-(4-indolyle)-pipérazine,
1-(4-indolyle)-4-[1-(3-métltylaminopropionyle-1,2,3,4-tétrahydroquinoline-2-ylméthyle]-pipérazine,
1-[1-(3-diméthylaminopropionyle)-1,2,3,4-tétrahydroquinoline-2-ylméthyle]-4-(4-indolyle)-pipérazine,
1-(4-indolyle)-4-(1-anilinocarbonyle-1,2,3,4-tétrahydroquinoline-2-ylméthyle]-pipérazine,
1-(1-cyclohexanecarbonyle-6-méthyle-1,2,3,4-tétrahydroquinoline-2-ylméthyle]-4-(4-indolyle)-pipérazine,
1-(4-indolyle)-4-(1-pyrrolidinecarbonyle-1,2,3,4-tétrahydroquinoline-2-ylméthyle)-pipérazine,
1-(6-bromo-1-cyclohexanecarbonyle-1,2,3,4-tétrahydroquinoline-2-ylméthyle)-4-(4-indolyle)-pipérazine,
1-(1-cyclohexanecarbonyle-1,2,3,4-tétrahydroquinoline-2-ylméthyle)-4-(4-fluoro-2-méthoxyphényle)-pipérazine,
1-(1-cyclohexanecarbonyle-1,2,3,4-tétrahydroquinoline-2-ylméthyle)-4-(2-méthyle-4-indolyle)-pipérazine,
1-(1-cyclohexanecarbonyle-1,2,3,4-tétrahydroquinoline-2-ylméthyle)-4-phénylpipérazine,
1-(1-cyclohexanecarbonyle-1,2,3,4-tétrahydroquinoline-2-ylméthyle)-4-(2-éthoxyphényle)-pipérazine,
1-(1-cyclohexanecarbonyle-1,2,3,4-tétrahydroquinoline-2-ylméthyle)-4-(2,5-diméthoxyphényle)-pipérazine,
1-(1-cyclohexanecarbonyle-1,2,3,4-tétrahydroquinoline-2-ylméthyle)-4-(2,3-dihydro-benzofurane-7-yl)-pipérazine,
1-(1-cyclohexanecarbonyle-1,2,3,4-tétrahydroquinoline-2-ylméthyle)-4-(5-fluoro-2-méthoxyphényle)-pipérazine,
1-(1-cyclohexanecarbonyle-1,2,3,4-tétrahydroquinoline-2-ylméthyle)-4-(2-méthylphényle)-pipérazine,
1-(1-cyclohexanecarbonyle-1,2,3,4-tétrahydroquinoline-2-ylméthyle)-4-(2-trifluorométhylphényle)-pipérazine,
1-(1-cyclohexanecarbonyle-1,2,3,4-tétrahydroquinoline-2-ylméthyle)-4-(2,4-dichlorophényle)-pipérazine,
1-(1-cyclohexanecarbonyle-1,2,3,4-tétrahydroquinoline-2-ylméthyle)-4-(2-hydroxyphényle)-pipérazine,
1-(1-cyclohexanecarbonyle-1,2,3,4-tétrahydroquinoline-2-ylméthyle)-4-(2-isopropoxyphényle)-pipérazine,
1-(1-cyclohexanecarbonyle-1,2,3,4-tétrahydroquinoline-2-ylméthyle)-4-(2-fluoro-5-méthylphényle)-pipérazine,
1-(1-cyclohexanecarbonyle-1,2,3,4-tétrahydroquinoline-2-ylméthyle)-4-(2,3-diméthyle-4-indolyle)-pipérazine,
1-(1-cyclohexanecarbonyle-1,2,3,4-tétrahydroquinoline-2-ylméthyle)-4-(2-chloro-5-fluorophényle)-pipérazine,
1-(1-cyclohexanecarbonyle-1,2,3,4-tétrahydroquinoline-2-ylméthyle)-4-(5-fluoro-2-méthylphényle)-pipérazine,
1-[(2,3-dihydro-1,4-benzodioxine-5-yl)]-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2-trifluorométhoxyphényle)-pipérazine,
1-(1-cyclohexanecarbonyle-1,2,3,4-tétrahydroquinoline-2-ylméthyle)-4-(4-fluoro-2-méthylphényle)-pipérazine,
1-(1-cyclohexanecarbonyle-1,2,3,4-tétrahydroquinoline-2-ylméthyle)-2,5-dichlorophényle)-pipérazine,
1-(1-cyclohexanecarbonyle-1,2,3,4-tétrahydroquinoline-2-ylméthyle)-4-[4-fluoro-2-(2,2,2-trifluoroéthoxyphényle)]-pipérazine,
1-(1-cyclohexanecarbonyle-1,2,3,4-tétrahydroquinoline-2-ylméthyle)-4-(2-pyrimidinyle)-pipérazine,
1-(1-cyclohexanecarbonyle-1,2,3,4-tétrahydroquinoline-2-ylméthyle)-4-(8-quinolinyle)-pipérazine,
1-(5-chloro-2-cyanophényle)-4-(1-cyclohexanecarbonyle-1,2,3,4-tétrahydroquinoline-2-ylméthyle)-pipérazine,
1-(5-cyano-2-méthoxyphényle)-4-(1-cyclohexanecarbonyle-1,2,3,4-tétrahydroquinoline-2-ylméthyle)-pipérazine
1-(1-acétyle-4-indolyle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(7-indolyle)-pipérazine,
1-(3-cyano-4-indolyle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2,4-difluorobenzyle)-pipérazine,
1-(2-bromobenzyle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2,5-difluorobenzyle)-pipérazine,
1-(1-cyclohexanecarbonyle-1,2,3,4-tétrahydroquinoline-2-ylméthyle)-4-(1-naphtyle)-pipérazine,
1-(7-bromo-4-indolyle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(3,4-dihydro-2H-benzo[b][1,4]dioxepine-6-yl)-pipérazine,
1-(2-chlorobenzyle)-4-(1-cyclohexanecarbonyle-1,2,3,4-tétrahydroquinoline-2-ylméthyle)-pipérazine,
1-(1-cyclohexanecarbonyle-1,2, 3,4-tétrahydroquinoline-2-ylméthyle)-4-(6-méthoxy-2-pyridyle)-pipérazine,
1-(1-cyclohexanecarbonyle-1,2,3,4-tétrahydroquinoline-2-ylméthyle)-4-(2, 5-dichlorobenzyle)-pipérazine,
1-(4-indolyle)-4-(1-piperidinocarbonyle-1,2,3,4-tétrahydroquinoline-2-ylméthyle)-pipérazine,
1-[1-(3-cyanopropionyle)-1,2,3,4-tétrahydroquinoline-2-ylméthyle]-4-(4-indolyle)-pipérazine,
1-(1-cyclohexanecarbonyle-8-fluoro-1,2,3,4-tétrahydroquinoline-2-ylméthyle)-4-(4-indolyle)-pipérazine,
1-[1-(3-acétylaminopropionyle)-1,2,3,4-tétrahydroquinoline-2-ylméthyle]-4-(4-indolyle)-pipérazine,
1-[1-(3-carbamoylaminopropionyle)-1,2,3,4-tétrahydroquinoline-2-ylméthyle]-4-(4-indolyle)-pipérazine,
1-{[1-[3-bis(acétylamino)-propionyle]-1,2,3,4-tétrahydroquinoline-2-ylméthyle}-4-(4-indolyle)-pipérazine,
1-(6-chloro-1-cyclohexanecarbonyle-1,2,3,4-tétrahydroquinoline-2-ylméthyle)-4-(4-indolyle)-pipérazine,
(R)-1-(1-cyclohexanecarbonyle-1,2,3,4-tétrahydroquinoline-2-ylméthyle)-4-(1-méthyle-4-indolyle)-pipérazine,
(Z)-1-[1-(4-hydroxycyclohexanecarbonyle)-1,2,3,4-tétrahydroquinoline-2-ylméthyle]-4-(4-indolyle)-pipérazine,
(E)-1-[1-(4-hydroxycyclohexanecarbonyle)-1,2,3,4-tétrahydroquinoline-2-ylméthyle]-4-(4-indolyle)-pipérazine,
1-(1-cyclohexanecarbonyle-7-fluoro-1,2,3,4-tétrahydroquinoline-2-ylméthyle)-4-(4-indolyle)-pipérazine,
1-(1-cyclohexanecarbonyle-6-phényle-1,2,3,4-tétrahydroquinoline-2-ylméthyle)-4-(4-indolyle)-pipérazine,
1-(1-cyclohexanecarbonyle-2,3-dihydroindole-2-ylméthyle)-4-(4-indolyle)-pipérazine,
1-(1-cyclohexanecarbonyle-8-méthoxy-1,2,3,4-tétrahydroquinoline-2-ylméthyle)-4-(4-indolyle)-pipérazine,
1-(1-cyclohexanecarbonyle-6-hydroxy-1,2,3,4-tétrahydroquinoline-2-ylméthyle)-4-(4-indolyle)-pipérazine,
1-(6-bromo-1-cyclohexanecarbonyle-1,2,3,4-tétrahydroquinoline-2-ylméthyle)-4-(4-fluoro-2-méthoxyphényle)-pipérazine,
1-(1-cyclohexanecarbonyle-8-hydroxy-1,2,3,4-tétrahydroquinoline-2-ylméthyle)-4-(4-indolyle)-pipérazine,
1-(1-cyclohexanecarbonyle-6-méthyle-1,2,3,4-tétrahydroqumoline-2-ylméthyle)-4-(4-fluoro-2-méthoxyphényle)-pipérazine,
1-[1-(3-cyanaminopropionyle-1,2,3,4-tétrahydroqumoline-2-ylméthyle)]-4-(4-indolyie)-pipérazine,
1-(5-chlore-1-cyclohexanecarbonyle-1,2,3,4-tétrahydroquinoline-2-ylméthyle)-4-(4-fluoro-2-méthoxyphényle)-pipérazine,
1-[5-chloro-1-(4-méthoxybenzoyle)-1,2,3,4-tétrahydroquinoline-2-ylméthyle]-4-(4-fluoro-2-méthoxyphényle)-pipérazine,
1-(7-chloro-1-cyclohexanecarbonyle-1,2,3,4-tétrahydroquinoline-2-ylméthyle)-4-(4-fluoro-2-méthoxyphényle)-pipérazine,
3-benzyle-1-(1-cyclohexanecarbonyle-1,2,3,4-tétrahydroquinoline-2-ylméthyle)-pipéridine (diastéréomère supérieur TLC Rf),
3-benzyle-1-(1-cyclohexanecarbonyle-1,2,3,4-tétrahydroquinoline-2-ylméthyle)-pipéridine (diastéréomère inférieur TLC Rf),
1-(4-chloro-2-*i*-propoxyphényle)-4-(1-cyclohexanecarbonyle-1,2,3,4-tétrahydroquinoline-2-ylméthyle)-pipérazine,
1-(5-chloro-2-fluorophényle)-4-(1-cyclohexanecarbonyle-1,2,3,4-tétrahydroquinoline-2-ylméthyle)-pipérazine,
1-[4-(2,1,3-benzothiadiazolyle)]-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2-éthoxy-4-fluorophényle)-pipérazine,
1-(1-cyclohexanecarbonyle-1,2,3,4-tétrahydroquinoline-2-ylméthyle)-4-(2-méthoxy-4-hydroxyphényle)-pipérazine,
1-(1-cyclohexanecarbonyle-1,2,3,4-tétrahydroquinoline-2-ylméthyle)-4-(7-méthoxy-4-indolyle)-pipérazine,
1-(1-cyclohexanecarbonyle-1,2,3,4-tétrahydroquinoline-2-ylméthyle)-4-(2-pyrazinyle)-pipérazine,
1-(2-cyano-4-nitrophényle)-4-(1-cyclohexanecarbonyle-1,2,3,4-tétrahydroquinoline-2-ylméthyle)-pipérazine,
1-[4-(2,1,3-benzoxadiazolyle)]-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2-méthoxy-5-trifluorométhylphényle)-pipérazine,
1-(1-cyclohexanecarbonyle-1,2,3,4-tétrahydroquinoline-2-ylméthyle)-4-[1-(1,2,3,4-tétrahydronaphtyle)]-pipérazine,
1-(7-chloro-2,3-dihydro-1,4-benzodioxine-5-yl)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(4-pyridyle)-pipérazine,
1-(1-cyclohexanecarbonyle-1,2,3,4-tétrahydroquinoline-2-ylméthyle)-4-[4-(6,7-diméthoxyquinazolinyle)]-pipérazine,
1-(4-fluoro-2-méthoxyphenyle)-4-(1-cyclohexanecarbonyle-6-nitro-1,2,3,4-tétrahydroquinoline-2-ylméthyle)-pipérazine,
1-(1-cyclohexanecarbonyle-1,2,3,4-tétrahydroquinoline-2-ylméthyle)-4-(1-éthyle-4-indolyle)-pipérazine,
1-(2-bromo-5-méthoxybenzyle)-4-(1-cyclohexanecarbonyle-1,2,3,4-tétrahydroquinoline-2-ylméthyle)-pipérazine,
1-(4-chloro-2-méthoxyphényle)-4-(1-cyclohexanecarbonyle-1,2,3,4-tétrahydroquinoline-2-ylméthyle)-pipérazine,
1-(4-chloro-2-éthoxyphényle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(4-fluoro-2-*i*-propoxyphényle)-pipérazine,
1-(1-cyclohexanecarbonyle-1,2,3,4-tétrahydroquinoline-2-ylméthyle)-4-(7-méthyle-4-indolyle)-pipérazine,
1-(1-cyclohexanecarbonyle-1,2,3,4-tétrahydroquinoline-2-ylméthyle)-4-(2-méthoxy-phénoxy)-pipéridine,
1-[6-(5-acétyle-2-thiényle)-1-cyclohexanecarbonyle-1,2,3,4-tétrahydroquinoline-2-ylméthyle)-4-(4-fluoro-2-méthoxyphényle)-pipérazine,
1-[1-cyclohexanecarbonyle-6-(3,5-diméthylisoxazole-4-yl)-1,2,3,4-tétrahydroquinoline-2-ylméthyle]-4-(4-fluoro-2-méthoxyphényle)-pipérazine,
1-(1-cyclohexanecarbonyle-7-méthyle-1,2,3,4-tétrahydroquinoline-2-ylméthyle)-4-(4-fluoro-2-méthoxyphényle)-pipérazine,
1-(1-cyclohexanecarbonyle-4-méthyle-1,2,3,4-tétrahydroquinoline-2-ylméthyle)-4-(4-fluoro-2-méthoxyphényle)-pipérazine,
1-(1-cyclohexanecarbonyle-8-méthyle-1,2,3,4-tétrahydroquinoline-2-ylméthyle)-4-(4-fluoro-2-méthoxyphényle)-pipérazine,
1-(4-indolyle)-4-[1-(2-pyrrolylcarbonyle)-1,2,3,4-tétrahydroquinoline-2-ylméthyle]-pipérazine,
1-[1-(4-diméthylaminobenzoyle)-1,2,3,4-tétrahydroquinoline-2-ylméthyle]-4-(4-indolyle)-pipérazine,
1-(4-indolyle)-4-[1-(2-phénylacétyle)-1,2,3,4-tétrahydroquinoline-2-ylméthyle]-pipérazine,
1-[1-(4-cyanobenzoyle)-1,2,3,4-tétrahydroquinoline-2-ylméthyle]-4-(4-indolyle)-pipérazine,
1-[1-(2-chlorobenzoyle)-1,2,3,4-tétrahydroquinoline-2-ylméthyle]-4-(4-indolyle)-pipérazine,
1-[1-(4-chlorobenzoyle)-1,2,3,4-tétrahydroquinoline-2-ylméthyle]-4-(4-indolyle)-pipérazine,
1-[1-(3-chlorobenzoyle)-1,2,3,4-tétrahydroquinoline-2-ylméthyle]-4-(4-indolyle)-pipérazine,
1-[1-(5-benzo[1,3]dioxolylcarbonyle)-1,2,3,4-tétrahydroquinoline-2-ylméthyle]-4-(4-indolyle)-pipérazine,
1-(1-cyclopropylcarbonyle-1,2, 3,4-tétrahydroquinoline-2-ylméthyle)-4-(4-indolyle)-pipérazine,
1-(4-indolyle)-4-[1-(2-méthylpropionyle)-1,2,3,4-tétrahydroquinoline-2-ylméthyle]-pipérazine,
1-(4-Indolyle)-4-[1-(2-méthoxyacétyle)-1,2,3,4-tétrahydroquinoline-2-ylméthyle]-pipérazine,
1-(2-cyclopropylacétyle-1,2,3,4-tétrahydroquinoline-2-ylméthyle)-4-(4-indolyle)pipérazine,
1-(4-indolyle)-4-[1-(3-méthylbutanoyle)-1,2,3,4-tétrahydroquinoline-2-ylméthyle]pipérazine,
1-(2-furoyle-1,2,3,4-tétrahydroquinoline-2-ylméthyle)-4-(4-indolyle)-pipérazine,
1-(4-indolyle)-4-[1-(5-isoxazolylcarbonyle)-1,2,3,4-tétrahydroquinoline-2-ylméthyle]-pipérazine,
1-(4-indolyle)-4-[1-(3-tétrahydrofurylcarbonyle)-1,2,3,4-tétrahydroquinoline-2-ylméthyle]-pipérazine,
1-(4-indolyle)-4-[1-(2-tétrahydrofurylcarbonyle)-1,2,3,4-tétrahydroquinoline-2-ylméthyle]-pipérazine,
1-[1-(3,3-diméthylbutanoyle)-1,2,3,4-tétrahydroquinoline-2-ylméthyle]-4-(4-indolyle)-pipérazine,
1-[1-(2-Acétoxyacétyle)-1,2,3,4-tétrahydroquinoline-2-ylméthyle]-4-(4-indolyle)-pipérazine,
1-(4-indolyle)-4-[1-(2-thiénylcarbonyle-1,2,3,4-tétrahydroquinoline-2-ylméthyle]-pipérazine,
1-(4-indolyle)-4-[1-(3-thiénylcarbonyle)-1,2,3,4-tétrahydroquinoline-2-ylméthyle]-pipérazine,
1-[1-(2-cyclohexylacétyle)-1,2,3,4-tétrahydroquinoline-2-ylméthyle]-4-(4-indolyle)-pipérazine,
1-(4-indolyle)-4-[1-(3-trifluorométhylbenzoyle)-1,2,3,4-tétrahydroquinoline-2-ylméthyle]-pipérazine,
1-(4-indolyle)-4-[1-(4-trifluorométhylbenzoyle)-1,2,3,4-tétrahydroquinoline-2-ylméthyle]-pipérazine,
1-(4-indolyle)-4-[1-(3-phénylpropionyle)-1,2,3,4-tétrahydroquinoline-2-ylméthyle]pipérazine,
1-(4-indolyle)-4-[1-(2-méthoxybenzoyle)-1,2,3,4-tétrahydroquinoline-2-ylméthyle]pipérazine,
1-(4-indolyle)-4-[1-(4-méthoxybenzoyle)-1,2,3,4-tétrahydroquinoline-2-ylméthyl]pipérazine,
1-[1-(4-fluorophénylacétyle)-1,2,3,4-tétrahydroquinoline-2-ylméthyle]-4-(4-indolyle)-pipérazine,
1-[1-(2,6-difluorobenzoyle)-1,2,3,4-tétrahydroquinoline-2-ylméthyle]-4-(4-indolyle)-pipérazine,
1-(4-indolyle)-4-[1-(2-méthoxyphénylacétyle)-1,2,3,4-tétrahydroquinoline-2-ylméthyle)-pipérazine,
1-[1-(4-chlorophénylacétyle)-1,2,3,4-tétrahydroquinoline-2-ylméthyle]-4-(4-indolyle)-pipérazine,
1-(4-indolyle)-4-[1-[2-(4-trifluorométhylphényle)-acétyle]-1,2,3,4-tétrahydroquinoline]-2-ylméthyl]-pipérazine,
1-(4-indolyle)-4-[1-(2-pyridylcarbonyle)-1,2,3,4-tétrahydroquinoline-2-ylméthyle]-pipérazine
1-(4-indolyle)-4-[1-(3-pyridylcarbonyle)-1,2,3,4-tétrahydroquinoline-2-ylméthyle]-pipérazine
1-(4-indolyle)-4-[1-(4-pyridylcarbonyle)-1,2,3,4-tétrahydroquinoline-2-ylméthyle]-pipérazine
1-[1-(3, 5-diméthyle-4-isoxazolylcarbonyle)-1,2,3,4-tétrahydroquinoline-2-ylméthyle]-4-(4-indolyle)-pipérazine,
1-[1-(2,2-diméthylpropionyle)-1,2,3,4-tétrahydroquinoline-2-ylméthyle]-4-(4-indolyle)-pipérazine,
1-(4-indolyle)-4-[1-[2-(3-pyridyle)-acétyle]-1,2,3,4-tétrahydroquinoline-2-ylméthyle]-pipérazine,
1-[1-(1-acétyle-4-pipéridinylcarbonyle)-1,2,3,4-tétrahydroquinoline-2-ylméthyle]-4-(4-indolyle)-pipérazine,
1-(4-indolyle)-4-[1-[2-(2-thiénylacétyle)]-1,2,3,4-tétrahydroquinoline-2-ylméthyle]-pipérazine,
1-(4-indolyle)-4-[1-[2-(3-thiénylacétyle)]-1,2,3,4-tétrahydroquinoline-2-ylméthyle]-pipérazine,
1-(4-indolyle)-4-[1-[3-(3-pyridylpropionyle)]-1,2,3,4-tétrahydroquinoline-2-ylméthyle]-pipérazine,
1-(4-indolyle)-4-[1-(2 phénoxyacétyle)-1,2,3,4-tétrahydroquinoline-2-ylméthyle]-pipérazine,
1-(4-indolyle)-4-[1-[2-(4-méthoxyphénylacétyle)]-1,2,3,4-tétrahydroquinoline-2-ylméthyle]-pipérazine,
1-(4-indolyle)-4-[1-(2-phénylméthoxyacétyle)-1,2,3,4-tétrahydroquinoline-2-ylméthyle]-pipérazine,
1-[1-[2-(2-chlorophénylacétyle)]-1,2,3,4-tétrahydroquinoline-2-ylméthyle]-4-(4-indolyle)-pipérazine,
1-{1-[2-(N-benzoyl-N-méthyle)-aminoacétyle]-1,2,3,4-tétrahydroquinolime-2-ylméthyle}-4-(4-indolyle)-pipérazine,
1-(4-indolyle)-4-[1-(1-méthyle-3-indolylcarbonyle)-1,2,3,4-tétrahydroquinoline-2-ylméthyle]-pipérazine,
1-[1-[2-(4-diméthylaminophénylacétyle)]-1,2,3,4-tétrahydroquinoline-2-ylméthyle]-4-(4-indolyle)-pipérazine,
1-[1-(5-benzo[1,3]dioxolylacétyle)-1,2,3,4-tétrahydroquinoline-2-ylméthyle]-4-(4-indolyle)-pipérazine,
1-[1-[2-(2-chlorophénoxyacétyle)-1,2,3,4-tétrahydroquinoline-2-ylméthyl]-4-(4-indolyle)-pipérazine,
1-[1-[2-(4-chlorophénoxyacétyle)-1,2,3,4-tétrahydroquinoline-2-ylméthyle]-4-(4-indolyle)-pipérazine,
1-(4-indolyle)-4-[1-(2-morpholino-5-pyridylcarbonyle]-1,2,3,4-tétrahydroquinoline-2-ylméthyle]-pipérazine,
1-(4-indolyle)-4-[1-(5-méthyle-4-isoxazolylcarbonyle)-1,2,3,4-tétrahydroquinoline-2-ylméthyle]-pipérazine,
1-(4-indolyle)-4-[1-(5-oxo-2-pyrrolidinylacétyle)-1,2,3,4-tétrahydroquinoline-2-ylméthyle]-pipérazine,
1-(4-indolyle)-4-[1-(2-méthyle-5-pipéridinosulfonyle-3-furoyle)-1,2,3,4-tétrahydroquinoline-2-ylméthyle]-pipérazine,
1-(4-indolyle)-4-[1-(2-méthyle-5-morpholinosulfonyle-3-furoyle)-1,2,3,4-tétrahydroquinoline-2-ylméthyle]-pipérazine,
1-(4-indolyle)-4-{1-[1-(p-tolylsulfonyle)-3-pyrrolylcarbonyle]-1,2,3,4-tétrahydroquinoline-2-ylméhyle}-pipérazine,
1-(4-indolyle)-4-[1-(2-méthyle-5-dimethylsulfamoyle-3-furoyle)-1,2,3,4-tétrahydroquinoline-2-ylméthyle]-pipérazine,
1-(4-indolyle)-4-{1-[2-méthyle-5-(1-pyrrolidinylsulfamoyle)-3-furoyle)-1,2,3,4-tétrahydroquinoline-2-ylméthyle]-pipérazine,
1-[(1-adamantylcarbonyle)-1,2,3,4-tétrahydroqumoline-2-ylméthyle]-4-(4-indolyle)-pipérazine,
1-(4-indolyle)-4-[1-(3-phénoxypropionyle)-1,2,3,4-tétrahydroquinoline-2-ylméthyle-pipérazine,
1-(4-indolyle)-1-[1-(4-phénoxybutanoyle)-1,2,3,4-tétrahydroquinoline-2-ylméthyle]-pipérazine,
1-(4-indolyle)-4-[1-(2-fluorophénylacétyle)-1,2,3,4-tétrahydroquinoline-2-ylméthyle]-pipérazine,
1-(4-indolyle)-4-[1-(2-trifluorométhyle-phénylacétyle)-1,2,3,4-tétrahydroquinoline-2-ylméthyle]-pipérazine,
1-[1-(2-bicyclo [2.2.2]octylcarbonyle)-1,2,3,4-tétrahydroquinoline-2-ylméthyle]-4-(4-indolyle)-pipérazine,
1-(4-indolyle)-1-[1-(4-phénylbutanoyle)-1,2,3,4-tétrahydroquinoline-2-ylméthyle]-pipérazine,
1-(4-indolyle)-4-[1-(3-méthoxybenzoyle)-1,2,3,4-tétrahydroquinoline-2-ylméthyle]-pipérazine,
1-[1-(4-hydroxybenzoyle)-1,2,3,4-tétrahydroquinoline-2-ylméthyle]-4-(4-indolyle)-pipérazine,
1-[1-(4-éthoxybenzoyle)-1,2,3,4-tétrahydroquinoline-2-ylméthyle]-4-(4-indolyle)-pipérazine,
1-(4-indolyle)-4-[1-(4-méthylbenzoyle)-1,2,3,4-tétrahydroquinoline-2-ylméthyle]-pipérazine,
1-[1-[(N-benzyle-N-méthanesulfonyle)-2-aminoacétyle]-1,2,3,4-tétrahydroquinoline-2-ylméthyle]-4-(4-indolyle)-pipérazine,
1-(4-indolyle)-4-[1-(2-(S)-5-oxopyrrolidinylcarbonyle)-1,2,3,4-tétrahydroquinoline-2-ylméthyle]-pipérazine,
1-(4-indolyle)-4-[1-(2-(R)-5-oxopyrrolidinylcarbonyle)-1,2,3,4-tétrahydroquinoline-2-ylméthyle]-pipérazine,
1-(4-indolyle)-4-[1-(2-méthylanilinocarbonyle)-1,2,3,4-tétrahydroquinoline-2-ylinéthyle]-pipérazine,
1-(4-indolyle)-4-[1-(3-méthylanilinocarbonyle)-1,2,3,4-tétrahydroquinoline-2-ylméthyle]-pipérazine,
1-[1-(4-fluoroanilinocarbonyle)-1,2,3,4-tétrahydroquinoline-2-ylméthyle)-4-(4-indolyle)-pipérazine,
1-(4-indolyle)-4-[1-(4-méthylanilinocarbonyle)-1,2,3,4-tétrahydroquinoline-2-ylméthyle]-pipérazine,
1-(4-indolyle)-4-[1-((1S)-1-phényléthylaminocarbonyle)-1,2,3,4-tétrahydroquinoline-2-ylméthyle]-pipérazine,
1-(4-indolyle)-4-[1-((1R)-1-phényléthylaminocarbonyle)-1,2,3,4-tétrahydroquinoline-2-ylméthyle)-pipérazine(diastéréisomère A),
1-(4-Indolyle)-4-[1-((1R)-1-phényléthylaminocarbonyle)-1,2,3,4-tétrahydroquinoline-2-ylméthyle]-pipérazine (mélange de diastéréisomères 1:1),
1-(4-indolyle)-4-[1-(1-méthyléthylaminocarbonyle)-1,2,3,4-tétrahydroquinoline-2-ylméthyle]-pipérazine,
1-(4-indolyle)-4-[1-méthylaminothiocarbonyle-1,2,3,4-tétrahydroquinoline-2-ylméthyle)-pipérazine,
1-(1-benzylaminocarbonyle-1,2,3,4-tétrahydroquinoline-2-ylinéthyle)-4-(4-indolyle)-pipérazine,
1-(4-indolyle)-4-[1-(2-phényléthylaminocarbonyle)-1,2,3,4-tétrahydroquinoline-2-ylméthyle]-pipérazine,
1-(4-indolyle)-4-(1-pentylaminocarbonyle-1,2,3,4-tétrahydroquinoline-2-ylméthyle]-pipérazine,
1-[1-(1,1-diméthyléthylaminocarbonyle-1,2,3,4-tétrahydroquinoline-2-ylméthyle]-4-(4-indolyl)-pipérazine,
1-(4-indolyle)-4-[(4-méthoxyphénylaminocarbonyle)-1,2,3,4-tétrahydroquinoline-2-ylméthyle]-pipérazine,
1-(4-indolyle)-4-[(4-méthoxyphénylméthylaminocarbonyle)-1,2,3,4-tétrahydroquinoline-2-ylméthyle]-pipérazine,
1-(4-indolyle)-4-[1-phénylsulfonyle-1,2,3,4-tétrahydroquinoline-2-ylméthyle]-pipérazine,
1-(4-indolyle)-4-[1-(2-thiénylsulfonyle)-1,2,3,4-tétrahydroquinoline-2-ylméthyle]-pipérazine,
1-(4-indolyle)-4-[1-(4-méthoxyphénylsulfonyle)-1,2,3,4-tétrahydroquinoline-2-ylméthyle]-pipérazine,
1-(4-indolyle)-4-[1-(4-méthylphénylsulfonyle)-1,2,3,4-tétrahydroquinoline-2-ylméthyle]-pipérazine,
1-[1-(4-fluorophénylsulfonyle)-1,2,3,4-tétrahydroquinoline-2-ylméthyle]-4-(4-indolyle)-pipérazine,
1-[1-(4-cyanophénylsulfonyle)-1,2,3,4-tétrahydroquinoline-2-ylméthyle]-4-(4-indolyle)-pipérazine,
1-(4-indolyle)-4-[1-benzylsulfonyle-1,2,3,4-tétrahydroquinoline-2-ylméthyle]-pipérazine,
1-(1-*tert*-butoxycarbonylaminoacétyle-1,2,3,4-tétrahydroquinoline-2-ylméthyle)-4-(4-indolyle)-pipérazine,
1-[1-(3-*tert*-butoxycarbonylamino-propionyle)-1,2,3,4-tétrahydroquinoline-2-ylméthyle]-4-(4-indolyle)-pipérazine,
1-[1-(1-*tert*-butoxycarbonyle-3-indolyle-acétyle)-1,2,3,4-tétrahydroquinoline-2-ylméthyle]-4-(4-indolyle]-pipérazine,
1-[1-(1-tert-butoxycarbonyle-4-pipéridylcarbonyle)-1,2,3,4-tétrahydroquinoline-2-ylméthyle]-4-(4-indolyle)-pipérazine,
1-[1-(1-tert-butoxycarbonyle-2R-pyrrolidine-2-ylcarbonyle)-1,2,3,4-tétrahydroquinoline-2-ylméthyle]-4-(4-indolyle)-pipérazine,
1-[1-(1-tert-butoxycarbonyle-2S-pyrrolidine-2-ylcarbonyle)-1,2,3,4-tétrahydroquinoline-2-ylméthyle]-4-(4-indolyle)-pipérazine,
1-(1-cyclohexanecarbonyle-1,2,3,4-tétrahydroquinoline-2-ylméthyle)-2-(α-hydroxybenzyle)-pipéridine,
1-(1-cyclohexanecarbonyle-1,2,3,4-tétrahydroquinoline-2-ylméthyle)-4-(2,6-diméthylphényle)-pipéridine,
2-(4-chlorophényle)-1-(1-cyclohexanecarbonyle-1,2,3,4-tétrahydroquinoline-2-ylméthyle)-pipéridine,
1-(1-cyclohexanecarbonyle-1,2,3,4-tétrahydroquinoline-2-yliméthyle)-4-phénylpipéridine,
4-benzoyle-1-(1-Cyclohexanecarbonyle-1,2,3,4-tétrahydroquinoline-2-yliméthyle)-pipéridine,
1-(1-cyclohexanecarbonyle-1,2,3,4-tétrahydroquinoline-2-ylméthyle)-4-(2-nitrophényle)-pipérazine,
1-(1-cyclohexanecarbonyle-1,2,3,4-tétrahydroquinoline-2-ylméthyle)-4-(3-trifluorométhyle-2-pyridyle)-pipérazine,
1-(1-cyclohexanecarbonyle-1,2,3,4-tétrahydroquinoline-2-ylméthyle)-4-(4-fluoro-2-nitrophényle)-pipérazine,
1-(1-cyclohexanecarbonyle-1,2,3,4-tétrahydroquinoline-2-ylméthyle)-4-(2,3-dichlorophényle)-pipérazine,
1-(1-cyclohexanecarbonyle-1,2,3,4-tétrahydroquinoline-2-ylméthyle)-4-(3-cyano-2-pyridyle)-pipérazine,
1-(1-cyclohexanecarbonyle-1,2,3,4-tétrahydroquinoline-2-ylméthyle)-4-(3,4-dichlorophényle)-pipérazine,
1-(1-cyclohexanecarbonyle-1,2,3,4-tétrahydroquinoline-2-ylméthyle)-4-(2-éthylphényle)-pipérazine,
1-(4-chloro-3-trifluorométhylphényle)-4-(1-cyclohexanecarbonyle-1,2,3,4-tétrahydroquinoline-2-ylméthyle)-pipérazine,
1-(2-cyanophényle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2,4-diméthoxyphényle)-pipérazine,
1-(1-cyclohexanecarbonyle-1,2,3,4-tétrahydroquinoline-2-ylméthyle)-4-(2-méthylthiophényle)-pipérazine,
1-(1-cyclohexanecarbonyle-1,2,3,4-tétrahydroquinoline-2-ylméthyle)-4-(3,4-diméthoxyphényle)-pipérazine,
1-(1-cyclohexanecarbonyle-1,2,3,4-tétrahydroquinoline-2-ylméthyle)-4-(3-hydroxyphényle)-pipérazine,
1-(1-cyclohexanecarbonyle-1,2,3,4-tétrahydroquinoline-2-ylméthyle)-4-(2,4-difluorophényle)-pipérazine,
1-(5-chloro-2-méthoxyphényle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(6-méthyle-2-pyridyle)-pipérazine,
1-(1-cyclohexanecarbonyle-1,2,3,4-tétrahydroquinoline-2-ylméthyle)-4-(4-méthylphényle)-pipérazine,
1-(1-cyclohexanecarbonyle-1,2,3,4-tétrahydroquinoline-2-yhnéthyle)-4-(4-trifluorométhylphényle)-pipérazine,
1-(3-chlorophényle)-4-(1-cyclohexanecarbonyle-1,2,3,4-tétrahydroquinoline-2-ylméthyle)-pipérazine,
1-(2-chlorophényle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(3-méthoxyphényle)-pipérazine,
1-(1-cyclohexanecarbonyle-1,2,3,4-tétrahydroquinoline-2-ylméthyle)-4-(4-fluorophényle)-pipérazine,
1-(4-acétylphényle)-4-(1-cyclohexanecarbonyle-1,2,3,4-tétrahydroquinoline-2-ylméthyle)-pipérazine,
1-(1-cyclohexanecarbonyle-1,2, 3,4-tétrahydroquinoline-2-ylméthyle)-4-(4-hydroxyphényle)-pipérazine,
1-(5-chloro-2-méthylphényle)-4-(1-cyclohexanecarbonyle-1,2,3,4-tétrahydroquinoline-2-ylméthyle)-pipérazine,
1-(1-cyclohexanecarbonyle-1,2, 3,4-tétrahydroquinoline-2-ylméthyle)-4-(3-méthylphényle)-pipérazine,
1-(1-cyclohexanecarbonyle-1,2,3,4-tétrahydroquinoline-2-ylméthyle)-4-(2-nitro-4-trifluorométhylphényle)-pipérazine,
1-(1-cyclohexanecarbonyle-1,2,3,4-tétrahydroquinoline-2-ylméthyle)-4-(5-trifluorométhyle-2-pyridyle)-pipérazine,
1-(1-cyclohexanecarbonyle-1,2,3,4-tétrahydroquinoline-2-ylméthyle)-4-(3-chloro-5-trifluorométhyle-2-pyridyle)-pipérazine,
1-(5-cyano-2-pyridyle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2-méthyle-4-quinolinyle)-pipérazine,
1-(1-cyclohexanecarbonyle-1,2,3,4-tétrahydroquinoline-2-ylméthyle)-4-benzyle-pipéridine,
1-(benzofurane-3-yl)-4-(1-Cyclohexanecarbonyle-1,2,3,4-tétrahydroquinoline-2-ylméthyle)-pipéridine,
1-(1-cyclohexanecarbonyle-1,2,3,4-tétrahydroquinoline-2-ylméthyle)-4-(4-fluorophényle)-pipéridine,
1-(1-cyclohexanecarbonyle-1,2,3,4-tétrahydroquinoline-2-ylméthyle)-4-(3-fluorophényle)-pipéridine,
1-(1-cyclohexanecarbonyle-1,2,3,4-tétrahydroquinoline-2-ylméthyle)-4-(2-fluorophényle)-pipéridine,
4-(4-chlorophényle)-1-(1-cyclohexanecarbonyle-1,2,3,4-tétrahydroquinoline-2-ylméthyle)-pipéridine,
1-(1-cyclohexanecarbonyle-1,2,3,4-tétrahydroquinoline-2-ylméthyle)-4-(4-trifluorométhylphényle)-pipéridine,
1-(1-cyclohexanecarbonyle-1,2,3,4-tétrahydroquinoline-2-ylméthyle)-4-(3-trifluorométhylphényle)-pipéridine,
1-(1-cyclohexanecarbonyle-1,2,3,4-tétrahydroquinoline-2-ylméthyle)-4-[5-(2-furyle)-2H-3-pyrazolyle]-pipéridine,
1-(1-cyclohexanecarbonyle-1,2,3,4-tétrahydroquinoline-2-ylméthyle)-4-(4-méthoxyphényle)-pipéridine,
1-(-cyclohexanccarbonyle-1,2,3,4-tétrahydroquinoline-2-ylméthyle)-4-(3-méthoxyphényle)-pipéridine,
1-(1-cyclohexanecarbonyle-1,2,3,4-tétrahydroquinoline-2-ylméthyle)-4-(4-fluorobenzoyle)-pipéridine,
1-(1-Cyclohexanecarbonyle-1,2,3,4-tétrahydroquinoline-2-ylméthyle)-4-benzylureido-pipéridine,
1-(1-cyclohexanecarbonyle-1,2,3,4-tétrahydroquinoline-2-ylméthyle)-2-(4-méthoxybenzyle)-pipéridine,
1-(1-cyclohexanecarbonyle-1,2,3,4-tétrahydroquinoline-2-ylméthyle)-2-(4-fluorobenzyle)-pipéridine,
1-(1-cyclohexanecarbonyle-1,2,3,4-tétrahydroquinoline-2-ylméthyle)-4-(2,3-dichlorophényle)-pipéridine,
1-(1-cyclohexanecarbonyle-1,2,3,4-tétrahydroquinoline-2-ylméthyle)-4-(2,6-difluorophényle)-pipéridine,
4-(2-chloro-6-fluorophényle)-1-(1-cyclohexanecarbonyle-1,2,3,4-tétrahydroquinoline-2-ylméthyle)-pipéridine,
1-(1-cyclohexanecarbonyle-1,2,3,4-tétrahydroquinoline-2-ylméthyle)-4-(2,5-diméthylphényle)-pipéridine,
1-(1-cyclohexanecarbonyle-1,2,3,4-tétrahydroquinoline-2-ylméthyle)-4-(3-méthylphényle)-pipéridine,
1-(1-cyclohexanecarbonyle-1,2,3,4-tétrahydroquinoline-2-ylméthyle)-4-(2-méthylphényle)-pipéridine,
1-(1-cyclohexanecarbonyle-1,2,3,4-tétrahydroquinoline-2-ylméthyle)-4-(3-fluoro-2-méthylphényle)-pipéridine,
1-(1-cyclohexanecarbonyle-1,2,3,4-tétrahydroquinoline-2-ylméthyle)-4-(3,5-difluorophényle)-pipéridine,
1-(1-cyclohexanecarbonyle-1,2,3,4-tétrahydroquinoline-2-ylméthyle)-4-(2,5-difluorophényle)-pipéridine,
1-(1-cyclohexanecarbonyle-1,2,3,4-tétrahydroquinoline-2-ylméthyle)-4-(3,5-diméthylphényle)-pipéridine,
4-(3-bromophényle)-1-(1-cyclohexanecarbonyle-1,2,3,4-tétrahydroquinoline-2-ylméthyle)-pipéridine,
4-(2-bromophényle)-1-(1-cyclohexanecarbonyle-1,2,3,4-tétrahydroquinoline-2-ylméthyle)-pipéridine,
4-(4-butoxyphényle)-1-(1-cyclohexanecarbonyle-1,2,3,4-tétrahydroquinoline-2-ylméthyle)-pipéridine,
1-(1-cyclohexanecarbonyle-1,2,3,4-tétrahydroquinoline-2-ylméthyle)-4-(2-fluorophényle)-pipérazine,
1-(1-cyclohexanecarbonyle-1,2,3,4-tétrahydroquinoline-2-ylméthyle)-4-(6-chloro-2-pyridyle)-pipérazine,
1-(1-cyclohexanecarbonyle-1,2,3,4-tétrahydroquinoline-2-ylméthyle)-4-(3,5-dichloro-4-pyridyle)-pipérazine,
1-(4-aminophényle)-4-(1-cyclohexanecarbonyle-1,2,3,4-tétrahydroquinoline-2-ylméthyle)-pipérazine,
4-(3-chlorophényle)-1-(1-cyclohexanecarbonyle-1,2,3,4-tétrahydroquinoline-2-ylméthyle)-pipéridine,
4-(2-chlorophényle)-1-(1-cyclohexanecarbonyle-1,2,3,4-tétrahydroquinoline-2-ylméthyle)-pipéridine,
1-(1-cyclohexanecarbonyle-1,2,3,4-tétrahydroquinoline-2-ylméthyle)-2-(2-pyridyle)-pyrrolidine,
1-(1-cyclohexanecarbonyle-1,2,3,4-tétrahydroquinoline-2-ylméthyle)-2-(2-thiényle)-pyrrolidine,
1-(1-cyclohexanecarbonyle-1,2,3,4-tétrahydroquinoline-2-ylméthyle)-2-phénylpyrrolidine,
1-(1-cyclohexanecarbonyle-1,2,3,4-tétrahydroquinoline-2-ylméthyle)-2-(2,4-diméthoxyphényle)-pyrrolidine,
1-(1-cyclohexanecarbonyle-1,2,3,4-tétrahydroquinoline-2-ylméthyle)-2-(3-pyridyle)-pyrrolidine,
1-(1-cyclohexanccarbonyle-1,2,3,4-tétrahydroquinoline-2-ylméthyle)-2-benzylpyrrolidine,
1-(1-cyclohexanecarbonyle-1,2,3,4-tétrahydroquinoline-2-ylméthyle)-4-(3,5-diméthoxyphényle)-pipérazine,
1-(1-cyclohexanecarbonyle-1,2,3,4-tétrahydroquinoline-2-ylméthyle)-2-(2-furyle)-pyrrolidine,
1-(1cyclohexanecarbonyle-1,2,3,4-tétrahydroquinoline-2-ylméthyle)-2-(4-méthoxybenzyle)-pyrrolidine,
1-(1-cyclohexanecarbonyle-1,2,3,4-tétrahydroquinoline-2-ylméthyle)-2-(4-chlorophényle)-pyrrolidine,
1-(1-cyclohexanecarbonyle-1,2,3,4-tétrahydroquinoline-2-ylméthyle)-2-(2-indolyle)-pyrrolidine,
1-(1-cyclohexanecarbonyle-1,2,3,4-tétrahydroquinoline-2-ylméthyle)-4-(2-trifluorométhylphényle)-pipéridine,
1-cyclohexanecarbonyle-2-{N-[2-(2-méthoxyphénoxy)-éthyle]-N-méthyle-aminométhyle}- 1,2,3,4-tétrahydroquinoline,
1-cyclohexanecarbonyle-2-[N-(2,3-dihydro-benzo[1,4]dioxine-2-ylméthyle)-N-méthyle-aminométhylé]-1,2,3,4-tetrahydroquinoline,
1-cyclohexanecarbonyle-2-{N-[2-(2-chlorophénoxy)-éthyle]-N-méthyle-aminométhyle}-1,2,3,4-tétrahydroquinoline,
1-cyclohexanecarbonyle-2-{N-[2-(2-trifluorométhoxyphénoxy)-éthyle]-N-méthyle-aminométhyle}-1,2,3,4-tétrahydroquinoline,
1-cyclohexanecarbonyle-2-{N-[2-(4-indolyloxy)-éthyle]-N-méthyle-aminométhyle}-1,2,3,4-tétrahydroquinoline,
1-cyclohexanecarbonyle-2-{N-[2-(8-quinolyloxy)-éthyle]-N-méthyle-aminométhyle}-1,2,3,4-tétrahydroquinoline,
1-(4-chloro-2-nitrophényle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(2-trifluorométhyle-4-quinolinyle)-pipérazine,
4-(1-benzimidazolyle)-1-(1-cyclohexanecarbonyle-1,2,3,4-tétrahydroquinoline-2-ylméthyle)-pipéridine,
1-(1-cyclohexanecarbonyle-1,2,3,4-tétrahydroquinoline-2-ylinéthyle)-2-(4-méthoxyphényle)-pipéridine,
1-(1-cyclohexanecarbonyle-1,2,3,4-tétrahydroquinoline-2-ylméthyle)-4-(2,6-dichlorophényle)-pipéridine,
1-(1-cyclohexanecarbonyle-1,2,3,4-tétrahydroquinoline-2-ylméthyle)-4-(2-pyridyle)-pipérazine,
1-(1-cyclohexanecarbonyle-1,2,3,4-tétrahydroquinoline-2-ylméthyle)-4-(4-quinolyle)-pipérazine,
1-(1-cyclohexanecarbonyle-1,2,3,4-tétrahydroquinoline-2-ylméthyle)-4-(7-méthoxy-4-quinolyle)-pipérazine,
1-(4-fluoro-2-méthoxyphényle)-4-[1-(2-méthylanilinocarbonyle)-1,2,3,4-tétrahydroquinoline-6-méthyle-2-ylméthyle]-pipérazine,
1-(4-fluoro-2-méthoxyphényle)-4-[1-(4-méthylanilinocarbonyle)-1,2,3,4-tétrahydroquinoline-6-méthyle-2-ylméthyle]-pipérazine,
1-[1-(t-butylcarbamoyle)-1,2,3,4-tétrahydroquinoline-6-méthyle-2-ylméthyle]-4-(4-fluoro-2-méthoxyphényle)-pipérazine,
1-(4-fluoro-2-méthoxyphényle)-4-(1-pentylaminocarbonyle-1,2,3,4-tétrahydroquinoline-6-méthyle-2-ylméthyle)-pipérazine,
1-(4-fluoro-2-méthoxyphényle)-4-[(2-thiénylacétyle)-1,2,3,4-tétrahydroquinoline-6-méthyle-2-ylméthyle]-pipérazine,
1-(4-fluoro-2-méthoxyphényle)-4-[(3-thiénylacétyle)-1,2,3,4-tétrahydroquinoline-6-méthyle-2-ylméthyle]-pipérazine,
1-(1-cyclohexylacétyle-1,2,3,4-tétrahydroquinoline-6-méthyle-2-ylméthyle)-4-(4-fluoro-2-méthoxyphényle)-pipérazine,
1-[1-(4-cyanobenzoyle)-1,2,3,4-tétrahydroquinoline-6-méthyle-2-ylméthyle]-4-(4-fluoro-2-méthoxyphényle)-pipérazine,
1-(4-fluoro-2-méthoxyphényle)-4-[1-(4-méthoxybenzoyle)-1,2,3,4-tétrahydroquinoline-6-méthyle-2-ylméthyle)-pipérazine,
1-[1-(4-chlorobenzoyle)-1,2,3,4-tétrahydroquinoline-6-méthyle-2-ylméthyle]-4-(4-fluoro-2-méthoxyphényle)-pipérazine,
1-[1-(benzo[1,3]dioxol-5-ylcarbonyle)-1,2,3,4-tétrahydroquinoline-6-méthyle-2-ylméthyle]-4-(4-fluoro-2-méthoxyphényle)-pipérazine,
1-[1-(2-chlorophénylacétyle)-1,2,3,4-tétrahydroquinoline-6-méthyle-2-ylméthyle]-4-(4-fluoro-2-méthoxyphényle)-pipérazine,
1-(4-fluoro-2-méthoxyphényle)-4-[1-(1-méthyle-3-indolylcarbonyle)-1,2,3,4-tétrahydroquinoline-6-méthyle-2-ylméthyle]-pipérazine,
1-(1-cycloheptanecarbonyle-1,2,3,4-tétrahydroquinoline-6-méthyle-2-ylméthyle)-4-(4-fluoro-2-méthoxyphényle)-pipérazine,
1-(4-fluoro-2-méthoxyphényle)-4-(1-phénylsulfonyle-1,2,3,4-tétrahydroquinoline-6-méthyle-2-ylméthyle)-pipérazine,
1-(4-fluoro-2-méthoxyphényle)-4-(1-benzylsulfonyle-1,2,3,4-tétrahydroquinoline-6-méthyle-2-ylméthyle)-pipérazine,
1-(4-fluoro-2-méthoxyphényle)-4-[1-(4-méthoxyphénylsulfonyle)-1,2,3,4-tétrahydroquinoline-6-méthyle-2-ylméthyle]-pipérazine,
1-(4-fluoro-2-méthoxyphényle)-4-[1-(4-méthylphénylsulfonyle)-1,2,3,4-tétrahydroquinoline-6-méthyle-2-ylméthyle]-pipérazine,
1-[1-(4-cyanophénylsulfonyle)-1,2,3,4-tétrahydroquinoline-6-méthyle-2-ylméthyle]-4-(4-fluoro-2-méthoxyphényle)-pipérazine,
1-[1-(4-fluorophénylsulfonyle)-1,2,3,4-tétrahydroquinoline-6-méthyle-2-ylméthyle]-4-(4-fluoro-2-méthoxyphényle)-pipérazine,
1-(4-fluoro-2-méthoxyphényle)-4-[1-(3-méthylbutanoyle)-1,2,3,4-tétrahydroquinoline-2-ylméthyle]-pipérazine,
1-(3,3-diméthylbutanoyle-1,2, 3,4-tétrahydroquinoline-2-ylméthyle)-4-(4-fluoro-2-méthoxyhényle)-pipérazine,
1-(1-cyclopropylacétyle-1,2,3,4-tétrahydroquinoline-2-ylméthyle)-4-(4-fluoro-2-méthoxyphényle)-pipérazine,
1-(4-fluoro-2-méthoxyphényle)-4-[1-(2-pyrrolylcarbonyle)-1,2,3,4-tétrahydroquinoline-2-ylméthyle]-pipérazine,
1-(4-fluoro-2-méthoxyphényle)-4-[1-(2-furoyle)-1,2,3,4-tétrahydroquinoline-2-ylméthyle]-pipérazine,
1-(4-fluoro-2-méthoxyphényle)-4-[1-(4-méthoxybenzoyle)-1,2,3,4-tétrahydroquinoline-2-ylméthyle]-pipérazine,
1-[1-(4-cyanobenzoyle)-1,2,3,4-tétrahydroquinoline-2-ylméthyle]-4-(4-fluoro-2-méthoxyphényle)-pipérazine,
1-[1-(1-acétyle-4-piperidinylcarbonyle)-1,2,3,4-tétrahydroquinoline-2-ylméthyle]-4-(4-fluoro-2-méthoxyphényle)-pipérazine,
1-(4-fluoro-2-méthoxyphényle)-4-[1-(2-thiénylacétyle)-1,2,3,4-tétrahydroquinoline-2-ylméthyle]-pipérazine,
1-(4-fluoro-2-méthoxyphényle)-4-[1-(3-thiénylacétyle)-1,2,3,4-tétrahydroquinoline-2-ylméthyle]-pipérazine,
1-(1-cyclobutylcarbonyle-1,2,3,4-tétrahydroquinoline-2-ylméthyle)-4-(4-fluoro-2-méthoxyphényle)-pipérazine,
1-(4-fluoro-2-méthoxyphényle)-4-[1-(4-phénoxybutanoyle)-1,2,3,4-tétrahydroquinoline-2-ylméthyle]-pipérazine,
1-(4-fluoro-2-méthoxyphényle)-4-(1-phénylsulfonyle-1,2,3,4-tétrahydroquinoline-2-ylméthyle)-pipérazine,
1-(1-benzylsulfonyle-1,2,3,4-tétrahydroquinoline-2-ylméthyle)-4-(4-fluoro-2-méthoxyphényle)-pipérazine,
1-(4-fluoro-2-méthoxyphényle)-4-[1-(2-méthylanilinocarbonyle)-1,2,3,4-tétrahydroquinoline-2-ylméthyle]-pipérazine,
1-[1-(t-butylcarbamoyle)-1,2,3,4-tétrahydroquinoline-2-ylméthyle]-4-(4-fluoro-2-méthoxyphényle)-pipérazine,
1-(1-isoquinolinyle)-4-[1-(3-méthylbutanoyle)-1,2,3,4-tétrahydroquinoline-2-ylméthyle]-pipérazine,
1-[1-(3,3-diméthylbutanoyle)-1,2,3,4-tétrahydroquinoline-2-ylméthyle]-4-(1-isoquinolinyle)-pipérazine,
1-[1-(2-cyclopropylacétyle)-1,2,3,4-tétrahydroquinoline-2-ylméthyle]-4-(1-isoquinolinyle)-pipérazine,
1-(1-isoquinolinyle)-4-[1-(2-pyrrolylcarbonyle)-1,2,3,4-tétrahydroquinoline-2-ylméthyle]-pipérazine,
1-[1-(2-furoyle)-1,2,3,4-tétrahydroquinoline-2-ylméthyle]-4-(1-isoquinolinyle)-pipérazine,
1-(1-isoquinolinyle)-4-[1-(4-méthoxybenzoyle)-1,2,3,4-tétrahydroquinoline-2-ylméthyle]-pipérazine,
1-[1-(4-cyanobenzoyle)-1,2,3,4-tétrahydroquinoline-2-ylméthyle]-4-(1-isoquinolinyle)-pipérazine,
1-[1-(1-acétyle-4-pipéridylcarbonyle)-1,2,3,4-tétrahydroquinoline-2-ylméthyle]-4-(1-isoquinolinyle)-pipérazine,
1-(1-isoquinolinyle)-4-[1-(2-thiénylacétyle)-1,2,3,4-tétrahydroquinoline-2-ylméthyle]-pipérazine,
1-(1-isoquinolinyle)-4-[1-(3-thiénylacétyle)-1,2,3,4-tétrahydroquinoline-2-ylméthyle]-pipérazine,
1-(1-cyclobutanecarbonyle-1,2,3,4-tétrahydroquinoline-2-ylméthyle)-4-(1-isoquinolinyle)-pipérazine,
1-(1-isoquinolinyle)-4-[1-(4-phénoxybutanoyle)-1,2,3,4-tétrahydroquinoline-2-ylméthyle]-pipérazine,
1-(1-isoquinolinyle)-4-(1-phénylsulfonyle-1,2,3,4-tétrahydroquinoline-2-ylméthyle)-pipérazine,
1-(1-benzylsulfonyle-1,2,3,4-tétrahydroquinoline-2-ylméthyle)-4-(1-Isoquinolinyle)-pipérazine,
1-(1-isoquinolinyle)-4-[1-(2-méthylanilinocarbonyle)-1,2,3,4-tétrahydroquinoline-2-ylméthyle]-pipérazine,
1-[1-(t-butylcarbamoyle)-1,2,3,4-tétrahydroquinoline-2-ylméthyle]-4-(1-Isoquinolinyle)-pipérazine,
1-(2,3-dihydrobenzo[1,4]dioxine-5-yl)-4-[1-(3-méthylbutanoyle)-1,2,3,4-tétrahydroquinoline-2-ylméthyle]-pipérazine,
4-[1-(2-cyclopropylacétyle)-1,2,3,4-tétrahydroquinoline-2-ylméthyle]-1-(2,3-dihydrobenzo[1,4]dioxine-5-yl)-pipérazine,
1-(2,3-dihydrobenzo[1,4]dioxine-5-yl)-4-[1-(2-pyrrolylcarbonyle)-1,2,3,4-tétrahydroquinoline-2-ylméthyle]-pipérazine,
1-(2,3-dihydrobenzo[1,4]dioxine-5-yl)-4-[1-(2-furoyle)-1,2,3,4-tétrahydroquinoline-2-ylméthyle]-pipérazine,
1-(2,3-dihydrobenzo[1,4]dioxine-5-yl)-4-[1-(4-méthoxybenzoyle)-1,2,3,4-tétrahydroquinoline-2-ylméthyle]-pipérazine,
4-[1-(4-cyanobenzoyle)-1,2,3,4-tétrahydroquinoline-2-ylméthyle]-1-(2,3-dihydrobenzo[1,4]dioxine-5-yl)-pipérazine,
1-(2,3-dihydrobenzo[1,4]dioxine-5-yl)-4-[1-(2-thiénylacétyle)-1,2,3,4-tébahydroquinoline-2-ylméthyle]-pipérazine,
1-(2,3-dihydrobenzo[1,4]dioxine-5-yl)-4-[1-(3-thiénylacétyle)-1,2,3,4-tétrahydroquinoline-2-ylméthyle]-pipérazine,
4-(1-cyclobutanecarbonyle-1,2,3,4-tétrahydroquinoline-2-ylméthyle)-1-(2,3-dihydrobenzo[1,4]dioxine-5-yl)-pipérazine,
1-(2,3-dihydrobenzo[1,4]dioxine-5-yl)-4-[1-(4-phénoxybutanoyle)-1,2,3,4-tétrahydroquinoline-2-ylméthyle]-pipérazine,
1-(2,3-dihydrobenzo[1,4]dioxine-5-yl)-4-(1-phénylsulfonyle-1,2,3,4-tétrahydroquinoline-2-ylméthyle]-pipérazine,
1-(1-benzylsulfonyle-1,2,3,4-tétrahydroquinoline-2-ylméthyle)-4-(2,3-dihydrobenzo[1,4]dioxine-5-yl)-pipérazine,
1-(2,3-dihydrobenzo[1,4]dioxine-5-yl)-4-[1-(2-méthylanilinocarbonyle)-1,2,3,4-tétrahydroquinoline-2-ylméthyle]-pipérazine,
1-[1-(t-butylcarbamoyle)-1,2,3,4-tétrahydroquinoline-2-ylméthyle]-4-(2,3-dihydrobenzo[1,4]dioxine-5-yl)-pipérazine,
1-(2,3-dihydrobenzo[1,4]dioxine-5-yl)-4-[1-(3,3-diméthylbutanoyle)-1,2,3,4-tétrahydroquinoline-2-ylméthyle]-pipérazine,
4-[1-(1-acétyle-4-pipéridylecatbonyle)-1,2,3,4-tétrahydroquinoline-2-ylméthyle]-1-(2,3-dihydrobenzo[1,4]dioxine-5-yl)-pipérazine,
1-(2,3-dihydrobenzo[1,4] dioxine-5-yl)-4-[1-(2,2-diméthylbutanoyle)-1,2,3,4-tétrahydroquinoline-2-ylméthyle]-pipérazine,
1-(2,3-dihydrobenzo[1,4]dioxine-5-yl)-4-[1-(1-trifluorométhylcyclopropanecarbonyle)-1,2,3,4-tétrahydroquinoline-2-ylméthyle]-pipérazine,
1-[1-(bicyclo[2.2.2]oct-2-ylcarbonyle)-1,2,3,4-tétrahydroquinoline-2-ylméthyle]-4-(2,3-dihydrobenzo[1,4]dioxine-5-yl)-pipérazine,
1-(1-cyclopentanecarbonyle-1,2,3,4-tétrahydroquinoline-2-ylméthyle)-4-(2,3-dihydrobenzo[1,4]dioxine-5-yl)-pipérazine,
1-(2,3-dihydrobenzo[1,4]dioxine-5-yl)-4-[1-(4-fluorobenzoyle)-1,2,3,4-tétrahydroquinoline-2-ylméthyle]-pipérazine,
1-[1-(benzo[1,3]dioxol-5-ylcarbonyle)-1,2,3,4-tétrahydroquinoline-2-ylméthyle]-4-(2,3-dihydrobenzo[1,4]dioxine-5-yl)-pipérazine,
1-(2,3-dihydrobenzo[1,4]dioxine-5-yl)-4-[1-(3-phénylpropenoyle)-1,2,3,4-tétrahydroquinoline-2-ylméthyle]-pipérazine,
1-(2,3-dihydrobenzo[1,4]dioxine-5-yl)-4-[1-[3-(4-fluorophényle)-propenoyle]-1,2,3,4-tétrahydroquinoline-2-ylméthyle]-pipérazine,
1-(2,3-dihydrobenzo[1,4]dioxine-5-yl)-4-[1-(3-méthyle-2-thiénylcarbonyle)-1,2,3,4-tétrahydroquinoline-2-ylméthyle]-pipérazine,
1-(2,3-dihydrobenzo[1,4]dioxine-5-yl)-4-[1-(3-furoyle)-1,2,3,4-tétrahydroquinoline-2-ylméthyle]-pipérazine,
1-(2,3-dihydrobenzo[1,4]dioxine-5-yl)-4-[1-(2-méthyle-3-furoyle)-1,2,3,4-tétrahydroquinoline-2-ylméthyle]-pipérazine,
1-(2,3-dihydrobenzo[1,4]dioxine-5-yl)-4-[1-[5-méthyle-4-(1,2,4-triazol-1-ylméthyle)-2-furoyle)-1,2,3,4-tétrahydroquinoline-2-ylméthyle]-pipérazine,
1-(2,3-dihydrobenzo[1,4]dioxine-5-yl)-4-[1-(5-méthyle-4-isoxazolecarbonyle)-1,2,3,4-tétrahydroquinoline-2-ylméthyle]-pipérazine,
1-[1-(4-acétyle-2-pyrrolecarbonyle)-1,2,3,4-tétrahydroquinoline-2-ylméthyle]-4-(2,3-dihydrobenzo[1,4]dioxine-5-yl)-pipérazine,
2-{N-[2-(2-méthoxyphénoxy)-éthyle]-N-méthyl-aminométhyle}-1-(3-méthylbutanoyle)-1,2,3,4-tétrahydroquinoline,
1-(3,3-diméthylbutanoyle)-2-{N-[2-(2-méthoxyphénoxy)-éthyle]-N-méthyle-aminométhyle}-1,2,3,4-tétrahydroquinoline,
1-cyclopropylacétyle-2-{N-[2-(2-méthoxyphénoxy)-éthyle]-N-méthyle-aminométhyle}-1,2,3,4-tétrahydroquinoline,
2- {N-[2-(2-méthoxyphénoxy)-éthyle]-N-méthyle-aminométhyle}-1-(2-pyrrolylcarbonyle)-1,2,3,4-tétrahydroquinoline,
1-(2-furoyle)-2-{N-[2-(2-méthoxyphénoxy)-éthyle]-N-méthyle-aminométhyle}-1,2,3,4-tétrahydroquinoline,
1-(4-méthoxybenzoyle)-2-{N-[2-(2-méthoxyphénoxy)-éthyle]-N-méthyle-aminométhyle}-1,2,3,4-tétrahydroquinoline,
1-(1-acétyle-4-pipéridylcarbonyle)-2-{N-[2-(2-méthoxyphénoxy)-éthyle]-N-méthyle-aminométhyle}-1,2,3,4-tétrahydroquinoline,
2- {N-[2-(2-méthoxyphénoxy)-éthyle]-N-méthyle-aminométhyle}-1-(2-thiénylacétyle)-1,2,3,4-tétrahydroquinoline,
2-{N-[2-(2-méthoxyphénoxy)-éthylé]-N-méthyle-aminométhyle}-1-(3-thiénylacétyle)-1,2,3,4-tétrahydroquinoline,
1-cyclobutanecarbonyle-2-{N-[2-(2-méthoxyphénoxy)-éthyle]-N-méthyle-ammométhyle}-1,2,3,4-tétrahydroquinoline,
2- {N-[2-(2-méthoxyphénoxy)-éthyle]-N-méthyle-aminométhyle}-1-(4-phénoxybutanoyle)-1,2,3,4-tétrahydroquinoline,
2- {N-[2-(2-méthoxyphénoxy)-éthyle]-N-méthyle-aminométhyle}-1-phénylsulfonyle-1,2,3,4-tétrahydroquinoline,
1-benzylsulfonyle-2-{N-[2-(2-méthoxyphénoxy)-éthyle]-N-méthyle-aminométhyle}-1,2,3,4-tétrahydroquinoline,
2- {N-[2-(2-méthoxyphénoxy)-éthyle]-N-méthyle-aminométhyle}-1-(2-méthylanilinocarbonyle)-1,2,3,4-tétrahydroquinoline,
1-(t-butylcarb amoyle)-2-{N-[2-(2-méthoxyphénoxy)-éthyle]-N-méthyle-aminométhyle}-1,2,3,4-tétrahydroquinoline
1-(1-cyclohexanecarbonyle-5-fluoro-1,2,3,4-tétrahydroquinoline-2-ylméthyle)-4-(4-fluoro-2-méthoxyphényle)-pipérazine,
1-(1-cyclohexanecarbonyle-1,2,3,4-tétrahydroquinoline-2-ylméthyle)-4-(2-triluorométhanesulfonyloxyphényle)-pipérazine,
1-(1-cyclohexanecarbonyle-1,2,3,4-tétrahydroquinoline-2-ylméthyle)-4-(1-isopropyle-4-indolyle)-pipérazine,
1-(6-*tert*-butoxycarbonylamino-1-cyclohexanecarbonyle-1,2,3,4-tétrahydroquinoline-2-ylméthyle)-4-(4-fluoro-2-méthoxyphényle)-pipérazine,
1-(1-cyclohexanecarbonyle-1,2,3,4-tétrahydroquinoline-2-ylméthyle)-4-(1-méthoxyméthyle-4-indolyle)-pipérazine,
1-(1-cyclohexanecarbonyle-2-méthyle-1,2,3,4-tétrahydroquinoline-2-ylméthyle)-4-(4-fluoro-2-méthoxyphényle)-pipérazine,
1-(1-cyclopentylcarbonyle-6-méthyle-1,2,3,4-tétrahydroquinoline-2-ylméthyle]-4-(4-fluoro-2-méthoxyphényle)-pipérazine,
1-(6-amino-1-cyclohexanecarbonyle-1,2,3,4-tétrahydroquinoline-2-ylméthyle)-4-(4-fluoro-2-méthoxyphényle)-pipérazine,
1-(1-cyclohexanecarbonyle-1,2,3,4-tétrahydroquinoline-2-ylméthyle)-4-(2-méthoxy-4-méthylphényle)-pipérazine,
1-(4-benzo[1,3]dioxol-5-ylméthyle)-4-(1-cyclohexanecarbonyle-1,2,3,4-tétrahydroquinoline-2-ylméthyle)-pipérazine,
1-cyclohexanecarbonyle-2-{N-[3-(2-méthoxyphénoxy)-propyle]-aminométhyle}-1,2,3,4-tétrahydroquinoline,
1-cyclohexanecarbonyle-2-{N-[2-(2-méthoxyphénoxy)-éthyle]-N-méthyle-aminométhyle}-1,2,3,4-tétrahydroquinoline,
4-cyano-1-(1-cyclohexanecarbonyle-1,2,3,4-tétrahydroquinoline-2-ylméthyle)-4-phényle-pipéridine,
4-benzyle-1-(1-cyclohexanecarbonyle-1,2,3,4-tétrahydroquinoline-2-ylméthyle)-4-hydroxy-pipéridine,
4-benzyloxy-1-(1-cyclohexanecarbonyle-1,2,3,4-tétrahydroquinoline-2-ylméthyle)-pipéridine,
4-benzoylamino-1-(1-cyclohexanecarbonyle-1,2,3,4-tétrahydroquinoline-2-ylméthyle)-pipéridine,
4-acétyle-1-(1-cyclohexanecarbonyle-1,2,3,4-tétrahydroquinoline-2-ylméthyle)-4-phényle-pipéridine,
1-(benzo[1,3]dioxol-4-yl)-4-(1-cyclohexanecarbonyle-1,2,3,4-tétrahydroquinoline-2-ylméthyle)-pipérazine,
2- {N-[2-(3-chlorophénoxy)-éthyle]-N-méthyle-aminométhyle}-1-cyclohexanecarbonyle-1,2,3,4-tétrahydroquinoline,
2- {N-[2-(4-chlorophénoxy)-éthyle]-N-méthyle-aminométhyle}-1-cyclohexanecarbonyle-1,2,3,4-tétrahydroquinoline,
2- {N-benzyle-N-[2-(2-méthoxyphénoxy)-éthyle]-aminométhyle}-1-cyclohexanecarbonyle-1,2,3,4-tétrahydroquinoline,
1-cyclohexanecarbonyle-2-[N-méthyle-N-(3-phénylpropyle)-aminométhyle]-1,2,3,4-tétrahydroquinoline,
1-(4-cyano-2-trifluorométhoxyphényle)-4-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-4-(4-fluoro-2-méthoxy-anilino)-pipéridine,
1-(3H-1,2,3-benzotriazole-4-yl)-4-(1-cyclohexanecarbonyle-1,2,3,4-tétrahydroquinoline-2-ylméthyle)-pipérazine,
1-(1H-1,3-benzodiazole-4-yl)-4-(1-cyclohexanecarbonyle-1,2,3,4-tétrahydroquinoline-2-ylmeéhyle)-pipérazine,
1-cyclohexanecarbonyle-2- {N-[2-(2-pyridyloxy)-éthyle]-aminométhyle}-1,2,3,4-tétrahydroquinoline,
1-cyclohexanecarbonyle-2-{N-méthyle-N-[2-(2-pyridyloxy)-éthyle]-aminométhyle}-1,2,3,4-tétrahydroquinoline,
1-cyclohexanecarbonyle-2-{N-méthyle-N-[3-(2-méthoxyphényle)-propyle]-aminométhyle}-1,2,3,4-tétrahydroquinoline,
4-benzoyle-1-(1-cyclohexanecarbonyle-1,2,3,4-tétrahydroquinoline-2-ylméthyle)-pipérazine,
1-(1-cyclohexanecarbonyle-1,2,3,4-tétrahydroquinoline-2-ylméthyle)-3-oxo-4-phénylpipérazine,
1-(4-fluoro-2-méthoxyphényle)-4-(1-hexanoyl-1,2,3,4-tétrahydroquinoline-2-ylméthyle)-pipérazine,
1-(2,3-dihydrobenzo[1,4]dioxine-5-yl)-4-[1-pentafluoropropionyle-1,2,3,4-tétrahydroquinoline-2-ylméthyle]-pipérazine,
1-(2,3-dihydrobenzo[1,4]dioxine-5-yl)-4-[1-(3,3,3-trifluoropropionyle)-1,2,3,4-tétrahydroquinoline-2-ylméthyle]-pipérazine,
(Z)-1-(2,3-dihydrobenzo[1,4]dioxine-5-yl)-4-[1-(4-hydroxy-cyclohexanecarbonyle)-1,2,3,4-tétrahydroquinoline-2-ylméthyle]-pipérazine, et
1-(1-cyclohexanecarbonyle-6-fluoro-1,2,3,4-tétrahydroquinoline-2-ylméthyle)-4-(4-fluoro-2-méthoxyphényle)-pipérazine.

15. Une composition pharmaceutique comprenant un composé selon l'une quelconque des revendications 1 à 14 ou un énantiomère, diastéréomère, N-oxyde, forme cristalline, hydrate, solvate ou un sel pharmaceutiquement acceptable de ce composé, en mélange avec un diluant ou support pharmaceutiquement acceptable.
